(19) Europäisches Patentamt
European Patent Office
Office européen des brevets

(11) EP 2 004 756 B1

(12) # FASCICULE DE BREVET EUROPEEN

(45) Date de publication et mention de la délivrance du brevet:
**04.05.2011 Bulletin 2011/18**

(21) Numéro de dépôt: **07731812.9**

(22) Date de dépôt: **23.03.2007**

(51) Int Cl.:
***C09B 23/14*** (2006.01) ***C09B 49/12*** (2006.01)
***A61Q 5/08*** (2006.01)

(86) Numéro de dépôt international:
**PCT/FR2007/051000**

(87) Numéro de publication internationale:
**WO 2007/110534 (04.10.2007 Gazette 2007/40)**

(54) **COMPOSITION DE TEINTURE COMPRENANT UN COLORANT FLUORESCENT THIOL/ DISULFURE, A CHARGE CATIONIQUE EXTERNE, PROCEDE D'ECLAIRCISSEMENT DES MATIERES KERATINIQUES A PARTIR DE CE COLORANT**

FÄRBEZUSAMMENSETZUNG MIT EINEM THIOL/DISULFID- LEUCHTFARBSTOFF MIT EINER EXTERNEN KATIONISCHEN LADUNG SOWIE VERFAHREN ZUR AUFHELLUNG VON KERATINMATERIAL MIT DIESEM FARBSTOFF

DYEING COMPOSITION COMPRISING A THIOL/DISULPHIDE FLUORESCENT COLORANT HAVING AN EXTERNAL CATIONIC CHARGE, AND METHOD FOR LIGHTENING KERATIN MATERIALS USING SAID COLORANT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **24.03.2006 FR 0651035**
**19.04.2006 US 792941 P**
**05.02.2007 FR 0753066**
**12.02.2007 US 900696 P**

(43) Date de publication de la demande:
**24.12.2008 Bulletin 2008/52**

(73) Titulaire: **L'Oréal**
**75008 Paris (FR)**

(72) Inventeurs:
- **GREAVES, Andrew**
  **F-77144 Montevrain (FR)**
- **DAUBRESSE, Nicolas**
  **F-78170 La Celles St Cloud (FR)**

(74) Mandataire: **Fevrier, Murielle Françoise E. et al**
**L'Oréal**
**D.I.P.I.**
**25-29 Quai Aulagnier**
**92600 Asnières-sur-Seine Cedex (FR)**

(56) Documents cités:
**EP-A1- 0 860 636** **WO-A-03/028685**
**WO-A-2005/004822** **WO-A-2006/134043**
**WO-A-2006/136617** **WO-A2-2004/091473**
**WO-A2-2005/097051** **GB-A- 2 180 215**
**US-A- 2 904 385**

- **GEOFFREY J. ASHWELL, ABDUL MOHIB AND JAMES R. MILLER: "Induced rectification from self-assembled monolayers of sterically hindered -bridged chromophores" JOURNAL OF MATERIALS CHEMISTRY, vol. 15, no. 11, 27 janvier 2005 (2005-01-27), pages 1160-1166, XP002419175**
- **GEOFFREY J. ASHWELL AND ABDUL MOHIB: "Improved Molecular Rectification from Self-Assembled Monolayers of a Sterically Hindered Dye" J. OF THE AMERICAN CHEMICAL SOCIETY, vol. 127, no. 46, 11 janvier 2005 (2005-01-11), pages 16238-16244, XP002419176**
- **GEOFFREY J. ASHWELL, WAYNE D. TYRRELL AND ANNE J. WHITTAM: "Molecular rectification: self-assembled monolayers of a donor?(-bridge)?acceptor chromophore connected via a truncated Au?S?(CH2)3 bridge" JOURNAL OF MATERIALS CHEMISTRY, vol. 13, no. 12, 3 novembre 2003 (2003-11-03), pages 2855-2857, XP002419177**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **TSUBOI K ET AL: "Formation of Merocyanine Self-Assembled Monolayer and Its Nonlinear Optical Properties Probed by Second-Harmonic Generation and Surface Plasmon Resonance" JAPANESE JOURNAL OF APPLIED PHYSICS, JAPAN SOCIETY OF APPLIED PHYSICS, TOKYO, JP, vol. 42, no. 2A, février 2003 (2003-02), pages 607-613, XP002355458 ISSN: 0021-4922**
- **NARAOKAA R ET AL: "Nonlinear optical property of hemicyanine self-assembled monolayers on gold and its adsorption kinetics probed by optical second-harmonic generation and surface plasmon resonance spectroscopy" CHEMICAL PHYSICS LETTERS, NORTH-HOLLAND, AMSTERDAM, NL, vol. 362, no. 1-2, 13 août 2002 (2002-08-13), pages 26-30, XP002355459 ISSN: 0009-2614**
- **OKAWA H ET AL: "SYNTHESIS AND CHARACTERIZATION OF AN ALKANETHIOL THIN FILM CONTAINING A HEMICYANINE DYE" MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, vol. 377, 2002, pages 137-140, XP008056025 ISSN: 1058-725X**
- **KAJIKAWA K ET AL: "PREPARATION AND OPTICAL CHARACTERIZATION OF HEMICYANINE SELF-ASSEMBLED MONOLAYER ON AU SUBSTRATE" MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, vol. 370, 2001, pages 277-283, XP008056026 ISSN: 1058-725X**
- **TSUBOI K ET AL: "REFLECTION SPECTROSCOPY OF MEROCYANINE SELF-ASSEMBLED MONOLAYER ON A GOLD SUBSTRATE" MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, CH, vol. 322, 1998, pages 191-196, XP008056027 ISSN: 1058-725X**
- **ZAYED A ET AL: "NOVEL SUBSTITUTED QUINOLINES WITH POSSIBLE ANTIMALARIAL ACTIVITY" PHARMAZIE, DIE, GOVI VERLAG, ESCHBORN, DE, vol. 33, no. 9, 1978, pages 572-575, XP009027864 ISSN: 0031-7144**

## Description

L'invention concerne la coloration de fibres kératiniques à l'aide de colorants fluorescents thiol/disulfure à charge cationique externe.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les matières kératiniques telles que les fibres kératiniques, notamment les cheveux, par un système oxydant fort, généralement constitué par du peroxyde d'hydrogène associé ou non à des persels, le plus souvent en milieu alcalin.

Ce système de décoloration présente l'inconvénient de dégrader des matières kératiniques, en particulier les fibres kératiniques, notamment humaines telles que les cheveux et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans les documents FR 2830189 et WO 2004/091473 permet de respecter la qualité de la fibre kératinique lors du traitement mais les colorants fluorescents employés ne présentent pas une résistance aux shampoings satisfaisante.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres kératiniques voir par exemple Journal of the Society of Dyers and Colourists, Guise et Stapleton, 91, 259-264 (1975); Journal of Cosmetic Chemistry, 42, 1-17 (1991); CA 2024509.

De plus il est connu de protéger la ou les fonctions thiols contenues dans une molécule à greffer aux cheveux avant de les appliquer auxdits cheveux WO99/51194. Cependant cette demande ne mentionne pas l'utilisation de colorants fluorescents permettant de colorer ou d'éclaircir des cheveux.

D'autres colorants disulfures connus pour la coloration des fibres kératiniques sont des dérivés disulfures de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un prétraitement réducteur du cheveu. Cependant, ces colorants peuvent occasionner des virages de couleur lors de l'application.

Enfin, le document WO 2005/097051 décrit des colorants disulfures aza-imidazoliums pour la coloration directe de fibres kératiniques.

Le but de la présente invention est de fournir de nouveaux systèmes de coloration de matières kératiniques en particulier les fibres kératiniques humaines, notamment les cheveux, qui ne présentent pas les inconvénients des procédés de décoloration existants. En particulier, un des buts de la présente invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Ce but est atteint avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent pyridinium à charge cationique externe choisi parmi les colorants de formules (I) ou (II) suivante :

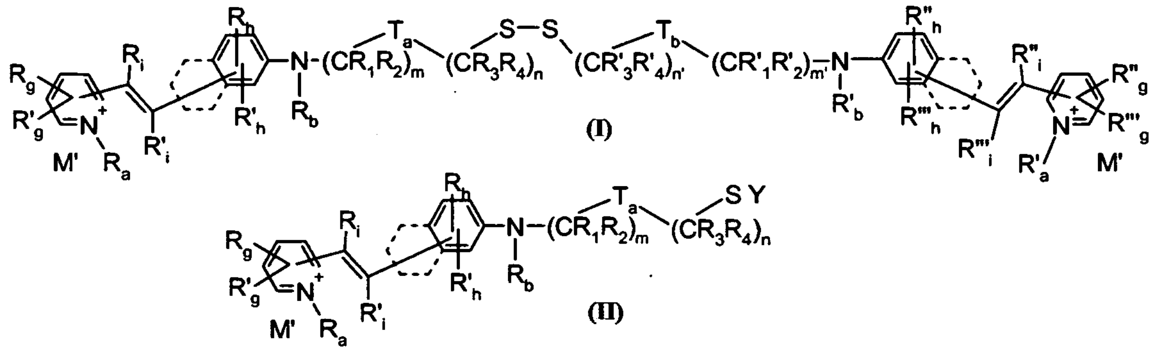

(I)

(II)

leurs sels d'acide organique ou minéral, isomères optiques, isomères géométriques, et les solvates tels que hydrates ; formules (I) ou (II) dans lesquelles :

➢ $R_a$ et $R'_a$, identiques ou différents, représentent un groupement aryl($C_1$-$C_4$)alkyle ou un groupement ($C_1$-$C_6$) alkyle éventuellement substitué par un groupement hydroxy ou amino, $C_1$-$C_4$ alkylamino, ($C_1$-$C_4$) dialkylamino, lesdits radicaux alkyle pouvant former avec l'atome d'azote qui les portent un hétérocyle comprenant de 5 à 7 chaînons, comprenant éventuellement un autre hétéroatome différent ou non de l'azote ; préférentiellement $R_a$ et/ou $R'_a$ représentent un groupement ($C_1$-$C_3$) alkyle éventuellement substitué par un groupement hydroxy, ou un groupement benzyle ;
➢ $R_b$ et $R'_b$, identiques ou différents, représentent un atome d'hydrogène, un groupement aryl($C_1$-$C_4$)alkyle ou un groupement ($C_1$-$C_6$) alkyle éventuellement substitué; notamment $R_b$ et/ou $R'_b$ représentent un atome d'hydrogène ou un groupement ($C_1$-$C_3$) alkyle ou benzyle ;
➢ $R_g$, $R'_g$, $R''_g$ et $R'''_g$, identiques ou différents, représentent un atome d'hydrogène, un groupement amino, ($C_1$-$C_4$) alkylamino, ($C_1$-$C_4$) dialkylamino, trifluorométhyle, un radical acylamino, ($C_1$-$C_4$)alcoxy ($C_1$-$C_4$)alkylcarbonyloxy, ($C_1$-$C_4$)alcoxycarbonyle, ($C_1$-$C_4$)alkylcarbonylamino, ($C_1$-$C_4$)alkylsulfonylamino, un radical ($C_1$-$C_3$)alkyle ;
➢ $R_h$, $R'_h$, $R''_h$ et $R'''_h$, identiques ou différents, représentent un atome d'hydrogène, un atome d'halogène, un groupement dialkylamino ($C_1$-$C_4$), ($C_1$-$C_4$)alkylcarbonylamino, un radical acylamino, ($C_1$-$C_4$)alkylsulfonylamino, ou un radical ($C_1$-$C_4$) alkyle ; particulièrement $R_h$, $R'_h$, $R''_h$ et $R'''_h$ représentent un atome d'hydrogène, ou un groupement ($C_1$-$C_3$)alkyle ;
➢ ou alors deux groupements $R_g$ et $R'_g$ ; $R''_g$ et $R'''_g$ ; $R_h$ et $R'_h$ ; $R''_h$ et $R'''_h$ portés par deux atomes de carbone adjacents, forment ensembles un cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, ($C_1$-$C_4$)alkylamino, ($C_1$-$C_4$)dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonylamino, un radical acylamino, carbamoyle , ($C_1$-$C_4$)alkylsulfonyl-amino, un radical aminosulfonyle, ou un radical ($C_1$-$C_{16}$)alkyle éventuellement substitué par un groupement choisi parmi ($C_1$-$C_{12}$)alcoxy, hydroxy, cyano, carboxy, amino, ($C_1$-$C_4$)alkylamino et ($C_1$-$C_4$)dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ; particulièrement $R_g$ et $R'_g$ ; $R''_g$ et $R'''_g$ forment ensemble un groupement benzo ;
➢ $R_i$, $R'_i$, $R''_i$ et $R'''_i$, identiques ou différents, représentent un atome d'hydrogène, où un groupement ($C_1$-$C_4$)alkyle ; particulièrement un atome d'hydrogène ;
➢ $R_1$, $R_2$, $R_3$, $R_4$, $R'_1$, $R'_2$, $R'_3$ et $R'_4$, identiques ou différents, représentent un atome d'hydrogène ou un groupement ($C_1$-$C_4$) alkyle particulièrement $R_1$, $R_2$, $R_3$, $R_4$, $R'_1$, $R'_2$, $R'_3$ et $R'_4$ représentent un atome d'hydrogène ;
➢

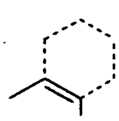 ,

présent ou absent, représente un groupement benzo ;
➢ $T_a$ et $T_b$, identiques ou différents, représentent :

i) soit une liaison covalente σ ;
ii) soit un radical choisi parmi -N(R)-, $-N^+(R)(R^\circ)-$, avec R, R°, identiques ou différents, représentant un atome d'hydrogène, un radical alkyle en $C_1$-$C_4$, hydroxyalkyle en $C_1$-$C_4$; ou un aryl($C_1$-$C_4$)alkyle,
iii) soit un radical hétérocycloalkyle ou hétéroaryle, cationique ou non, préférentiellement monocycliques, contenant un ou deux hétéroatomes, particulièrement deux atomes d'azote, et comportant notamment de 5 à 7 chaînons tel que l'imidazolium ; ou un hétérocycle saturé à 6 chaînons :

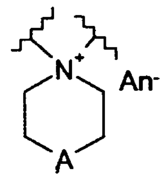

avec A représentant un atome d'oxygène, de soufre ou un groupement $CH_2$, NR°, où R° est tel que défini précédemment et $An^-$ représente un contre-ion anionique ;

préférentiellement $T_a$ et $T_b$ représentent une liaison covalente ; ➢ m, m', n et n', identiques ou différents, représentent un entier compris inclusivement entre 0 et 6 avec m+n et m'+n', identiques ou différents, représentent un entier compris inclusivement entre 1 et 10 ; particulièrement la somme m+n=m'+n' est un entier compris inclusivement entre 2 et 4 ; préférentiellement lorsque $T_a$ et $T_b$ représentent une liaison covalente σ, m+n=m'+n' est un entier égal à 2 ;
➢ lorsque $T_a$ représente une liaison covalente σ, Y représente un groupement choisi parmi :

o aryle éventuellement substitué tel que le phényle, dibenzosubéryle, ou 1,3,5-cycloheptatriényle ;
o hétéroaryle éventuellement substitué notamment aromatique, cationiques ou non, comprenant de 1 à 4 hétéroatomes tel que :

i) hétéroaryle monocyclique à 5, 6 ou 7 chaînons comme furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;
ii) hétéroaryle bicyclique à 8 à 11 chaînons comme indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupements tels que ($C_1$-$C_4$) alkyle comme méthyle, ou polyhalogéno ($C_1$-$C_4$)alkyle comme trifluorométhyle ;
iii) ou hétéroaryle tricyclique ABC suivant :

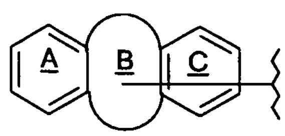

dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

o hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme ($C_1$-$C_4$) alkyle, oxo ou

thioxo ; ou l'hétérocycle représente le groupement suivant :

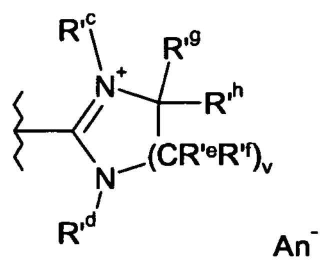

dans lequel R'c, R'd, R'e, R'f, R'g et R'h, identiques ou différents, représentent un atome d'hydrogène ou un groupement alkyle, ou alors deux groupement R'g avec R'h, et/ou R'e avec R'f forment un groupement oxo ou thioxo, ou alors R'g avec R'e forment ensemble un cycloalkyle; et v représente un entier compris inclusivement entre 1 et 3 ; préférentiellement R'c à R'h représentent un atome d'hydrogène ; et $An^-$ représente un contre-ion anionique ;

o (di)arylalkyle éventuellement substitué tel que le 9-anthracénylméthyle, phénylméthyle ou diphénylméthyle éventuellement substitué par un plusieurs groupements notamment choisis parmi $(C_1-C_4)$ alkyle, $(C_1-C_4)$ alcoxy comme le méthoxy, hydroxy, $(C_1-C_4)$ alkylcarbonyle,, (di) $(C_1-C_4)$ (alkyl)amino comme le diméthylamino ;

o (di)hétéroarylakyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que $(C_1-C_4)$ alkyle particulièrement méthyle, avantageusement le (di)hétéroarylakyle est (di)hétéroarylméthyle ou (di)hétéroaryléthyle ;

o cyclique stériquement encombré tel que le groupe adamantyle ;

➢ lorsque $T_a$ représente -N(R)-, $-N^+(R)(R^\circ)-$, un radical hétérocycloalkyle ou hétéroaryle, cationique ou non ; Y représente un groupement choisi parmi i) un atome d'hydrogène ; ii) un métal alcalin ; iii) un métal alcalino-terreux ; iv) un groupement ammonium : $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe $(C_1-C_4)$alkyle ; ou v) un groupement protecteur de fonction thiol ;

et

➢ M' représentant un contre-ion anionique ;

étant entendu que lorsque le composé de formule (I) ou (II) contient d'autres parties cationiques, il se trouve associé à un ou plusieurs contre-ions anioniques permettant d'atteindre l'électroneutralité de la formule (I) ou (II).

Un autre objet de l'invention est une composition tinctoriale comprenant, dans un milieu cosmétique approprié au moins un colorant fluorescent choisi parmi les colorants de formule (I) ou (II) tels que définis précédemment, et éventuellement un agent réducteur.

L'invention a aussi pour objet de nouveaux colorants fluorescents de formule (I) ou (II) tels que définis précédemment.

Le procédé de coloration selon l'invention permet de colorer de façon visible les matières kératiniques foncées, en particulier les fibres kératiniques humaines foncées, notamment les cheveux foncés.

De plus le procédé de l'invention permet d'obtenir une coloration des matières kératiniques, en particulier les fibres kératiniques humaines, notamment les cheveux, sans dégrader ladite matière, rémanente vis-à-vis de shampooings, des agressions courantes (soleil, transpiration), et des autres traitements capillaires. Le procédé de l'invention permet également d'obtenir un éclaircissement des matières kératiniques telles que les fibres kératiniques particulièrement les fibres kératiniques foncées et plus particulièrement les cheveux foncés.

Au sens de l'invention, on entend par matière kératinique foncée celle qui présente une luminescence L* chiffrée dans le système C.I.E. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de « hauteur de ton » avant et après application du composé de formule (I) ou (II).

La notion « ton » repose sur la classification des nuances naturelles, un ton séparant chaque nuance de celle

qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

De préférence, la composition doit, après application sur des cheveux, par exemple châtains, amener aux résultats ci-dessous.

- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :

les radicaux « aryle » ou « hétéroaryle » peuvent être substitués par au moins un substituant porté par un atome de carbone, choisi parmi :

- un radical alkyle en $C_1$-$C_{16}$, de préférence en $C_1$-$C_6$ ;
- un atome d'halogène tel que chlore, fluor ou brome ;
- un groupement hydroxy ;
- un radical alcoxy en $C_1$-$C_2$ ;
- un radical alkylthio en $C_1$-$C_2$ ;
- un radical (poly)-hydroxyalcoxy en $C_2$-$C_4$ ;
- un radical amino ;
- un radical amino substitué par un ou deux radicaux alkyle, identiques ou différents, en $C_1$-$C_6$ éventuellement porteurs d'au moins :

  i) un groupement hydroxy,
  ii) un groupement amino ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
- un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ;
- un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$ ;
- un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy ;
- un radical carboxylique sous forme acide ou salifiée (de préférence avec un métal alcalin ou un ammonium, substitué ou non) ;
- un groupement cyano ;
- un groupement polyhalogénoalkyle comprenant de 1 à 6 atomes de carbones et de 1 à 6 atome d'halogène, identiques ou différents, le groupement polyhalogénoalkyle est par exemple le trifluorométhyle ;

- la partie cyclique ou hétérocyclique d'un radical non aromatique peut être substituée par au moins un substituant porté par un atome de carbone choisi parmi les groupements :

  • hydroxy,
  • alkyle en $C_1$-$C_4$,
  • alcoxy en $C_1$-$C_4$,
  • (poly)hydroxyalcoxy en $C_2C_4$,
  • un radical alkylthio en $C_1$-$C_2$ ;
  • RCO-NR'- dans lequel le radical R' est un atome d'hydrogène ou un radical alkyle en $C_1$-$C_4$ et le radical R est un atome d'hydrogène, un radical alkyle en $C_1$-$C_2$, ou amino substitué par deux groupements alkyle identiques ou différents en $C_1$-$C_4$ lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
  • RCO-O- dans lequel le radical R est un radical alkyle en $C_1$-$C_4$, amino substitué par deux groupements alkyle identiques ou différents en $C_1$-$C_4$, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
  • RO-CO- dans lequel le radical R est un radical alkyle en $C_1$-$C_4$, amino substitué par deux groupements alkyle identiques ou différents en $C_1$-$C_4$ lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;

- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un radical « diarylalkyle » représente un groupement comportant sur le même atome de carbone d'un groupement alkyle deux groupement aryle, identiques ou différents tel que diphénylméthyle ou 1,1-diphényléthyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un radical « dihétéroarylalkyle » représente un groupement comportant sur le même atome de carbone d'un groupement alkyle deux groupement hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1,3,5-trimétylsillylphényle et l'adamantyle ;
- un « radical hétérocyclique » est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en $C_1$-$C_{16}$, linéaire ou ramifié, de préférence en $C_1$-$C_8$ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en $C_1$-$C_4$, iii) acylamino, iv) amino éventuellement substitué par un ou deux radicaux alkyle, identiques ou différents, en $C_1$-$C_4$, lesdits radicaux alkyle pouvant former avec l'atome d'azote qui les portent un hétérocycle comprenant de 5 à 7 chaînons, comprenant éventuellement un autre hétéroatome différent ou non de l'azote ;
- un « radical alcoxy » est un radical alkyle-oxy ou alkyl-O- pour lequel le radical alkyle est un radical hydrocarboné, linéaire ou ramifié, en $C_1$-$C_{16}$ préférentiellement en $C_1$-$C_8$ ;
- un « radical alkylthio » est un radical alkyl-S- pour lequel le radical alkyle est un radical hydrocarboné, linéaire ou

ramifié, en $C_1$-$C_{16}$ préférentiellement en $C_1$-$C_8$ ;

- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-S(O)$_2$OH tels que d'acide méthylsulfonique et d'acide éthylsulfonique ; v) d'acides arylsulfoniques : ArS(O)$_2$OH tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3COOH$ ; xiv) d'acide triflique $CF_3SO_3H$ et xv) d'acide tétrafluoroborique $HBF_4$ ;
- un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les $C_1$-$C_6$ alkylsulfonates : Alk-S(O)$_2$O$^-$ tels que le méthylsulfonate ou mésylate et l'éthylsulfonate ; iv) les arylsulfonates : Ar-S(O)$_2$O$^-$ tel que le benzènesulfonate et le toluènesulfonate ou tosylate ; v) le citrate ; vi) le succinate ; vii) le tartrate ; viii) le lactate ; ix) les alkylsulfites : Alk-O-S(O)O$^-$ tels que le méthysulfite et l'éthylsulfite ; x) les arylsulfites : Ar-O-S(O)O$^-$ tels que le benzènesulfite et le toluènesulfite ; xi) les alkylsulfates : Alk-O-S(O)$_2$O$^-$ tel que le méthyl sulfate et l'éthylsulfate ; xii) les arylsulfates : Ar-O-S(O)$_2$O$^-$, xiii) le phosphate ; xiv) l'acétate ; xv) le triflate ; et, xvi) les borates tels que le tétrafluoroborate.

Les colorants fluorescents de formule (I) ou (II) sont des composés capables d'absorber dans le rayonnement UV ou visible à une longueur d'onde $\lambda_{abs}$ comprise entre 250 et 800 nm et capables de réémettrent dans le domaine du visible à une longueur d'onde d'émission $\lambda_{ém}$ comprise entre 400 et 800 nm.

De préférence les composés fluorescents de formule (I) ou (II) sont des colorants capables d'absorber dans le visible, $\lambda_{abs}$ comprise entre 400 et 800 nm, et de réémettre dans le visible, $\lambda_{ém}$ comprise entre 400 et 800 nm. Plus particulièrement les colorants fluorescents sont des colorants capables d'absorber à une longueur d'onde $\lambda_{abs}$ comprise entre 420 nm et 550 nm et de réémettre dans le visible à une longueur d'onde $\lambda_{ém}$ comprise entre 470 et 600 nm.

Un mode particulier de l'invention concerne les colorants de formule (I) ou (II) dans lesquels $T_a$, $T_b$ représentent -N(R)-, -N$^+$(R)(R°)-, un radical hétérocycloalkyle ou hétéroaryle, cationique ou non. Plus particulièrement les colorants fluorescents thiols de formule (II) à fonction SY comportent alors un groupe Y qui représente un atome d'hydrogène ou un métal alcalin, préférentiellement Y représente un atome d'hydrogène.

Conformément à un autre mode de réalisation particulier de l'invention, dans la formule (II) précitée dans lesquels $T_a$ représente -N(R)-, -N$^+$(R)(R°)-, Y est un groupement protecteur et le groupement protecteur est choisi parmi ceux connus par l'homme du métier comme par exemple ceux décris dans les ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Les composés fluorescents de l'invention de formule (II) contiennent une fonction SY qui peut se trouver sous la forme covalente -S-Y ou ionique -S$^-$ Y$^+$ selon la nature de Y et du pH du milieu.

Particulièrement lorsque Y représente un groupement protecteur de la fonction thiol du composé de formule (II), Y est choisi parmi les radicaux suivants :

➢ ($C_1$-$C_4$)alkylcarbonyle ;
➢ ($C_1$-$C_4$)alkylthiocarbonyle ;
➢ ($C_1$-$C_4$)alcoxycarbonyle ;
➢ ($C_1$-$C_4$)alcoxythiocarbonyle;
➢ ($C_1$-$C_4$)alkylthio-thiocarbonyle ;
➢ (di) ($C_1$-$C_4$) (alkyl)aminocarbonyle ;
➢ (di) ($C_1$-$C_4$) (alkyl)aminothiocarbonyle;
➢ arylcarbonyle comme phénylcarbonyle ;
➢ aryloxycarbonyle ;
➢ aryl($C_1$-$C_4$)alkcoxycarbonyle ;
➢ (di) ($C_1$-$C_4$) (alkyl)aminocarbonyle comme diméthylaminocarbonyle ;
➢ ($C_1$-$C_4$)(alkyl)arylaminocarbonyle ;
➢ carboxy ;
➢ $SO_3^-$ ; M$^+$ avec M$^+$ représentant un métal alcalin tel que le sodium ou le potassium, ou alors M' de la formule (II) et M$^+$ sont absents ;
➢ hétéroaryle éventuellement substitué dont notamment les groupements aromatiques, cationiques ou non, comprenant de 1 à 4 hétéroatomes suivants :

i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;

ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupements tels que alkyle comme méthyle, ou polyhalogénoalkyle comme trifluorométhyle ;

iii) ou tricyclique ABC suivant :

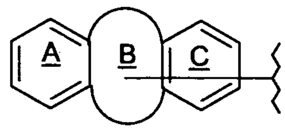

dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

➢ hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme alkyle, oxo ou thioxo ; ou l'hétérocycle représente le groupement suivant :

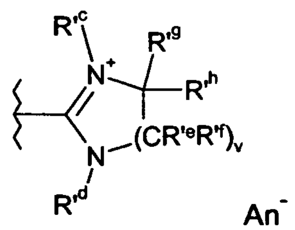

dans lequel R'$^c$, R'$^d$, R'$^e$, R'$^f$, R'$^g$ et R'$^h$, identiques ou différents représentent un atome d'hydrogène ou un groupement alkyle, ou alors deux groupement R'$^g$ avec R'$^h$, et/ou R'$^e$ avec R'$^f$ forment un groupement oxo ou thioxo, ou alors R'$^g$ avec R'$^e$ forment ensemble un cycloalkyle, et v représente un entier compris inclusivement entre 1 et 3 ; préférentiellement R'$^c$ à R'$^h$ représentent un atome d'hydrogène ; et $An^-$ représente le contre-ion ;

➢ isothiouronium -C(NR'$^c$R'$^d$)=$N^+$R'$^e$R'$^f$; $An^-$ avec R'$^c$, R'$^d$, R'$^e$ et R'$^f$, identiques ou différents représentent un atome d'hydrogène ou un groupement alkyle ; préférentiellement R'$^c$ à R'$^f$ représentent un atome d'hydrogène; et $An^-$ représente le contre-ion ;

➢ isothiourée -C(NR'$^c$R'$^d$)=NR'$^e$ avec R'$^c$, R'$^d$, R'$^e$ sont tels que définis précédemment ;

➢ (di)arylalkyle éventuellement substitué tel que le 9-anthracénylméthyle, phénylméthyle ou diphénylméthyle éventuellement substitué par un plusieurs groupements notamment choisis parmi alkyle, alcoxy comme le méthoxy, hydroxy, alkylcarbonyle" (di)(alkyl)amino comme le diméthylamino ;

➢ (di)hétéroarylakyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que alkyle particulièrement méthyle, avantageusement le (di)hétéroarylakyle est (di)hétéroarylméthyle ou (di)hétéroaryléthyle ;

➢ CR'$^1$R'$^2$R'$^3$ avec R'$^1$, R'$^2$ et R'$^3$ identiques ou différents, représentant un atome d'halogène ou un groupe choisi parmi :

■ alkyle éventuellement substitué tel que méthyle, éthyle ;
■ alcoxy éventuellement substitué tel que méthoxy ou éthoxy ;
■ aryle éventuellement substitué tel que phényle éventuellement substitué par un ou plusieurs groupements comme alkyle, alcoxy, hydroxy ;
■ hétéroaryle éventuellement substitué tel que thiophényle, furanyle, pyrrolyle, pyranyle, pyridyle, éventuellement substitué par un groupement alkyle ;

➢ $P(Z^1)R''^1R''^2R''^3$ avec $R''^1$, et $R''^2$ identiques ou différents représentent un groupement hydroxy, alcoxy ou alkyle, $R''^3$ représente un groupement hydroxy ou alcoxy, et $Z^1$ représente un atome d'oxygène ou de soufre ;
➢ cyclique stériquement encombré ; et
➢ alcoxyalkyle éventuellement substitué tels que le méthoxyméthyle (MOM), éthoxyéthyle (EOM) et l'isobutoxyméthyle.

En particulier le groupement Y du colorant fluorescent de formule (II) représente un métal alcalin ou un groupement protecteur tel que:

➢ $(C_1\text{-}C_4)$alkylcarbonyle comme méthylcarbonyle ou éthylcarbonyle ;
➢ arylcarbonyle comme phénylcarbonyle ;
➢ $(C_1\text{-}C_4)$alcoxycarbonyle ;
➢ aryloxycarbonyle ;
➢ aryl$(C_1\text{-}C_4)$alcoxycarbonyle ;
➢ (di) $(C_1\text{-}C_4)$ (alkyl)aminocarbonyle comme diméthylaminocarbonyle ;
➢ $(C_1\text{-}C_4)$(alkyl)arylaminocarbonyle ;
➢ aryle éventuellement substitué tel que le phényle ;
➢ hétéroaryle monocyclique cationique à 5, 6 chaînons tels que pyrylium, pyridinium, pyrimidinium, pyrazinium, pyridazinium, triazinium, imidazolium ; ces groupements étant éventuellement substitué par un ou plusieurs, identiques ou différents, groupes $(C_1\text{-}C_4)$alkyle tel que méthyle;
➢ hétéroaryle bicyclique cationique à 8 à 11 chaînons tels que benzoimidazolium, ou le benzoxazolium ; ces groupements étant éventuellement substitué par un ou plusieurs, identiques ou différents, groupes $(C_1\text{-}C_4)$alkyle tel que méthyle ;
➢ hétérocycle cationique de formule suivante :

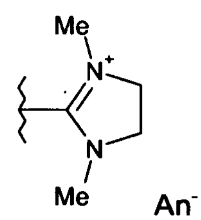

➢ isothiouronium $-C(NH_2)=N^+H_2$; $An^-$;
➢ isothiourée $-C(NH_2)=NH$ ;
➢ $SO_3^-$, $M^+$ avec $M^+$ représentant un métal alcalin tel que le sodium ou le potassium, ou alors M' de la formule (II) et $M^+$ sont absents.

Selon un mode de réalisation particulier les colorants fluorescents thiols protégés de formule (II) comportent un groupement Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, , isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium; pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme méthyle, ou polyhalogéno$(C_1\text{-}C_4)$alkyle comme trifluorométhyle ; iii) ou hétérocyclique suivant :

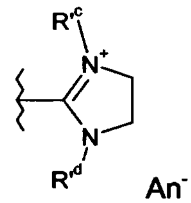

dans lequel R'$^c$ et R'$^d$, identiques ou différents, représentent un atome d'hydrogène ou un groupement ($C_1$-$C_4$)alkyle ; préférentiellement R'$^c$ à R'$^d$ représentent un groupement ($C_1$-$C_4$)alkyle tel que méthyle ; et An$^-$ représente un contre-ion.

Selon un autre mode de réalisation particulier lorsque $T_a$, $T_b$ représentent une liaison covalente. Particulièrement les colorants fluorescents thiols protégés de formule (II) comportent alors un groupement Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme méthyle, ou polyhalogénoalkyle comme trifluorométhyle ; iii) ou hétérocyclique suivant :

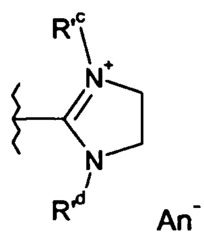

dans lequel R'$^c$ et R'$^d$, identiques ou différents, représentent un groupement alkyle ; préférentiellement R'$^c$ à R'$^d$ représentent un groupement alkyle tel que méthyle ; et An$^-$ représente un contre-ion anionique.

Particulièrement Y représente un groupement choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzimidazolium, benzoxazolium, benzothiazolium, ces groupements étant éventuellement substitués par un ou plusieurs groupes ($C_1$-$C_4$) alkyle notamment méthyle.

Selon un mode particulier de l'invention, les colorants fluorescents de l'invention sont de formule (Ia), (IIa), (Ib), (IIb), (Ic) ou (IIc) possédant chacun un groupement éthylène qui relie la partie pyridinium au phényle en position ortho ou para soit en positions 4-4', 4-2', ou 2-4' pour (Ia), IIa), (Ib) ou (IIb), ou alors en positions 4-5' ou 2-5' pour (Ic) ou (IIc) :

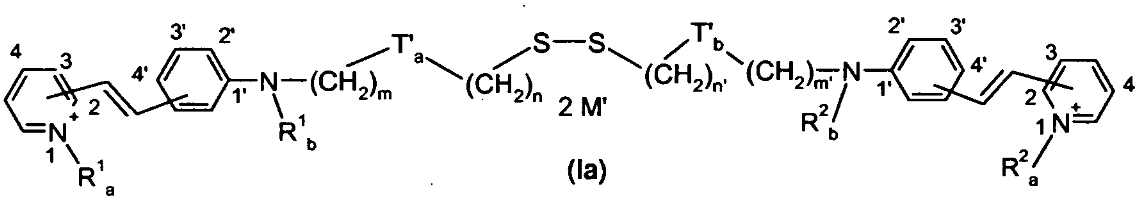

(Ia)

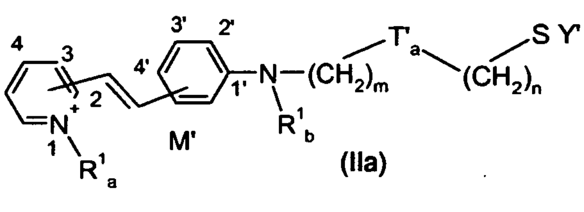

(IIa)

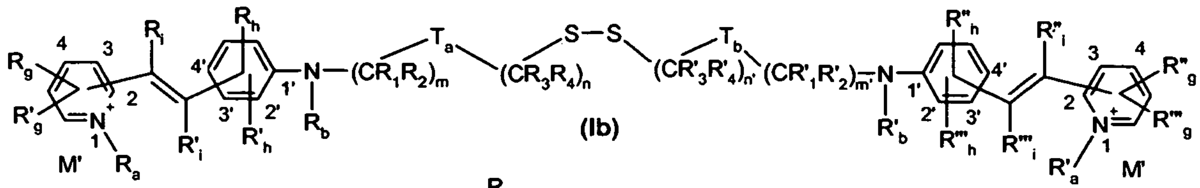

(Ib)

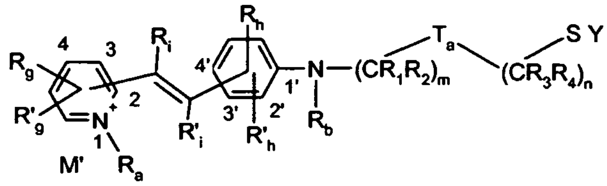

(IIb)

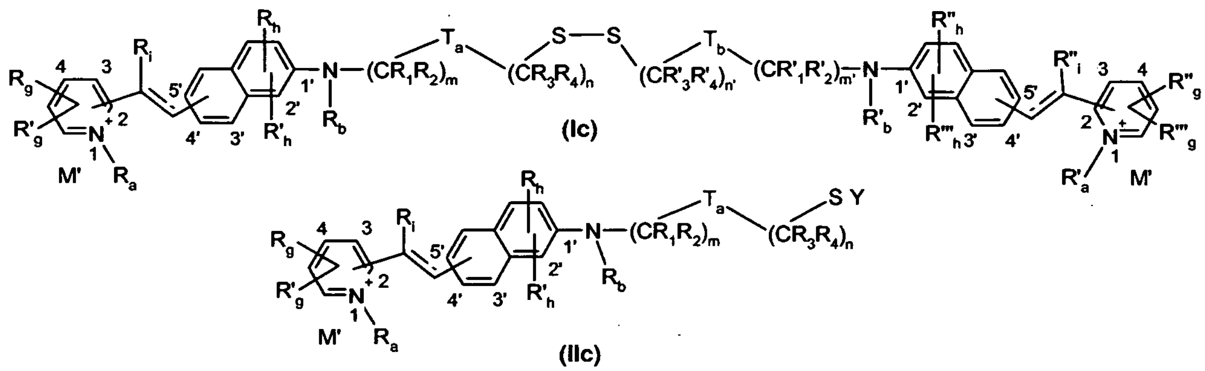

(Ic)

(IIc)

formules (Ia) ou (IIa) dans lesquelles :

- $R^1{}_a$ et $R^2{}_a$, identiques ou différents, représentent un groupement benzyle ou alkyle éventuellement substitué par un groupement hydroxy tel que méthyle, éthyle ou hydroxyéthyle ;
- $R^1{}_b$ et $R^2{}_b$, identiques ou différents, représentent un atome d'hydrogène ; un groupement benzyle ou alkyle tel que le méthyle ou l'éthyle ;
- $T'_a$ et $T'_b$, identiques ou différents, représentent une liaison σ ; un groupement $-N^+(R)(R^°)-$ avec R et R°, identiques ou différents, représentant un groupement alkyle tel que le méthyle ; ou alors $T'_a$ et $T'_b$ représentent un groupement imidazolium ;
- Y' représente un groupement choisi parmi imidazolium, thiazolium ; oxazolium ; 1,2,4-triazolium ; pyridinium ; pyrimidinium ; benzoxazolium et benzothiazolium ; ces groupements étant substitués par un ou plusieurs groupements, identiques ou différents, groupement alkyle tel que méthyle ou éthyle ;
- m, n, m' et n', identiques ou différents, représentent un entier compris inclusivement entre 1 et 6 avec m+n=m'+n' représentent un entier compris inclusivement entre 2 et 6; particulièrement lorsque $T'_a$ et $T'_b$ représentent une liaison σ, alors la somme m+n=m'+n' est un entier égal à 2 ;
- M' représente un contre-ion anionique ;

formules (Ib) ou (IIb) dans lesquelles :

- ➢ $R_a$, $R'_a$, $R_b$, $R'_b$, $R_i$, $R'_i$, $R''_i$, $R'''_i$, $R_1$, $R'_1$, $R_2$, $R'_2$, $R_3$, $R'_3$, $R_4$, $R'_4$, $T_a$, $T_b$, m, m', n, n', Y sont tels que définis précédemment ;
- ➢ $R_g$ et $R'_g$ ; $R''_g$ et $R'''_g$ identiques ou différents, représentent un atome d'hydrogène, un atome d'halogène, un groupement amino, $(C_1\text{-}C_4)$alkylamino, $(C_1\text{-}C_4)$ dialkylamino, cyano, carboxy, hydroxy, trifluorométhyle, acylamino, alcoxy en $C_1\text{-}C_4$, (poly)hydroxyalcoxy en $C_2\text{-}C_4$, $(C_1\text{-}C_4)$alkylcarbonyloxy, $(C_1\text{-}C_4)$alcoxycarbonyle, $(C_1\text{-}C_4)$alkylcarbonylamino, acylamino, carbamoyle , $(C_1\text{-}C_4)$alkylsulfonylamino, amino-sulfonyle, ou un radical $(C_1\text{-}C_{16})$ alkyle éventuellement substitué par un groupement choisi parmi $(C_1\text{-}C_{12})$ alcoxy, hydroxy, cyano, carboxy, amino, $(C_1\text{-}C_4)$ alkylamino et $(C_1\text{-}C_4)$ dialkylamino, ou alors les deux radicaux alkyle portés par l'atome d'azote du groupement amino forment un hétérocycle comprenant de 5 à 7 chaînons et comprenant éventuellement un autre hétéroatome

identique ou différent à celui de l'atome d'azote ; particulièrement $R_g$ et $R'_g$ ; $R''_g$ et $R'''_g$ représentent un atome d'hydrogène, d'halogène ou un groupement ($C_1$-$C_3$) alkyle, avantageusement $R_g$ et $R'_g$ ; $R''_g$ et $R'''_g$ représentent un atome d'hydrogène ;

➢ $R_h$ et $R'_h$ ; $R''_h$ et $R'''_h$ portés par deux atomes de carbone adjacents, forment ensembles un cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, ($C_1$-$C_4$)alkylamino, ($C_1$-$C_4$)dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonylamino, un radical acylamino, carbamoyle, ($C_1$-$C_4$)alkylsulfonyl-amino, un radical aminosulfonyle, ou un radical ($C_1$-$C_{16}$)alkyle éventuellement substitué par un groupement choisi parmi ($C_1$-$C_{12}$)alcoxy, hydroxy, cyano, carboxy, amino, ($C_1$-$C_4$)alkylamino et ($C_1$-$C_4$) dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ; particulièrement $R_h$ et $R'_h$ ; $R''_h$ et $R'''_h$ forment ensemble un groupement benzo ; et

formules (Ic) ou (IIc) dans lesquelles :

➢ $R_a$, $R'_a$, $R_b$, $R'_b$, $R_n$ $R''_i$, $R_1$, $R'_1$, $R_2$, $H'_2$, $R_3$, $R'_3$, $R_4$, $R'_4$, $T_a$, $T_b$, m, m', n, n', Y sont tels que définis précédemment ;

➢ $R_g$, $R'_g$, $R''_g$, $R'''_g$, $R_h$, $R'_h$, $R''_h$ et $R'''_h$, identiques ou différents, représentent un atome d'hydrogène, un atome d'halogène, un groupement amino, ($C_1$-$C_4$)alkylamino, ($C_1$-$C_4$) dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy, ($C_1$-$C_4$)alcoxycarbonyle, ($C_1$-$C_4$)alkylcarbonylamino, acylamino, carbamoyle , ($C_1$-$C_4$)alkylsulfonylamino, amino-sulfonyle, ou un radical ($C_1$-$C_{16}$) alkyle éventuellement substitué par un groupement choisi parmi ($C_1$-$C_{12}$) alcoxy, hydroxy, cyano, carboxy, amino, ($C_1$-$C_4$) alkylamino et ($C_1$-$C_4$) dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ; particulièrement $R_g$, $R'_g$, $R_h$ et $R'_h$ représentent un atome d'hydrogène, d'halogène ou un groupement ($C_1$-$C_3$) alkyle, avantageusement $R_g$, $R'_g$, $R_h$ et $R'_h$ représentent un atome d'hydrogène ;

étant entendu que lorsque le composé de formule **(Ia), (IIa), (Ib), (IIb), (Ic)** ou **(IIc)** contient d'autres parties cationiques, il se trouve associé à un ou plusieurs contre-ions anioniques permettant d'atteindre l'électroneutralité de la formule **(Ia), (IIa), (Ib), (IIb), (Ic)** ou **(IIc).**

Un autre mode de réalisation particulier de l'invention concerne des colorants disulfure de formule (I) symétriques ie $R_a$, $R_b$, $R_g$, $R'_g$, $R_h$, $R'_h$, $R_i$, $R'_i$, $R_1$, $R_2$, $R_3$, $R_4$, $T_a$, m, n sont identiques à $R'_a$, $R'_b$, $R''_g$, $R'''_g$, $R''_h$, $R'''_h$, $R''_i$, $R'''_i$, $R'_1$, $R'_2$, $R'_3$, $R'_4$, $T_b$, m' et n' respectivement.

A titre d'exemple de colorants fluorescents de l'invention, on peut citer notamment les colorants suivants :

| | |
|---|---|
| 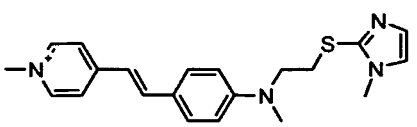 $An^-$ | 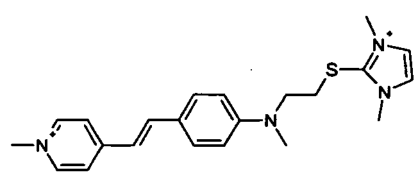 $2An^-$ |
| **1** | **2** |
| 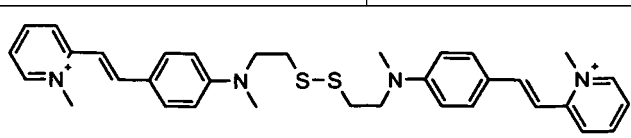 2M' | |
| **3** | |
| 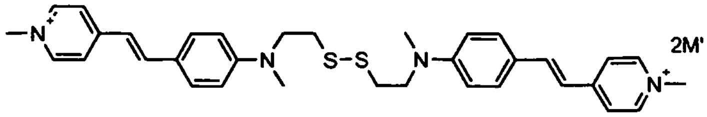 2M' | |
| **4** | |

(suite)

| |
|---|
| 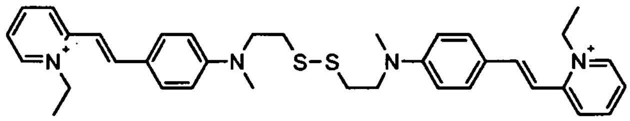 2M' |
| **5** |
| 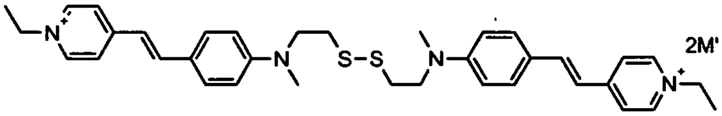 2M' |
| **6** |
| 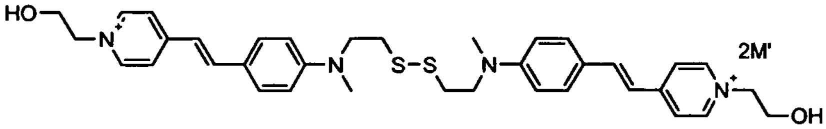 2M' |
| **7** |
| 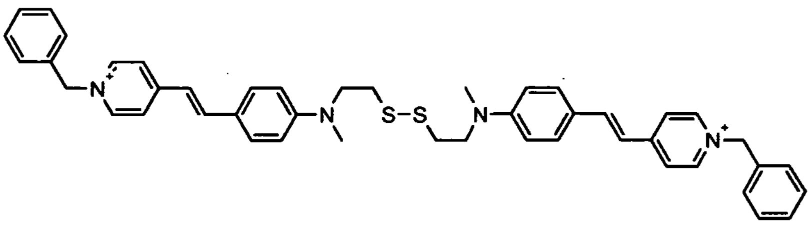 2M' |
| **8** |
| 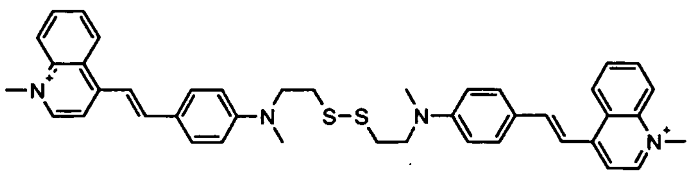 2M' |
| **9** |
| 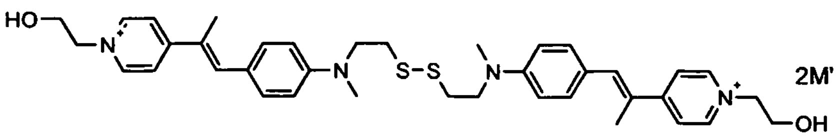 2M' |
| **10** |
| 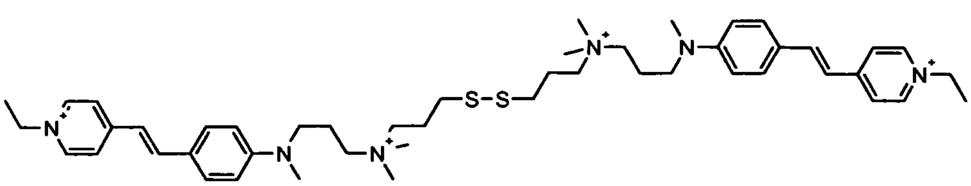 4M' |
| **11** |

(suite)

| | |
|---|---|
| 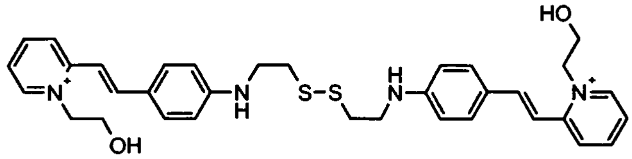 2M' | |
| **12** | |
| 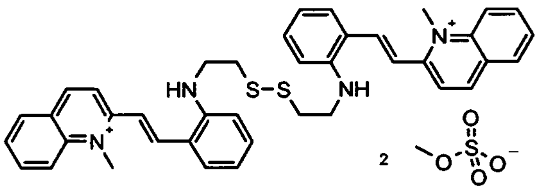 2 | 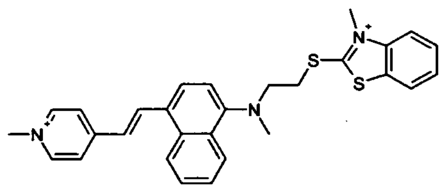 2An$^-$ |
| **13** | **14** |
| 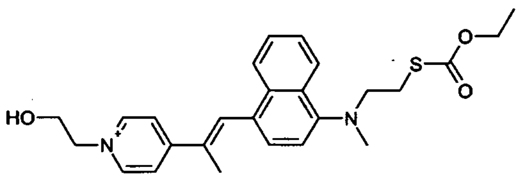 An$^-$ | 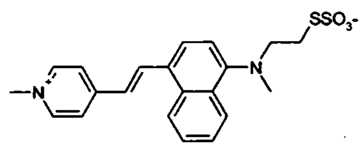 An$^-$, M$^+$ |
| **15** | **16** |
| 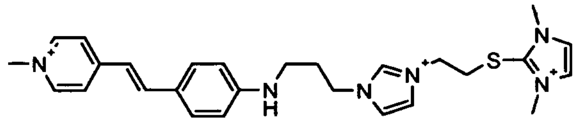 3An$^-$ | 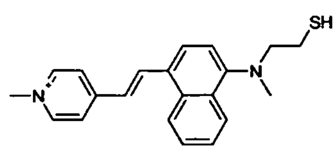 An$^-$ |
| **17** | **18** |
| 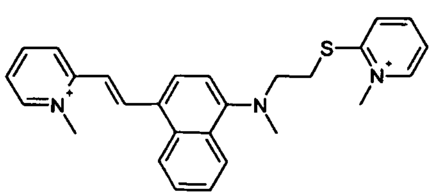 2An$^-$ | 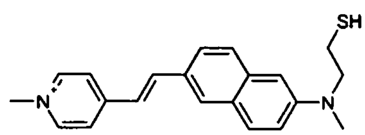 An$^-$ |
| **19** | **- 20** |
| 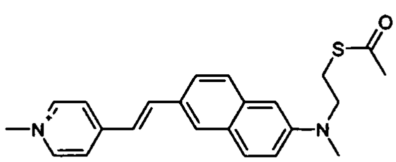 An$^-$ | 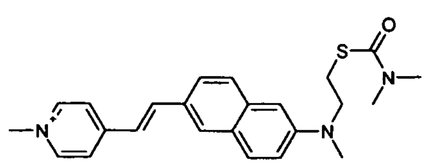 An$^-$ |
| **21** | **22** |

(suite)

| | |
|---|---|
| 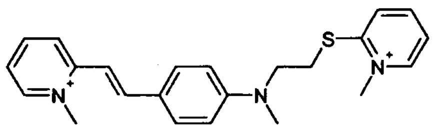 2An$^-$ | 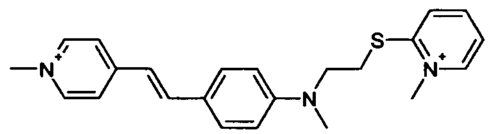 2An$^-$ |
| **23** | **24** |
| 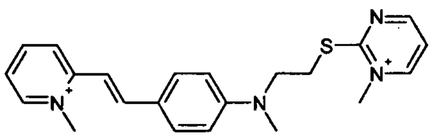 2An$^-$ | 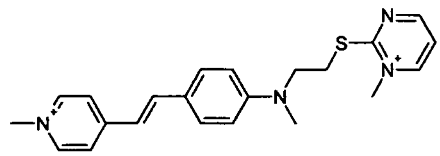 2An$^-$ |
| **25** | **26** |
| 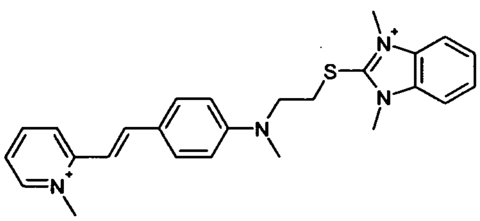 2An$^-$ | 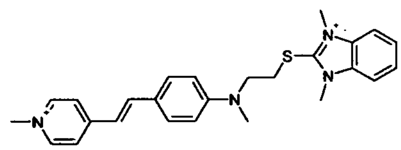 2An$^-$ |
| **27** | **28** |
| 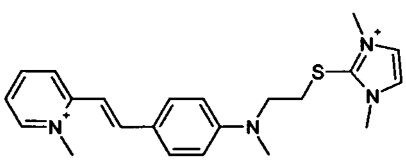 2An$^-$ | 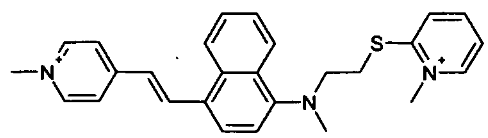 2 An$^-$ |
| **29** | **30** |
| 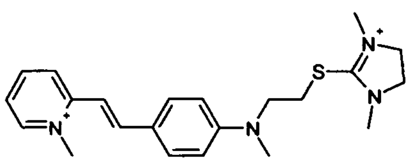 2An$^-$ | 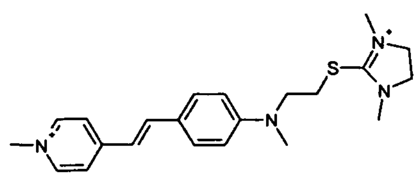 2An$^-$ |
| **31** | **32** |
| 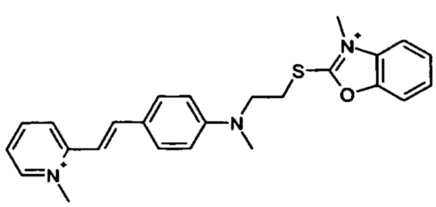 2An$^-$ | 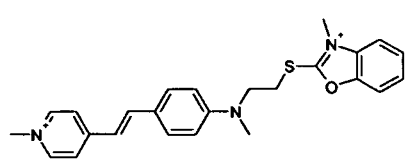 2An$^-$ |
| **33** | **34** |

(suite)

| | |
|---|---|
| 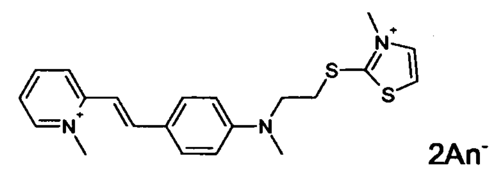 | 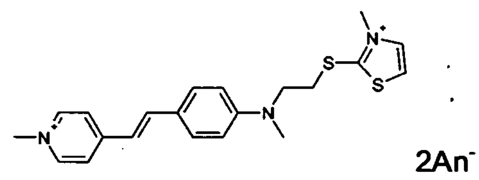 |
| **35** | **36** |
| 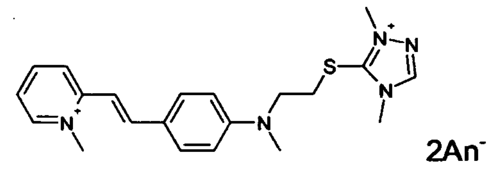 | 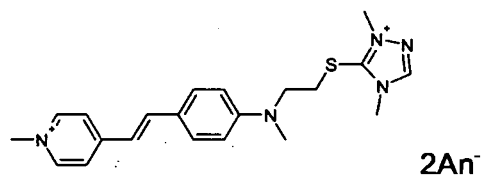 |
| **37** | **38** |
| 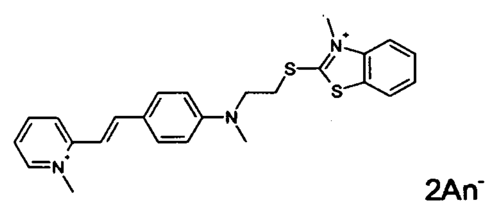 | 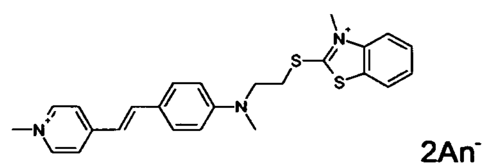 |
| **39** | **40** |
| 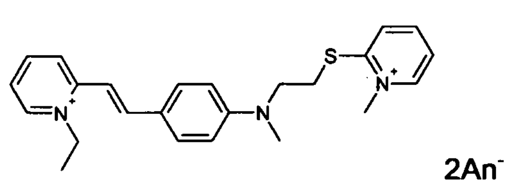 | 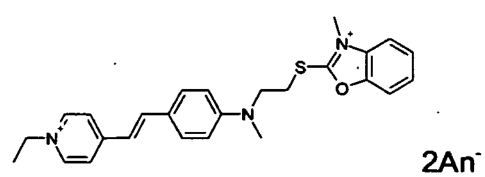 |
| **41** | **42** |
| 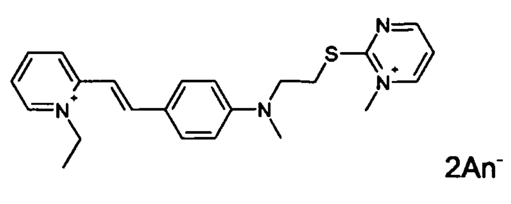 | 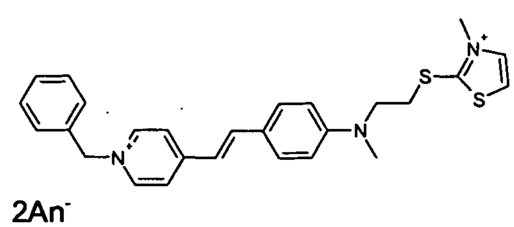 |
| **43** | **44** |
| 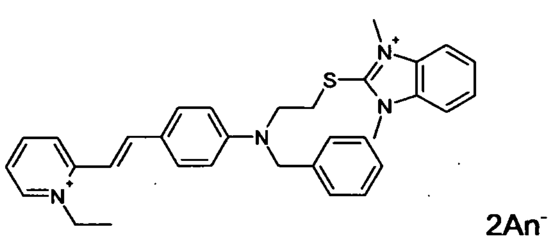 | 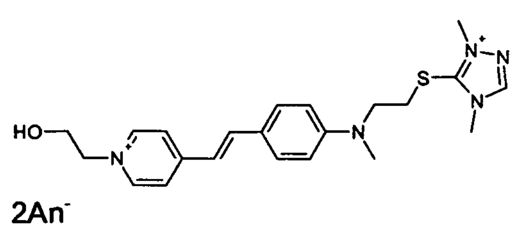 |
| **45** | **46** |

(suite)

| | |
|---|---|
| 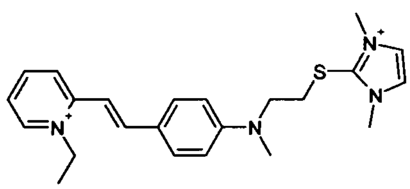 $2An^-$ | 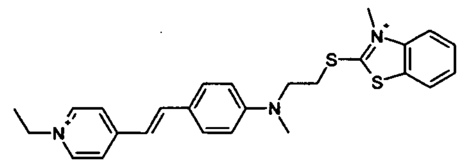 2A $n^-$ |
| **47** | **48** |
| 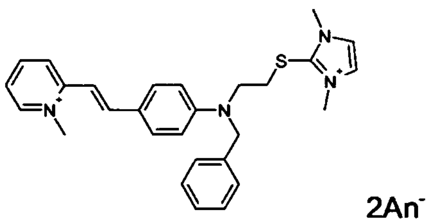 $2An^-$ | 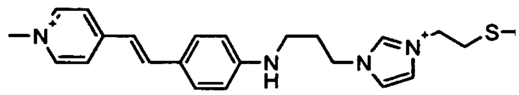 $3An^-$ |
| **49** | **50** |
| 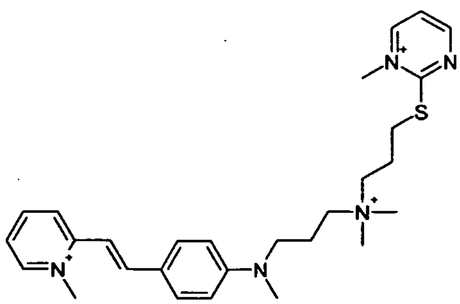 $3An^-$ | 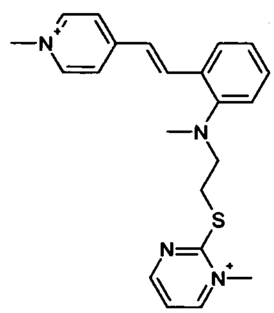 $2An^-$ |
| **51** | **52** |
| 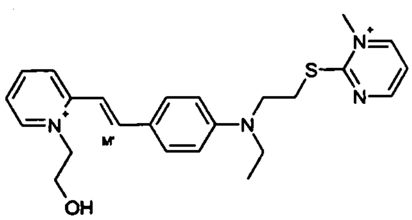 $2An^-$ | 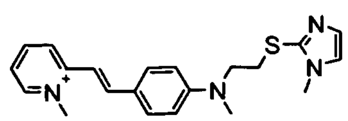 $An^-$ |
| **53** | **54** |
| 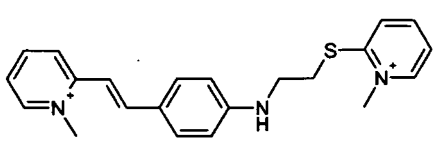 $2An^-$ | 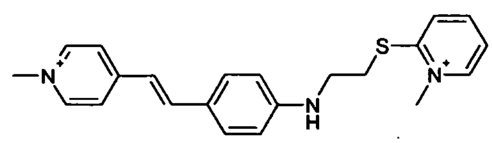 2 $An^-$ |
| **55** | **56** |

(suite)

| | |
|---|---|
| 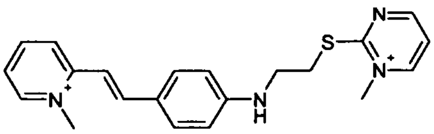 2An⁻ | 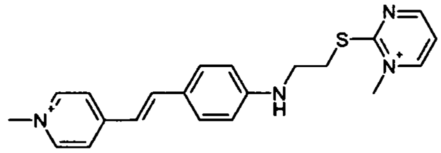 2An⁻ |
| **57** | **58** |
| 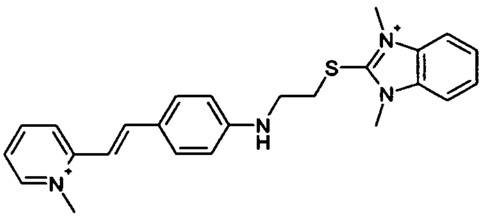 2An⁻ | 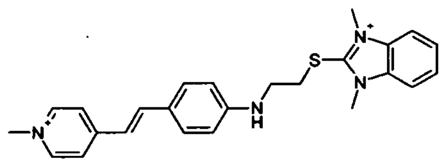 2An⁻ |
| **59** | **60** |
| 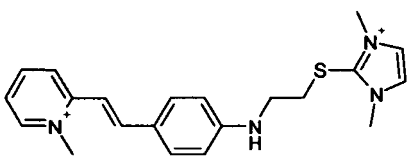 2An⁻ | 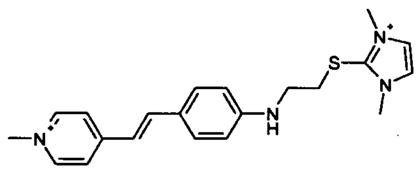 2An⁻ |
| **61** | **62** |
| 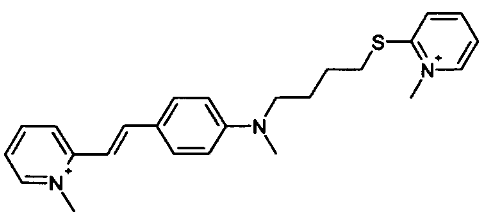 2An⁻ | 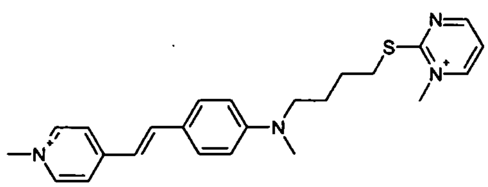 2 An⁻ |
| **63** | **64** |
| 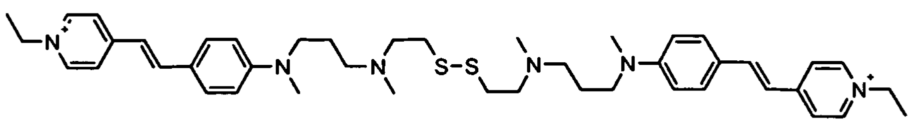 2M' | |
| **65** | |
| 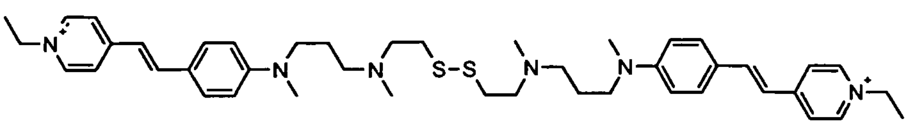 2M' | |
| **66** | |
| 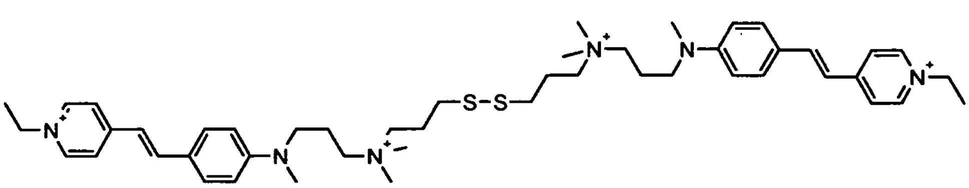 4M' | |

(suite)

| 67 |
|---|
| 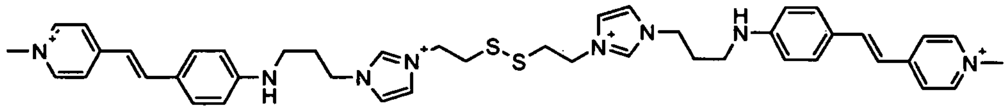 4M' |
| 68 |
| 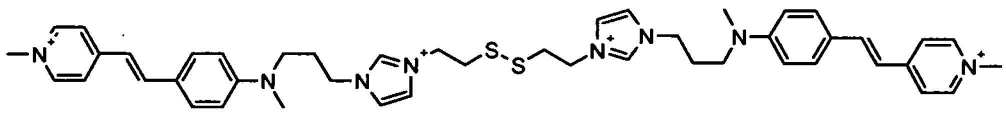 4M' |
| 69 |
| 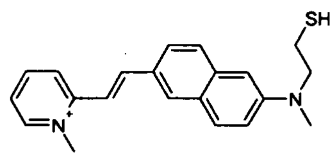 An⁻ |
| 70 |
| 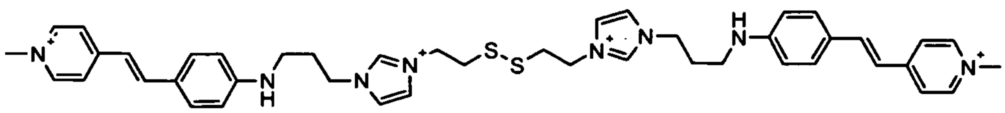 4M' |
| 71 |
| 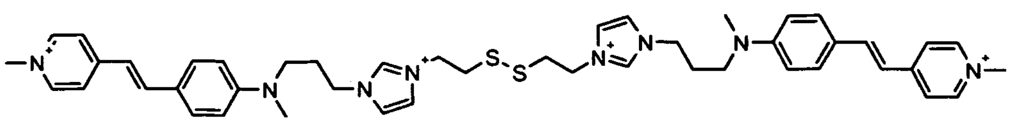 4M' |
| 72 |
| 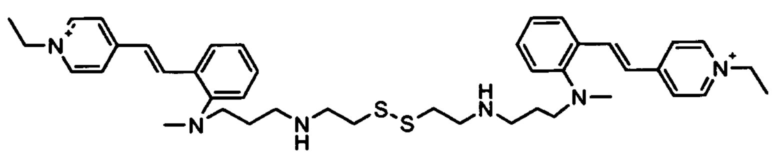 2M' |
| 73 |
| 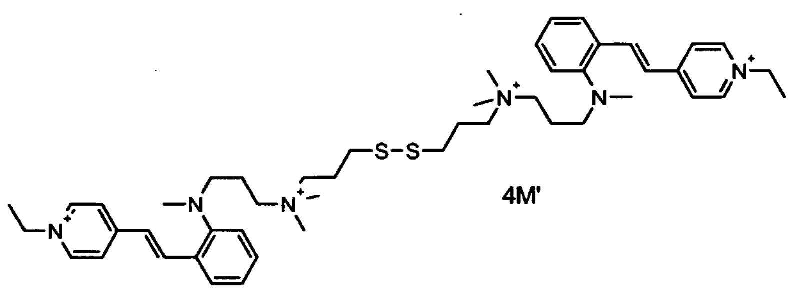 4M' |
| 74 |

(suite)

| |
|---|
| 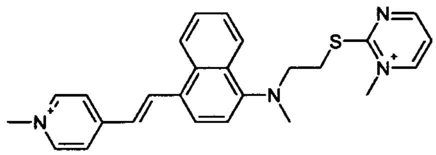 $2An^-$ |
| **75** |
| 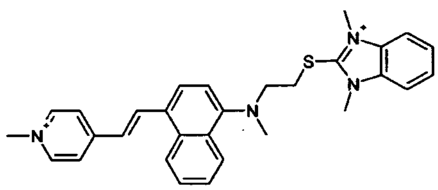 $2An^-$ |
| **76** |
| 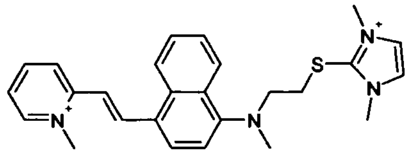 $2An^-$ |
| **77** |
| 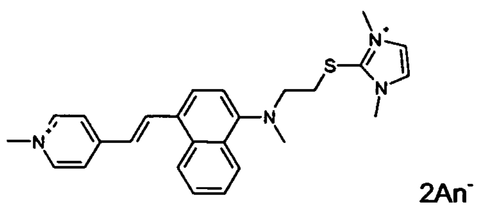 $2An^-$ |
| **78** |
| 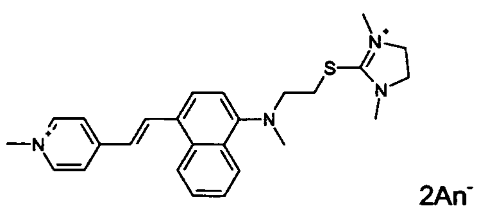 $2An^-$ |
| **79** |
| 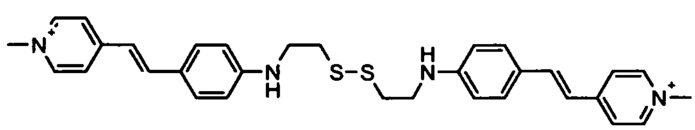 2M' |
| **80** |
| 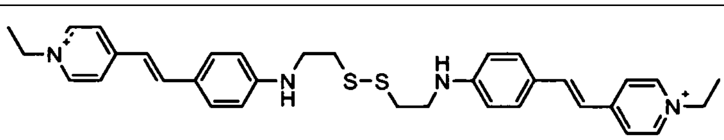 2M' |
| **82** |

(suite)

| |
|---|
| 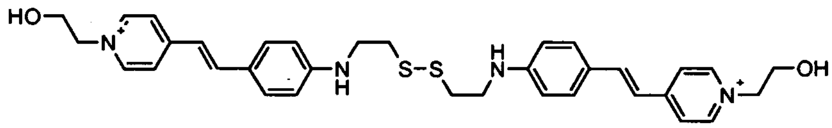 2M' |
| **83** |
| 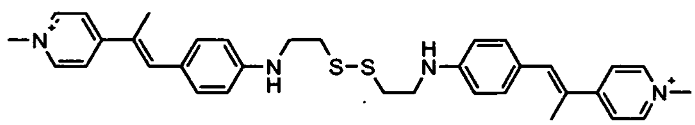 2M' |
| **84** |
| 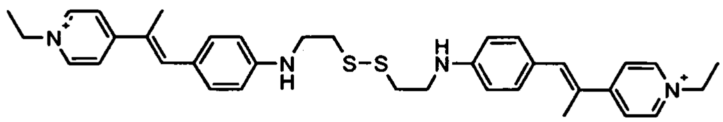 2M' |
| **85** |
| 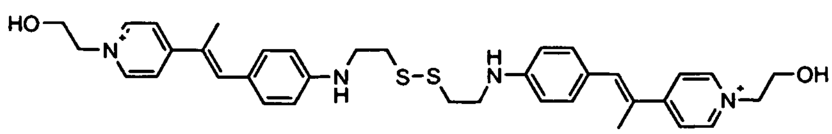 2M' |
| **86** |
| 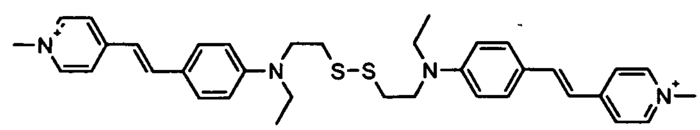 2M' |
| **87** |
| 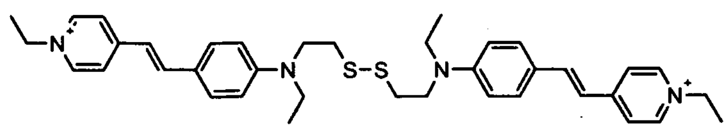 2M' |
| **88** |
| 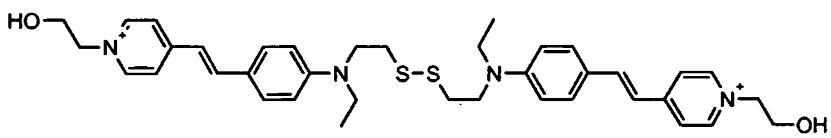 2M' |
| **89** |
| 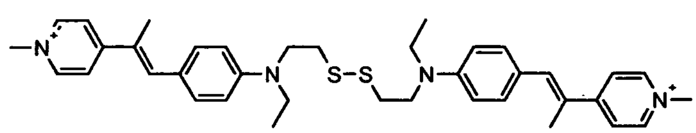 2M' |
| **90** |

(suite)

| |
|---|
| 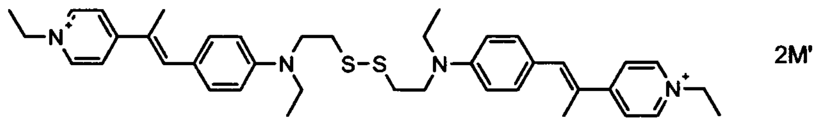 |
| **91** |
| 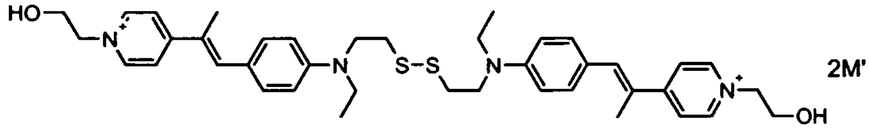 |
| **92** |
| 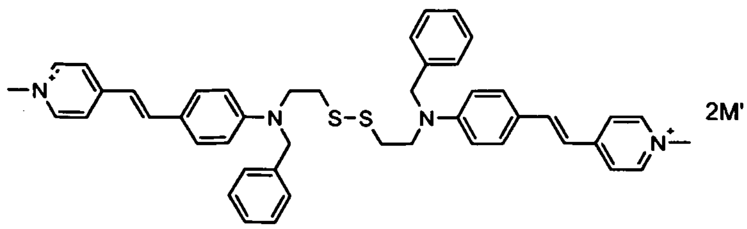 |
| **93** |
| 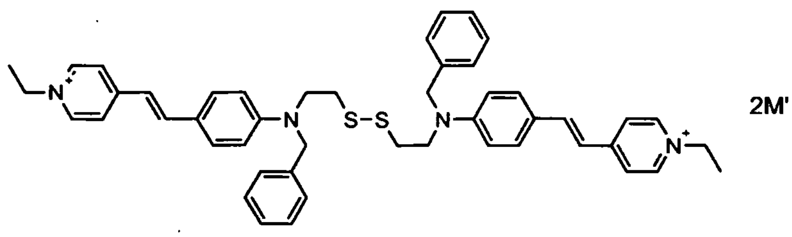 |
| **94** |
| 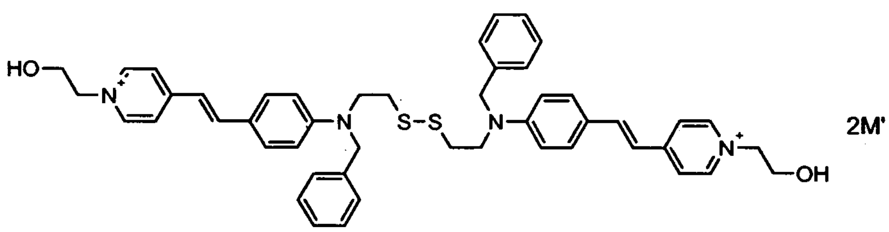 |
| **95** |
| 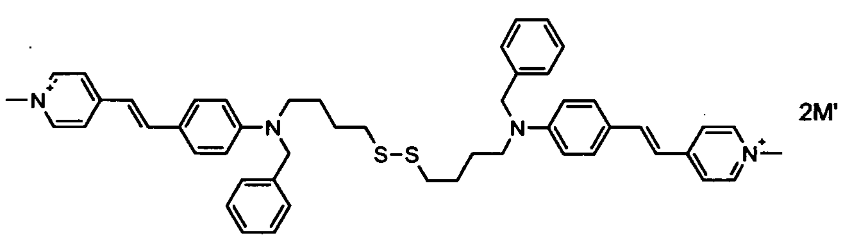 |
| **96** |

(suite)

| |
|---|
| 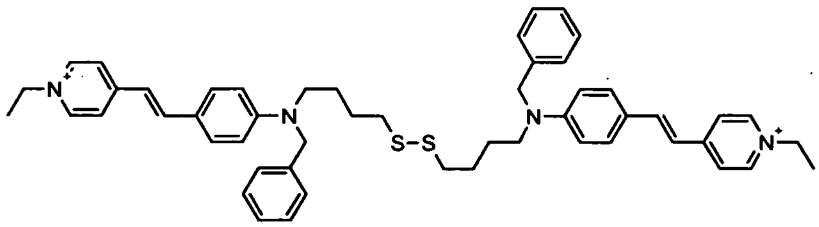 2M' |
| **97** |
| 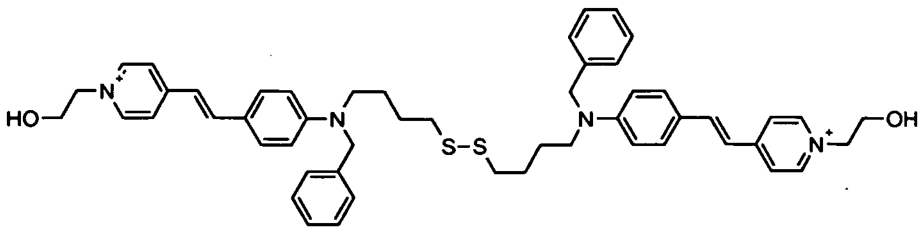 2M' |
| **98** |
| 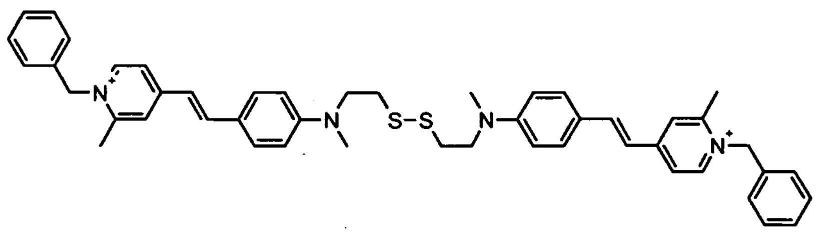 2M' |
| **99** |
| 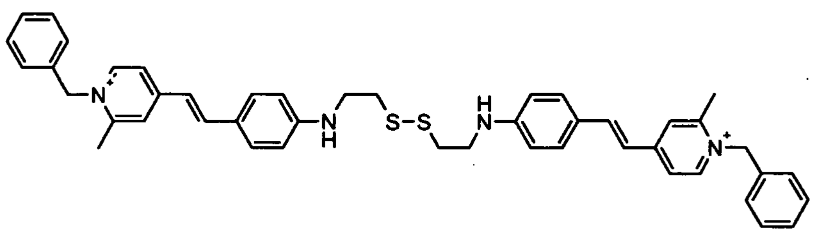 2M' |
| **100** |
| 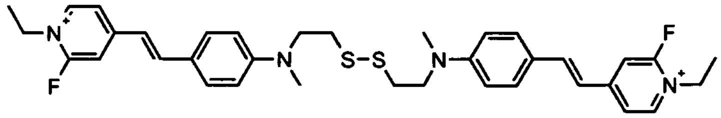 2M' |
| **101** |
| 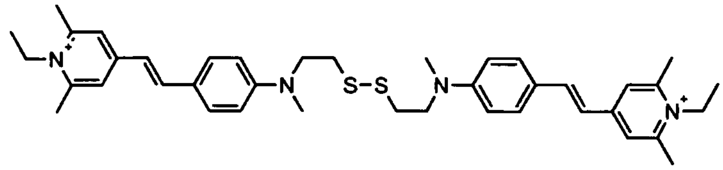 2M' |
| **102** |

(suite)

| |
|---|
| 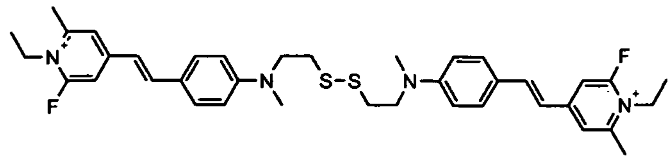 2M' |
| **103** |
| 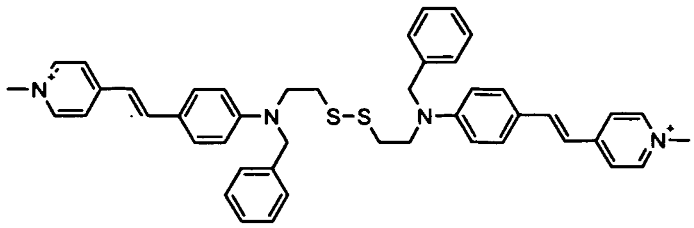 2M' |
| **104** |
| 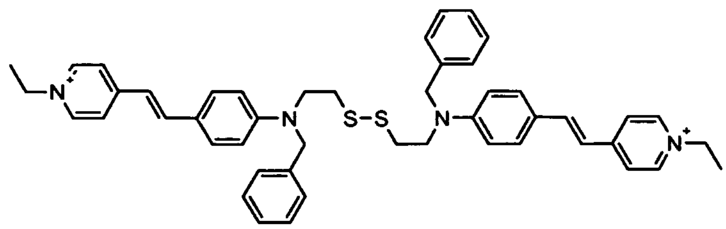 2M' |
| **105** |
| 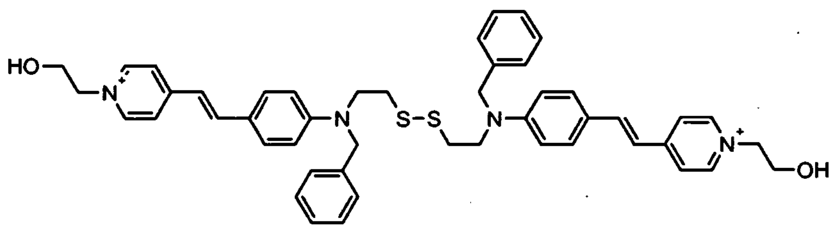 2M' |
| **106** |
| 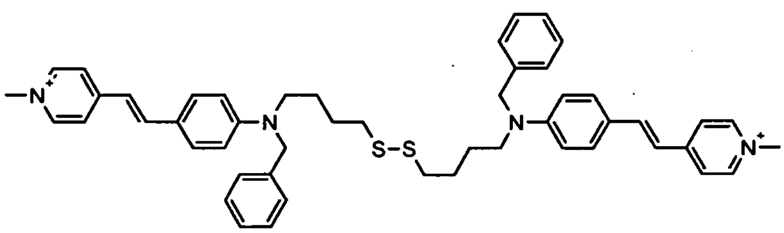 2M' |
| **107** |
| 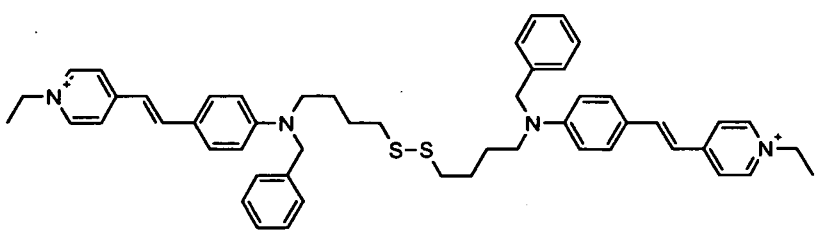 2M' |

(suite)

| 108 |
|---|
| 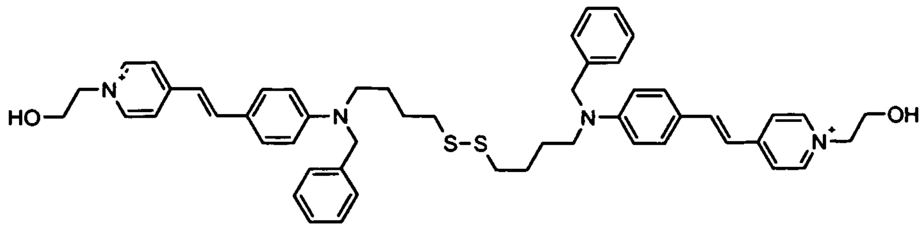 2M' |
| 109 |
| 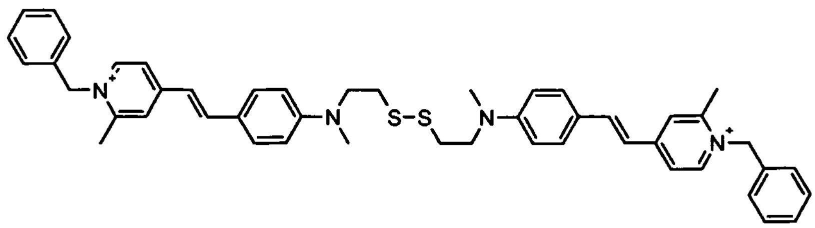 2M' |
| 110 |
| 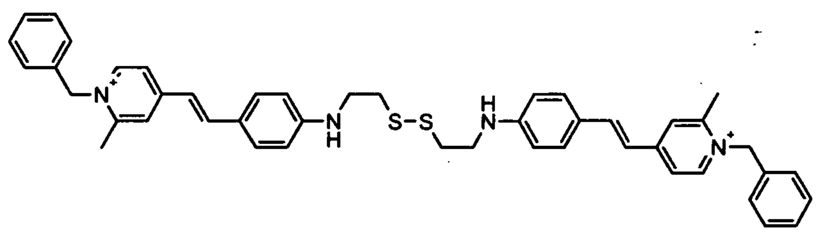 2M' |
| 111 |
| 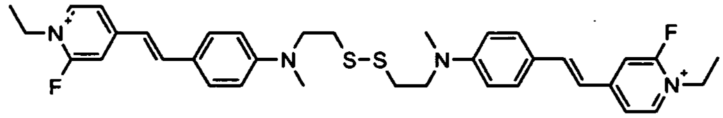 2M' |
| 112 |
| 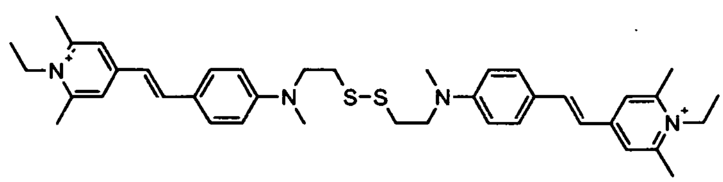 2M' |
| 113 |
| 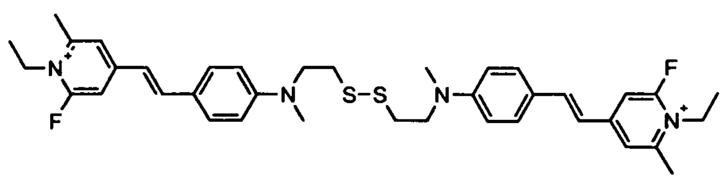 2M' |
| 114 |

(suite)

| |
|---|
| 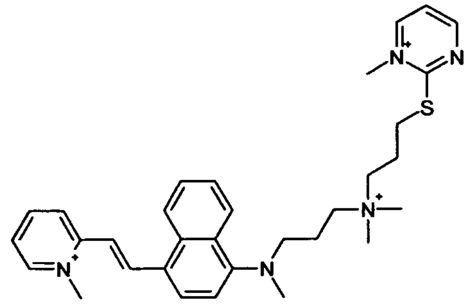 3An⁻ |
| **115** |
| 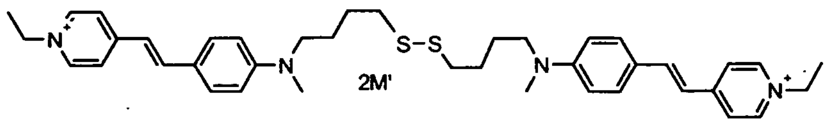 2M' |
| **116** |
| 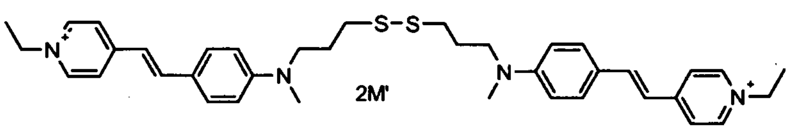 2M' |
| **117** |
| 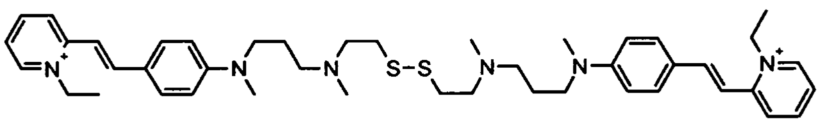 2M' |
| **118** |
| 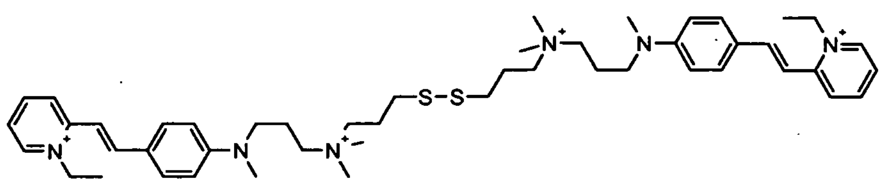 4M' |
| **119** |
| 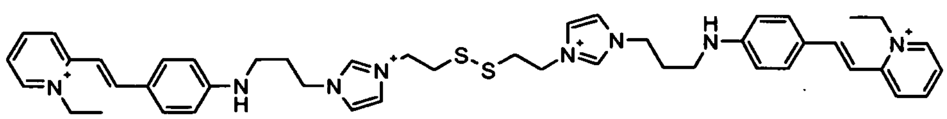 4M' |
| **120** |
| 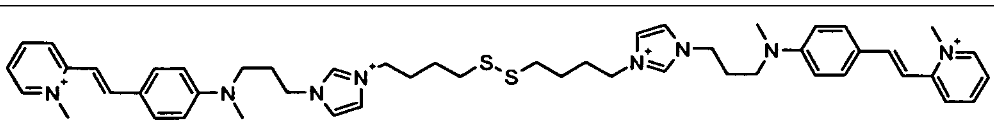 4M' |
| **121** |

(suite)

| |
|---|
| 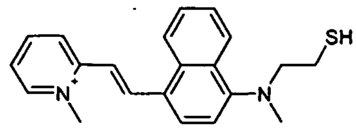 |
| **122** |
| 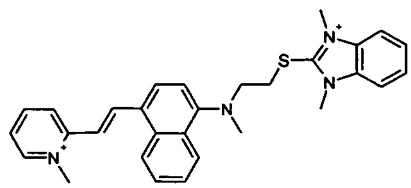 2M' |
| **123** |
| 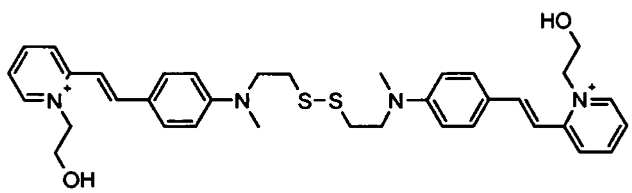 2M' |
| **124** |
| 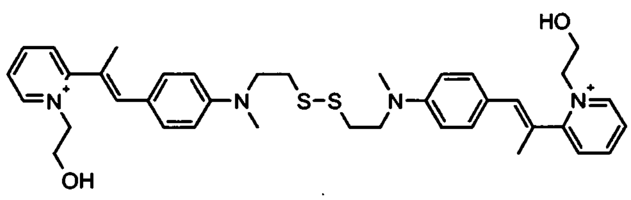 2M' |
| **125** |
| 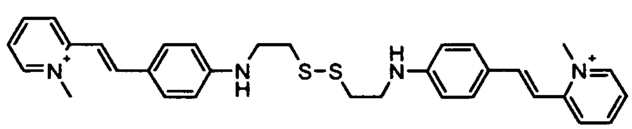 2M' |
| **126** |
| 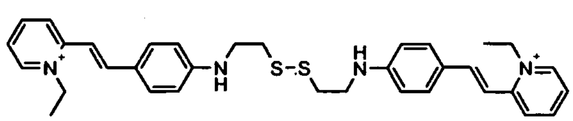 2M' |
| **127** |
| 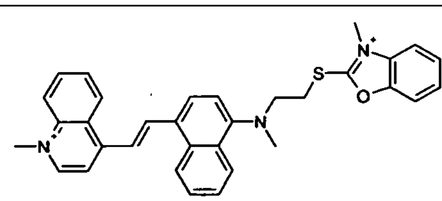 2An$^-$ |
| 128 |

(suite)

| |
|---|
| 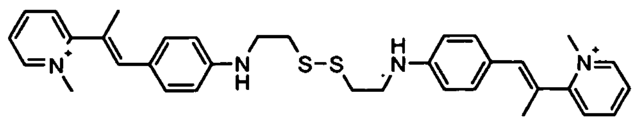 2M' |
| **129** |
| 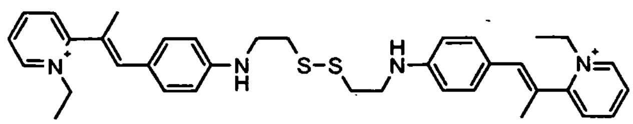 2M' |
| **130** |
| 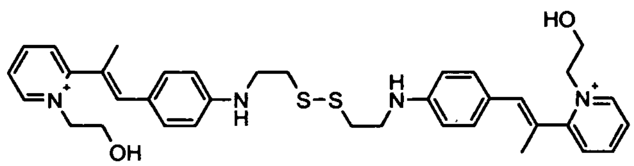 2M' |
| **131** |
| 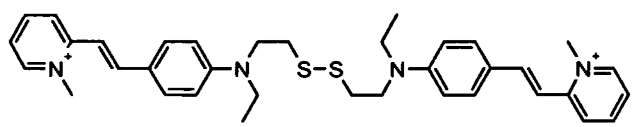 2M' |
| **132** |
| 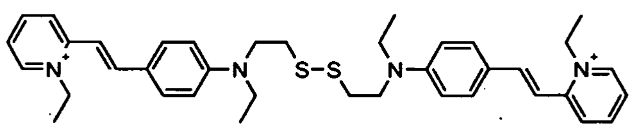 2M' |
| **133** |
| 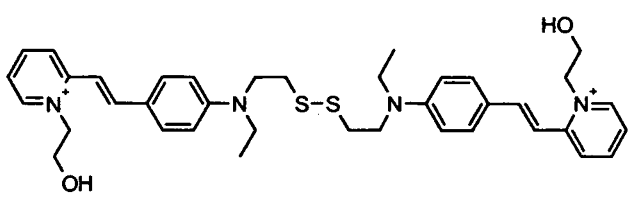 2M' |
| **134** |
| 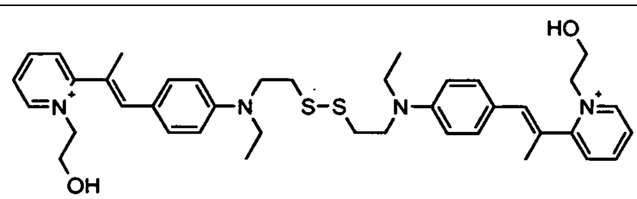 2M' |
| **135** |

(suite)

| |
|---|
| 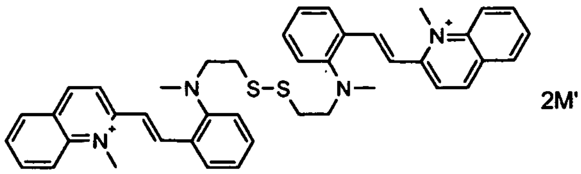 |
| **135** |
| 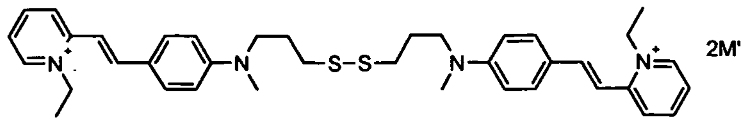 |
| **137** |
| 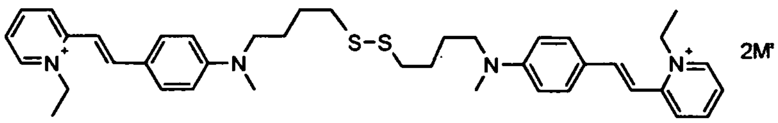 |
| **138** |
| 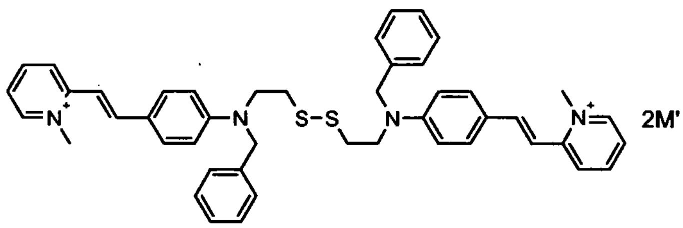 |
| **139** |
| 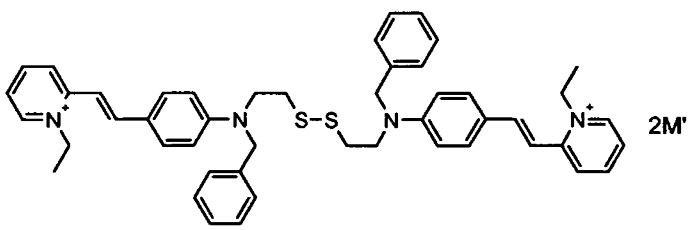 |
| **140** |
| 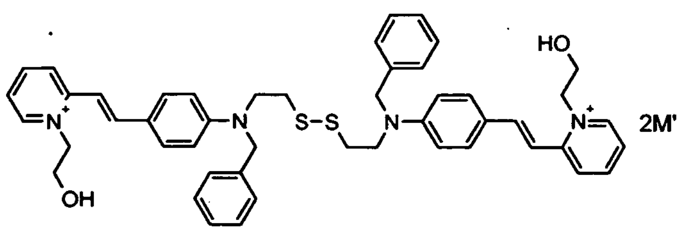 |
| **141** |

(suite)

| | |
|---|---|
| 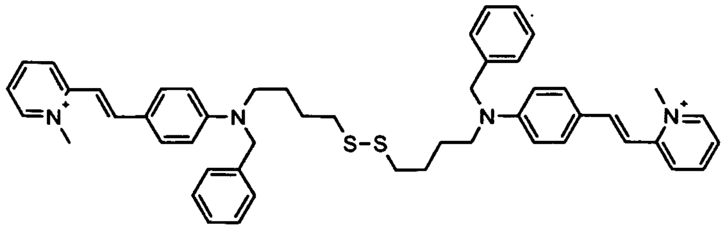 2M' | |
| 142 | |
| 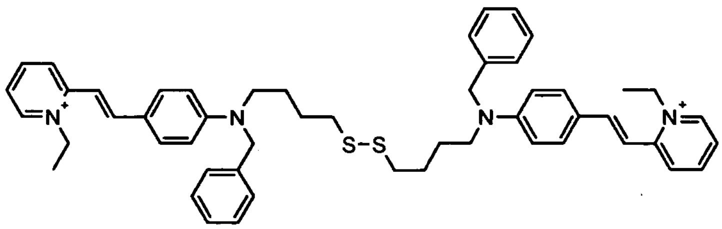 2M' | |
| 143 | |
| 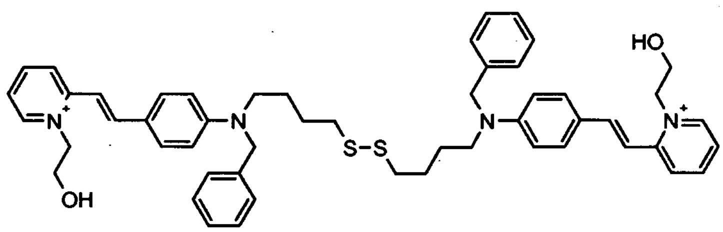 2M' | |
| 144 | |
| 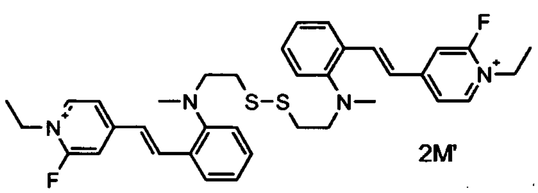 2M' | 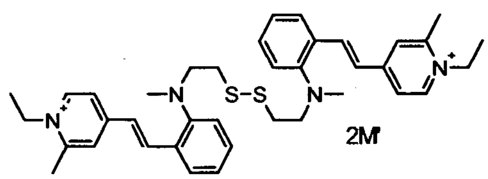 2M' |
| 145 | 146 |
| 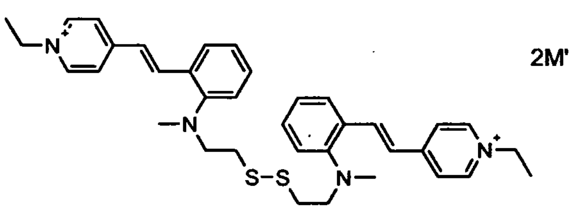 2M' | 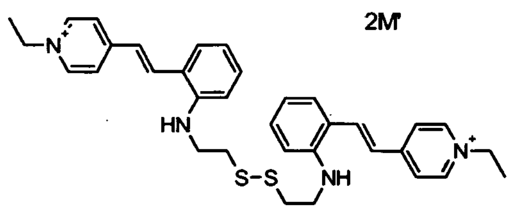 2M' |
| *147 | 148 |
| 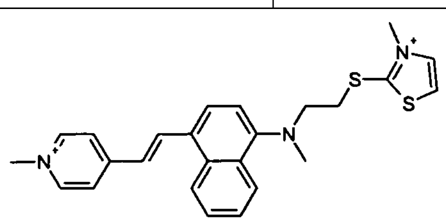 2An⁻ | |
| 149 | |

avec $An^-$, et M', identiques ou différents, représentant un contre-ion anionique ; et $M^+$ représentant un métal alcalin.

Avec $An^-$, M', identiques ou différents, représentent un contre-ion anionique tel que les halogénures, tels que le chlorure, le bromure ; les nitrates ; les sulfonates parmi lesquels les $C_1$-$C_6$ alkylsulfonates : Aik-$S(O)_2O^-$ tels que le méthylsulfonate ou mésylate et l'éthylsulfonate; les arylsulfonates : Ar-$S(O)_2O^-$ tel que le benzènesulfonate et le toluènesulfonate ou tosylate ; le citrate ; le succinate ; le tartrate ; le lactate ; les alkylsulfates : Alk-O-$S(O)O^-$ tels que le méthylsulfate et l'éthylsulfate ; les arylsulfates : Ar-O-$S(O)O^-$ tels que le benzènesulfate et le toluènesulfate ; les alcoxysulfates : Alk-O-$S(O)_2O^-$ tel que le méthoxy sulfate et l'éthoxysulfate ; les aryloxysulfates : Ar-O-$S(O)_2O^-$, le phosphate ; l'acétate; le triflate ; et les borates tels que le tétrafluoroborate.

Les colorants thiols protégés de formule **(II")** peuvent être synthétisés en deux étapes. La première étape consistant à préparer le colorant thiol non protégé **(II')** selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule **(II").** A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Nous pouvons illustrer cette méthode par la méthode consistant i) à générer des colorants fluorescents thiols par réduction d'un colorant fluorescent à deux chromophores portant une fonction disulfure -S-S- tels que **(I')** et ii) à protéger selon les méthodes classiques ladite fonction thiol les composés **(II')** pour accéder au colorants fluorescents thiols protégés de formule **(II")** :

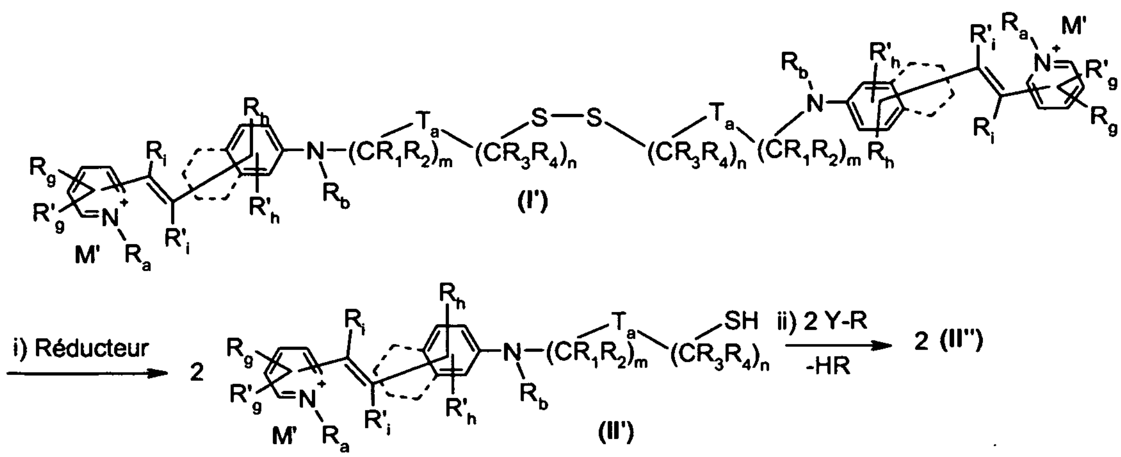


avec $R_1$, $R_2$, $R_3$, $R_4$, $R_a$, $R_b$, $R_g$, $R'_g$, $R_h$, $R'_h$, $R_i$, $R'_i$, Y, m, n et M' sont tels que définis précédemment et R représente un groupe partant nucléofuge, comme par exemple mésylate, tosylate, triflate ou halogénure.

Selon une autre possibilité, on peut faire réagir un composé thiol protégé (b) par un groupement protecteur Y tel que défini précédemment préparé selon une des procédures décrites dans les ouvrages cités précédemment, ledit composé thiol protégé comprenant au moins une fonction nucléophile avec une quantité suffisante, préférentiellement équimolaire, d'un "chromophore fluorescent réactif'' ou d'un composé comprenant un tel "chromophore fluorescent réactif'' (a). En d'autres termes (a) comprend une fonction nucléofuge pour former une liaison covalente Σ comme cela peut être schématisé ci-dessous:

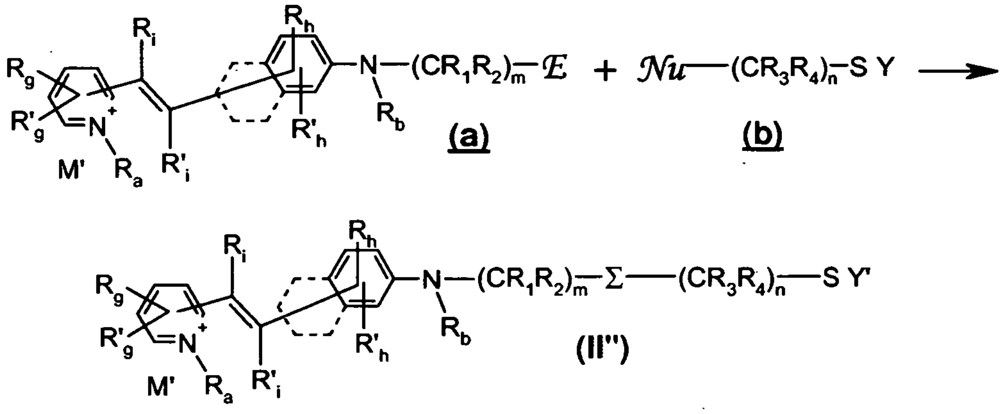

(a)

(b)

(II'')

avec $R_1$, $R_2$, $R_3$, $R_4$, $R_a$, $R_b$, $R_g$, $R'_g$, $R_i$, $R'_h$, $R_i$, $R'_i$, Y, m, n et M' sont tels que définis précédemment ; *Nu* représentant un groupement nucléophile aminé ou hétérocycloalkyle, hétéroaryle; *E* représentant un groupement nucléofuge comme par exemple mésylate, tosylate, triflate ou halogénure comme le brome, l'iode ou le chlore. Σ représente un radical choisis parmi -N(R)-, $-N^+(R)(R^°)$- avec R et R° tels que définis précédemment, hétérocycloalkyle et hétéroaryle, cationique ou non.

On pourra également utiliser un réactif thiol Y-SH comprenant un groupement Y dont la fonction nucléophile SH peut réagir sur l'atome de carbone en alpha de l'atome d'halogène porté par le chromophore fluorescent **(a')** pour conduire au colorant fluorescent thiol protégé de formule (**II**):

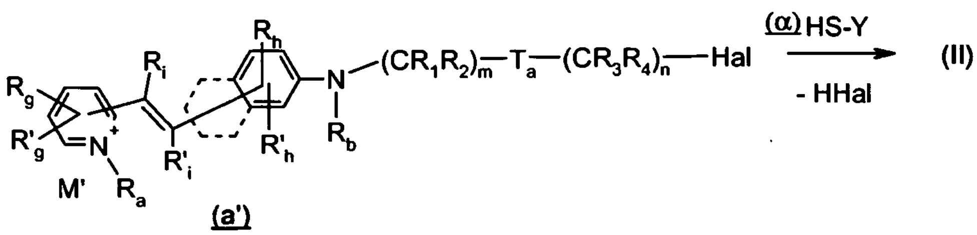


(II)

(a')

avec $R_1$, $R_2$, $R_3$, $R_4$, $R_a$, $R_b$, $R_g$, $R'_g$, $R_h$, $R'_h$, $R_i$, $R'_i$, $T_a$, Y, m, n et M' sont tels que définis précédemment, et Hal représentant un atome d'halogène nucléofuge tel que le brome, l'iode ou le chlore.

On pourra également utiliser un thioacide (α') (par exemple l'acide de thioacétique) qui agira sur halogénure porté par le chromophore fluorescent (a') :

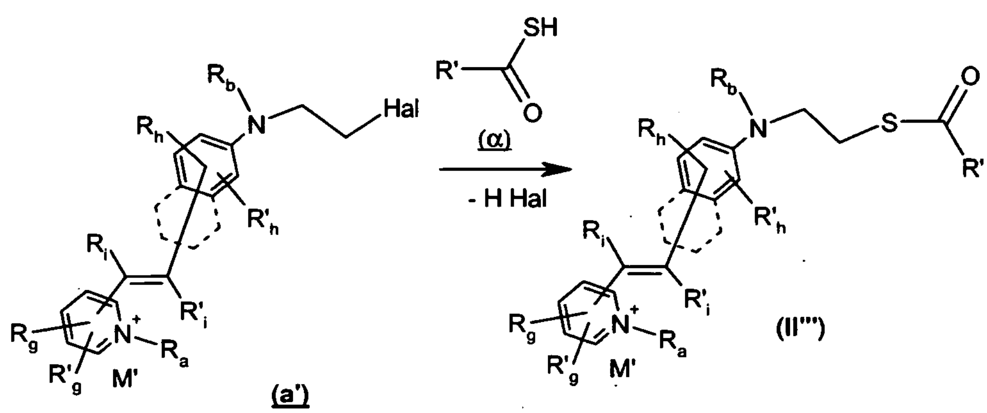


(a')

(II''')

avec $R_a$, $R_b$, $R_g$, $R'_g$, $R_h$, $R'_h$, $R_i$, $R'_i$, m, n, Hal, M' sont tels que définis précédemment et R' représentant un groupement $(C_1-C_6)$alkyle.

Plus particulièrement on pourra substituer un groupement partant nucléofuge par un groupement thiourée (S=C(NRR)NRR) pour générer les isothiouroniums. Par exemple si le groupement thiourée est une thioimidazolinium

(β), le schéma réactionnel est le suivant :

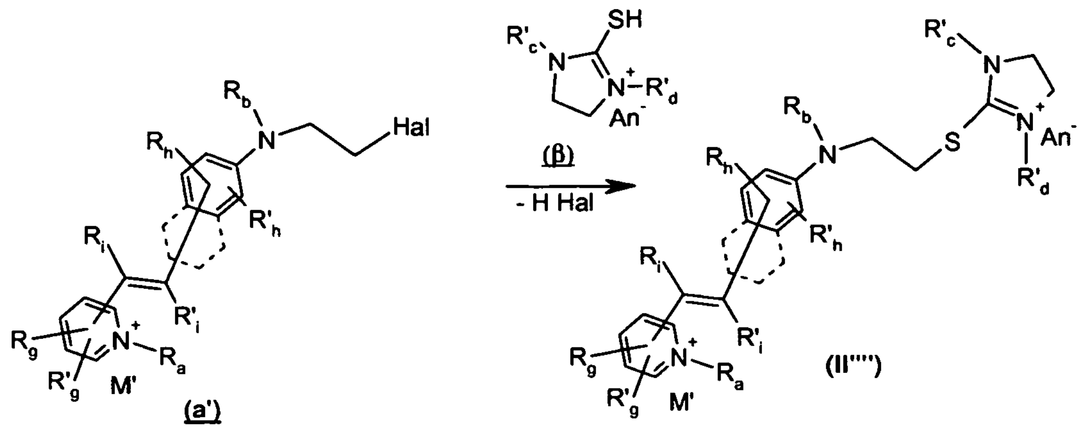


avec $R_a$, $R_b$, $R'_c$, $R'_d$, $R_g$, $R'_g$, $R_h$, $R'_h$, $R_i$, $R'_i$, m, n, Hal, $An^-$ et M' sont tels que définis précédemment.

Une variante est d'utiliser à la place de l'halogénure comprenant le chromophore fluorescent (a') un chromophore comprenant un autre type de nucléofuge tel que le tosylate, mésylate.

Selon une autre variante il est possible de générer un intermédiaire imidazoline à partir de l'halogénure comprenant le chromophore fluorescent (a') et une thioimidazoline (b') pour conduire après alkylation par un R-Gp avec R représentant un groupement alkyle et Gp un groupe partant tel qu'un halogène comme le chlore, brome, iode, ou un groupement mésylate ou tosylate.

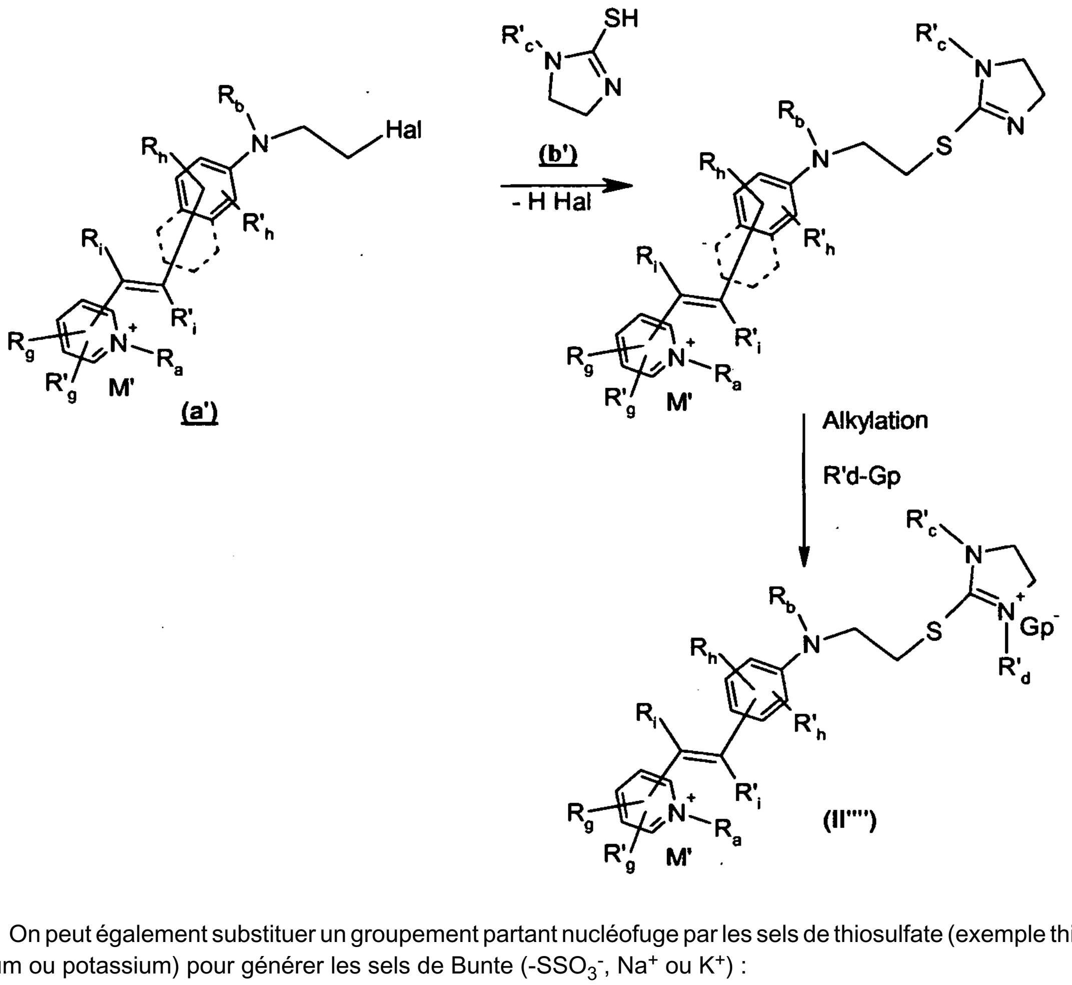


On peut également substituer un groupement partant nucléofuge par les sels de thiosulfate (exemple thiosulfate de sodium ou potassium) pour générer les sels de Bunte ($-SSO_3^-$, $Na^+$ ou $K^+$) :

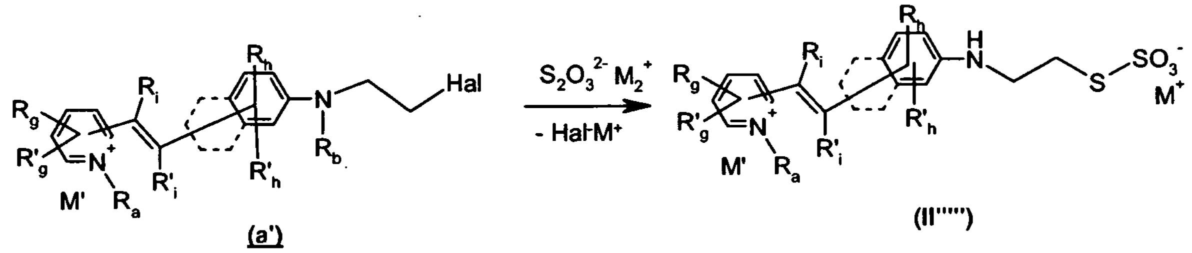


(a') (II''''')

avec $R_a$, $R_b$, $R_g$, $R'_g$, $R_h$, $R'_h$, $R_i$, $R'_i$, m, n, Hal, M' sont tels que définis précédemment et $M^+$ représentant un métal alcalin tel que le sodium ou potassium.

Conformément à une autre possibilité, certains colorants fluorescents thiols protégés **(II")** peuvent être obtenus en faisant réagir un composé thiol protégé, avec un composé portant une fonction nucléofuge <u>(**d**)</u> mis à réagir avec un chromophore fluorescent porteur d'une fonction nucléophile <u>(**c**)</u>, de type amine primaire ou secondaire, hétérocycloalkyle, ou hétéroaryle :

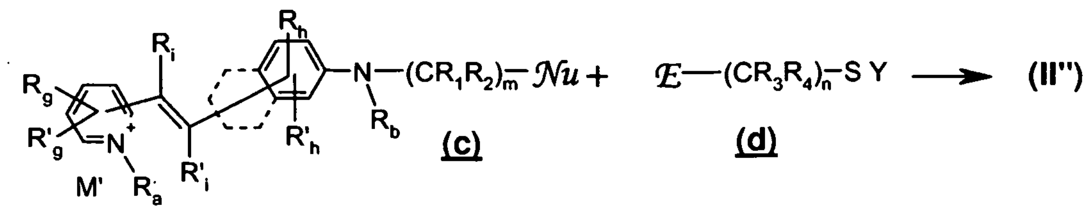


(c) (d)

avec $R_1$, $R_2$, $R_3$, $R_4$, $R_a$, $R_b$, Rg, $R'_g$, $R_h$, $R'_h$, $R_i$, $R'_i$, $T_a$, Y, m, n, M', *E, Nu* et **(II")** tels que définis précédemment.

Conformément à une autre possibilité, les colorants fluorescents thiols protégés de formule **(II)** peuvent être obtenus par réaction d'un composé comprenant un groupement thiol protégé par un groupement Y, et un groupement hydroxy activé préalablement en groupement partant nucléofuge <u>**(d')**</u>, comme par exemple mésylate, tosylate, triflate ou halogénure avec un chromophore fluorescent <u>**(c')**</u> portant une fonction nucléophile amine primaire lorsque $R_b$ représente un atome d'hydrogène, ou amine secondaire.

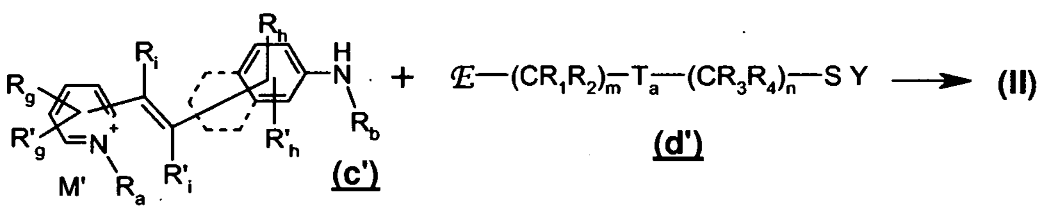


(c') (d')

Avec $R_1$, $R_2$, $R_3$, $R_4$, $R_a$, $R_b$, $R_g$, $R'_g$, $R_h$, $R'_h$, $R_i$, $R'_i$, $T_a$, Y, m, n, M' et *E* sont tels que définis précédemment.

A titre d'exemple un composé contenant un groupement thiol protégé, contient un groupement partant nucléofuge R, comme par exemple mésylate, tosylate, ou triflate qui peut subir l'attaque nucléophile de l'amine portée par le chromophore fluorescent styrylique :

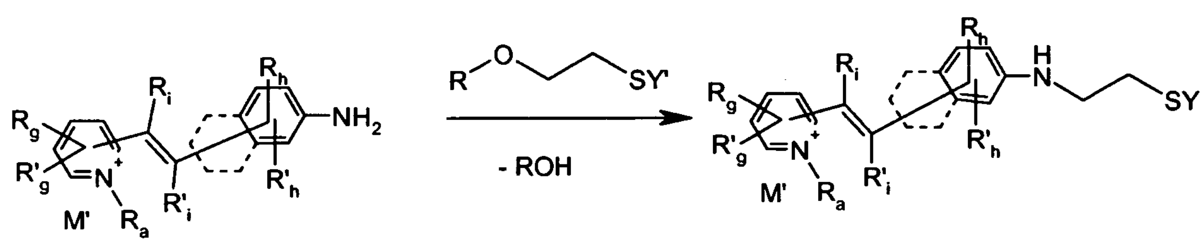


Une autre alternative provient de l'utilisation d'halogénures comme groupement partant nucléofuge sur un

composé thiol pouvant être substitué par un fonction amine primaire par exemple portée par un chromophore fluorescent styrylique :

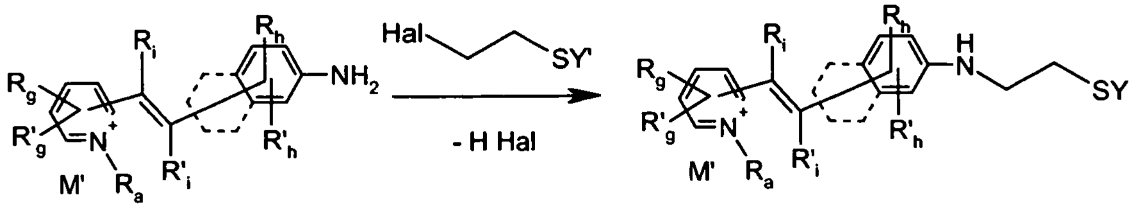


Conformément à une autre possibilité, les colorants fluorescents thiols de formule (II) selon l'invention peuvent être obtenus par réaction d'un composé comprenant un groupement thiol protégé et un groupement électrophile (f) avec un composé comprenant un groupement nucléophile. A titre d'exemple, on pourra condenser un aldéhyde ou cétone lorsque G représente un atome d'oxygène avec un « méthylène activé » tels que l'alkylpyridinium (e) pour générer un liaison éthylène >C=C<. Cette réaction est communément appelée condensation de « Knoevenagel ». Par « méthylènes activés » on sous entend ceux par exemple mentionnés dans le brevet DE19951134 on peut citer particulièrement le 1,2-dialkylpyridinium et notamment le 1,2-diméthylpyridinium :

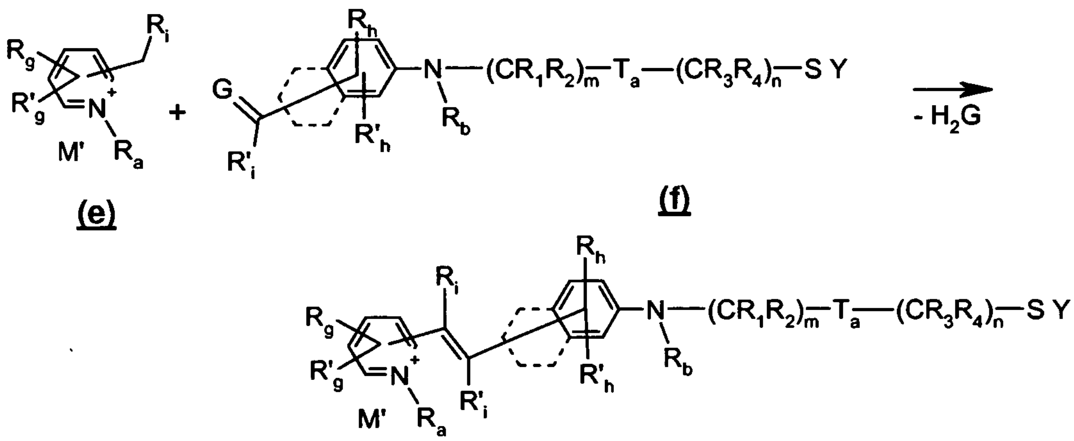


**(e)** **(f)**

avec $R_1$, $R_2$, $R_3$, $R_4$, $R_a$, $R_b$, $R_g$, $R'_g$, $R_h$, $R'_h$, $R'_i$, $T_a$, Y, m, n, M' tels que définis précédemment et G représentant un atome d'oxygène, de soufre ou un groupement N(R) avec R représentant un atome d'hydrogène ou un groupement $(C_1\text{-}C_4)$alkyle.

Une autre variante à ce schéma réactionnel est de réaliser la réaction de couplage de « Knoevenagel » ultérieurement ie à partir de l'intermédiaire **(f')**, le groupement amino NR'R" peut réagir avec un équivalent de réactif Gp-$(CR_3R_4)$-Gp pour conduire à **(i')** qui lui-même peut réagir avec un dérivé thiol protégé Y'SH pour donner l'intermédiaire aldéhyde **(j')**. Cet intermédiaire peut subir la réaction de condensation de « Knoevenagel » avec l'alkylpyridinium **(e)** pour conduire au colorant fluorescent naphtyle thiol protégé **(II")**.

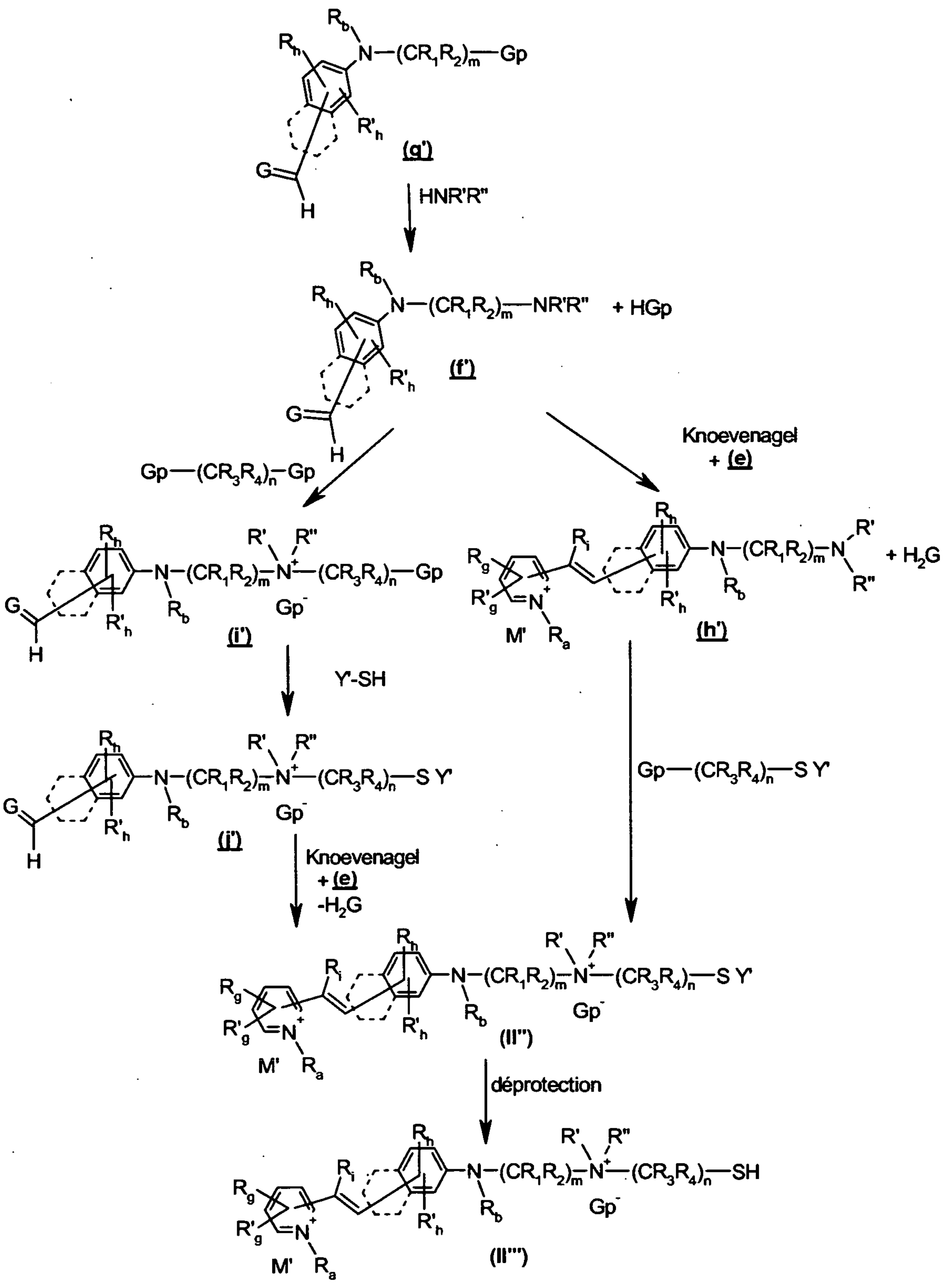


avec R', R", $R_1$, $R_2$, $R_3$, $R_4$, $R_a$, $R_b$, $R_g$, $R'_g$, $R_h$, $R'_h$, $R_i$, m, n, Y', et M' tels que définis, Gp représente un groupe partant tel qu'un atome d'halogène notamment Br, I, ou un groupement tosylate, mésylate, triflate, et $Gp^-$ représente le contre-ion anionique consécutif au départ du groupe partant Gp.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupements protecteurs classiques. Les colorants fluorescents thiols peuvent également êtres métallés en utilisant les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par voies classiques connues par l'homme du métier. A titre d'exemple pour synthétiser le colorant fluorescent à deux chromophores phényle/naphtyle, portant une fonction disulfure -S-S- (I'), on peut partir d'un réactif naphtyle difonctionnalisé (k), comprenant en position 2 un groupement nucléofuge G'-R avec R représentant un groupement ($C_1$-$C_4$)alkyle, mésylate, tosylate, ou triflate et G' représentant un atome d'oxygène, de soufre ou un groupement N(R) avec R représentant un atome d'hydrogène ou un groupement ($C_1$-$C_4$)alkyle et comprenant en position 6 un groupement électrophile aldéhyde ou thioaldéyde. Deux équivalents de ce réactif peuvent réagir avec la diamine-dialkyl-disulfure (l) pour conduire après condensation au dialdéhyde/thioaldéhyde disulfure (m) pouvant se condenser avec deux équivalents d'alkylpyridinium (e) pour former le composé (I').

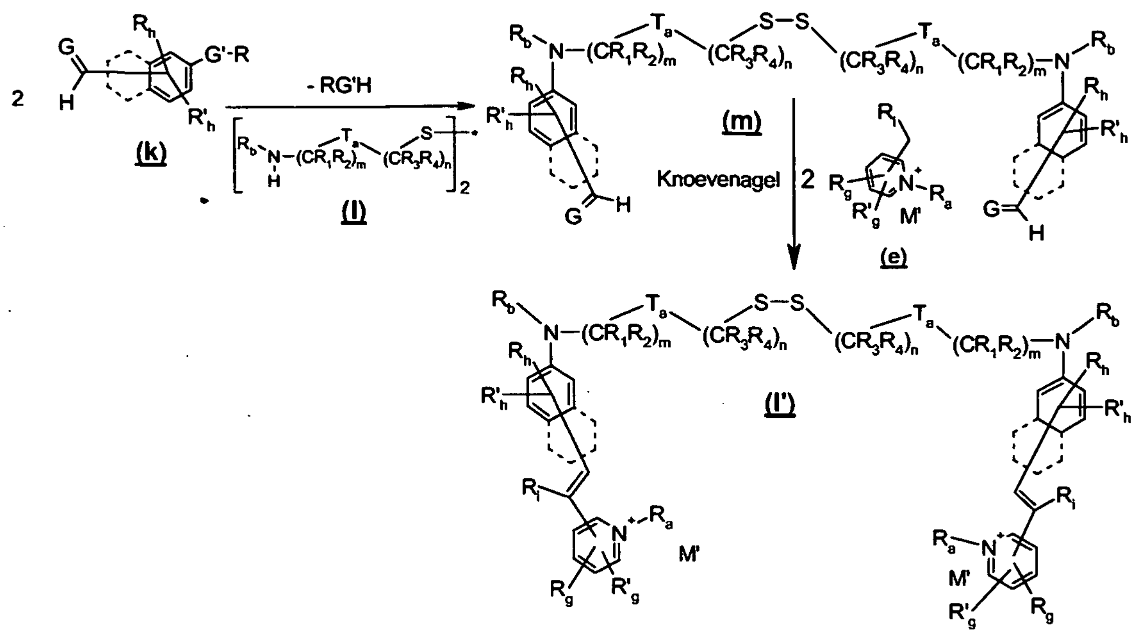


On pourra se référer à l'ouvrage Advanced Organic Chemistry, J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants disulfures disymetriques de formule **(I)** peuvent être synthétisés en une étape en faisant réagir un colorant fluorescent thiol non protégé avec un colorant fluorescent thiol protégé pour former le colorant disulfure de formule **(I)** disymétrique.

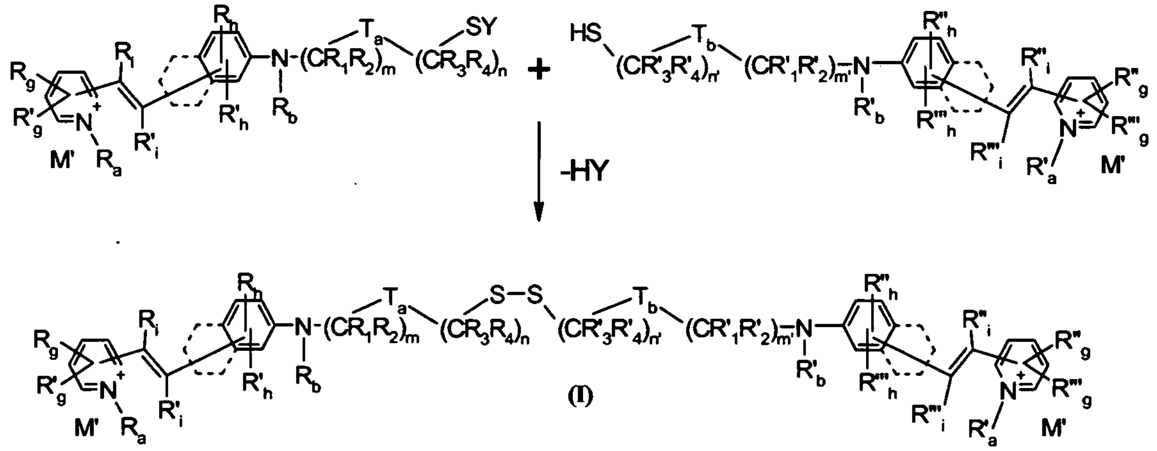


(I)

avec $R_a$, $R'_a$, $R_b$, $R'_b$, $R_g$, $R'_g$, $R''_g$, $R'''_g$, $R_h$, $R'_h$, $R''_h$, $R''_h$, $R_i$, $R'_i$, $R''_i$, $R'''_i$, $R_1$, $R'_1$, $R_2$, $R'_2$, $R_3$, $R'_3$, $R_4$, $R'_4$, m, m', n, n', $T_a$, $T_b$, et M' sont tels que définis précédemment ; Y' représente un groupement protecteur de fonction thiol.

La composition de l'invention contient au moins un colorant fluorescent de formule (I) ou (II). Outre la présence d'au moins un colorant fluorescent de formule (I) ou (II), la composition de l'invention peut également contenir un agent réducteur. Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut également être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catéchol-borane.

La composition tinctoriale utile dans l'invention contient en général une quantité de colorants fluorescents de formule (I) ou (II) comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

Parmi ces coupleurs, on peut notamment citer les méta-phénylènediamines, les méta-aminophénols, les métadiphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on

peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (y) suivante :

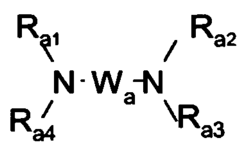

(γ)

dans laquelle $W_a$ est un reste propylène éventuellement substitué par un groupement hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Le procédé de coloration de l'invention consiste à appliquer la composition de l'invention sur les matières kératiniques, en particulier les fibres kératiniques, notamment humaines telles que les cheveux, plus particulièrement les cheveux foncés, la composition tinctoriale de l'invention comprenant au moins un colorant fluorescent de formule (I) ou (II).

Selon un mode de réalisation particulier dans le procédé de l'invention un agent réducteur peut être appliqué en pré-traitement avant l'application de la composition contenant au moins un colorant fluorescent de formule (I) ou (II).

Cet agent réducteur peut être choisi parmi les thiols par exemple, la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Ce prétraitement peut être de courte durée, notamment de 0,1 seconde à 30 minutes, de préférence de 1 minute à 15 minutes avec un agent réducteur tel que cité précédemment.

Selon un autre procédé, la composition comprenant au moins un colorant fluorescent de formule (I) ou (II)

contient également au moins un agent réducteur tel que défini précédemment. Cette composition est alors appliquée aux cheveux.

Lorsque le colorant fluorescent est de formule (II) comprend un groupement Y protecteur de la fonction thiol, le procédé de l'invention peut être précédé d'une étape de déprotection visant à restituer *in-situ* la fonction SH.

A titre d'exemple il est possible de déprotéger la fonction S-Y avec Y groupement protecteur en ajustant le pH comme suit :

| Y : groupe protecteur | déprotection |
|---|---|
| | |
| alkylcarbonyle, | pH>9 |
| arylcarbonyle, | pH>9 |
| alcoxycarbonyle, | pH>9 |
| aryloxycarbonyle, | pH>9 |
| arylalcoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| Hétéroaryle bicyclique à 8 à 11 chaînons tels que benzoimidazolium, ou benzoxazolium | pH>9 |

L'étape de déprotection peut également être réalisée au cour d'une étape de prétraitement des cheveux comme par exemple, le prétraitement réducteur des cheveux.

Selon un autre procédé de coloration, la composition comprenant au moins un colorant fluorescent de formule (I) ou (II) contient également au moins un agent réducteur tel que défini précédemment. Cette composition est alors appliquée aux cheveux.

Selon une variante, l'agent réducteur est ajouté à la composition tinctoriale contenant au moins un colorant fluorescent de formule (I) ou (II) au moment de l'emploi.

Dans le procédé de coloration une autre variante est d'appliquer le colorant fluorescent de formule (I) ou (II) en même temps que le réducteur.

Selon une autre variante, l'agent réducteur est appliqué en post-traitement, après l'application de la composition contenant au moins un colorant fluorescent de formule (I) ou (II). La durée du post traitement avec l'agent réducteur peut être courte, par exemple de 0,1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent de formule (I) ou (II) peut être appliqué directement aux cheveux sans réducteurs, exempt de pré ou post-traitement réducteurs.

Un traitement avec un agent oxydant peut éventuellement être associé. On pourra utiliser n'importe quel type d'agent oxydant classiques dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant au moins un colorant fluorescent de formule (I) ou (II).

L'application de la composition tinctoriale selon l'invention est généralement effectuée à température ambiante. Elle peut cependant être réalisée à des températures variant de 20 à 180°C.

L'invention a aussi pour objet un dispositif à plusieurs compartiments ou "kit" de teinture dans lequel un premier compartiment renferme une composition tinctoriale comprenant au moins un colorant fluorescent de formule (I) ou (II) et un deuxième compartiment renferme un agent réducteur capable de réduire les fonctions disulfures des matières kératiniques et/ou de la fonction disulfure du colorant de formule (I).

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent de formule (I) ou (II) ; un deuxième compartiment contient un agent réducteur capable de réduire la liaison disulfure des matières kératiniques et/ou de la fonction disulfure du colorant de formule (I) ; un troisième compartiment contient un agent oxydant.

Une autre variante concerne un dispositif à plusieurs compartiment ou « kit » de teinture comprenant un premier compartiment qui contient une composition avec au moins un colorant fluorescent protégé de formule (II) et un deuxième compartiment comprenant une composition contenant un agent de déprotection telle qu'une base.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents thiols des exemples ci-après ont été entièrement caractérisés par les méthodes spectroscopiques et spectrométriques classiques.

**EXEMPLES**

**EXEMPLES DE SYNTHESE**

**Exemple 1 : Synthèse du chlorure de 1-méthyl-4-{(E)-2-[4-(méthyl{2-[(1-méthyl-1H-imidazol-2-yl)sulfanyl]éthyl} amino)phényl]vinyl}pyridinium [1]**

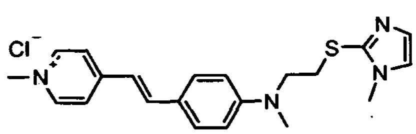

**[1]**

**Schéma de synthèse :**

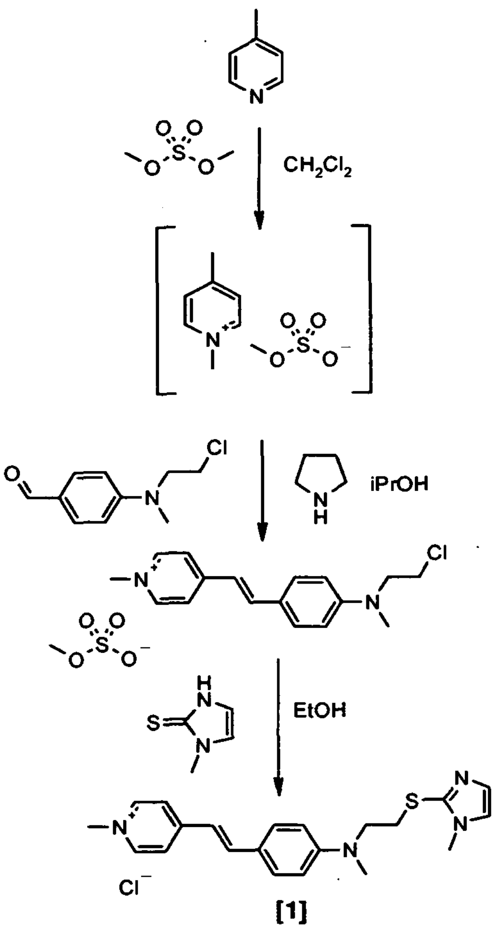


[1]

**Mode opératoire :**

**Etapes 1 et 2: Synthèse du méthyl sulfate de 4-((E)-2-{4-[(2-chloroéthyl)-(méthyl)amino]phényl}vinyl)-1-méthyl-pyridinium**

4,99 g de 4-picoline dilués dans 25 mL de dichlorométhane ($CH_2Cl_2$) sont agités et chauffés à 40˚C. 5 mL de diméthylsulfate dilués dans 20 mL de $CH_2Cl_2$ sont ajoutés goutte à goutte au milieu réactionnel. Le mélange est maintenu à 42,˚C pendant 30 min après l'addition, puis 20 mL d'isopropanol sont ajoutés.

1,87 g de pyrrolidine dilués dans 30 mL d'isopropanol sont additionnés. Le mélange est agité et chauffé à 60 ˚C pendant 5 min. 10,5 g de 4-[(2-chloroéthyl)(méthyl)-amino]benzaldéhyde sont ajoutés. Le mélange est maintenu agité 2h à 52,˚C puis à température ambiante pendant 48,h. Le mélange réactionnel est dilué dans 50 mL d'éthanol puis conservé à -25,˚C pendant 12,h. Le précipité obtenu est filtré à température ambiante, lavé par 100 mL d'acétone, par 3 fois 100 mL d'éther éthylique et séché sous vide en présence de $P_2O_5$.

15,1 g de cristaux orangés sont recueillis. Les analyses indiquent que le produit est conforme.

Etape 3 : Synthèse du chlorure de 1-méthyl-4-{(E)-2-[4-(méthyl{2-[(1-méthyl-1H-imidazol-2 yl)sulfanyl]éthyl}amino)phényl]vinyl}pyridinium [1]

5 g de méthyl sulfate de 4-((E)-2-{4-[(2-ch)oroéthyl)(méthyl)amino]phényl}vinyl) méthylpyridinium, 2,15 g de 2-mercapto-1-méthylimidazole et 20 mL d'éthanol (EtOH) sont agités et chauffés à 70 ˚C pendant 48 h. 2.15 g de 2-mercapto-1-méthylimidazole dilués dans 3 mL d'EtOH sont additionnés au mélange réactionnel, le mélange est maintenu agité à 72˚C pendant 72 h. 20 mL d'isopropanol et 2 mL d'eau sont ajoutés au mélange réactionnel refroidi à -10˚C. L'huile est séparée du surnageant, lavée avec de l'acétate d'éthyle (AcOEt), puis solubilisée dans 100 mL d'éthanol et conservée à - 25˚C pendant 48 h.

Les cristaux obtenus sont filtrés, lavés par 20 mL d'éthanol, par 3 fois 100 mL d'AcOEt, puis séchés sous vide en présence de $P_2O_5$. 5,79 g de poudre brune sont recueillis. Les analyses indiquent que le produit est conforme. RMN$^1$

H (400 MHz, ppm, DMSO-$d_6$) 2.96 (s, 3 H), 3.51 (t, 2 H), 3.67 (s, 3 H), 3.68 (t, 2 H), 4.20 (s, 3H), 6.75 (d, 2 H), 7.20 (d, 1 H), 7.57 (d 2 H), 7.72 (, 1 H), 7.76 (d, 1 H), 7.91 (d, 1 H), 8.07 (d, 2 H), 8.73 (d, 2 H).

**Exemple 2 : Synthèse du méthyl sulfate / chlorure de 4-((E)-2-{4-[{2-[(1,3-diméthyl-1H-imidazol-3-ium-2-yl)sulfanyl]éthyl}(méthyl)amino]phényl}vinyl)-1-méthylpyridinium [2]**

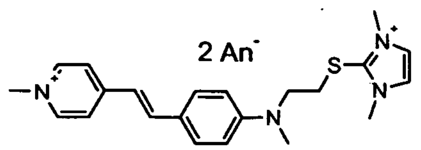

**[2]**

**Schéma de synthèse**

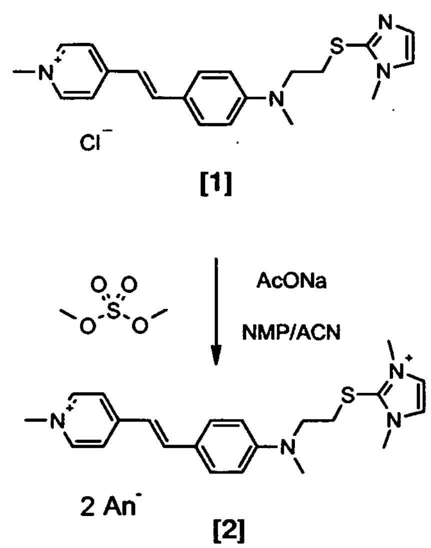


**[1]**

**[2]**

**Mode opératoire**

Une solution de 2 g de chlorure de 1-méthyl-4-{(E)-2-[4-(méthyl{2-[(1-méthyl-1H-imidazol-2yl)sulfanyl]éthyl} amino)phényl]vinyl}pyridinium [1] dans 2 mL de N-méthylpyrrolidinone (NMP) et 8 mL d'acétonitrile (ACN) sont agités à 70°C pendant 20 min. 500 µL de diméthylsulfate sont ajoutés au mélange. L'agitation et le chauffage sont maintenus pendant 1 h30. 500 mg d'acétate de sodium sont ajoutés. Après 4 h, 1 mL de diméthylsulfate sont ajoutés et l'agitation est maintenue pendant 15 h à température ambiante. 50 mL d'acétonitrile sont additionnés. Le précipité obtenu est filtré. 100 mL d'acétone sont introduits dans le filtrat, l'huile ainsi obtenue est lavée par 3 fois 100 mL d'acétone puis resolubilisée dans 50 mL d'isopropanol et conservée à -25°C pendant 48 h. Le précipité formé est filtré, lavé par 10 mL d'iPrOH, par 3 fois 20 mL d'acétate d'éthyle puis séché sous vide en présence de $P_2O_5$. 1.11 g de poudre brune sont recueillis. Les analyses indiquent que le produit est conforme, sous forme de sels mixtes de chlorure et de méthylsulfate.

RMN $^1$H (400 MHz, ppm, MeOH-d6) 3.06 (s, 3 H), 3.35 (t, 2 H), 6.68 (s, 5.44 H, méthylsulfate), 3.77 (t, 2 H), 3.79 (t, 6 H), 4.24 (s, 3 H), 6.82 (d, 2 H), 7.15 (d, 1 H), 7.64 (d, 2 H), 7.75 (s, 2 H), 7.83 (d, 1 H), 8.00 (d, 2 H), 8.55 (d, 2 H).

**Exemple 3: Synthèse du dichlorure de 2,2'-{disulfanediylbis[éthane-2,1-diyl(méthylimino)-4,1-phénylène(E) éthène-2,1-diyl]}bis(1-méthylpyridinium) [3]**

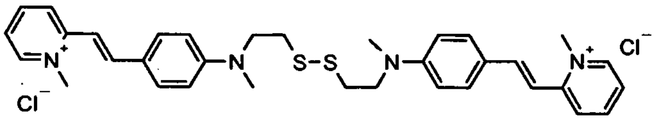

**[3]**

**Schéma de synthèse :**

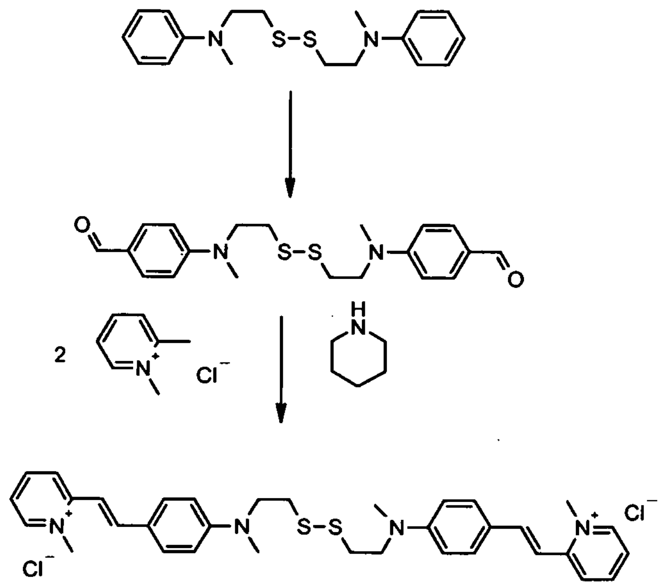


**[3]**

**Mode opératoire :**

**Etape 1 : 4,4'-{disulfanediylbis[éthane-2,1-diyl(méthylimino)]}dibenzaldéhyde**

82,3 g d'oxychlorure de phosphore sont ajoutés à 500 mL de DMF à 0 °C. Après 30 min d'agitation à 0 °C une solution de 47 g de *N,N'*-(disulfanediyldiéthane-2,1-diyl)bis(N-méthylaniline) sont ajoutés goutte à goutte. Le mélange est agité 90 min. à 0 °C puis 75 min. à 10°C et 105 min. à 40 °C. Il est ensuite versé sur 2,5 L d'eau glacée, 700 mL de soude 5N sont ajoutés. Le précipité jaune obtenu est filtré sur célite, solubilisé dans 200 mL de dichlorométhane et la solution obtenue est lavée par 200 mL de solution aqueuse saturée en chlorure de sodium. Après séchage sur sulfate de magnésium et évaporation du dichlorométhane, le résidu jaune (80 g) est purifié par chromatographie sur gel de silice. Après séchage, une poudre jaune clair est recueillie.

Les analyses indiquent que le produit est conforme à la structure attendue.

**Etape 2: dichlorure de 2,2'-{disulfanediylbis[éthane-2,1-diyl(méthylimino)-4,1-phénylène(E)éthène-2,1-diyl]} bis(1-méthylpyridinium) [3]**

25 g de 4,4'-(disulfanediylbis[éthane-2,1-diyl(méthylimino)]}dibenzaldehyde et 18,5 g de chlorure de N-méthylpicolinium sont dissous dans 300 mL de méthanol. 12,7 mL de pipéridine sont ajoutés au mélange. Le tout est chauffé, sous agitation à 55°C pendant 11 h. Le méthanol est éliminé sous vide à 40°C. Le solide est mélangé à 300 mL d'isopropanol. Après un nouveau séchage par évaporation on introduit 200 mL d'isopropanol. Le mélange prend en masse, il est étendu par addition de 100 mL d'iospropanol et essoré sur verre fritté. Le solide recueilli est lavé par de l'isopropanol puis de l'acétone, puis séché sous vide. Après séchage, 36,7g de poudre orange sont recueillis. Par recristallisation dans l'isopropanol, 27 g de poudre rouge orange de pureté supérieure sont recueillis. Les analyses

indiquent que le produit est conforme au composé [3] et pur.

RMN $^1H$ (400 MHz, MeOH-$d_4$) 2.99 (t, 4H), 3.81 (t, 4H), 4.31 (s, 6H), 6.86 (d, 4H), 7.22 (d, 2H), 7.63 (m, 2H), 7.69 (d, 4H), 7.83 (d, 2H), 8.29 (m, 2H), 8.36 (m, 2H), 8.61 (m, 2H).

**Exemple 4 : Synthèse du diméthoxysulfate de 4,4'-{disulfanediylbis[éthane-2,1-diyl(méthylimino)-4,1-phénylène(E)éthène-2,1-diyl]}bis(1-méthylpyridinium) [4]**

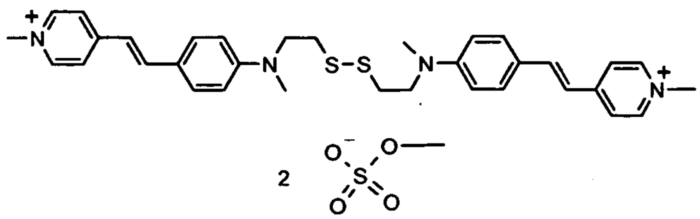

**[4]**

**Schéma de synthèse :**

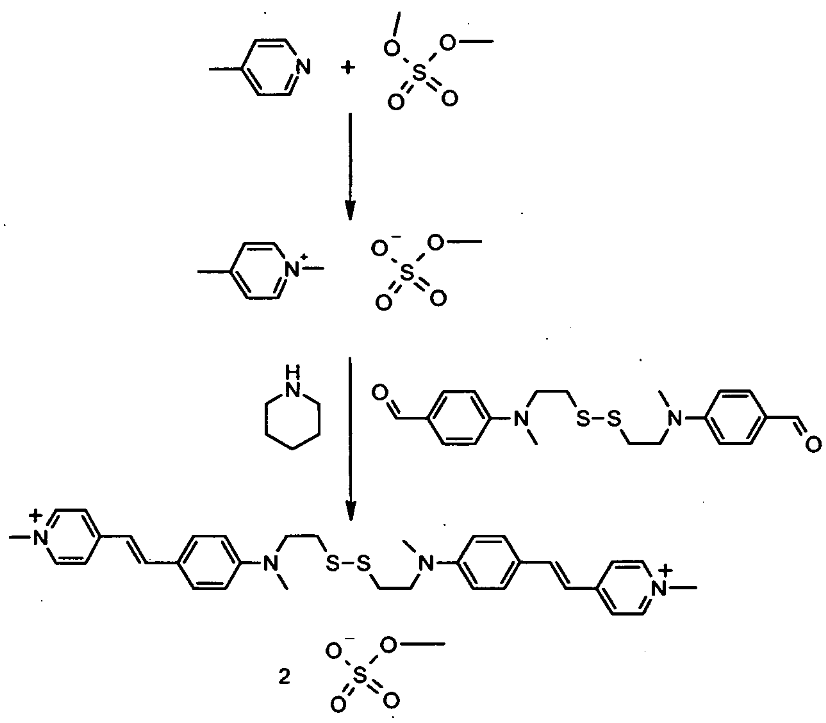

**[4]**

**Mode opératoire :**

**Diméthoxysulfate de 4,4'-{disulfanediylbis[éthane-2,1-diyl(méthylimino)-4,1-phénylène(E)éthène-2,1-diyl]}bis (1-méthylpyridinium) [4]**

2,62 g de 4-picoline sont dilués dans 25 mL de dichlorométhane, 3 mL de sulfate de diméthyle sont ajoutés à la solution, dont la température s'élève au reflux (40 °C). Après 40 min. d'agitation, 50 mL d'isopropanol sont ajoutés,

et le mélange est concentré par distillation du dichlorométhane (mélange chauffé à 60 °C). 1,83 g de pyrrolidine sont introduits dans le mélange suivis de 4,99 g de 4,4'-{disulfanediylbis[éthane-2,1-diyl(méthylimino)]}dibenzaldéhyde. Après 2 h d'agitation à 65 °C, le mélange réactionnel est refroidi à température ambiante, le précipité formé est filtré, lavé par 3 fois 100 mL d'isopropanol. La pâte rouge obtenue est dispersée dans 200 mL d'isopropanol, le mélange ainsi obtenu est porté à reflux puis refroidi. Le précipité rouge formé est filtré puis séché. 8,94 g de poudre rouge sont recueillis. Les analyses indiquent que le produit est conforme à la structure de [4] et pur. RMN $^1$ H (400 MHz, DMSO-$d_6$) 2.96 (t, 4 H), 3.02 (s, 6 H), 3.36 (s, 6 H), 3.72 (t, 4 H), 4.16 (s, 6 H), 6.81 (d, 4 H), 7.15 (d, 2 H), 7.57 (d, 4 H), 7.87 (d, 2 H), 8.02 (d, 4 H), 8.66 (d, 4 H).

**<u>Exemple 5</u> : Synthèse du dichlorure de 2,2'-{disulfanediylbis[éthane-2,1-diyl(méthylimino)-4,1-phénylène-(E) éthène-2,1-diyl]}bis(1-éthylpyridinium) [5]**

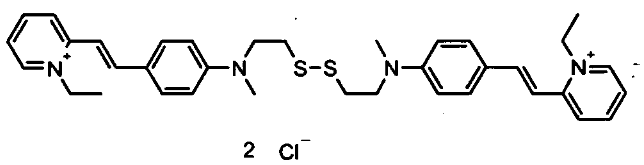

**[5]**

**Schéma de synthèse :**

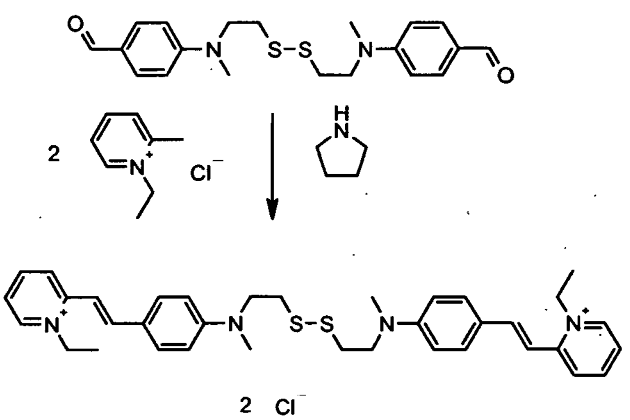

**[5]**

**Mode opératoire :**

**Synthèse du dichlorure de 2,2'-{disulfanediylbis[éthane-2,1-diyl(méthylimino)-4,1-phénylène-(E)éthène-2,1-diyl]}bis(1-éthylpyridinium) [5]**

10 g de 4,4-{disulfanediylbis[éthane-2,1-diyl(méthylimino)]}dibenzaldéhyde et 8,1 g de chlorure de N-éthylpicolinium sont dissous dans 100 mL d'isopropanol. 1,3 g de pipéridine sont ajoutés au mélange. Le tout est chauffé, sous agitation à reflux pendant 5 h. L'isopropanol est éliminé sous vide à 50°C. La gomme obtenue est triturée avec de l'acétone. 18 g de solide sont recueillis et traités au noir de charbon. 7,1 g de produit sont collectés et 4 g sont purifiés par chromatographie liquide liquide (eau/BuOH). Après séchage 1,65 g de poudre rouge sont recueillis. Les analyses indiquent que le produit est conforme à la structure du colorant [5] et pur. RMN $^1$H (400 MHz, MeOH-$d_4$) 1.57 (t, 6 H), 2.98 (t, 4 H), 3.11 (s, 6 H), 3.8 (t, 4 H), 4.73 (q, 4 H), 6.85 (m, 4 H), 7.23 (d, 2 H), 7.69 (m, 6 H), 7.85 (d, 2 H), 8.29 (m, 2 H), 8.38 (m, 2 H), 8.67 (m, 2 H).

Exemple 6 : **Synthèse du di bromure de 4,4'-{disulfanediylbis[éthane-2,1-diyl(méthylimino)-4,1-phénylène(E)ethene-2,1-diyl]}bis(1-éthylpyridinium) [6]**

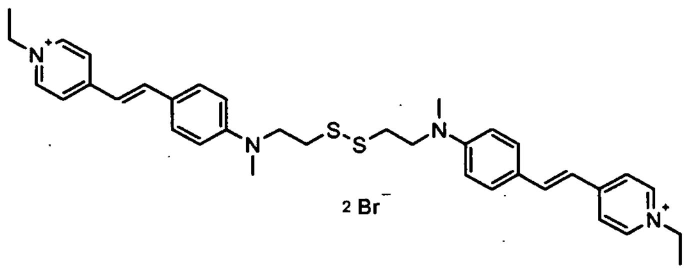

**[6]**

**Schéma de synthèse**

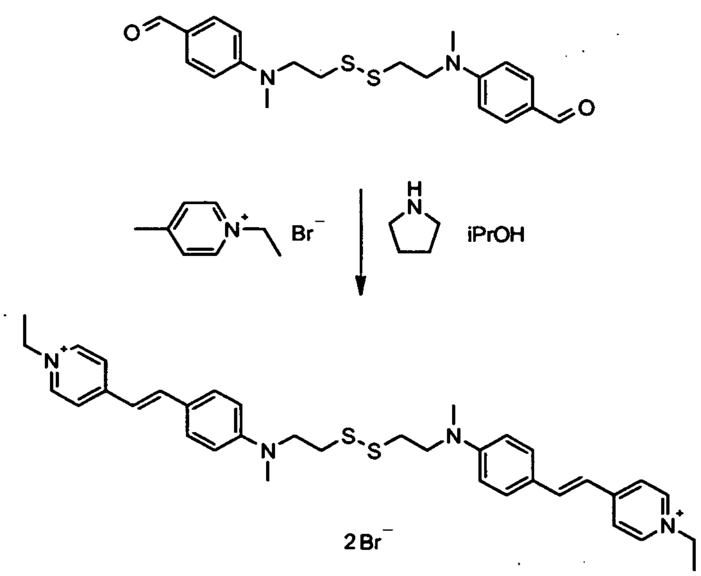


**[6]**

**Mode opératoire**

**Synthèse du dibromure de 4,4'-{disulfanediylbis[éthane-2,1-diyl(méthylimino)-4,1-phénylène(E)ethene-2,1-diyl]}bis(1-éthylpyridinium) [6]**

4,1 g de 4,4'-(disulfanediylbis[éthane-2,1-diyl(méthylimino)]}dibenzaldéhyde, 40 mL d'isopropanol, 0,86 mL de pyrrolidine sont chauffés à 75°C pendant 20 minutes. 4.26 g de bromure de 1-éthyl-4-méthylpyridinium sont ajoutés au milieu réactionnel, agité et chauffé pendant 5h. 200 mL d'acétone sont introduits dans le mélange réactionnel. Le précipité obtenu est filtré, lavé à l'acétone et séché. 7,37 g de poudre rouge sont recueillis. Les analyses indiquent que le produit est conforme et pur. RMN $^1$ H (400 MHz, DMSO-$d_6$) 1.5 (t, 6 H), 2.98 (t, 4 H), 3.04 (s, 6 H), 3.74 (t, 4 H), 4.46 (q, 4 H), 6.84 (d, 4 H), 7.19 (d, 2 H), 7.61 (d, 4 H), 7.93 (d, 2 H), 8.08 (d, 4 H), 8.82 (d, 4 H),

**Exemple 7 : Synthèse du di bromure de 4,4'-{disulfanediylbis[éthane-2,1-diyl(méthylimino)-4,1-phénylène(E) éthène-2,1-diyl]}bis[1-(2-hydroxyéthyl)pyridinium] [7]**

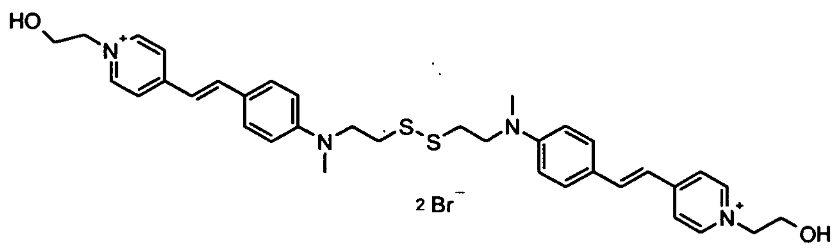

**[7]**

**Schéma de synthèse**

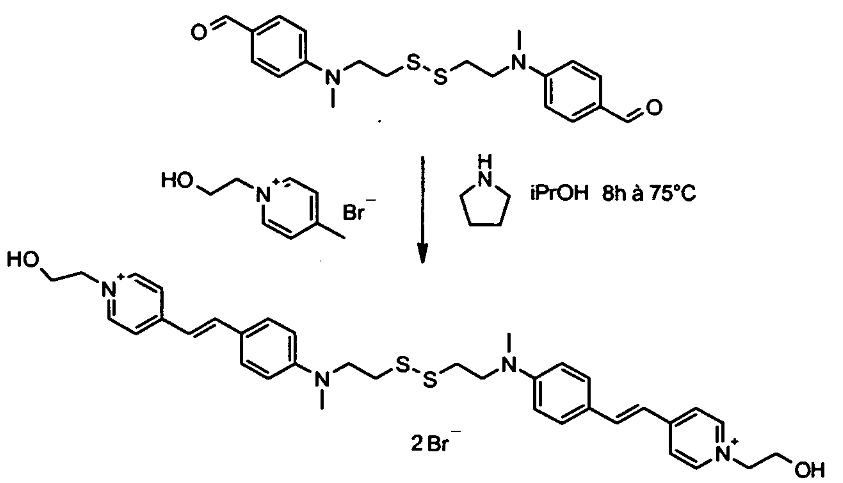


**[7]**

**Mode opératoire**

**Synthèse du di bromure de 4,4'-{disulfanediylbis[éthane-2,1-diyl(méthylimino)-4,1-phénylène(E)éthène-2,1-diyl]}bis[1-(2-hydroxyéthyl)pyridinium] [7]**

4,1 g de 4,4'-{disulfanediylbis[éthane-2,1-diyt(méthylimino)]}dibenzaldéhyde, 40 mL d'isopropanol, 0.86 mL de pyrrolidine sont chauffés à 75 °C pendant 1 h. 4,6 g de bromure de 1-(2-hydroxy-éthyl)-4-méthyl-pyridinium sont ajoutés au milieu réactionnel, agité et chauffé pendant 1h. 200 mL d'acétone sont introduits dans le mélange réactionnel. La gomme obtenue est brisée, filtrée, lavée par 3 fois 150 mL d'isopropanol et séchée. 6.59 g de poudre sont recueillis. Les analyses indiquent que le produit est conforme. RMN $^1H$ (400 MHz, MeOH-$d_4$) 2.97 (t, 4 H), 3.1 (s, 6 H), 3.78 (t, 4 H), 3.97 (t, 4 H), 4.51 (t, 4 H), 6.83 (d, 4 H), 7.09 (d, 2 H), 7.61 (d, 4 H), 7.83 (d, 2 H), 7.98 (d, 4 H), 8.56 (d, 4 H).

**Exemple 8 : Synthèse du di bromure de 4,4'-{disulfanedlylbis[éthane-2,1-diyl(méthylimino)-4,1-phénylène(E) éthene-2,1-diyl]}bis(1-benzylpyridinium) [8]**

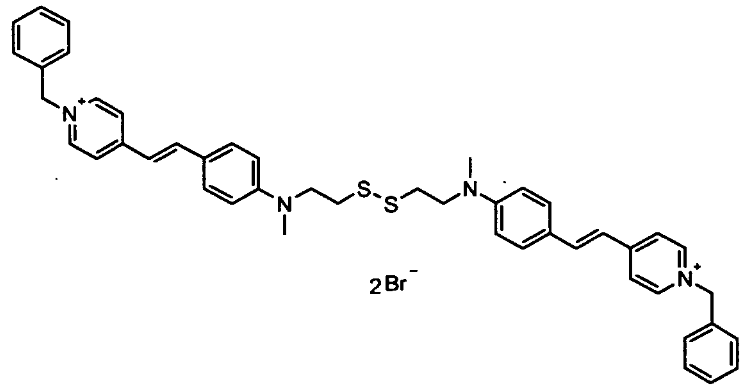

**[8]**

**Schéma de synthèse**

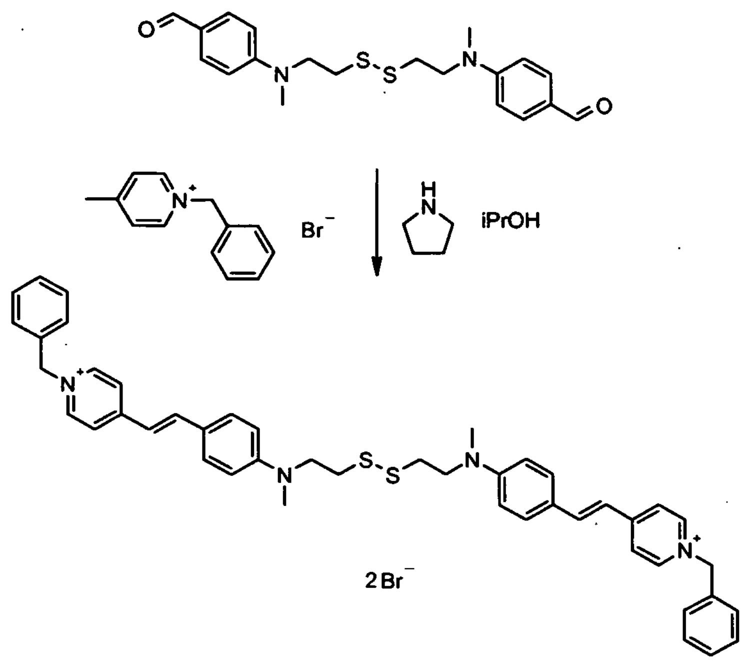


**[8]**

**Mode opératoire**

**Etape 1 : Synthèse du diméthanesulfonate de 4,4'-{disulfanediylbis[éthane-2,1-diyl(méthylimino)-4,1-phénylène(E)éthène-2,1-diyl]}bis(1-benzylpyridinium) [8]**

4,1 g de 4,4'-{disulfanediylbis[éthane-2,1-diyl(méthylimino)]}dibenzaldéhyde, 40 mL d'isopropanol, 0,86 mL de pyrrolidine sont chauffés à 75 °C pendant 20 minutes. 5,85 g de bromure de 1-Benzyl-4-méthyl-pyridinium sont ajoutés au milieu réactionnel, agité et chauffé pendant 5h. Le mélange réactionnel est agité à température ambiante pendant 17 h. Le surnageant est filtré, le solide noir obtenu est brisé, lavé avec 200 mL d'isopropanol, filtré, puis séché. 9,12 g de poudre rouge sont recueillis. Les analyses indiquent que le produit est conforme. RMN $^1$H (400 MHz, DMSO-$d_6$) 2.98 (t, 4 H), 3.03 (s, 6 H), 3.78 (t, 4 H), 5.67 (s, 4 H), 6.82 (d, 4 H), 7.15 (d, 2 H), 7.40 (m, 5 H), 7.61 (d, 4 H), 7.90

(d, 2 H), 8.07 (d, 4 H), 8.90 (d, 4 H).

**Exemple 9 : Synthèse du diméthylsulfate de 4,4'-{disulfanediylbis[éthane-2,1-diyl(méthylimino)-4,1-phénylène (E)éthène-2,1-diyl]}bis(1-méthylquinolinium [9]**

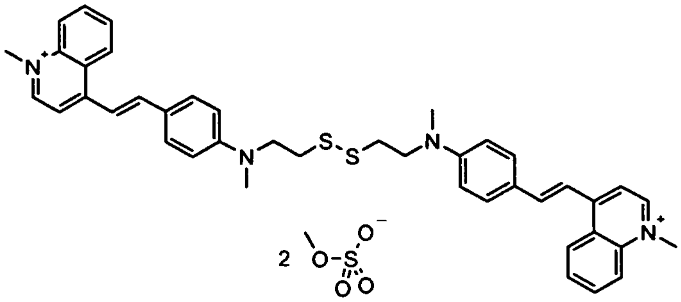

**[9]**

**Schéma de synthèse**

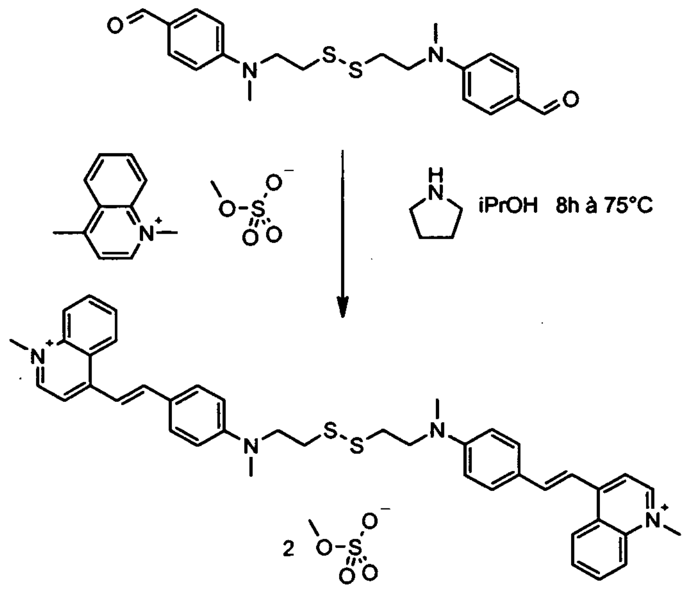


**[9]**

**Mode opératoire**

**Synthèse du diméthylsulfate de 4,4'-{disulfanediylbis[éthane-2,1-diyl(méthylimino)-4,1-phénylène(E)éthène-2,1-diyl]}bis(1-méthylquinolinium) [9]**

4,1 g de 4,4'-{disulfanediylbis[éthane-2,1-diyl(méthylimino)]}dibenzaldéhyde, 40 mL d'isopropanol, 0,86 mL de pyrrolidine sont chauffés à 75 °C pendant 20 minutes. 5,9 g de bromure de 1,4-diméthyl-quinolinium sont ajoutés au milieu réactionnel, agité et chauffé pendant 5 h. 200 mL d'acétone sont introduits dans le mélange réactionnel. Le précipité obtenu est filtré, lavé avec 200 mL d'acétone puis séché. 8,73 g de poudre noire sont recueillis. Les analyses indiquent que le produit est conforme. LC-MS m/z 334, L max 524nm

**Exemple 10 : Synthèse du dibromure de 4,4'-{disulfanediylbis[éthane-2,1-diyl(méthylimino)-4,1-phénylène(1E) prop-1-ène-1,2-diyl]}bis[1-(2-hydroxyéthyl)-pyridinium] [10]**

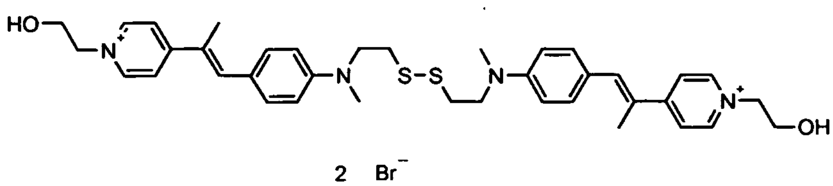

**[10]**

**Schéma de synthèse**

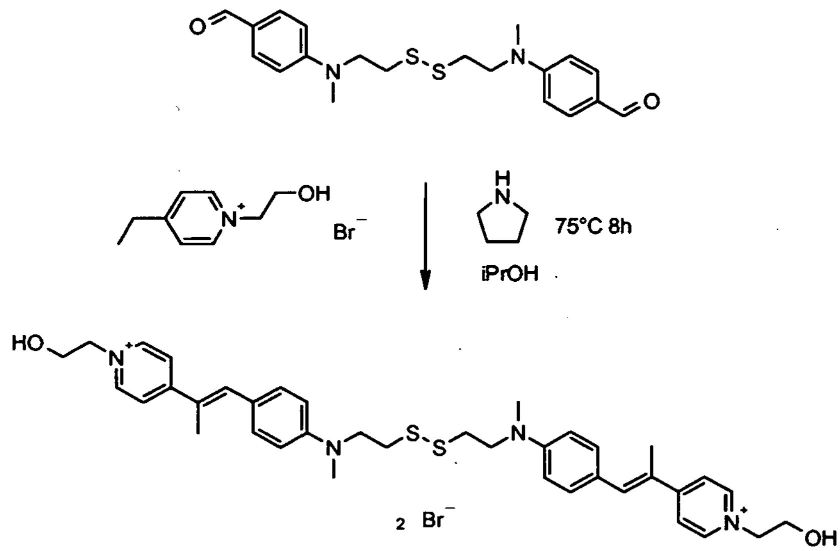


**[10]**

**Mode opératoire**

**Etape 1 : Synthèse du dibromure de 4,4'-{disulfanediylbis[éthane-2,1-diyl(méthylimino)-4,1-phénylène(1 E) prop-1-ène-1,2-diyl]}bis[1-(2-hydroxyéthyl)pyridinium]** [10]

4,1 g de 4,4'-{disulfanediylbis[éthane-2,1-diyl(méthylimino)]}dibenzaldéhyde, 40 mL d'isopropanol, 0,86 mL de pyrrolidine sont chauffés à 75 °C pendant 20 minutes. 5,9 g de bromure de 4-éthyl-1-(2-hydroxyéthyl)pyridinium sont ajoutés au milieu réactionnel, agité et chauffé pendant 5 h. 200 mL d'acétone sont introduits dans le mélange réactionnel. Le précipité obtenu est filtré, lavé avec 200 mL d'acétone puis séché. 9,67 g de poudre violette sont recueillis. Les analyses indiquent que le produit est conforme et pur. RMN $^1H$ (400 MHz, MeOH-$d_4$) 2.38 (s; 6 H), 2.96 (t; 4 H), 3.06 (s; 6 H), 3.76 (t; 4 H), 3.99 (t; 4 H), 4.59 (t; 4 H), 6.84 (d; 4 H), 7.49 (d; 4 H), 7.52 (s; 2 H), 8.13 (d; 4 H), 8.68 (d; 4 H).

**Exemple 11 : Synthèse du sel de 1-éthyl-4-((E)-2-{4-[(17-{4-[(E)-2-(1-éthyl-pyridinium-4-yl)vinyl]phényl}-4,4,13,13-tétraméthyl-8,9-dithia-17-aza-4,13-diazoniaoctadéc-1-yl)(méthyl)amino]phényl}vinyl)pyridinium [11]**

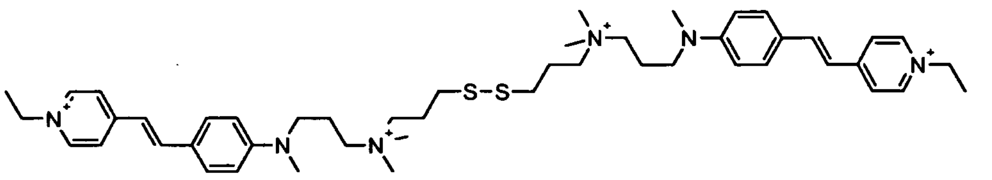

4M'

[11]

**Schéma de synthèse**

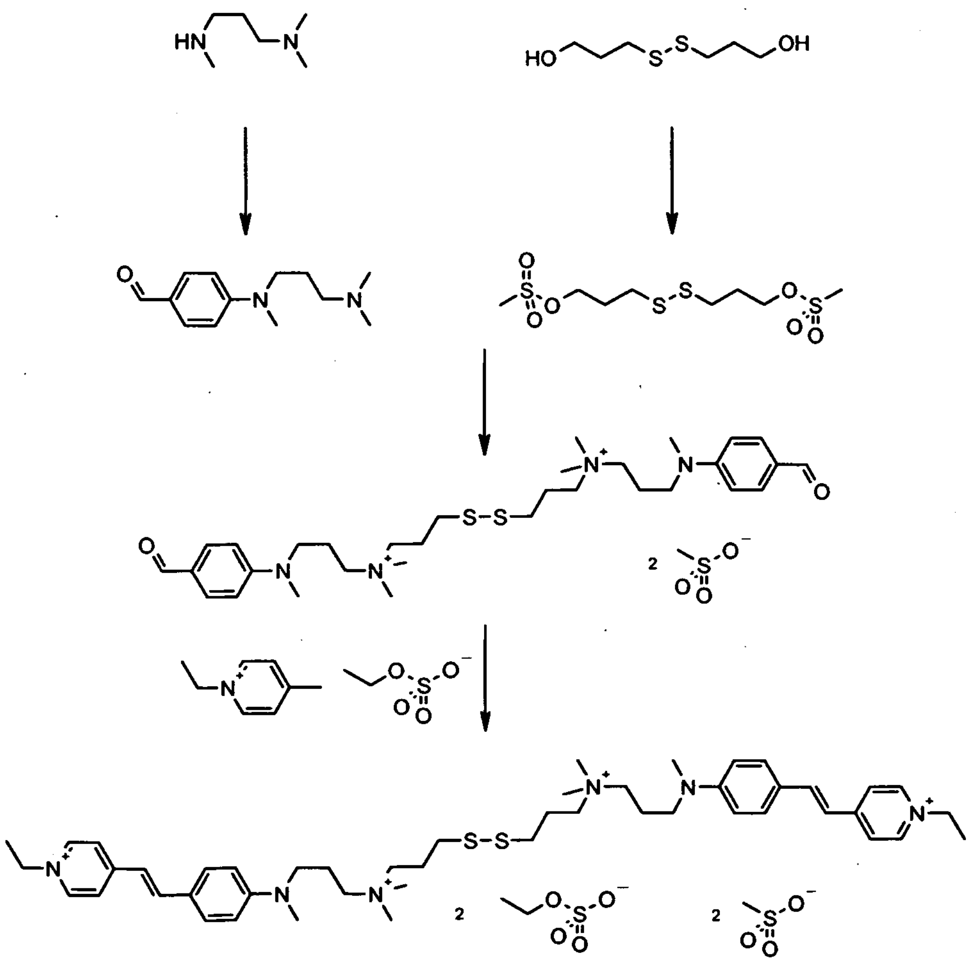

[11]

**Mode opératoire**

**Etape 1 : Synthèse du 4-[[3-(diméthylamino)propyl](méthyl)amino]benzaldéhyde**

10 g de 4-fluorobenzaldéhyde et 12 g de carbonate de potassium sont mélangés dans 20 mL de N-méthylpyrrolidinone (NMP) et portés à 50 °C. 13 mL de N.N.N'-triméthyl-1.3-propanediamine sont ajoutés et le mélange est porté à 80 °C pendant 10 h. Après refroidissement, 100 mL d'acétone sont ajoutés. Le mélange obtenu est filtré et concentré sous vide. 21,85 g d'huile sont recueillis, qui contiennent le composé attendu et 0,7 équivalents molaires de NMP.

**Etape 2 : synthèse du diméthanesulfonate de disulfanediyldipropane-3,1-diyl**

9 g de 3,3'-disulfanediyldipropan-1-ol sont dilués dans 50 mL d'acétate d'éthyle. 20 g de triéthylamine sont ajoutés, et le mélange est refroidi à 0 °C. 14,3 g de chlorure de mésyle sont ajoutés progressivement en maintenant la température inférieure à 5 °C. A la fin de l'addition, le mélange est maintenu agité une heure à température ambiante puis il est versé sur 100 mL de mélange eau/glace. Le produit est extrait par 100 mL d'acétate d'éthyle, lavé par 2 x 100 mL de solution molaire d'acide chlorhydrique, 2 x 100 mL d'eau , 100 mL d'une solution saturée d'hydrogénocarbonate de sodium et 2 x 100 mL de solution saturée de chlorure de sodium. La phase organique est séchée sur sulfate de sodium et concentrée sous vide. 6 g d'huile jaunâtre sont recueillis ; les analyses montrent que le produit obtenu est conforme.

**Etape 3 : synthèse du diméthanesulfonate de N,N'-(disulfanediyldipropane-3,1-diyl)bis{3-[(4-formylphényl)(methyl)amino]-N,N-diméthylpropan-1-aminium}**

2,2 g de 4-[[3-(diméthylamino)propyl](méthyl)amino]benzaldéhyde et 1,7 g de diméthanesulfonate de disulfanediyldipropane-3,1-diyl sont mélangés dans 2 mL de N-méthylpyrrolidinone à 80 °C pendant 22 h. Après refroidissement du mélange réactionnel, 20 mL d'éthanol sont ajoutés. Le solide obtenu est filtré, est très hygroscopique. Une purification par trituration dans le dichlorométhane conduit à l'isolement de 232 mg de solide conforme au produit attendu.

**Etape 4 : Synthèse du sel de 1-éthyl-4-((E)-2-{4-[(17-{4-[(E)-2-(1-éthylpyridinium-4-yl)vinyl]phényl}-4,4,13,13-tétraméthyl-8,9-dithia-17-aza-4,13-diazoniaoctadéc-1-yl)(méthyl)amino]phényl}vinyl)pyridinium [11]**

230 mg de diméthane sulfonate de N,N'-(disulfanediyldipropane-3,1-diyl)bis{3-[(4-formylphényl)(méthyl)amino]-N,N-diméthylpropan-1-aminium} et 163 mg de bromure de 1-éthyl-4-méthylpyridinium sont mélangés dans 5 mL d'isopropanol. 53 µL de pyrrolidine sont ajoutés, le mélange est maintenu agité à 70 °C pendant 6 h et agité à température ambiante pendant 72 h. Après refroidissement du mélange à -20 °C, le solide formé est filtré, repris dans du méthanol et reprécipité par ajout de 200 mL de terbutylméthyléther. 118 mg de solide noir sont recueillis. Les analyses indiquent que le produit est conforme à la structure attendue (MS (ESI+): pic de masse m/z =199)

<u>Exemple 12 :</u> **Synthèse du di bromure de 2,2'-{disulfanediylbis[éthane-2,1-diylimino-4,1-phénylène(E)éthène-2,1-diyl]}bis[1-(2-hydroxyéthyl)pyridinium] [12]**

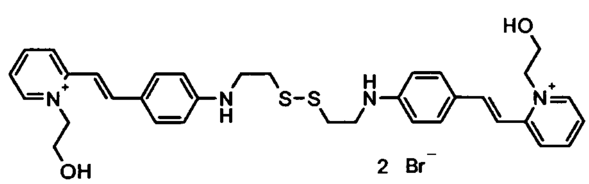

**Schéma de synthèse**

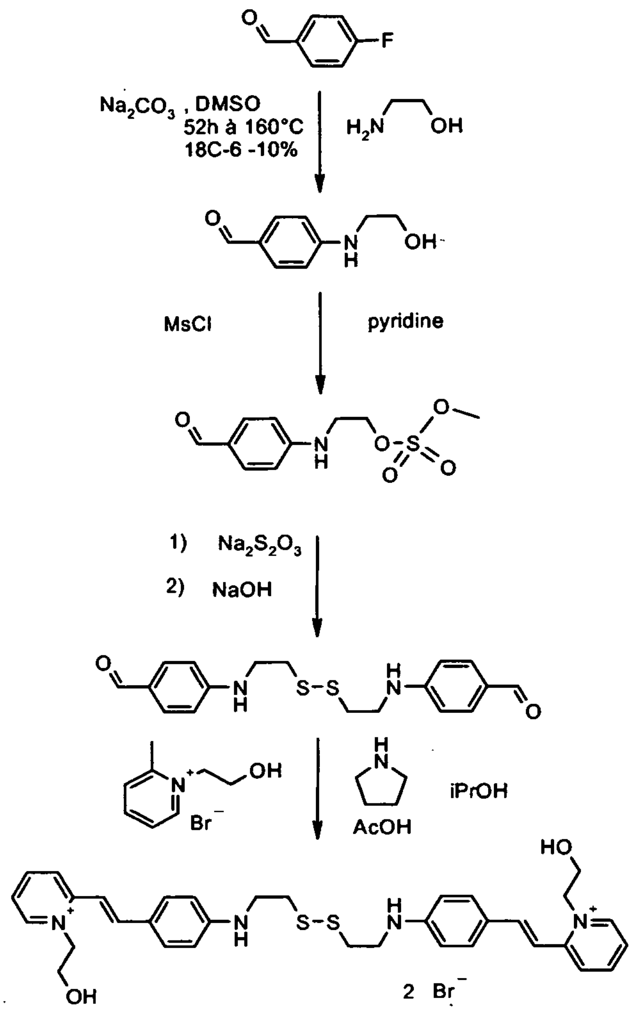


## Mode opératoire

### Etape 1 : Synthèse 4-[(2-hydroxyéthyl)amino]benzaldéhyde

1,24 g de 4-fluorobenzaldéhyde, 10 mL de DMSO distillé sur hydrure de calcium, 1,16 g de carbonate de sodium et une quantité catalytique d'éther 18-couronne-6 (5% mole) sont mélangés sous agitation pendant 10 minutes. 1,25 mL d'éthanolamine sont ajoutés lentement au milieu réactionnel maintenu sous agitation et chauffage à 160 ˚C pendant 52 h. Après refroidissement à 75 ˚C le mélange réactionnel est hydrolysé avec de l'eau distillée. Une solution diluée d'acide chlorhydrique est additionnée au mélange jusqu'à atteindre un pH compris entre 6 et 7 puis maintenu sous agitation et chauffage à 40 ˚C pendant 6 h. L'alcool obtenu est extrait à l'acétate d'éthyle. La phase organique est lavée à plusieurs reprises par de l'eau distillée et de l'eau salée, puis séchée sur sulfate de magnésium. Le mélange est concentré sous vide, purifié par chromatographie sur gel de silice.1,2 g de solide marron sont recueillis. Les analyses montrent que le produit est conforme à la structure attendue.

### Etape 2 : Synthèse du méthylsulfate de 2-[(4-formylphényl)amino]éthyle

0,4 g de 4-[(2-hydroxyéthyl)amino]benzaldéhyde et 10 mL de pyridine sont mélangés sous agitation. Le milieu réactionnel est refroidi dans un bain de carboglace-acétone. A 0 ˚C, 0,18 mL de chlorure de mésyle sont ajoutés goutte à goutte. Le mélange réactionnel est agité pendant 17h à une température comprise entre 0 et 5 ˚C, puis de l'eau glacée est incorporée au mélange. Le solide formé est trituré, filtré, lavé par de l'eau distillée puis séché sous vide. 550 mg de poudre blanche sont recueillis. Les analyses indiquent que le produit est conforme.

**Etape 3: Synthèse du 4,4'-[disulfanediylbis(éthane-2,1-diylimino)]dibenzaldéhyde**

0,5 g de 2-[(4-formylphényl)amino]éthyl méthyl sulfate, 3 mL de solution éthanol-eau (1 : 2) et 0,546 g de thiosulfate de sodium sont mélangés sous agitation à reflux pendant 2 h. La solution jaune obtenue est refroidie, puis 2,8 mL de soude à 35 % est ajoutée goutte à goutte. Le mélange est maintenu sous agitation à 75 °C pendant 1 h. Après refroidissement, un solide jaune clair est filtré, lavé à l'eau puis séché sous vide. 237 mg de poudre jaune sont recueillis. Les analyses indiquent que le produit est conforme et pur. RMN $^1$H (400 MHz, $CDCl_3$) 2.85 (t, 4H), 3.5 (m, 4 H), 4.61 (t, 2 H), 63.57 (d, 2 H), 7.63 (d, 4 H), 9.66 (s, 2 H)

**Etape 4 : Synthèse du di bromure de 2,2'-{disulfanedlylbis[éthane-2,1-diylimino-4,1-phénylène(E)éthène-2,1-diyl]}bis[1-(2-hydroxyéthyl)pyridinium] [12]**

200 mg de bromure de 1-(2-hydroxyethyl)-2-méthylpyridinium et 260 mg de 4,4'-[disulfanediylbis(éthane-2,1-diylimino)]dibenzaldéhyde sont dispersés dans 4 mL d'isopropanol. 1 mL de solution composée de 666 mg d'acide acétique, 790 mg de pyrrolidine et 10 mL d'isopropanol est ajouté à la suspension. Le mélange est maintenu agité pendant 16 h. 130 mg de de bromure de 1-(2-hydroxyéthyl)-2-méthylpyridinium sont ajoutés et le mélange est chauffé à 60 °C pendant 6 h. Le solide formé est filtré, trituré dans 5 mL d'isopropanol et 0,5 mL d'eau puis refiltré et séché. 118 mg de solide noir sont recueillis. Les analyses indiquent que le produit est conforme à la structure attendue. RMN $^1$H (400 MHz, DMSO$d_6$) 2.96 (t, 4H), 3.46 (m, 4 H), 3.84 (m, 4 H), 4.8 (t, 4 H), 5.22 (t, 2 H), 6.70 (d, 4 H), 6.80 (t, 2 H), 7.28 (d, 2 H), 7.65 (d, 4 H), 7.72 (dd, 2 H), 7.86 (d, 2 H), 8.35 (dd, 2 H), 8.45 (d, 2 H), 8.67 (d, 2 H).

**<u>Exemple 13</u> : Synthèse du di méthyl sulfate de 2,2'-{disulfanediylbis[éthane-2,1-diylimino-2,1-phénylène(E) éthène-2,1-diyl]}bis(1-méthylquinolinium) [13]**

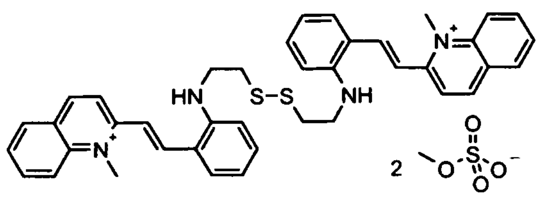

**[13]**

**Schéma de synthèse**

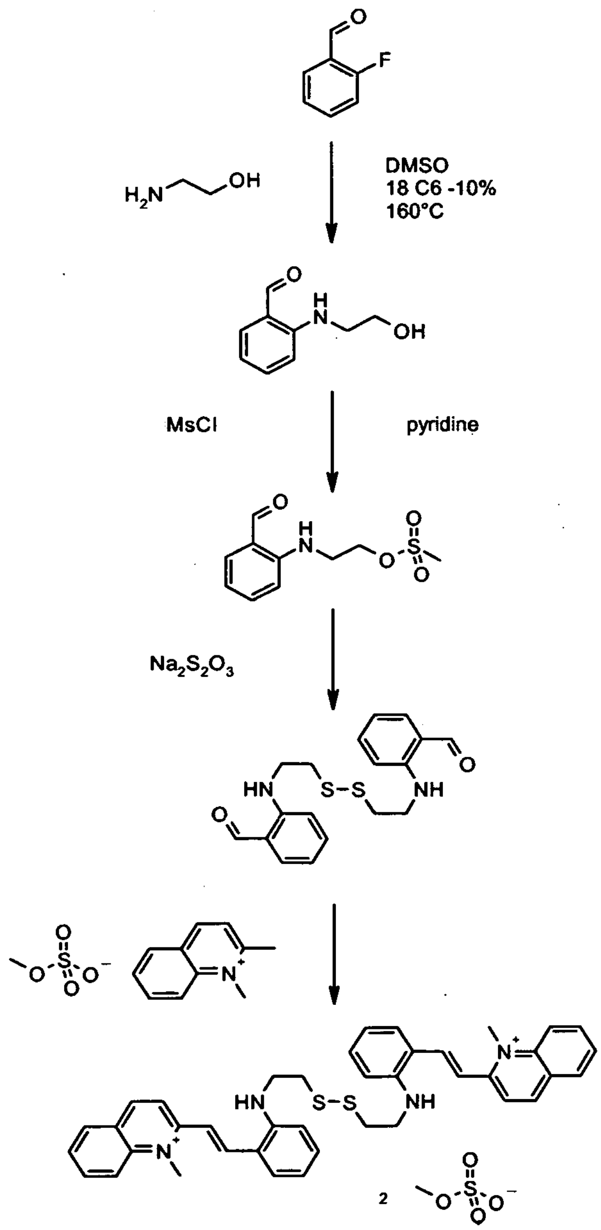


[13]

**Mode opératoire**

**Etape 1 : Synthèse du 2-[(2-hydroxyéthyl)amino]benzaldéhyde**

1,24 g de 2-fluorobénzaldéhyde, 10 mL de DMSO distillé sur hydrure de calcium, 1,16 g de carbonate de sodium et une quantité catalytique d'éther 18-couronne-6 (5% mole) sont mélangés sous agitation pendant 10 minutes. 1,3 mL g d'éthanolamine sont ajoutés. Après agitation à 160°C pendant 3 jours, le mélange est ramené à 75°C et hydrolysé avec de l'eau distillée. Le pH de la solution est abaissé à 6 par ajout d'acide chlorhydrique dilué maintenu sous agitation et chauffage à 40 °C pendant 18 h. L'alcool obtenu est extrait à l'acétate d'éthyle. La phase organique est lavée à plusieurs reprises par de l'eau distillée et de l'eau salée, puis séchée sur sulfate de magnésium. Le mélange est concentré sous vide puis purifié par chromatographie sur gel de silice. 1,2 g de produit marron sont recueillis.

**Etape 2 : Synthèse 2-[(2-formylphényl)amino]éthyl méthanesulfonate**

0,5 g de 2-[(2-hydroxyéthyl) amino]benzaldéhyde et 10 mL de pyridine distillée sont mélangés sous agitation à 0˚C. 0,23 mL de chlorure de mésyle sont ajoutés goutte à goutte. Le mélange réactionnel est agité pendant 17 h à une température comprise entre 0 et 5˚C, puis de l'eau glacée est incorporée au mélange. Le solide blanc obtenu est filtré, rincé à l'eau distillée et séché sous vide (650 mg) . Les analyses indiquent que le produit est conforme et pur.

**Etape 3 : Synthèse du 2,2'-[disulfanediylbis(éthane-2,1-diylimino)]di-benzaldéhyde**

0,5 g de 2-[(2-formylphényl)amino]éthyl méthanesulfonate et 0,546 g de thiosulfate de sodium pentahydrate sont chauffés dans 3 mL de solution éthanol/eau (1/2) à 90 ˚C pendant 15 g. La solution obtenue est refroidie et 2,8 mL de soude à 35 % sont ajoutés et le mélange est agité à 75 ˚C pendant 1 h. Après refroidissement, le solide jaune formé est filtré, lavé à l'eau distillée et séché sous vide. 222 mg de poudre sont recueillies. Les analyses montrent que le produit est conforme à l'attendu : RMN 1H (400 MHz, CDCl3) 2.96 (t, 4 H), 3.63 (dt, 4 H), 6.74 (t, 2 H), 6.76 (d, 2 H), 7.41 (t, 2 H), 7.50 (d, 2 H), 8.54 (t, 2 H), 9.84 (s, 2 H).

**Etape 4 : Synthèse du diméthyl sulfate de 2,2'-{disulfanediylbis[éthane-2,1-diylimino-2,1-phénylène(E)éthène-2,1-diyl]}bis(1-méthylquinolinium) [13]**

210 mg de 2,2'-[disulfanediylbis(éthane-2,1-diylimino)]dibenzaldéhyde et 360 mg de 1,2-diméthylquinolinium sont dispersés dans 4 ml d'isopropanol. 1 mL de solution composée d 790 ml de pyrrolidine, 666 mg d'acide acétique et qsp isopropanol 10 mL sont ajoutés Le mélange est maintenu agité à température ambiante pendant 10 jours, 180 mg de quinolinium sont ajoutés et l'agitation est maintenue pendant 3 jours à température ambiante. Le mélange est filtré, le solide recueilli est séché (214 mg). Les analyses indiquent que le produit est conforme à la structure attendue. LCMS (ESI+) 320, $\lambda_{max}$ 494 nm.

**EXEMPLES DE COLORATION**

**Exemple 1 : Procédé de coloration - composé [1]**

Une mèche de 1 g de cheveux de hauteur de ton 4 sont disposées au fond d'un bol. 1 mL de solution molaire de thioglycolate d'ammonium à pH 8,5 sont dilués par 9 mL d'eau. La solution ainsi obtenue est appliquée sur les mèches et laissée agir pendant 30 min. Les mèches sont rincées à l'eau courante puis redisposées dans un bol. 10 mL de solution aqueuse contenant 49 mg de composé [1] sont appliqués pendant 30 minutes à température ambiante (les mèches sont retournées et réimprégnées après 15 minutes).

Les mèches sont ensuite rincées à l'eau courante et séchées.

Après coloration, la mèche de hauteur de ton 4 est devenue visuellement plus claire que des mèches témoins non traitées.

**Exemple 2 : Procédé de coloration - composé [2]**

Une mèche de 1 g cheveux de hauteur de ton 4 sont disposées au fond d'un bol. 1 mL de solution molaire de thioglycolate d'ammonium à pH 8.5 sont dilués par 9 mL d'eau. La solution ainsi obtenue est appliquée sur les mèches et laissée agir pendant 30 min. Les mèches sont rincées à l'eau courante puis redisposées dans un bol. 10 mL de solution aqueuse contenant 63 mg de composé [2] sont appliqués pendant 30 minutes à température ambiante (les mèches sont retournées et réimprégnées après 15 minutes).

Les mèches sont ensuite rincées à l'eau courante et séchées.

Après coloration, la mèche de hauteur de ton 4 est devenue visuellement plus claire que des mèches témoins non traitées.

**Exemple 3 : Procédé de coloration - composés [3] à [12]**

Préparation d'une composition A

| | |
|---|---|
| Colorant disulfure de [3] à [12] | $10^{-3}$ mol % |

(suite)

| | |
|---|---|
| Alcool Benzylique | 4 g |
| Polyéthylèneglycol 6OE | 6 g |
| Hydroxyethylcellulose | 0,7 g |
| Alkylpolyglucoside en solution aqueuse à 65%MA | 4.5 g |
| Eau déminéralisée | qsp 100g |

Préparation d'une composition B

| | |
|---|---|
| Acide thioglycolique | 1M |
| Hydroxyde de Sodium | qsp pH 8,5 |
| Eau déminéralisée | qsp 100g |

Au moment de l'emploi, on mélange les compositions A (9 mL) et B (1 mL), puis on applique le mélange obtenu sur une mèche de 1 g de cheveux foncés (hauteur de ton 4) pendant 30 minutes à température ambiante (les mèches sont retournées et réimprégnées après 15 minutes).
Après rinçage à l'eau courante et séchage, on observe un éclaircissement des cheveux ainsi traités : la mèche de hauteur de ton 4 est devenue visuellement plus claire que des mèches témoins non traitées.

Rémanence vis-à-vis des shampooings successifs :

Les mèches ainsi traitées sont divisées en deux, une moitié est soumise à 5 shampooings successifs selon un cycle qui comprend le mouillage des mèches à l'eau, le lavage avec un shampooing classique, un rinçage à l'eau suivi d'un séchage.

*Observations visuelles*

Lors des shampooings des exemples [1], [2], [3], [4], [5], [7], [8], [9], [10] et [12] il n'y a pas de dégorgement visible, la mousse des shampooings et les eaux de rinçage ne sont pas colorées.
La couleur observée est conservée et l'effet d'éclaircissement reste visible sur les cheveux de hauteur de ton 4 ainsi traités.

*Résultats dans le système L*a*b* :*

La couleur des mèches avant et après les 5 lavages a été évaluée dans le système L*a*b* au moyen d'un spectrophotomètre CM 2600D MINOLTA ®, (Illuminant D65).
Dans ce système L* a* b*, L* représente la luminosité, a* indique l'axe de couleur vert/rouge et b* l'axe de couleur bleu/jaune. Plus la valeur de L est élevée, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.
La variation de la coloration entre les mèches de cheveux HT4 ou HT4,5 colorées et lavées est mesurée par (ΔE) selon l'équation suivante :

$$\Delta E = \sqrt{(L^* - L_o^*)^2 + (a^* - a_o^*)^2 + (b^* - b_o^*)^2}$$

Dans cette équation, L*, a* et b* représentent les valeurs mesurées avant coloration et $L_0^*$, $a_0^*$ et $b_0^*$ représentent les valeurs mesurées avant coloration (ou shampooing).
Plus la valeur de ΔE est importante, plus la différence de couleur entre les mèches HT4 ou 4,5 et les mèches

colorées est importante.

| Composé | L* | a* | b* | ΔE |
|---|---|---|---|---|
| | | | | |
| Référence mèches HT4 | 16,16 | 2,88 | 2,95 | |
| Composé 1 | 19,79 | 11,02 | 11,72 | 12,50 |
| Composé 1 - 5 shampooings | 21,45 | 11,36 | 12,87 | 14,08 |
| Composé 2 | 20,76 | 8,65 | 14,18 | 13,44 |
| Composé 2 - 5 shampooings | 22,16 | 9,13 | 14,43 | 14,38 |
| | | | | |
| Référence mèches HT4 | 17,27 | 2,78 | 3,19 | |
| Composé 3 | 19,09 | 6,45 | 9,30 | 7,36 |
| Composé 3 - 5 shampooings | 18,75 | 5,41 | 8,02 | 5,70 |
| Composé 4 | 17,94 | 9,33 | 9,24 | 8,95 |
| Composé 4 - 5 shampooings | 17,85 | 8,51 | 7,73 | 7,34 |
| Composé 5 | 19,57 | 6,31 | 10,14 | 8,13 |
| Composé 5 - 5 shampooings | 19,02 | 5,84 | 9,31 | 7,06 |
| Composé 7 | 17,76 | 7,76 | 7,65 | 6,71 |
| Composé 7 - 5 shampooings | 18,45 | 9,01 | 7,85 | 7,88 |
| Composé 8 | 17,47 | 5,46 | 5,75 | 3,72 |
| Composé 8 - 5 shampooings | 17,89 | 6,82 | 6,99 | 5,59 |
| Composé 9 | 16,91 | 4,67 | 2,15 | 2,19 |
| Composé 9 - 5 shampooings | 16,62 | 4,73 | 2,81 | 2,10 |
| Composé 10 | 17,31 | 6,42 | 6,65 | 5,02 |
| Composé 10 - 5 shampooings | 18,55 | 6,47 | 6,82 | 5,34 |
| Composé 12 | 20,06 | 3,66 | 9,15 | 6,64 |
| Composé 12 - 5 shampooings | 18,73 | 3,27 | 7,71 | 4,78 |

Les résultats du tableau ci-dessus montrent que la coloration évolue très peu après 5 shampooings sur les mèches originellement de HT4 colorées avec les colorants de l'invention. Ainsi la coloration et l'effet éclaircissant sur les cheveux restent quasiment inchangés ce qui montre une très bonne résistance aux shampooings des colorants de l'invention.

Résultats obtenus sur des mèches de HT4,5 traité avec les composés 3 à 5, suivi d' une série de 30 shampooings

| | L | a* | b* | ΔE |
|---|---|---|---|---|
| Référence mèches HT4,5 | 23,09 | 3,13 | 3,59 | - |
| Composé 3 | 25,49 | 10,56 | 9,96 | 10,08 |
| Composé 3 après 30 shampooings | 25,98 | 11,41 | 9,86 | 10,78 |
| Composé 4 | 25,87 | 6,06 | 9,60 | 7,24 |
| Composé 4 après 30 shampooings | 24,38 | 5,86 | 9,20 | 6,37 |
| Composé 5 | 25,08 | 1,19 | 7,51 | 4,81 |
| Composé 5 après 30 shampooings | 25,72 | 1,38 | 6,01 | 3,98 |

Les résultats du tableau ci-dessus montrent que la coloration évolue très peu même après 30 shampooings sur les mèches originellement de HT4,5 colorées avec les colorants de l'invention. Ainsi la coloration et l'effet éclaircissant sur les cheveux restent quasiment inchangés ce qui montre une très bonne résistance aux shampooings des colorants de l'invention.

*Résultats de Réflectance :*

Les performances d'éclaircissement des compositions conformes à l'invention et leur rémanence vis-à-vis de shampooings successifs ont été exprimées en fonction de la réflectance des cheveux. Ces réflectances sont comparées à la réflectance d'une mèche de cheveux non traitées de hauteur de ton HT4 .

La réflectance est mesurée au moyen d'un appareil spectrophotocolorimètre KONIKA-MINOLTA ®, CM 2600d et après irradiation des cheveux avec une lumière visible dans la gamme de longueurs d'onde allant de 400 à 700 nanomètres.

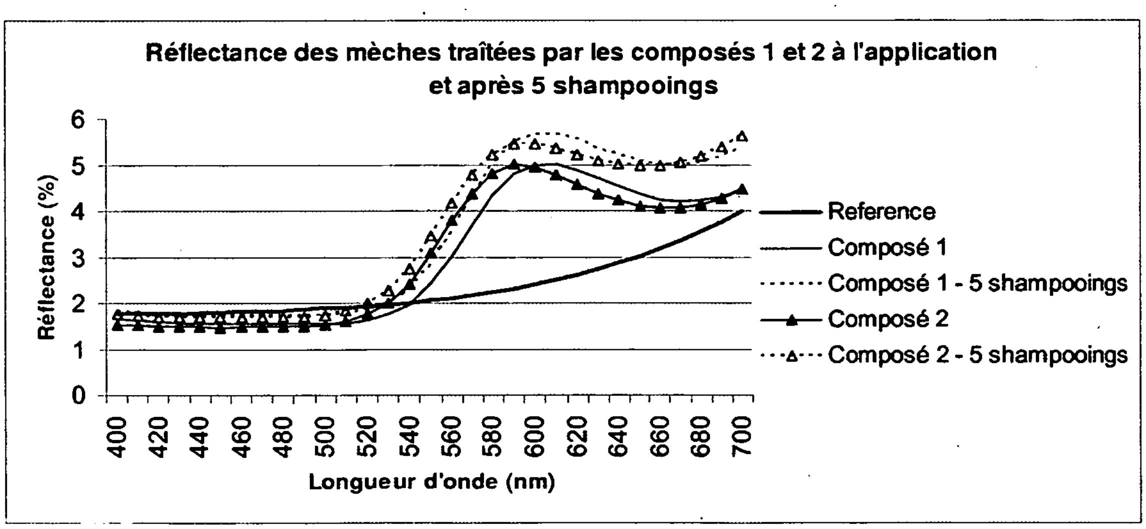


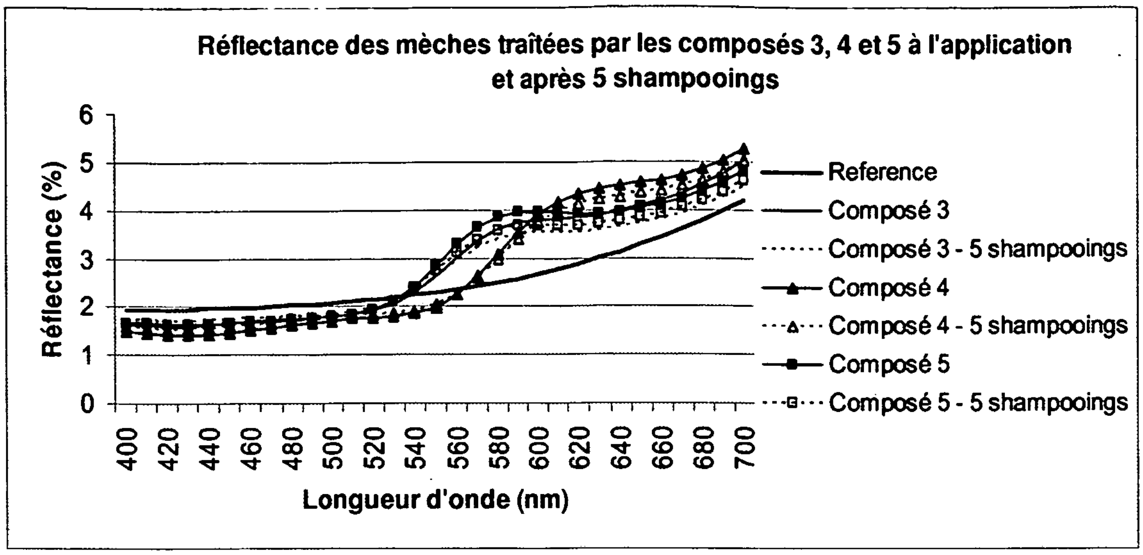

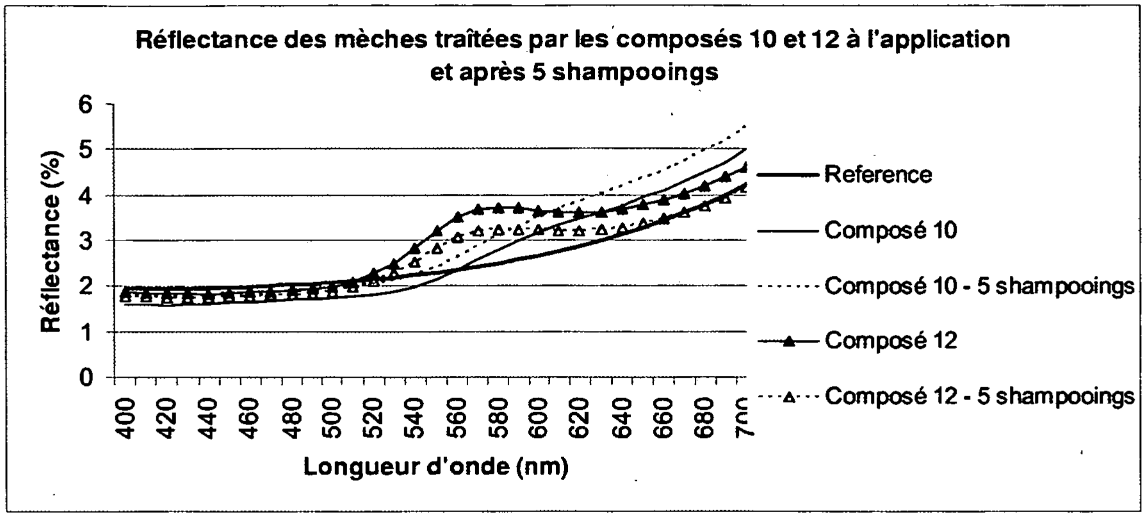


On constate tout d'abord que la réflectance d'une mèche de cheveux traitée avec une composition selon l'invention est supérieure à celle des cheveux non traités. Les mèches traitées apparaissent donc plus claires.

De plus, les résultats montrent que la réflectance des mèches de cheveux de hauteur de ton 4 traitées avec la composition de l'invention évoluent très peu après 5 shampooings. Ainsi la coloration et l'effet éclaircissant sur les cheveux restent quasiment inchangés ce qui montre une très bonne résistance aux shampooings des colorants de l'invention.

## Revendications

1. Colorant fluorescent de formule (I) ou (II) suivante :

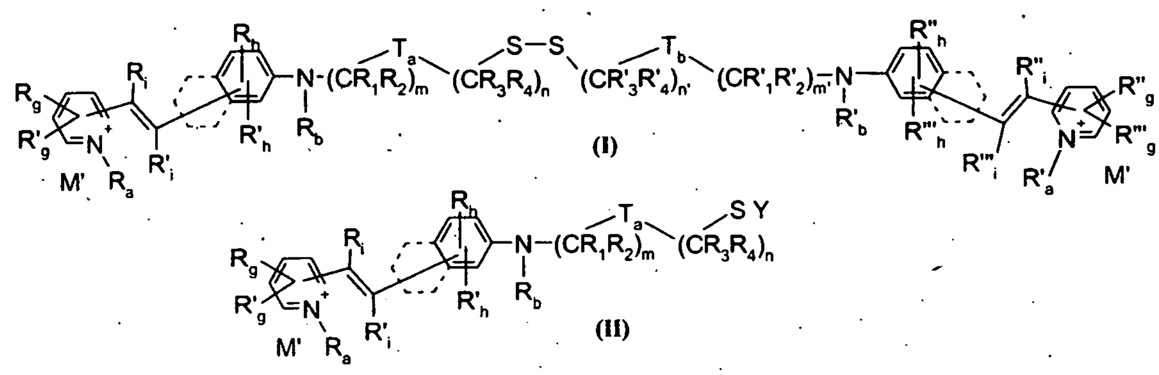

(I)

(II)

ses sels d'acide organique ou minéral, isomères optiques, isomères géométriques, et les solvates tels que hydrates ; formule (I) ou (II) dans laquelle :

➢ $R_a$ et $R'_a$, identiques ou différents, représentent un groupement aryl($C_1$-$C_4$)alkyle ou un groupement ($C_1$-$C_6$) alkyle éventuellement substitué par un groupement hydroxy ou amino, $C_1$-$C_4$ alkylamino, ($C_1$-$C_4$) dialkylamino, lesdits radicaux alkyle pouvant former avec l'atome d'azote qui les portent un hétérocyle comprenant de 5 à 7 chaînons, comprenant éventuellement un autre hétéroatome différent ou non de l'azote ;

➢ $R_b$ et $R'_b$, identiques ou différents, représentent un atome d'hydrogène, un groupement aryl($C_1$-$C_4$)alkyle ou un groupement ($C_1$-$C_6$) alkyle éventuellement substitué;

➢ $R_g$, $R'_g$, $R''_g$ et $R'''_g$, identiques ou différents, représentent un atome d'hydrogène, un groupement amino, ($C_1$-$C_4$) alkylamino, ($C_1$-$C_4$) dialkylamino, trifluorométhyle, un radical acylamino, ($C_1$-$C_4$)alcoxy ($C_1$-$C_4$)alkylcarbonyloxy, (C1-C4)alcoxycarbonyle, ($C_1$-$C_4$)alkylcarbonylamino, ($C_1$-$C_4$)alkylsulfonylamino, un radical ($C_1$-$C_3$)alkyle ;

➢ $R_h$, $R'_h$, $R''_h$ et $R'''_h$, identiques ou différents, représentant un atome d'hydrogène, un atome d'halogène, un

groupement dialkylamino ($C_1$-$C_4$), ($C_1$-$C_4$)alkylcarbonylamino, un radical acylamino, ($C_1$-$C_4$)alkylsulfonylamino, ou un radical ($C_1$-$C_4$) alkyle ;

➢ ou alors deux groupements $R_g$ et $R'_g$ ; $R''_g$ et $R'''_g$ ; $R_h$ et $R'_h$ ; $R''_h$ et $R'''_h$ portés par deux atomes de carbone adjacents, forment ensembles un cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, ($C_1$-$C_4$)alkylamino, ($C_1$-$C_4$)dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonylamino, un radical acylamino, carbamoyle , ($C_1$-$C_4$)alkylsulfonyl-amino, un radical aminosulfonyle, ou un radical ($C_1$-$C_{16}$)alkyle éventuellement substitué par un groupement choisi parmi ($C_1$-$C_{12}$)alcoxy, hydroxy, cyano, carboxy, amino, ($C_1$-$C_4$)alkylamino et ($C_1$-$C_4$)dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

➢ $R_i$, $R'_i$, $R''_i$ et $R'''_i$, identiques ou différents, représentent un atome d'hydrogène, où un groupement ($C_1$-$C_4$) alkyle ;

➢ $R_1$, $R_2$, $R_3$, $R_4$, $R'_1$, $R'_2$, $R'_3$ et $R'_4$, identiques ou différents, représentent un atome d'hydrogène ou un groupement ($C_1$-$C_4$) alkyle ;

➢

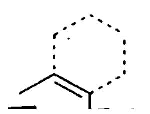,

présent ou absent, représente un groupement benzo ;

➢ $T_a$ et $T_b$, identiques ou différents, représentent :

i) soit une liaison covalente σ ;

ii) soit un radical choisi parmi -N(R)-, $-N^+(R)(R^\circ)-$, avec R, R°, identiques ou différents, représentant un atome d'hydrogène, un radical alkyle en $C_1$-$C_4$, hydroxyalkyle en $C_1$-$C_4$, ou un aryl($C_1$-$C_4$)alkyle,

iii) soit un radical hétérocycloalkyle ou hétéroaryle, cationique ou non ;

➢ m, m', n et n', identiques ou différents, représentent un entier compris inclusivement entre 0 et 6 avec m+n, m'+n', identiques ou différents, représentent un entier compris inclusivement entre 1 et 10 ;

➢ lorsque $T_a$ représente une liaison covalente σ, Y représente un groupement choisi parmi :

o aryle éventuellement substitué ;
o hétéroaryle éventuellement substitué ;
o hétérocycloalkyle événtuellemént substitué ;
o (di)arylalkyle éventuellement substitué ;
o (di)hétéroarylakyle éventuellement substitué ;
o cyclique stériquement encombré ;

➢ lorsque $T_a$ représente -N(R)-, $-N^+(R)(R^\circ)-$, un radical hétérocycloalkyle ou hétéroaryle, cationique ou non ; Y représente un groupement choisi parmi i) un atome d'hydrogène ; ii) un métal alcalin ; iii) un métal alcalino-terreux ; iv) un groupement ammonium : $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe ($C_1$-$C_4$)alkyle ; ou v) un groupement protecteur de fonction thiol ;

et

➢ M' représentant un contre-ion anionique ;

étant entendu que lorsque le composé de formule (I) ou (II) contient d'autres parties cationiques, il se trouve associé à un ou plusieurs contre-ions anioniques permettant d'atteindre l'électroneutralité de la formule (I) ou (II).

**2.** Colorant fluorescent de formule (II) selon la revendication 1 dans lesquels lorsque $T_a$ représente -N(R)-, $-N^+(R)(R^\circ)-$, un radical hétérocycloalkyle ou hétéroaryle, cationique ou non ; et Y représente un atome d'hydrogène ou un métal alcalin.

**3.** Colorant fluorescent de formule (II) selon la revendication 1 dans lesquels lorsque $T_a$ représente -N(R)-, $-N^+(R)$

(R°)-, un radical hétérocycloalkyle ou hétéroaryle, cationique ou non ; Y représente un groupement protecteur de la fonction thiol suivants :

- ➢ $(C_1-C_4)$alkylcarbonyle ;
- ➢ $(C_1-C_4)$alkylthiocarbonyle ;
- ➢ $(C_1-C_4)$alcoxycarbonyle ;
- ➢ $(C_1-C_4)$alcoxythiocarbonyle ;
- ➢ $(C_1-C_4)$alkylthio-thiocarbonyle ;
- ➢ (di) $(C_1-C_4)$ (alkyl)aminocarbonyle ;
- ➢ (di) $(C_1-C_4)$ (alkyl)aminothiocarbonyle;
- ➢ arylcarbonyle ;
- ➢ aryloxycarbonyle ;
- ➢ aryl$(C_1-C_4)$alkcoxycarbonyle ;
- ➢ (di) $(C_1-C_4)$ (alkyl)aminocarbonyle ;
- ➢ $(C_1-C_4)$(alkyl)arylaminocarbonyle
- ➢ carboxy ;
- ➢ $SO3^-$ ; $M^+$ avec $M^+$ représentant un métal alcalin ou alors M' de la formule (II) et $M^+$ sont absents ;
- ➢ aryle éventuellement substitué ;
- ➢ hétéroaryle éventuellement substitué ;
- ➢ hétérocycloalkyle éventuellement substitué ;
- ➢ isothiouronium $-C(NR'^cR'^d)=N^+R'^eR'^f$ ; $An^-$ avec $R'^c$, $R'^d$, $R'^e$ et $R'^f$, identiques ou différents représentent un atome d'hydrogène ou un groupement alkyle ;
- ➢ isothiourée $-C(NR'^cR'^d)=NR'^e$; avec $R'^c$, $R'^d$, $R'^e$ tels que définis précédemment ;
- ➢ (di)arylalkyle éventuellement substitué ;
- ➢ (di)hétéroarylakyle éventuellement substitué ;
- ➢ $CR'^1R'^2R'^3$ avec $R'^1$, $R'^2$ et $R'^3$ identiques ou différents, représentant un atome d'halogène ou un groupe choisi parmi :

  - ■ alkyle éventuellement substitué ;
  - ■ alcoxy éventuellement substitué ;
  - ■ aryle éventuellement substitué ;
  - ■ hétéroaryle éventuellement substitué ;

- ➢ $P(Z^1)R''^1R''^2R''^3$ avec. $R''^1$ et $R''^2$, identiques ou différents, représentent un groupement hydroxy, alcoxy ou alkyle, $R''^3$ représente un groupement hydroxy ou alcoxy, et $Z^1$ représente un atome d'oxygène ou de soufre ;
- ➢ cyclique stériquement encombré ; et
- ➢ alcoxyalkyle éventuellement substitué.

**4.** Colorant fluorescent de formule (II) selon l'une quelconque des revendications 1 et 3 dans lequel lorsque $T_a$ représente -N(R)-, $-N^+(R)(R°)$-, un radical hétérocycloalkyle ou hétéroaryle, cationique ou non ; Y représente un groupement protecteur choisi parmi :

- ■ $(C_1-C_4)$alkylcarbonyle ;
- ■ arylcarbonyle ;
- ■ $(C_1-C_4)$alcoxycarbonyle ;
- ■ aryloxycarbonyle ;
- ■ aryl$(C_1-C_4)$alcoxycarbonyle ;
- ■ (di)$(C_1-C_4)$(alkyl)aminocarbonyle ;
- ■ $(C_1-C_4)$(alkyl)arylaminocarbonyle ;
- ■ aryle éventuellement substitué ;
- ■ hétéroaryle monocyclique à 5 ou 6 chaînons ;
- ■ hétéroaryle bicyclique cationique à 8 à 11 chaînons ;
- ■ hétérocycle cationique de formule suivante : .

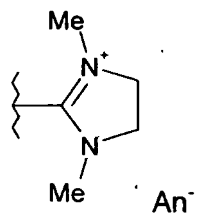

- ■ isothiouronium $-C(NH_2)=N^+H_2$; $An^-$;
- ■ isothiourée $-C(NH_2)=NH$ ;et
- ■ $SO_3^-$, $M^+$ avec $M^+$ représentant un métal alcalin ou alors M' de la formule (II) et $M^+$ sont absents.

**5.** Colorant fluorescent de formule (II) selon la revendication 1 dans lequel lorsque $T_a$ représente une liaison Y représente un i) hétéroaryle monocyclique à 5 ou 6 chaînons aromatiques, cationiques comprenant de 1 à 4 hétéroatomes choisis parmi l'oxygène, le soufre et l'azote ; ii) un hétéroaryle cationique bicyclique à 8 à 11 chaînons, ces groupes mono ou bicycliques étant éventuellement substitués par un ou plusieurs groupements ; iii) ou l'hétérocyclique suivant :

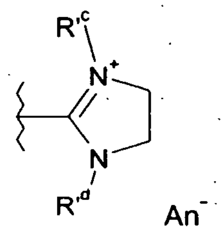

dans lequel $R'^c$ et $R'^d$, identiques ou différents, représentent un atome d'hydrogène ou un groupement alkyle ; et $An^-$ représente le contre-ion

**6.** Colorant fluorescent selon l'une quelconque des revendications précédentes, choisi parmi les colorants fluorescents de formule (Ia), (IIa), (Ib), (IIb), (Ic) ou (IIc) possédant chacun un groupement éthylène qui relie la partie pyridinium au phényle en position ortho ou para soit en positions 4-4', 4-2', ou 2-4' pour (Ia), (IIa), (Ib) ou (IIb), ou alors en positions 4-5' ou 2-5' pour (Ic) ou (IIc) :

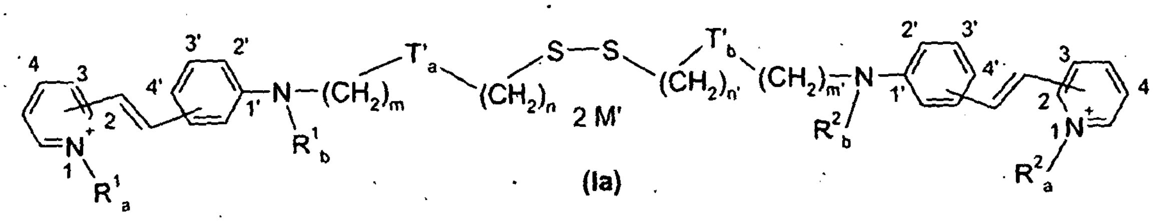

(Ia)

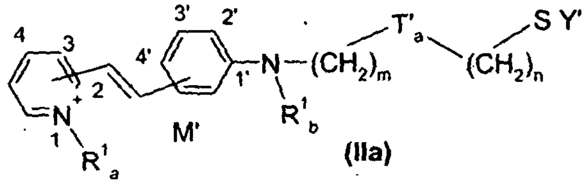

(IIa)

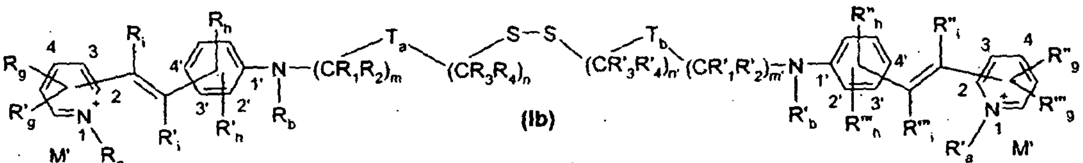

(Ib)

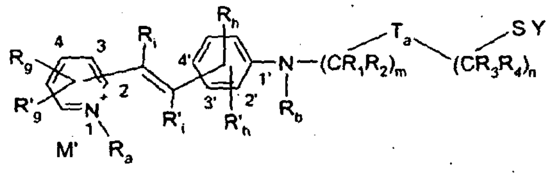

(IIb)

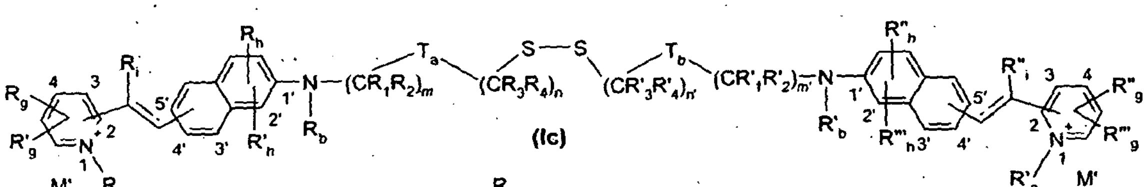

(Ic)

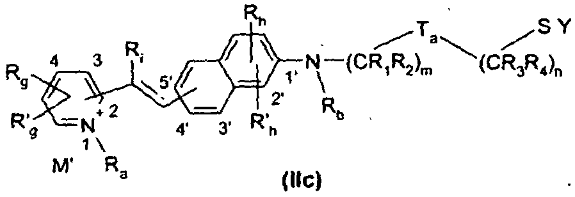

(IIc)

formules (**Ia**) ou (**IIa**) dans lesquelles :

- ■ $R^1_a$ et $R^2_a$ identiques ou différents, représentent un groupement benzoyle ou alkyle éventuellement substitué par un groupement hydroxy ;
- ■ $R^1_b$ et $R^2_b$, identiques ou différents, représentent un atome d'hydrogène ; un groupement benzyle ou alkyle ;
- ■ $T'_a$ et $T'_b$, identiques ou différents, représentent une liaison $\sigma$ ; un groupement $-N^+(R)(R^\circ)-$ avec R et R°, identiques ou différents, représentant un groupement alkyle ; ou alors $T'_a$ et $T'_b$ représentent un groupement imidazolium ;
- ■ Y' représente un groupement choisi parmi imidazolium, thiazolium ; oxazolium ; 1,2,4-triazolium ; pyridinium ; pyrimidinium ; benzoxazolium et benzothiazolium ; ces groupements étant substitués par un ou plusieurs groupements, identiques ou différents, groupement alkyle ;
- ■ m, n, m' et n', identiques ou différents, représentent un entier compris inclusivement entre 1 et 6 avec m+n=m'+n' représentent un entier compris inclusivement entre 2 et 6 ;
- ■ M' représente un contre-ion anionique ;

formules (**Ib**) ou (**IIb**) dans lesquelles :

➢ $R_a$, $R'_a$, $R_b$, $R'_b$, $R_i$, $R'_i$, $R''_i$, $R'''_i$, $R_1$, $R'_1$, $R_2$, $R'_2$, $R_3$, $R'_3$, $R_4$, $R'_4$, $T_a$, $T_b$, m, m', n, n', Y dans une quelconque des revendications précédentes ;

➢ $R_g$ et $R'_g$; $R''_g$ et $R'''_g$ identiques ou différents, représentent un atome d'hydrogène, un atome d'halogène, un groupement amino, ($C_1$-$C_4$)alkylamino, ($C_1$-$C_4$) dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy, ($C_1$-$C_4$)alcoxycarbonyle, ($C_1$-$C_4$)alkylcarbonylamino, acylamino, carbamoyle, ($C_1$-$C_4$)alkylsulfonylamino, amino-sulfonyle, ou un radical ($C_1$-$C_{16}$) alkyle éventuellement substitué par un groupement choisi parmi ($C_1$-$C_{12}$) alcoxy, hydroxy, cyano, carboxy, amino, ($C_1$-$C_4$) alkylamino et ($C_1$-$C_4$) dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

➢ $R_h$ et $R'_h$ ; $R''_h$ et $R'''_h$ portés par deux atomes de carbone adjacents, forment ensembles un cycle benzo, indéno, un groupement hétérocycloalkyle fusionné ou hétéroaryle fusionné ; le cycle benzo, indéno, hétérocycloalkyle ou hétéroaryle étant éventuellement substitué par un atome d'halogène, un groupement amino, ($C_1$-$C_4$) alkylamino, ($C_1$-$C_4$)dialkylamino, cyano, carboxy, hydroxy, trifluorométhyle, acylamino, alcoxy en $C_1$-$C_4$, (poly) hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonylamino, un radical acylamino, carbamoyle , ($C_1$-$C_4$)alkylsulfonyl-amino, un radical aminosulfonyle, ou un radical ($C_1$-$C_{16}$) alkyle éventuellement substitué par un groupement choisi parmi ($C_1$-$C_{12}$)alcoxy, hydroxy, cyano, carboxy, amino, ($C_1$-$C_4$)alkylamino et ($C_1$-$C_4$)dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

et

formules (**Ic**) ou (**IIc**) dans lesquelles :

➢ $R_a$, $R'_a$, $R_b$, $R'_b$, $R_i$, $R''_i$, $R_1$, $R'_1$, $R_2$, $R'_2$, $R_3$, $R'_3$, $R_4$, $R'_4$, $T_a$, $T_b$, m, m', n, n', Y sont tels que définis dans une quelconque des revendications précédentes ;

➢ $R_g$, $R'_g$, $R''_g$, $R'''_g$, $R_h$, $R'_h$, $R''_h$ et $R'''_h$, identiques ou différents, représentent un atome d'hydrogène, un atome d'halogène, un groupement amino, ($C_1$-$C_4$)alkylamino, ($C_1$-$C_4$) dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy, ($C_1$-$C_4$)alcoxycarbonyle, ($C_1$-$C_4$)alkylcarbonylamino, acylamino, carbamoyle , ($C_1$-$C_4$)alkylsulfonylamino, amino-sulfonyle, ou un radical ($C_1$-$C_{16}$) alkyle éventuellement substitué par un groupement choisi parmi ($C_1$-$C_{12}$) alcoxy, hydroxy, cyano, carboxy, amino, ($C_1$+$C_4$) alkylamino et ($C_1$-$C_4$) dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

étant entendu que lorsque les composés de formule (**Ia**), (**IIa**), (**Ib**), (**IIb**), (**Ic**) ou (**IIc**) contiennent d'autres parties cationiques, ils se trouvent associés à un ou plusieurs contre-ions anioniques permettant d'atteindre l'électroneutralité de la formule (**Ia**), (**IIa**), (**Ib**), (**IIb**), (**Ic**) ou (**IIc**).

**7.** Colorant fluorescent de formule (I) selon une des revendications 1 ou 6 qui est symétrique.

**8.** Colorant fluorescent selon l'une quelconque des revendications précédentes, choisi parmi les colorants suivants :

<table>
<tr><td>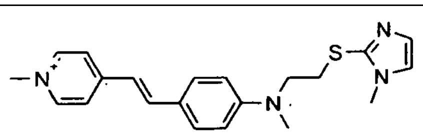 An⁻</td><td>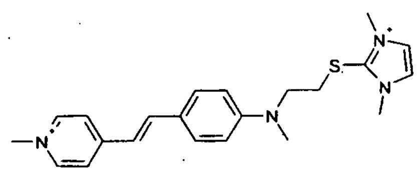 2An'</td></tr>
<tr><td>1</td><td>2</td></tr>
<tr><td colspan="2">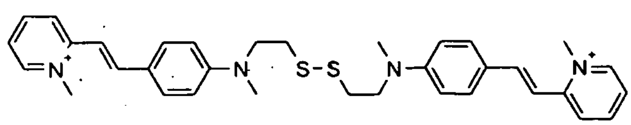 2M'</td></tr>
<tr><td colspan="2">3</td></tr>
</table>

(suite)

| |
|---|
| 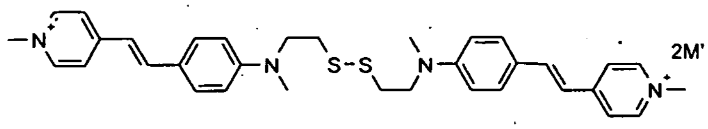 2M' |
| **4** |
| 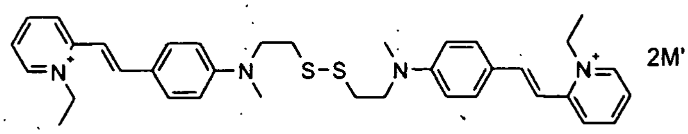 2M' |
| **5** |
| 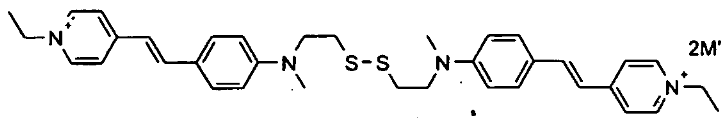 2M' |
| **6** |
| 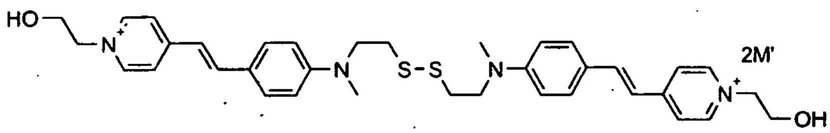 2M' |
| **7** |
| 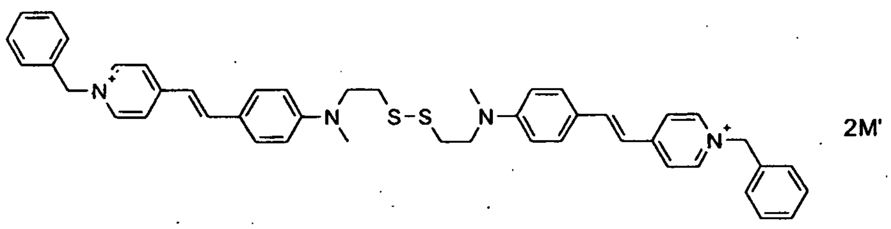 2M' |
| **8** |
| 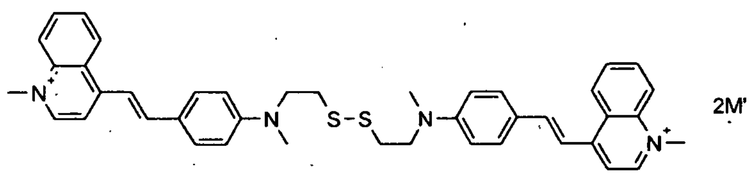 2M' |
| **9** |
| 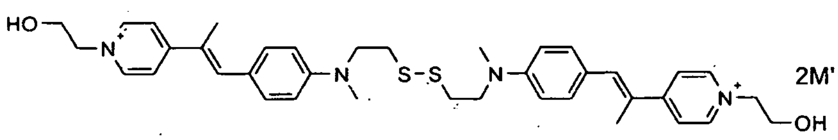 2M' |
| **10** |
| 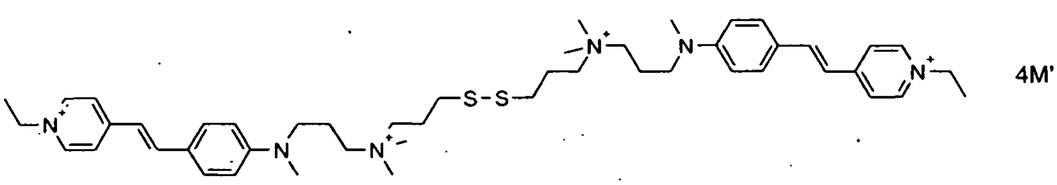 4M' |
| **11** |

(suite)

| | |
|---|---|
| 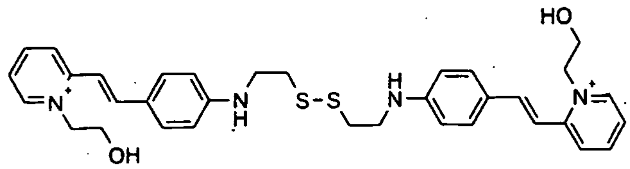 2M' | |
| 12 | |
| 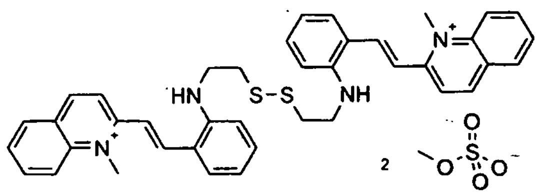 | 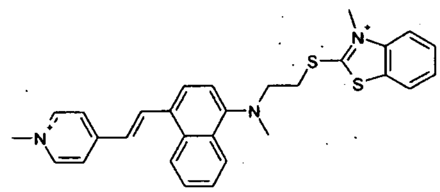 2An- |
| 13 | 14 |
| 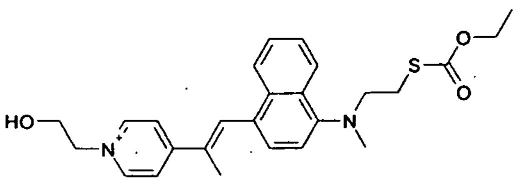 An- | 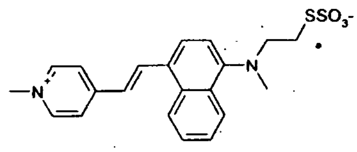 An-, M+ |
| 15 | 16 |
| 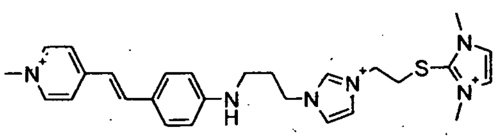 3An- | 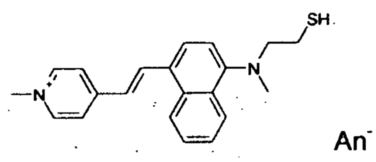 An- |
| 17 | 18 |
| 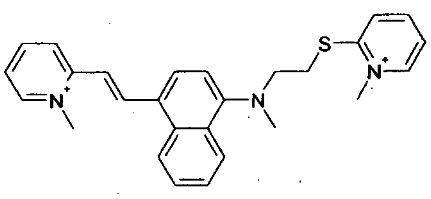 2An- | 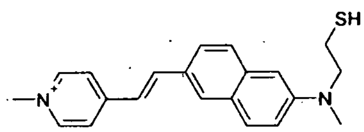 An- |
| 19 | 20 |
| 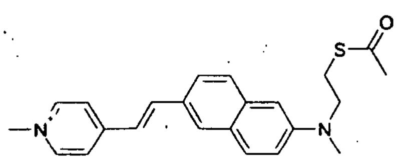 An- | 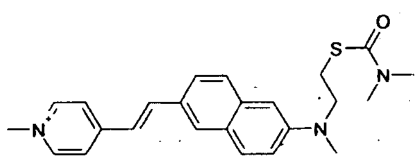 An- |
| 21 | 22 |
| 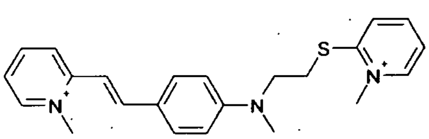 2An- | 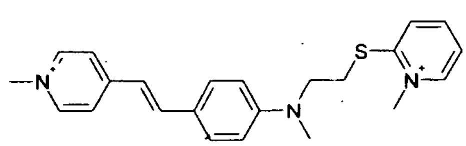 2An |
| 23 | 24 |

(suite)

| | |
|---|---|
| 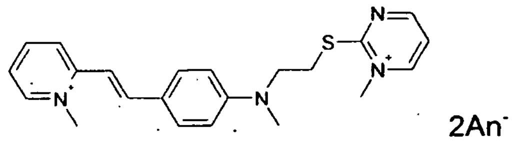 | 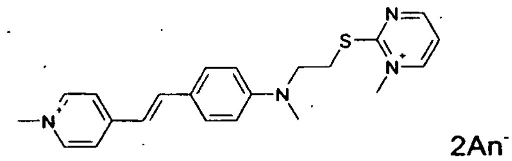 |
| **25** | **26** |
| 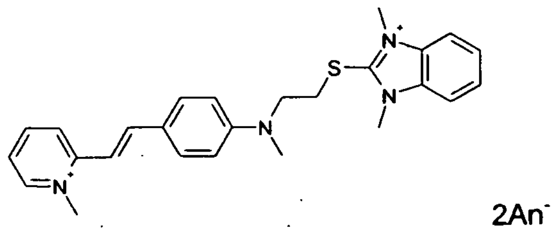 | 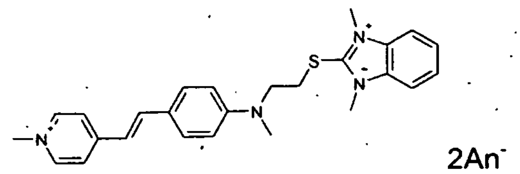 |
| **27** | **28** |
| 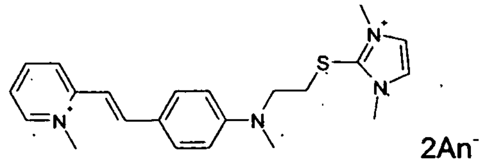 | 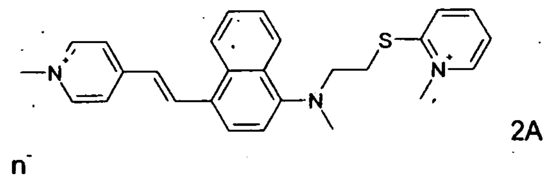 |
| **29** | **30** |
| 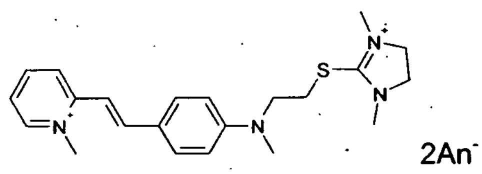 | 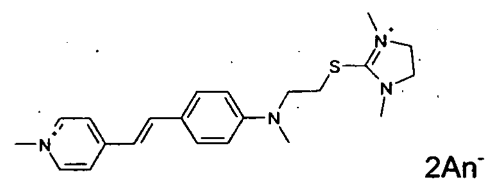 |
| **31** | **32** |
| 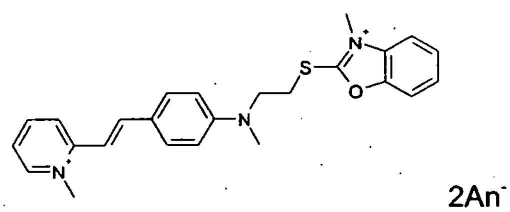 | 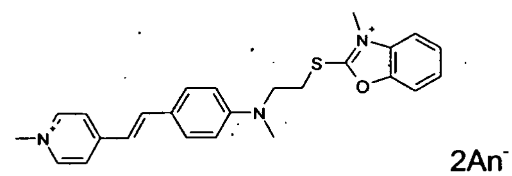 |
| **33** . | **34** |
| 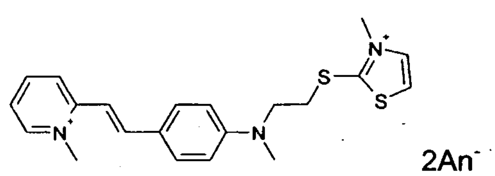 | 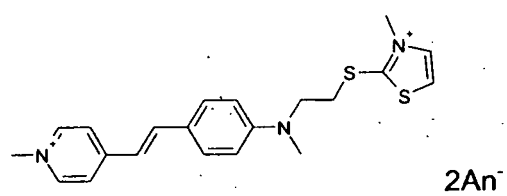 |
| **35** | **36** |

(suite)

| | |
|---|---|
| 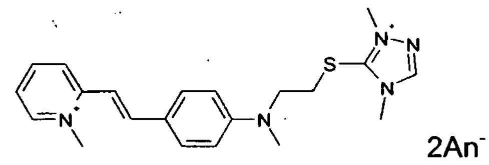 | 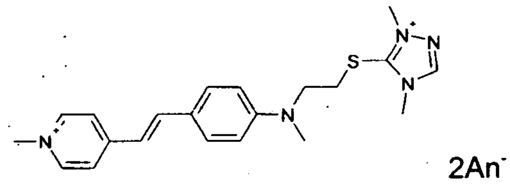 |
| **37** | **38** |
| 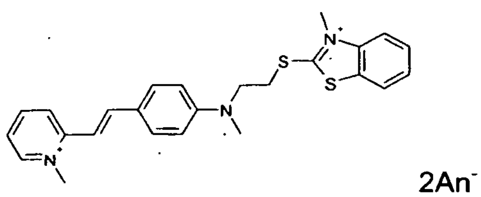 | 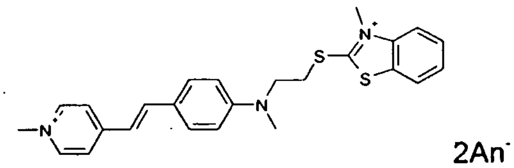 |
| **39** | **40** |
| 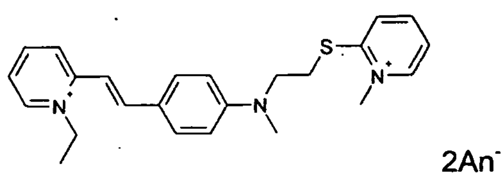 | 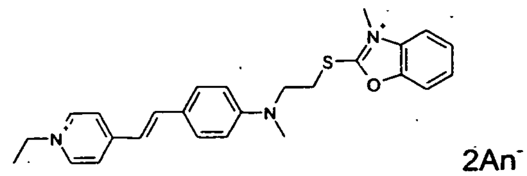 |
| **41** | **42** |
| 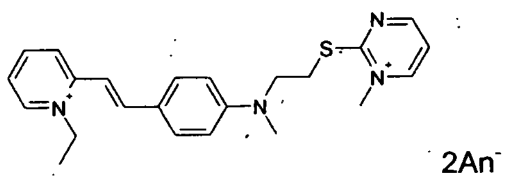 | 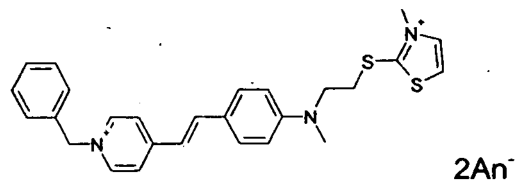 |
| **43** | **44** |
| 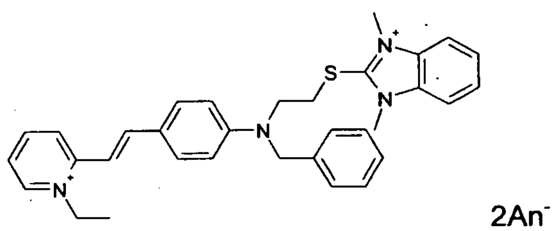 | 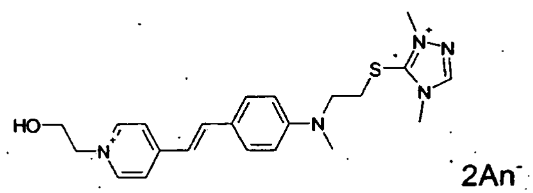 |
| **45** | **46** |
| 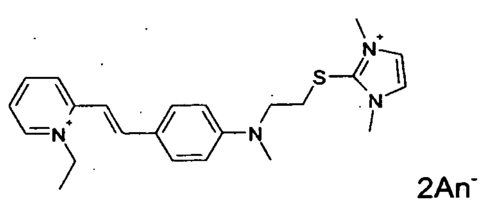 | 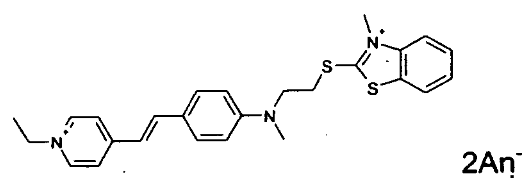 |
| **47** | **48** |

(suite)

| | |
|---|---|
| 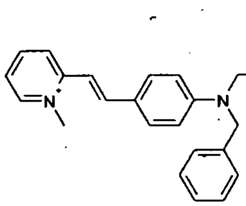 $2An^-$ | 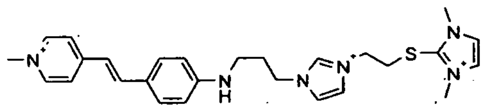 $3An^-$ |
| **49** | **50** |
| 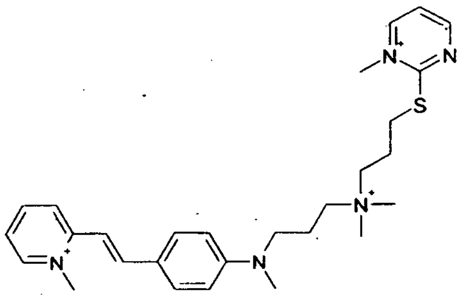 $3An^-$ | 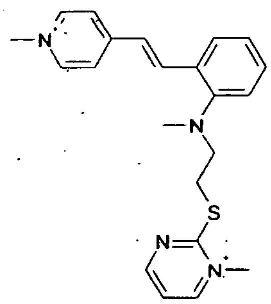 $2An^-$ |
| **51** | **52** |
| 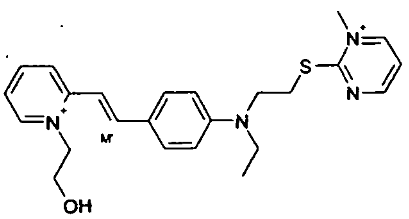 $2An^-$ | 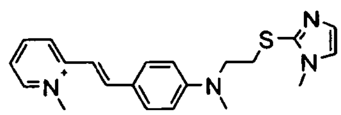 $An^-$ |
| **53** | **54** |
| 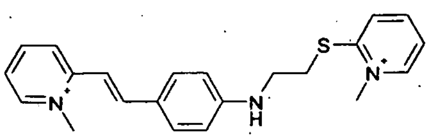 $2An^-$ | 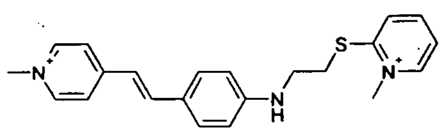 $2An^-$ |
| **55** | **56** |
| 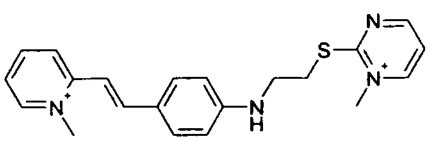 $2An^-$ | 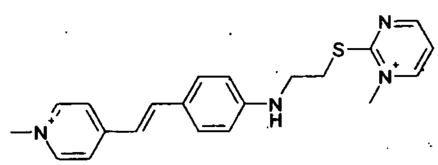 $2An^-$ |
| **57** | **58** |
| 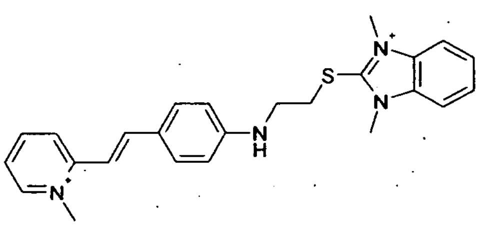 $2An^-$ | 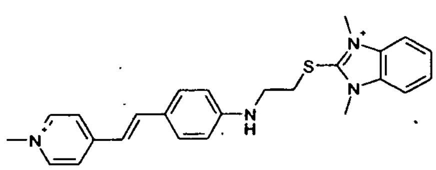 $2An^-$ |
| **59** | **60** |

(suite)

| | |
|---|---|
| 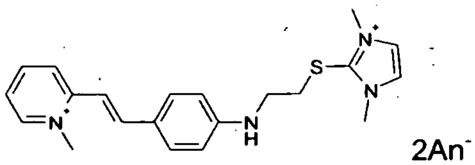 2An⁻ | 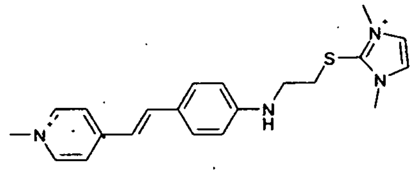 2An⁻ |
| **61** | **62** |
| 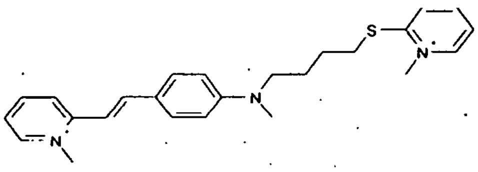 2An⁻ | 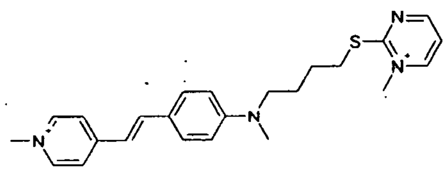 2An⁻ |
| **63** | **64** |
| 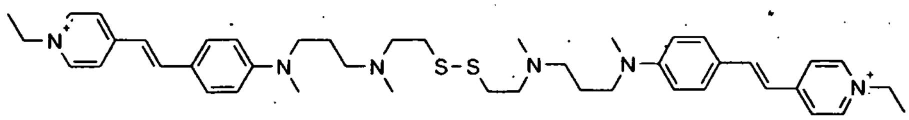 2M' | |
| **65** | |
| 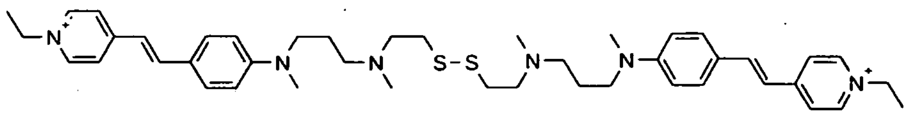 2M' | |
| **66** | |
| 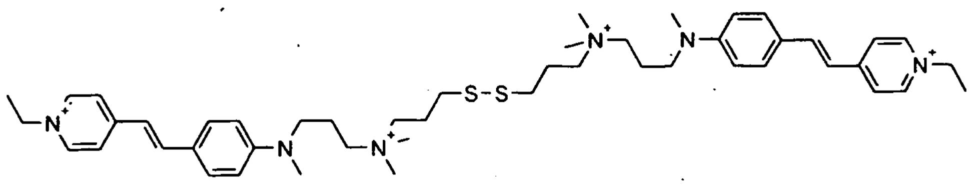 4M' | |
| **67** | |
| 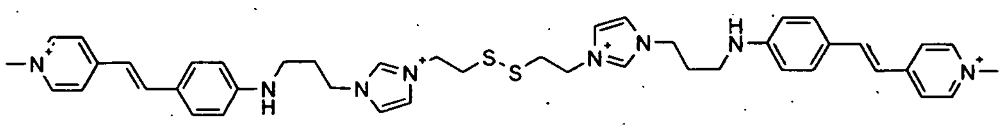 4M' | |
| **68** | |
| 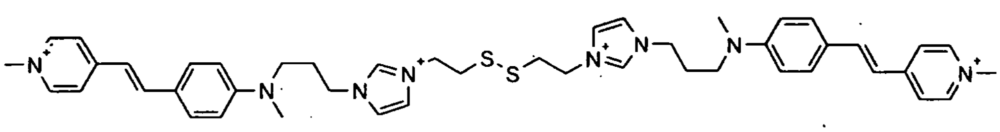 4M' | |
| **69** | |
| 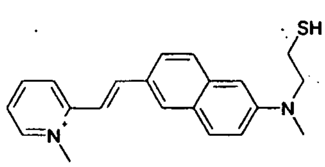 An⁻ | |
| **70** | |

(suite)

| |
|---|
| 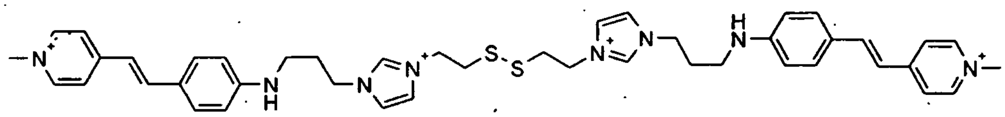 4M' |
| **71** |
| 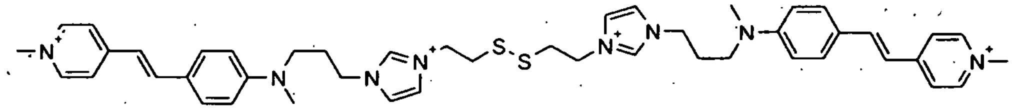 4M' |
| **72** |
| 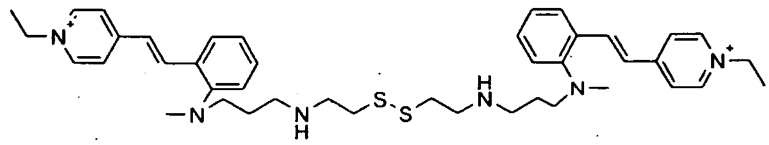 2M' |
| **73** |
| 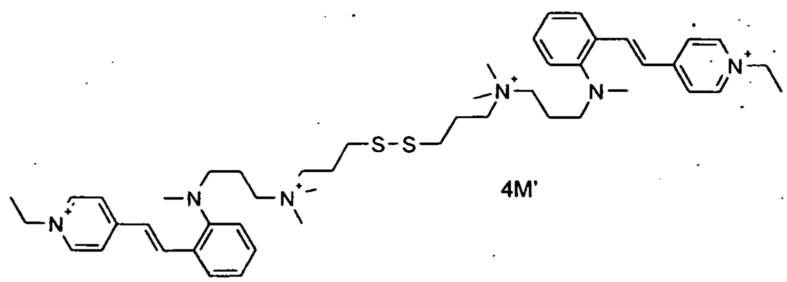 4M' |
| **74** |
| 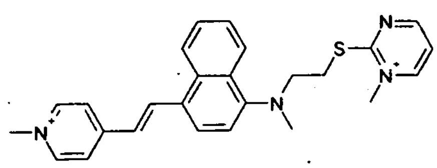 2An⁻ |
| **75** |
| 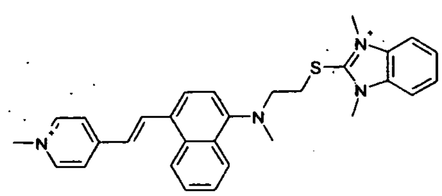 2An⁻ |
| **76** |
| 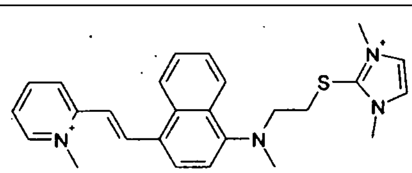 2An⁻ |
| **77** |

(suite)

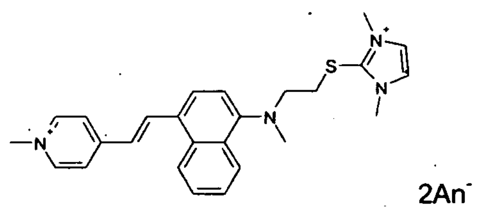

78

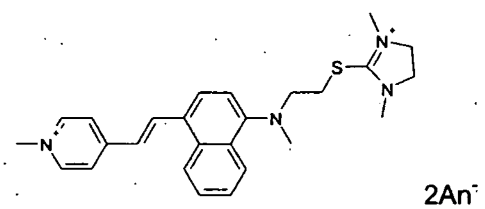

79

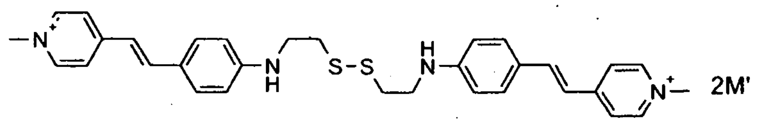

80

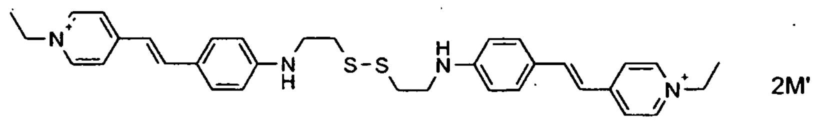

82

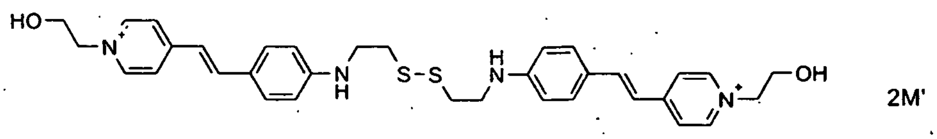

83

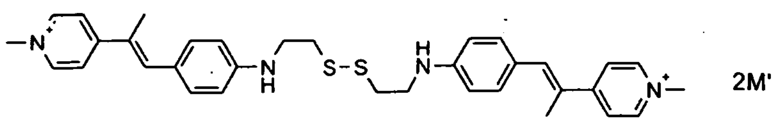

84

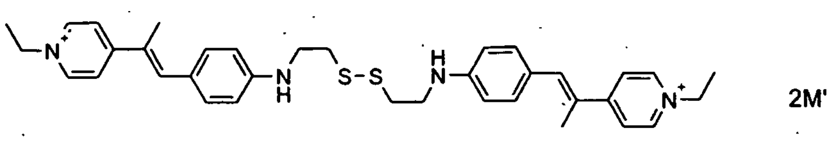

85

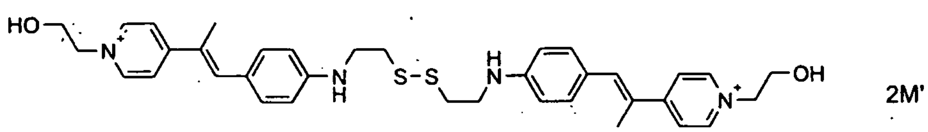

86

(suite)

| |
|---|
| 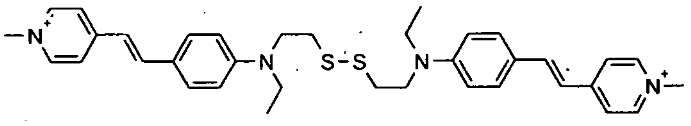 2M' |
| **87** |
| 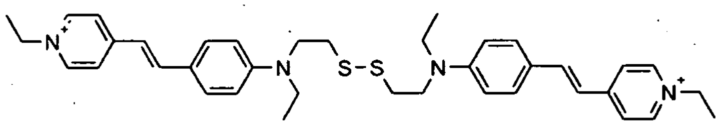 2M' |
| **88** |
| 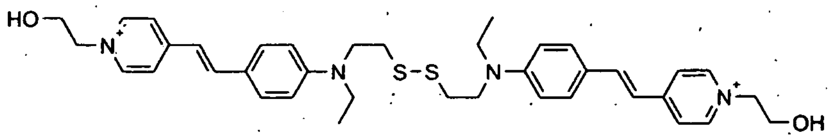 2M' |
| **89** |
| 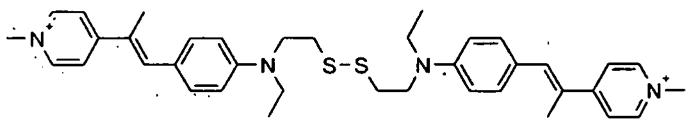 2M' |
| **90** |
| 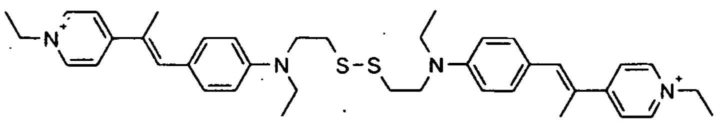 2M' |
| **91** |
| 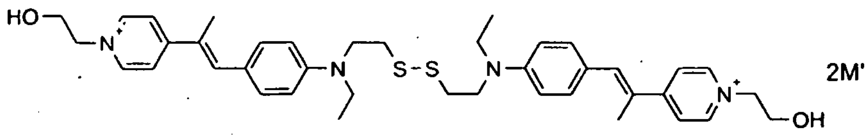 2M' |
| **92** |
| 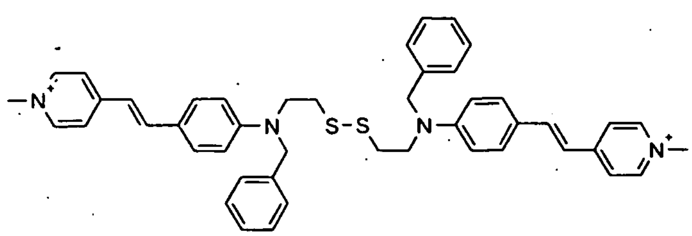 2M' |
| **93** |
| 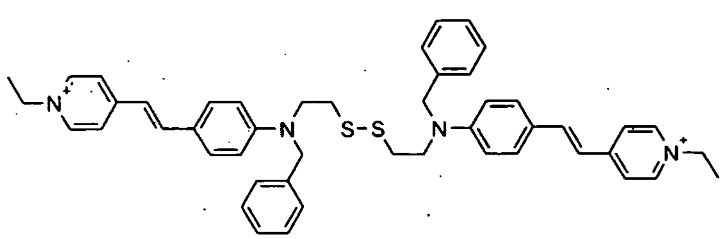 2M' |
| **94** |

(suite)

| |
|---|
| 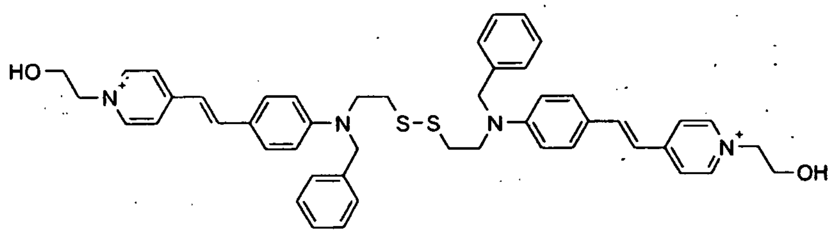 2M' |
| **95** |
| 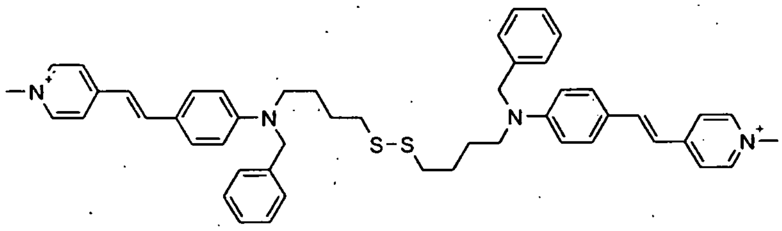 2M' |
| **96** |
| 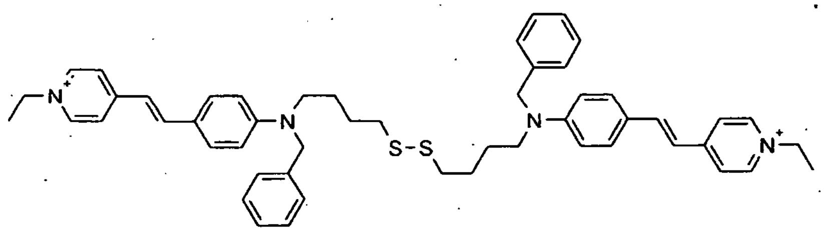 2M' |
| **97** |
| 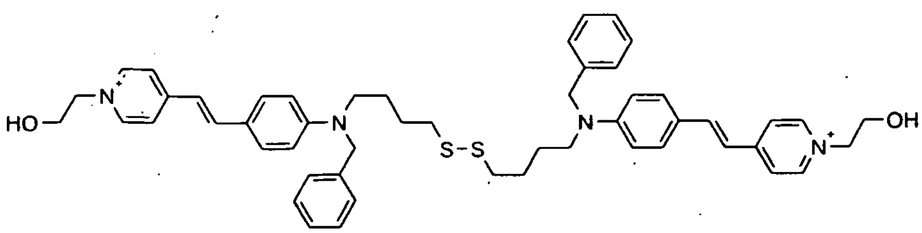 2M' |
| **98** |
| 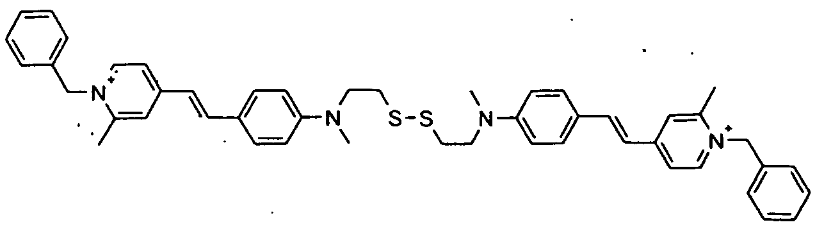 2M' |
| **99** |
| 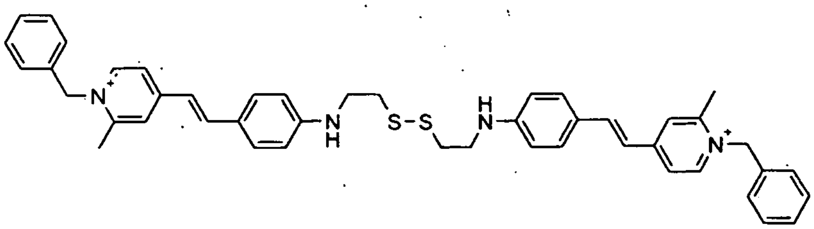 2M' |

(suite)

| |
|---|
| **100** |
| 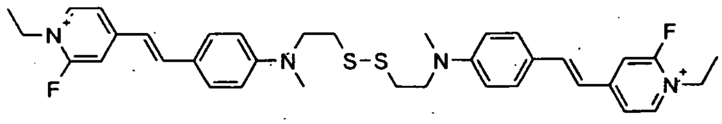 2M' |
| **101** |
| 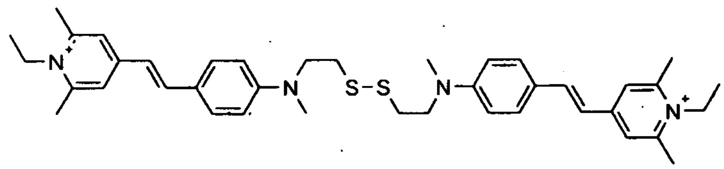 2M' |
| **102** |
| 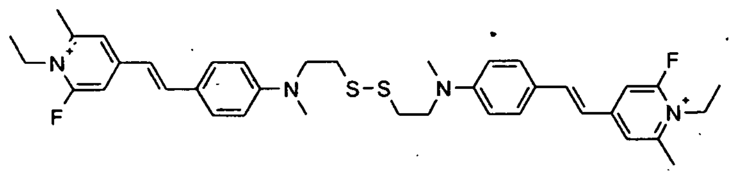 2M' |
| **103** |
| 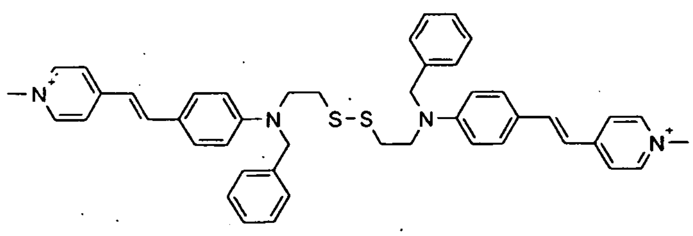 2M' |
| **104** |
| 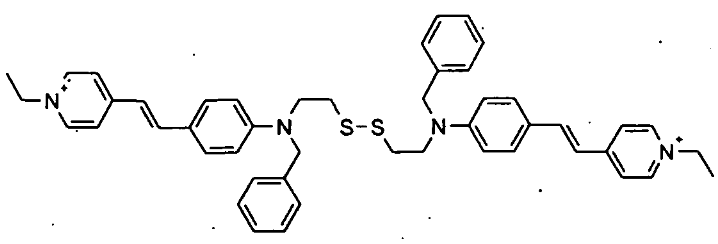 2M' |
| **105** |
| 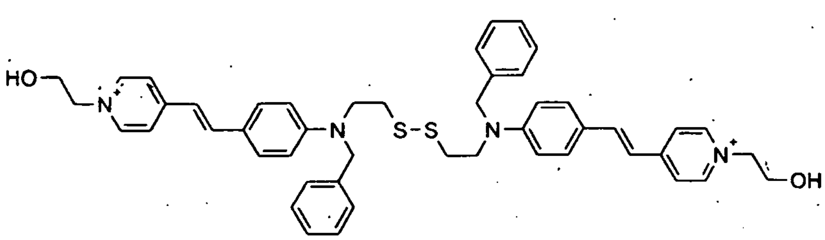 2M' |
| **106** |

(suite)

| |
|---|
| 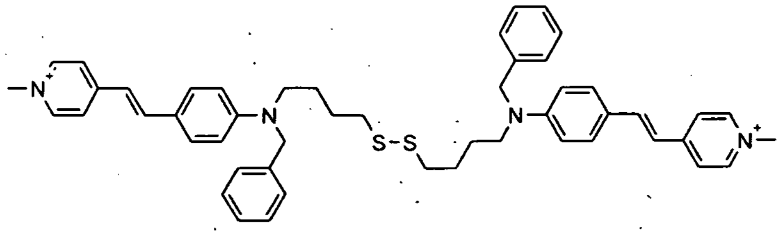 2M' |
| **107** |
| 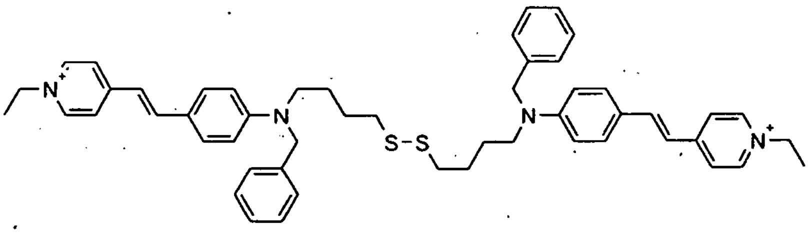 2M' |
| **108** |
| 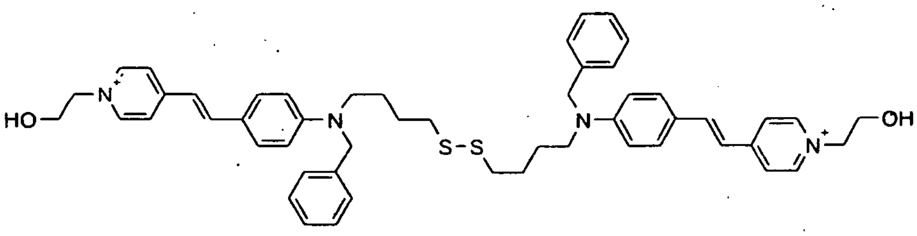 2M' |
| **109** |
| 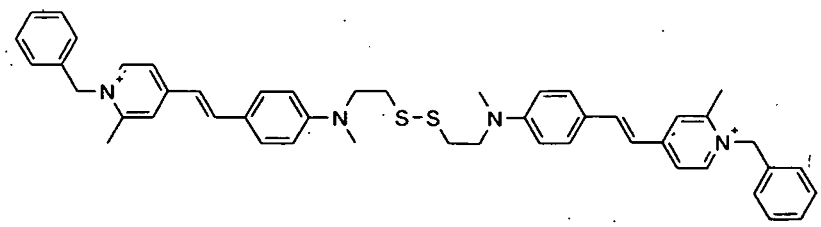 2M' |
| **110** |
| 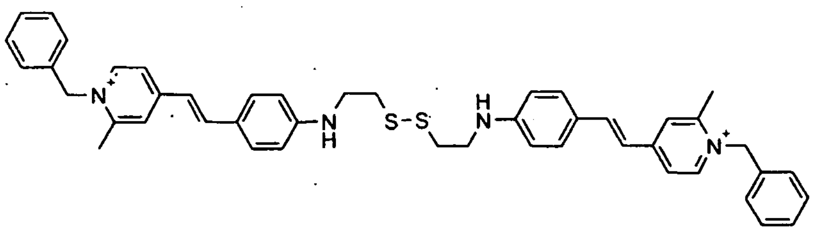 2M' |
| **111** |
| 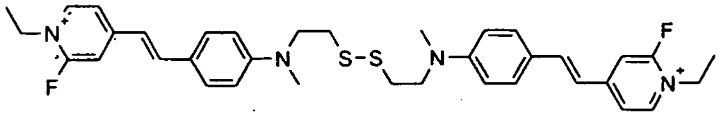 2M' |
| **112** |

(suite)

| |
|---|
| 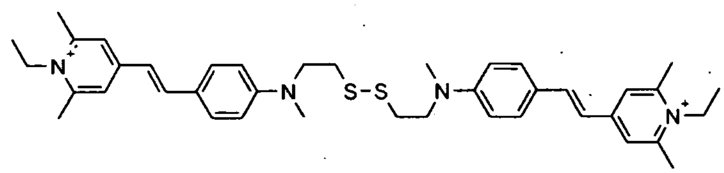 2M' |
| **113** |
| 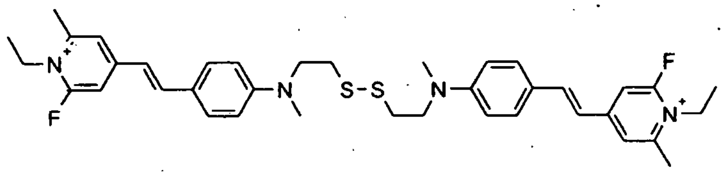 2M' |
| **114** |
| 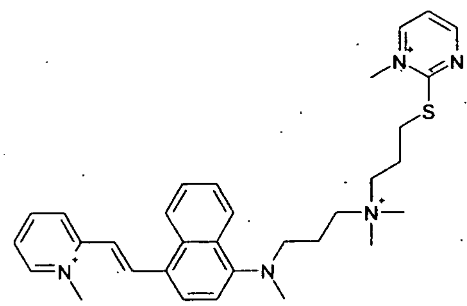 3An⁻ |
| **115** |
| 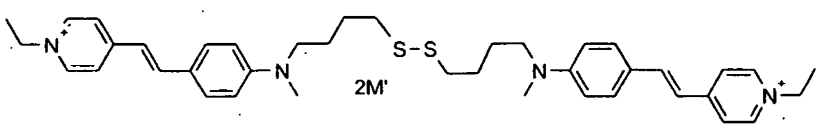 2M' |
| **116** |
| 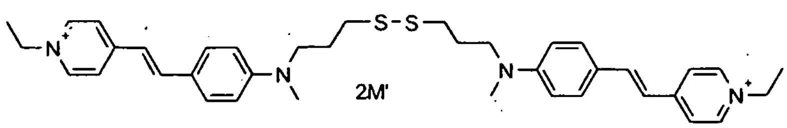 2M' |
| **117** |
| 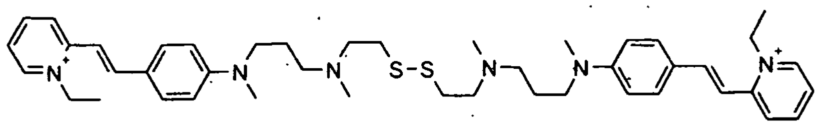 2M' |
| **118** |
| 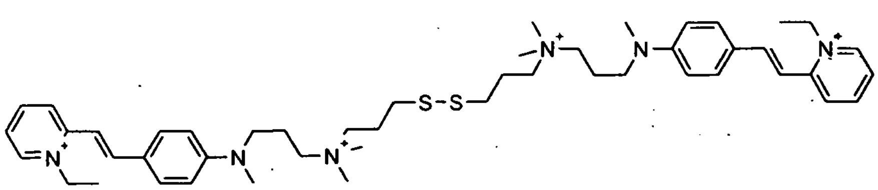 4M' |
| **119** |

(suite)

| |
|---|
| 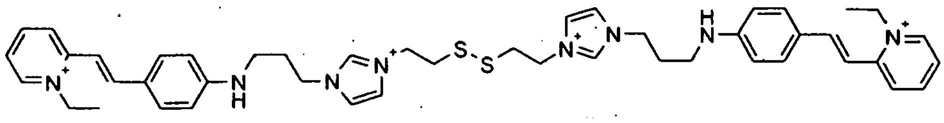 4M' |
| **120** |
| 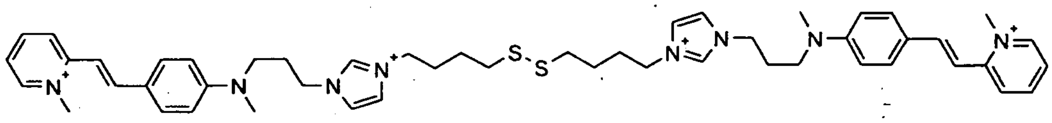 4M' |
| **121** |
| 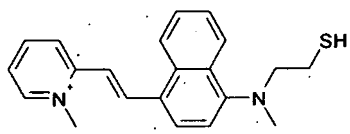 |
| **122** |
| 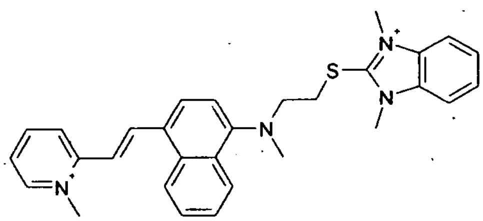 2M' |
| **123** |
| 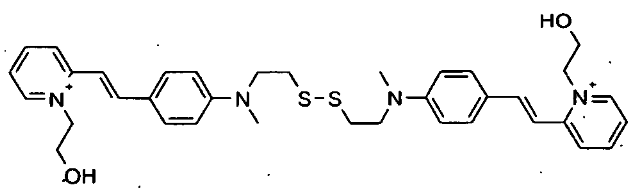 2M' |
| **124** |
| 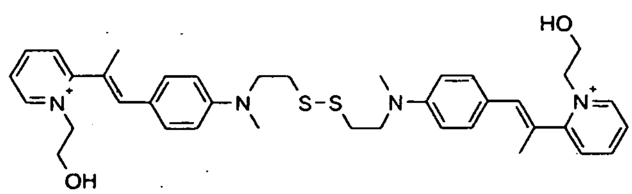 2M' |
| **125** |
| 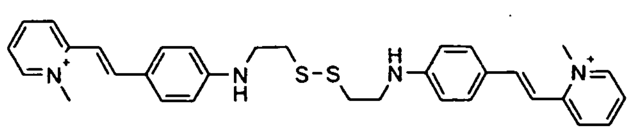 2M' |
| **126** |
| 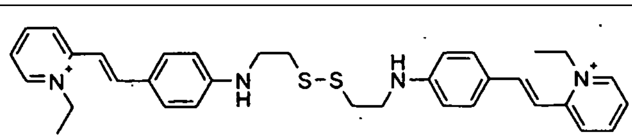 2M' |
| **127** |

(suite)

| |
|---|
| 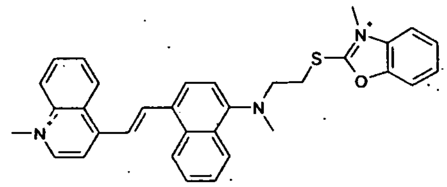 $2An^-$ |
| **128** |
| 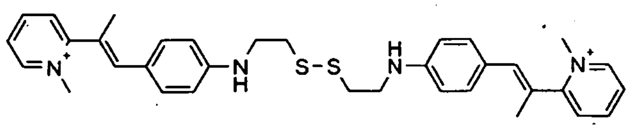 2M' |
| **129** |
| 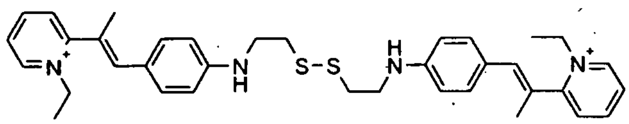 2M' |
| **130** |
| 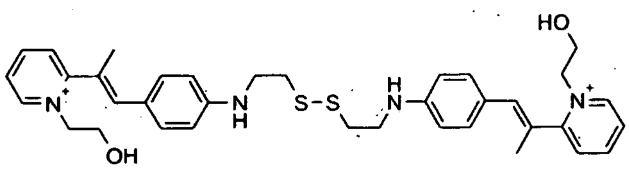 2M' |
| **131** |
| 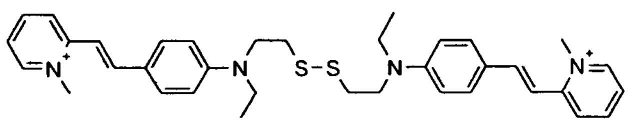 2M' |
| **132** |
| 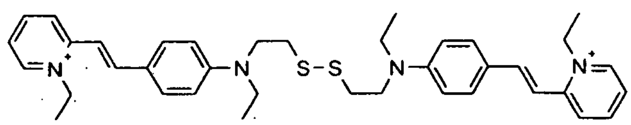 2M' |
| **133** |
| 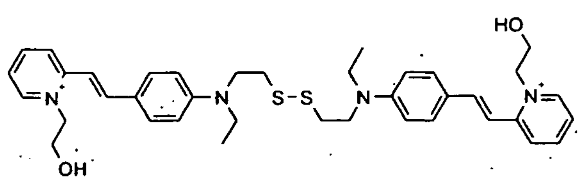 2M' |
| **134** |
| 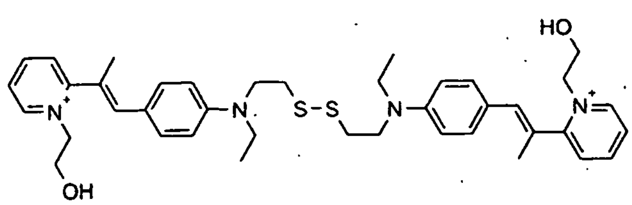 2M' |

(suite)

| 135 |
|---|
| 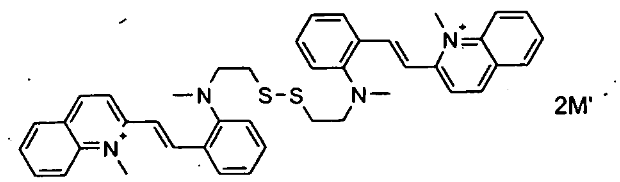 |
| 135 |
| 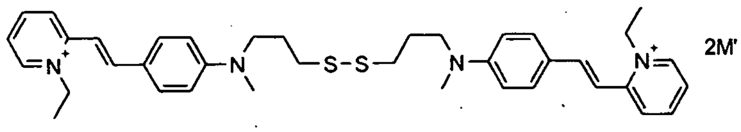 |
| 137 |
| 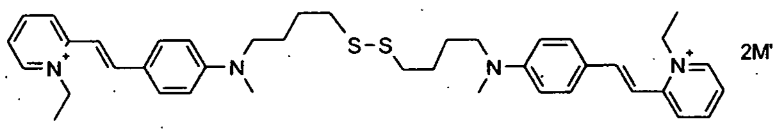 |
| 138 |
| 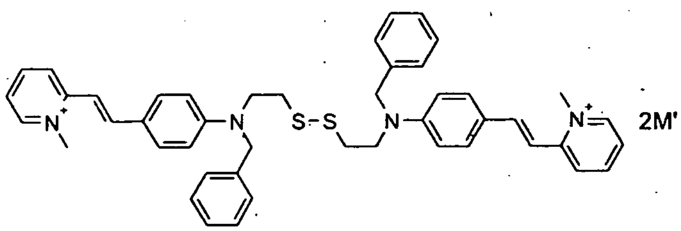 |
| 139 |
| 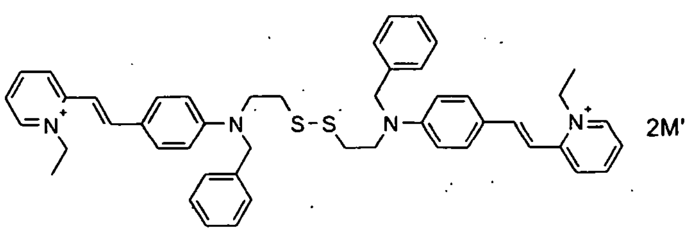 |
| 140 |
| 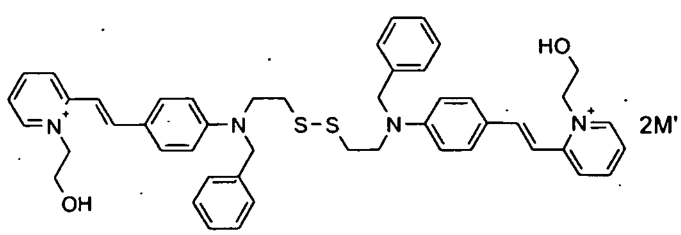 |
| 141 |

(suite)

| | |
|---|---|
| 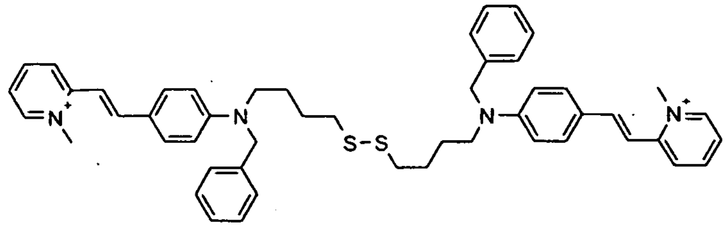 2M' | |
| **142** | |
| 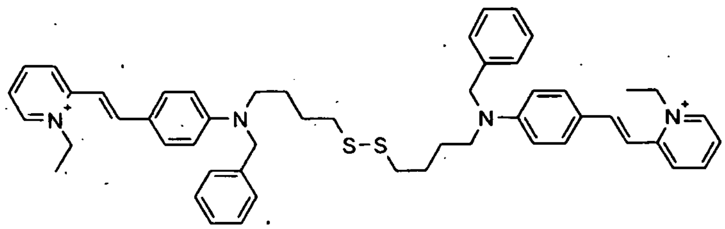 2M' | |
| **143** | |
| 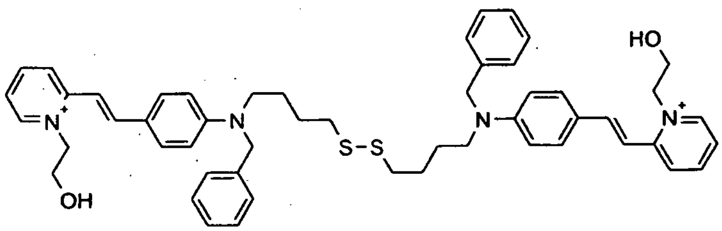 2M' | |
| **144** | |
| 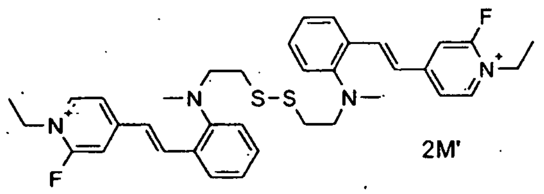 2M' | 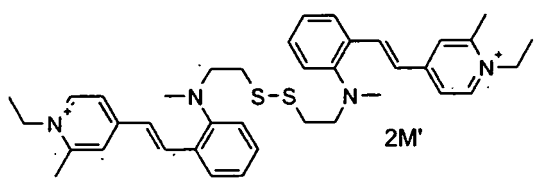 2M' |
| **145** | **146** |
| 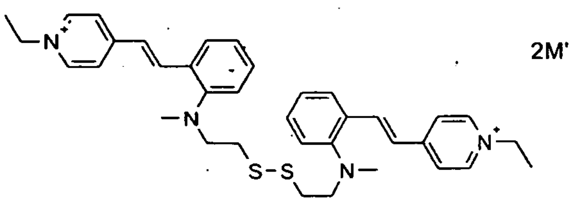 2M' | 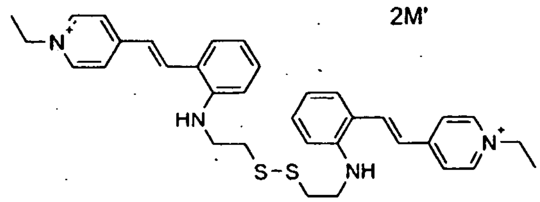 2M' |
| **147** | **148** |
| 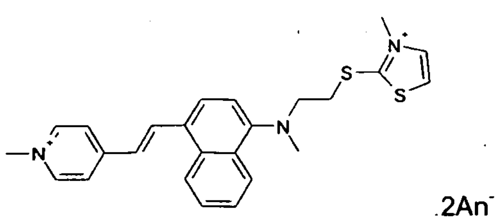 .2An⁻ | |
| **149** | |

avec An⁻, et M', identiques ou différents, représentant un contre-ion anionique ; et $M^+$ représentant un métal alcalin.

**9.** Composition tinctoriale comprenant dans un milieu cosmétique approprié, un colorant fluorescent de formule (I) ou (II) tel que défini dans une quelconque des revendications 1 à 8.

**10.** Composition tinctoriale comprenant dans un milieu cosmétique approprié, au moins un colorant fluorescent de formule (I) ou (II) tel que défini dans une quelconque des revendications 1 à 8 et au moins un agent réducteur.

**11.** Procédé de coloration de matières kératiniques, dans lequel on applique sur les matières, une composition tinctoriale, comprenant dans un milieu cosmétique approprié, au moins un colorant fluorescent de formule (I) ou (II) tel que défini dans une quelconque des revendications 1 à 8, éventuellement en présence d'un agent réducteur capable de réduire les liaisons disulfures des matières kératiniques.

**12.** Procédé de coloration de matières kératiniques selon la revendications 11 ou **caractérisé en ce que** les matières kératiniques sont des fibres kératiniques foncées.

**13.** Procédé selon les revendications 11 ou 12 comprenant une étape supplémentaire consistant à appliquer sur les fibres kératiniques un agent oxydant.

**14.** Procédé selon l'une quelconque des revendications 11 à 13, dans lequel le colorant fluorescent de formule (I) ou (II) est présent en quantité comprise entre 0,001 et 50% en poids par rapport au poids total de la composition.

**15.** Dispositif à plusieurs compartiments dans lequel un premier compartiment contient une composition tinctoriale comprenant un colorant fluorescent tel que défini aux revendications 1 à 8 et un deuxième compartiment contient un agent réducteur capable de réduire les liaisons disulfures des matières kératiniques.

**16.** Utilisation des colorants fluorescents de formule (I) ou (II) tels que définis aux revendications 1 à 8 pour l'éclaircissement des fibres kératiniques foncées.

**Claims**

**1.** Fluorescent dye of formula (I) or (II) below:

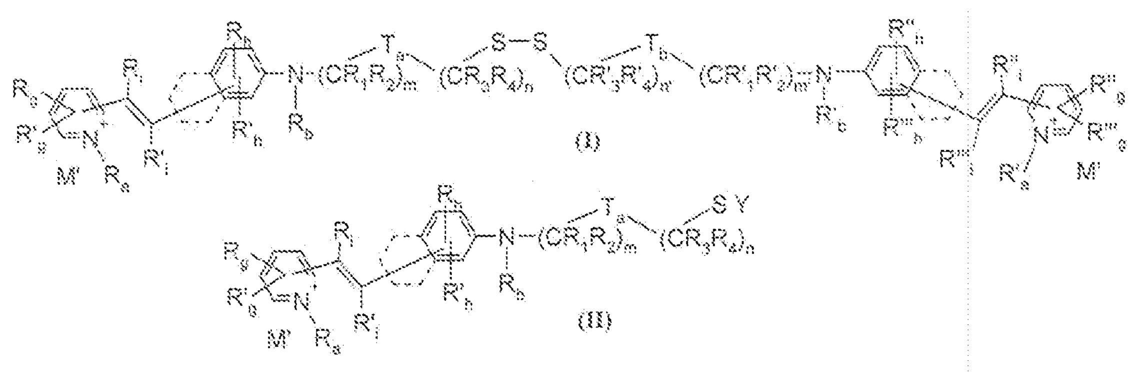

(I)

(II)

the organic or mineral acid salts, optical isomers and geometrical isomers thereof, and the solvates such as hydrates; in which formulae (I) and (II):

> ➢ $R_a$ and $R'_a$, which may be identical or different, represent an aryl ($C_1$-$C_4$)alkyl group or a ($C_1$-$C_6$) alkyl group optionally substituted with a hydroxyl, amino, $C_1$-$C_4$ alkylamino or ($C_1$-$C_4$) dialkylamino group, it being possible for said alkyl radicals to form, with the nitrogen atom which bears them, a heterocycle comprising from 5 to 7 members, optionally comprising another heteroatom which may or may not be different from nitrogen ;
> ➢ $R_b$ and $R'_b$, which may be identical or different, represent a hydrogen atom, an aryl ($C_1$-$C_4$) alkyl group or a ($C_1$-$C_6$) alkyl group which is optionally substituted;
> ➢ $R_g$, $R'_g$, $R''_g$ a $R'''_g$, which may be identical or different, represent a hydrogen atom, an amino, ($C_1$-$C_4$)

alkylamino, $(C_1-C_4)$dialkylamino or trifluoromethyl group, an acylamino, $(C_1-C_4)$alkoxy, $(C_1-C_4)$alkylcarbonyloxy, $(C_1-C_4)$alkoxycarbonyl, $(C_1-C_4)$alkylcarbonylamino or $(C_1-C_4)$alkylsulphonylamino radical, or a $(C_1-C_3)$ alkyl radical;

➢ $R_h$, $R'_h$, $R''_h$ and $R'''_h$, which may be identical or different, represent a hydrogen atom, a halogen atom, a $(C_1-C_4)$dialkylamino or $(C_1-C_4)$alkycarbonylamino group, an acylamino or $(C_1-C_4)$alkylsulphonylamino radical, or a $(C_1-C_4)$alkyl radical;

➢ or else two groups $R_g$ and $R'_g$, $R''_g$ and $R'''_g$; $R_h$ and $R'_h$, $R''_h$ and $R'''_h$, borne by two adjacent carbon atoms, together form a benzo or indeno ring, or a fused heterocycloalkyl or fused heteroaryl group; the benzo, indeno, heterocycloalkyl or heteroaryl ring being optionally substituted with a halogen atom, an ami$(C_1-C_4)$alkylamino, $(C_1-C_4)$dialkylamino, cyano, carboxyl, hydroxyl, trifluoromethyl, acylamino, $C_1-C_4$ alkoxy, $C_2-C_4$(poly)hydroxyalkoxy, $(C_1-C_4)$alkylcarbonyloxy, $(C_1-C_4)$alkoxycarbonyl or $(C_1-C_4)$alkylcarbonylamino group, an acylamino, carbamoyl or $(C_1-C_4)$alkylsulphonylamino radical, an aminosulphonyl radical, or a $(C_1-C_{16})$alkyl radical optionally substituted with a group chosen, from $(C_1-C_{12})$alkoxy, hydroxyl, cyano, carboxyl, amino, $(C_1-C_4)$alkylamino and $(C_1-C_4)$dialkylamino, or else the two alkyl radicals borne by the nitrogen atom of the amino group form a heterocycle comprising from 5 to 7 members and optionally comprising another heteroatom identical to or different from that of the nitrogen atom;

➢ $R_i$, $R'_i$, $R''_i$ and $R'''_i$, which may be identical or different, represent a hydrogen atom or a $(C_1-C_4)$alkyl group;

➢ $R_1$, $R_2$, $R_3$, $R_4$, $R'_1$, $R'_2$, $R'_3$ and $R'_4$, which may be identical or different, represent a hydrogen atom or a $(C_1-C_4)$alkyl group;

➢

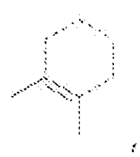

which may be present or absent, represents a benzo group;

➢ $T_a$ and $T_b$, which may be identical or different, represent:

i) either a σ covalent bond;
ii) or a radical chosen from -N(R)- and $-N^+(R)(R^o)-$, with R and $R^o$, which may be identical or different, representing a hydrogen atom, a $C_1-C_4$ alkyl or $C_1-C_4$ hydroxyalkyl radical, or an aryl$(C_1-C_4)$alkyl,
iii) or a cationic or noncationic, heterocycloalkyl or heteroaryl radical;

➢ m, m', n and n', which may be identical or different, represent an integer between 0 and 6 inclusive, with m+n and m'+n', which may be identical or different, representing an integer between 1 and 10 inclusive;

➢ when $T_a$ represents a σ covalent bond, Y represents a group chosen from:

o optionally substituted aryl;
o optionally substituted heteroaryl;
o optionally substituted heterocycloalkyl;
o optionally substituted (di)arylalkyl;
o optionally substituted (di)heteroarylalkyl;
o a sterically hindered cyclic group;

➢ when $T_a$ represents -N(R)-, -N'(R)($R^o$)-, or a cationic or noncationic, heterocycloalkyl or heteroaryl radical; Y represents a group chosen from i) a hydrogen atom; ii) an alkali metal; iii) an alkaline earth metal; iv) an ammonium group: $N^+R^{\alpha}R^{\beta}R^{\gamma}R^{\delta}$ or a phosphonium group: $P^+R^{\alpha}R^{\beta}R^{\gamma}R^{\delta}$ with $R^{\alpha}$, $R^{\beta}$, $R^{\gamma}$ and $R^{\delta}$, which may be identical or different, representing a hydrogen atom or a $(C_1-C_4)$alkyl group; or v) a thiol-function-protecting group;

and

➢ M' representing an anionic counterion;

it being understood that, when the compound of formula (I) or (II) contains other cationic parts, it is associated with one or more anionic counterions allowing formula (I) or (II) to achieve electroneutrality.

**2.** Fluorescent dye of formula (II) according to Claim 1, in which, when $T_a$ represents -N(R)-, $-N^+(R)$ $(R^o)-$, or a cationic or noncationic heterocycloalkyl or heteroaryl radical, Y represents a hydrogen atom or an alkali metal.

**3.** Fluorescent dye of formula (II) according to Claim 1, in which, when $T_a$ represents -N(R)-, -N+(R) (Ro)-, or a cationic or noncationic heterocycloalkyl or heteroaryl radical, Y represents a thiol-function-protecting group below:

➢ $(C_1-C_4)$alkylcarbonyl;
➢ $(C_1-C_4)$alkylthiocarbonyl;
➢ $(C_1-C_4)$alkoxycarbonyl;
➢ $(C_1-C_4)$alkoxythiocarbonyl;
➢ $(C_1-C_4)$alkylthiothiocarbonyl;
➢ (di)$(C_1-C_4)$(alkyl)aminocarbonyl;
➢ (di)$(C_1-C_4)$(alkyl)aminothiocarbonyl;
➢ arylcarbonyl such as phenylcarbonyl;
➢ aryloxycarbonyl;
➢ aryl$(C_1-C_4)$alkoxycarbonyl;
➢ (di)$(C_1-C_4)$(alkyl)aminocarbonyl;
➢ $(C_1-C_4)$(alkyl)arylaminocarbonyl;
➢ carboxyl;
➢ $SO_3^-$; $M^+$ with $M^+$ representing an alkali metal or else M' of formula (II) and $M^+$ are absent;
➢ optionally substitituted acryl;
➢ optionally substituted heteroaryl;
➢ optionally substituted heterocycloalkyl;
➢ isothiouronium $-C(NR'^cR'^d)=N^+R'^eR'^f$; An with $R'^c$, $R'^d$, $R'^e$ and $R'^f$, which may be identical or different, representing a hydrogen atom or an alkyl group;
➢ isothiourea $-C(NR'^cR'^d)=NR'^e$; with $R'^c$, $R'^d$ and $R'^e$ as defined above;
➢ optionally substituted (di)arylalkyl;
➢ optionally substituted (di)heteroarylalkyl;
➢ $CR'^1R'^2R'^3$ with $R'^1$, $R'^2$ and $R'^3$, which may be identical or different, representing a halogen atom or a group chosen from:

■ optionally substituted alkyl;
■ optionally substituted alkoxy;
■ optionally substituted aryl;
■ optionally substituted heteroaryl;
■ $P(Z^1)\ R''^1R''^2R''^3$ with $R''^1$ and $R''^2$, which may be identical or different, representing a hydroxyl, alkoxy or alkyl group, $R''^3$ representing a hydroxy or alkoxy group and $Z^1$ representing an oxygen or sulphur atom;

➢ a sterically hindered cyclic group; and
➢ optionally substituted alkoxyalkyl.

**4.** Fluorescent dye of formula (II) according to either one of Claims 1 and 3, in which, when $T_a$ represents -N(R)-, -N+(R)(Ro)-, or a cationic or noncationic heterocycloalkyl or heteroaryl radical; Y represents a protecting group chosen from:

➢ $(C_1-C_4)$alkylcarbonyl;
➢ arylcarbonyl;
➢ $(C_1-C_4)$alkoxycarbonyl;
➢ aryloxycarbonyl;
➢ aryl$(C_1-C_4)$alkoxycarbonyl;
➢ (di)$(C_1-C_4)$(alkyl)aminocarbonyl;
➢ $(C_1-C_4)$(alkyl)arylaminocarbonyl;
➢ optionally substituted aryl;
➢ 5- or 6-membered monocyclic heteroaryl;
➢ 8- to 11-membered cationic bicyclic heteroaryl;
➢ cationic heterocycle of formula below:

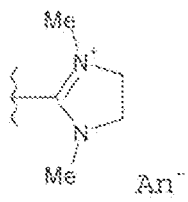

- ➢ isothiouronium $-C(NH_2)=N^+H_2$; $An^-$;
- ➢ isothiourea $-C(NH_2)=NH$; and
- ➢ $SO_3^-$, $M^+$ with $M^+$ representing an alkali metal or else M' of formula (II) and $M^+$ are absent.

**5.** Fluorescent dye of formula (II) according to Claim 1, in which, when $T_a$ represents a bond, Y represents a i) cationic, aromatic, 5- or 6-membered monocyclic heteroaryl comprising from 1 to 4 heteroatoms chosen from oxygen, sulphur and nitrogen; ii) a cationic 8- to 11-membered bicyclic heteroaryl, these monocyclic or bicyclic groups being optionally substituted with one or more groups; iii) or the heterocyclic group below:

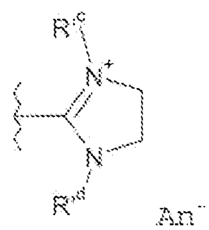

in which $R'^c$ and $R'^d$, which may be identical or different, represent a hydrogen atom or an alkyl group; and An represents the counterion.

**6.** Fluorescent dye according to any one of the preceding claims, chosen from the fluorescent dyes of formula **(Ia), (IIa)) , (Ib), (IIb) (Ic)** or **(IIc),** each having an ethylene group which links the pyridinium part to the phenyl in ortho- or para-position, in the 4-4', 4.-2', or 2-4' positions for **(Ia), (IIa), (Ib)** or **(IIb),** or else in the 4-5' or 2-5' positions for (Ic) or **(IIc):**

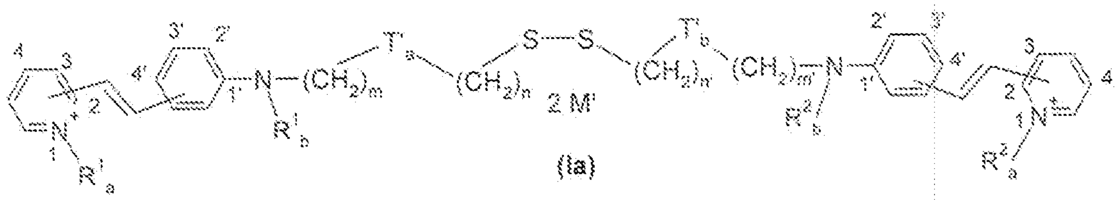

(Ia)

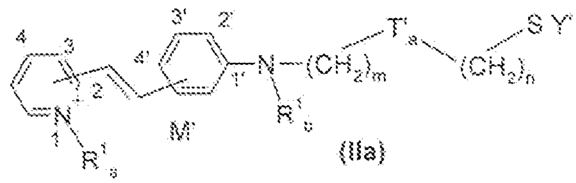

(IIa)

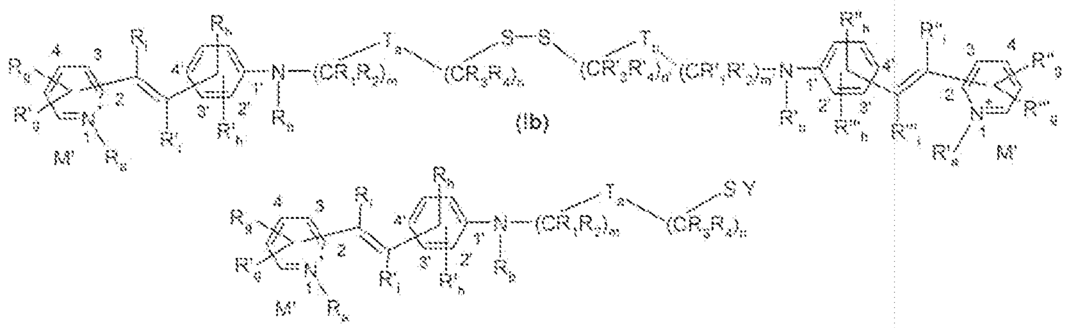

(Ib)

(IIb)

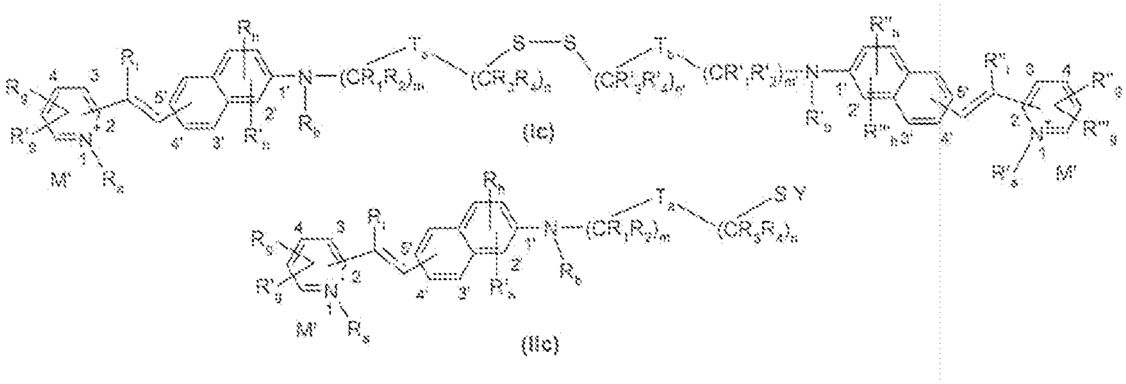

(Ic)

(IIc)

in which formula **(Ia)** or **(IIa):**

- $R^1a$ and $R^2a$, which ay be identical or different, represent a benzyl or alkyl group optionally substituted with a hydroxy group;
- ■ $R^1b$ and $R^2b$, which may be identical or different, represent a hydrogen atom; of a benzyl or alkyl group ;
- ■ T' a and T'b, which may be identical or different, represent a σ bond; or a $N^+(R)(R^o)$ - group with R and $R^o$, which may be identical or different, representing an alkyl group; or else $T'_a$ and $T'_b$ represent an imidazolium group;
- ■ Y' represents a group chosen from imidazolium, thiazolium; oxazolium; 1, 2, 4-triazolium; yridinium; pyrimidinium; benzoxazolium and benzothiazolium; these groups being substituted with one or more alkyl groups, which may be identical or different;
- ■ m, n, m, and n', which may be identical or m, n, m, and ', which may be identical or different, represent an integer between 1 and 6 inclusive, with m+n=m'+n' representing an integer between 2 and 6 inclusive;
- M' represents an anionic counterion;

in which formula **(Ib)** or **(IIB);**

➢ $R_a$, $R'_a$, $R_b$, $R'_b$, $R_1$, $R'_1$, $R''_1$, $R'''_1$, $R_1$, $R'_1$, $R_2$, $R'_2$, $R_3$, $R'_3$, $R_4$, $R'_4$, $T_a$, $T_b$, m, m', n, n' and Y are as defined in any one of the preceding claims;

➢ $R_g$ and $R'_g$; $R''_g$ and $R'''_g$, which may be identical or different, represent a hydrogen atom, a halogen atom, an amino, ($C_1$-$C_4$)alkylamino, ($C_1$-$C_4$)dialkylamino, cyano, carboxyl, hydroxyl, trifluoromethyl, acylamino, $C_1$-$C_4$ alkoxy, $C_2$-$C_4$(poly)hydroxyalkoxy, ($C_1$-$C_4$)alkylcarbonyloxy, ($C_1$-$C_4$)alkoxycarbonyl, ($C_1$-$C_4$)alkylcarbonylamino, acylamino, carbamoyl, ($C_1$-$C_4$)alkylsulphonylamino or aminosulphonyl group, or a ($C_1$-$C_{16}$)alkyl radical, optionally substituted with a group chosen from ($C_1$-$C_{12}$)alkoxy, hydroxy, cyano, carboxyl, amino, ($C_1$-$C_4$) alkylamino and ($C_1$-$C_4$)dialkylamino, or else the two alkyl radicals borne by the nitrogen atom of the amino group form a heterocycle comprising from 5 to 7 members and optionally comprising another hereroatom identical to or different from that of the nitrogen atom;

➢ $R_h$ and $R'_h$; $R''_h$ and $R'''_h$ borne by two adjacent carbon atoms, together form a benzo or indeno ring, or a fused heterocycloalkyl or fused heteroaryl group; the benzo, indeno, heterocycloalkyl or heteroaryl ring being optionally substituted with a halogen, atom, an amino, ($C_1$-$C_4$)alkylamino, ($C_1$-$C_4$)dialkylamino, cyano, carboxyl, hydroxyl, trifluoromethyl, acylamino, $C_1$-$C_4$ alkoxy, $C_2$-$C_4$(poly)hydroxyalkoxy, ($C_1$-$C_4$)alkylcarbonyloxy, ($C_1$-$C_4$) alkoxycarbonyl or ($C_1$-$C_4$)alkylcarbonylamino group, an acylamino, carbamoyl or ($C_1$-$C_4$)alkylsulphonylamino radical, an aminosulphonyl radical, or a ($C_1$-$C_{16}$)alkyl radical optionally substituted with a group chosen from ($C_1$-$C_{12}$)alkoxy, hydroxyl, cyano, carboxyl, amino, ($C_1$-$C_4$)alkylamino and ($C_1$-$C_4$)dialkylamino, or else the two alkyl radicals borne by the nitrogen atom of the amino group form a heterocycle comprising from 5 to 7 members and optionally comprising another heteroatom identical to or different from that of the nitrogen atom; and

in which formula (Ic) or (IIc) :

➢ $R_a$, $R'_a$, $R_b$, $R'_b$, $R_i$, $R''_i$, $R_1$, $R'_1$, $R_2$, $R'_2$, $R_2$, $R'_3$, $R_4$, $R'_4$, $T_a$, $T_b$, m, m, n, n' and Y are as defined in any one of the preceding claims;

➢ $R_g$, $R'_g$, $R''_g$, $R'''_g$, $R_h$, $R'_h$, $R''_b$ and $R'''_h$, which may be identical or different, represent a hydrogen tom, a halogen atom, an amino, ($C_1$-$C_4$)alkylamino, ($C_1$-$C_4$)dialkylamino, cyano, carboxyl, hydroxyl, trifluoromethyl, acylamino, $C_1$-$C_4$ alkoxy, $C_2$-$C_4$(poly)hydroxyalkoxy, ($C_1$-$C_4$alkylcarbonyloxy, ($C_1$-$C_4$)alkoxycarbonyl, ($C_1$-$C_4$) alkylcarbonylamino, acylamino, carbamocyl, ($C_1$-$C_4$)alkylsulphonylamino or aminozulphonyl group, or a ($C_1$-$C_{16}$)alkyl radical optionally substituted with a group chosen from ($C_1$-$C_{12}$) alkoxy, hydroxyl, cyano, carboxyl, amino, ($C_1$-$C_4$) alkylamino and ($C_1$-$C_4$)dialkylamino, or else the two alkyl radicals borne by the nitrogen atom of the amino group form a he-terocycle comprising from 5 to 7 members and optionally comprising another heteroatom identical to or different from that of the nitrogen atom ;

it being understood that, when the compounds of formula **(Ia), (IIa) , (Ib), (IIb), (Ic)** or **(IIc)** contain other cationic parts, they y are associated with one or more anionic cc-unterions allowing formula **(Ia), (IIa), (Ib), (IIb), (Ic)** or **(IIc)** to achieve electroneutrality.

**7.** Fluorescent dye of formula **(I)** according to one of Claims 1 or 6, which is symmetrical.

**8.** Fluorescent dye according to any one of the preceding claims, chosen from the following dyes:

<table>
<tr><td>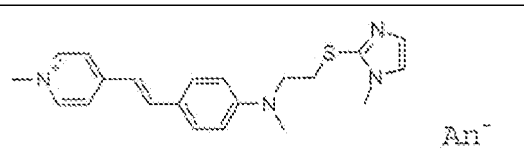</td><td>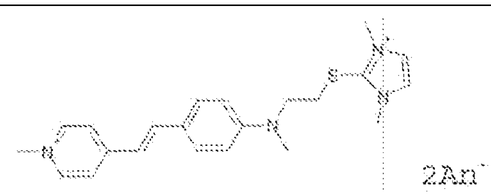</td></tr>
<tr><td>1</td><td>2</td></tr>
<tr><td colspan="2">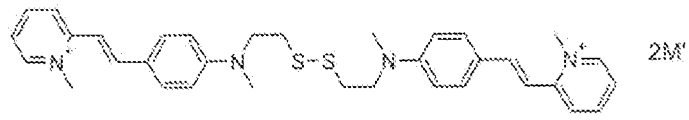</td></tr>
<tr><td colspan="2">3</td></tr>
</table>

(continued)

| |
|---|
| 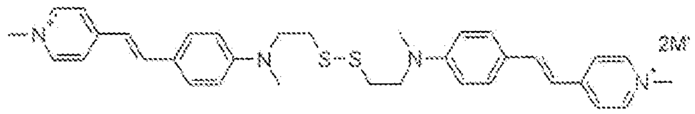 |
| **4** |
| 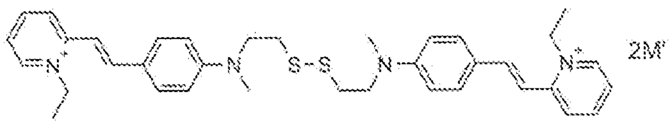 |
| **5** |
| 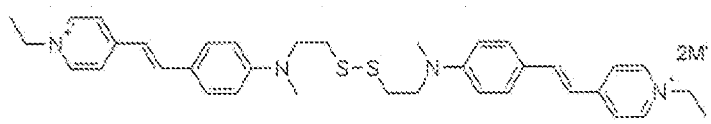 |
| **6** |
| 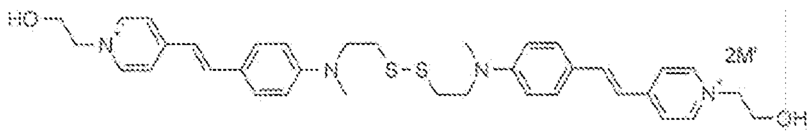 |
| **7** |
| 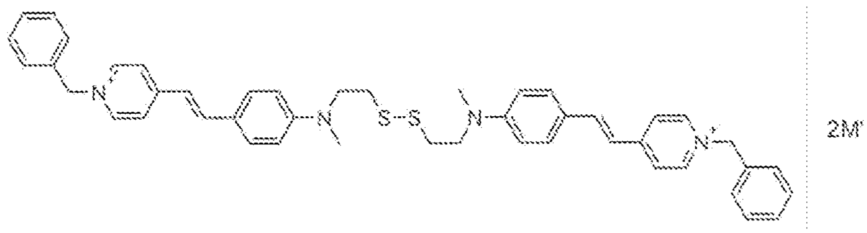 |
| **8** |
| 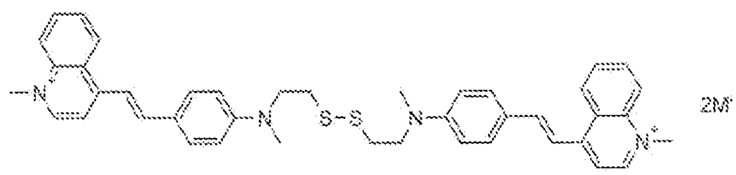 |
| **9** |
| 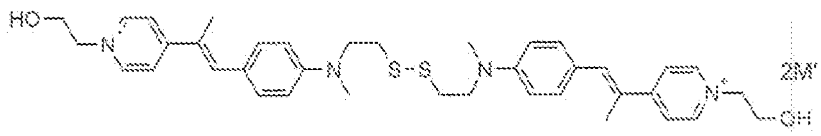 |
| **10** |
| 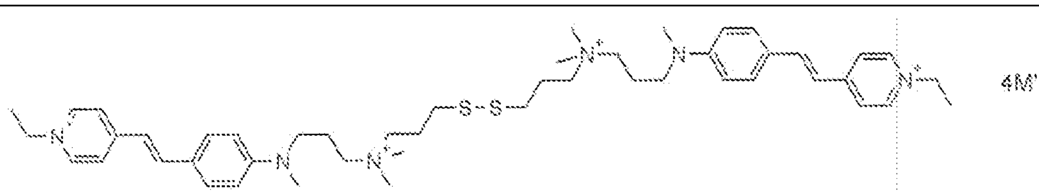 |
| **11** |

(continued)

| | |
|---|---|
| 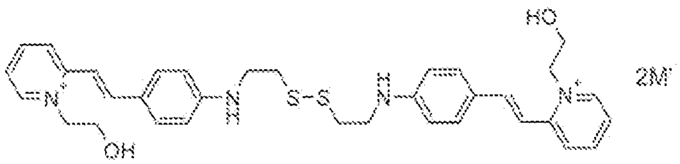 | |
| 12 | |
| 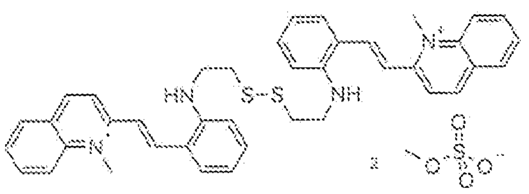 | 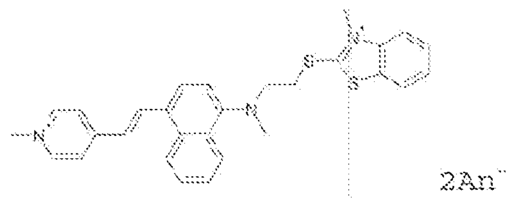 |
| 13 | 14 |
| 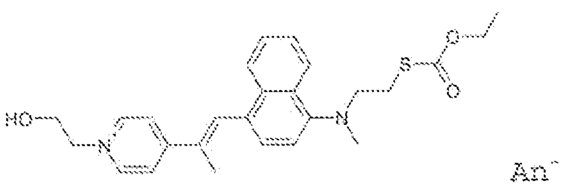 | 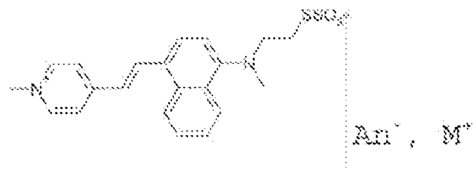 |
| 15 | 16 |
| 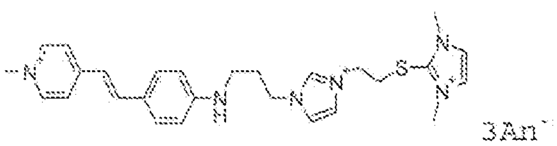 | 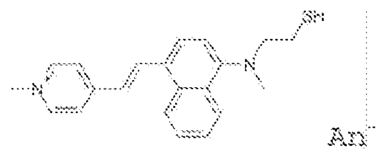 |
| 17 | 18 |
| 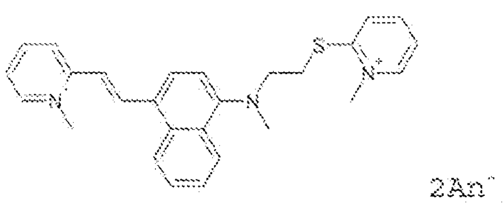 | 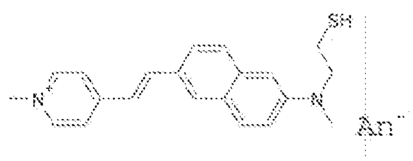 |
| 19 | 20 |
| 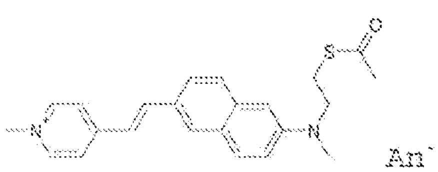 | 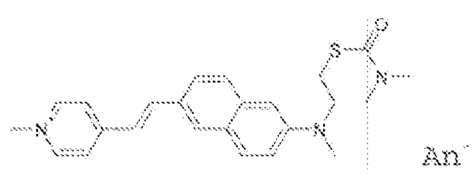 |
| 21 | 22 |
| 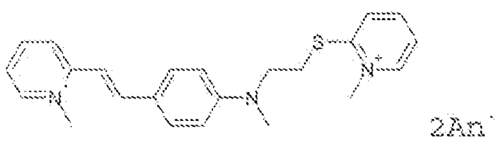 | 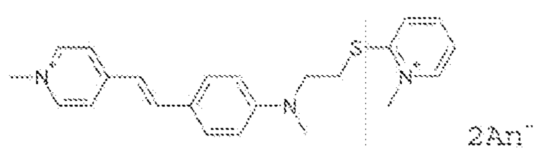 |
| 23 | 24 |

(continued)

| | |
|---|---|
| 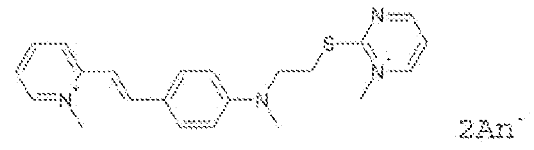 | 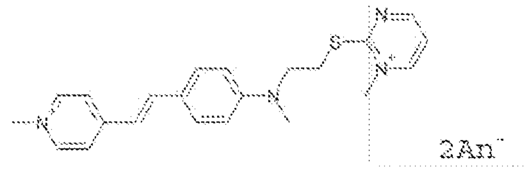 |
| **25** | **26** |
| 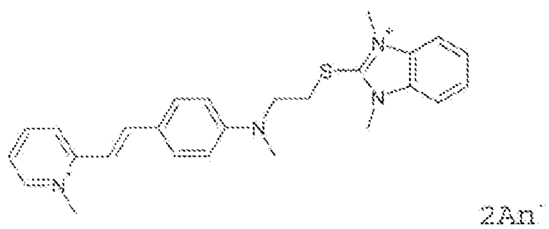 | 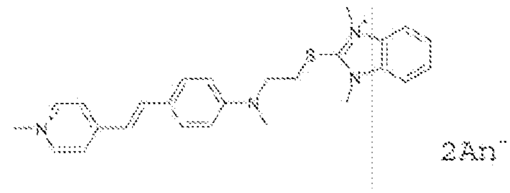 |
| **27** | **28** |
| 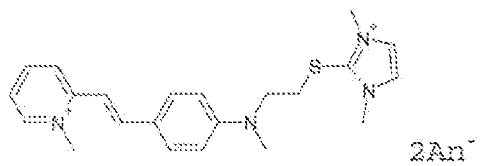 | 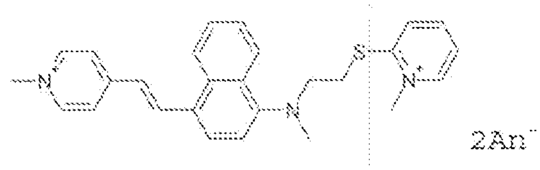 |
| **29** | **30** |
| 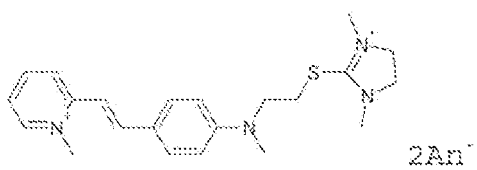 | 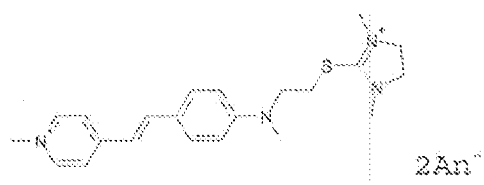 |
| **31** | **32** |
| 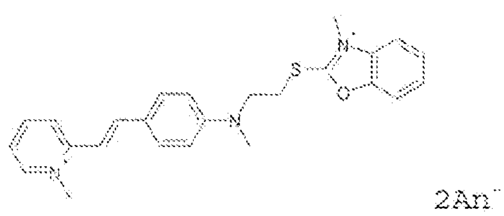 | 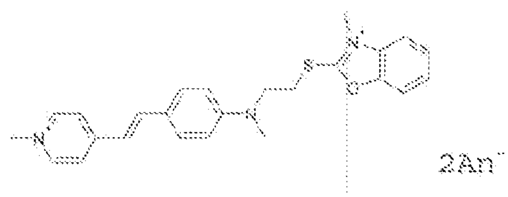 |
| **33** | **34** |
| 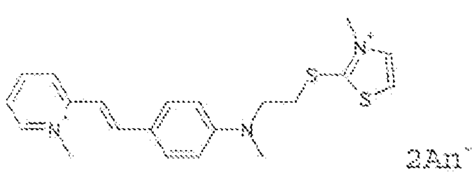 | 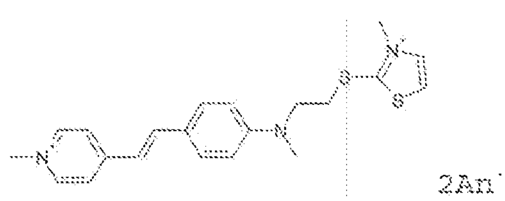 |
| **35** | **36** |

(continued)

| | |
|---|---|
| 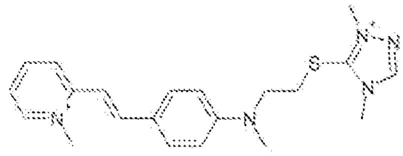 $2An^-$ | 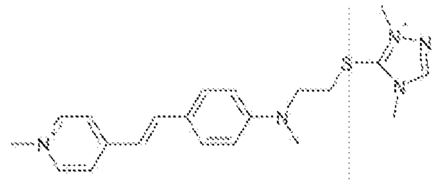 $2An^-$ |
| **37** | **38** |
| 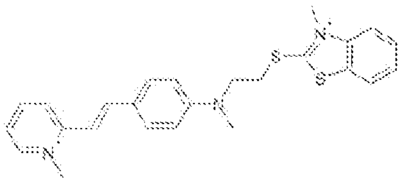 $2An^-$ | 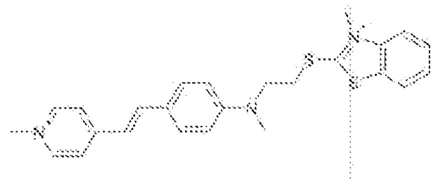 $2An^-$ |
| **39** | **40** |
| 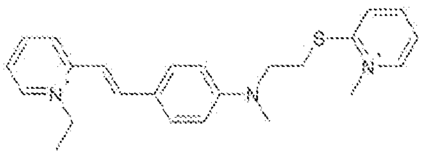 $2An^-$ | 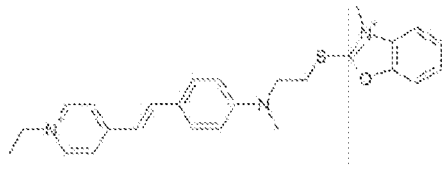 $2An^-$ |
| **41** | **42** |
| 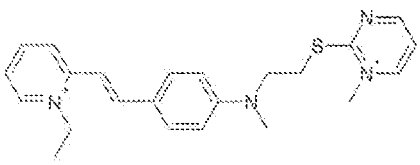 $2An^-$ | 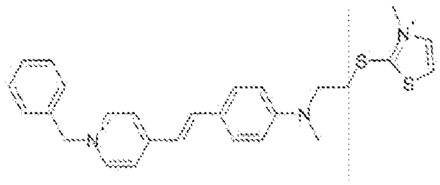 $2An^-$ |
| **43** | **44** |
| 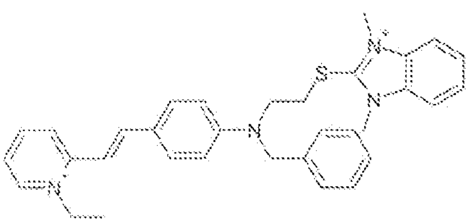 $2An^-$ | 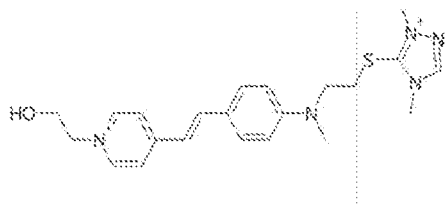 $2An^-$ |
| **45** | **46** |
| 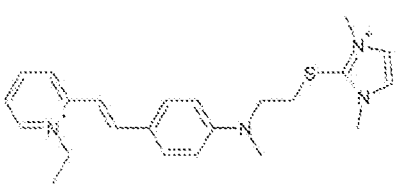 $2An^-$ | 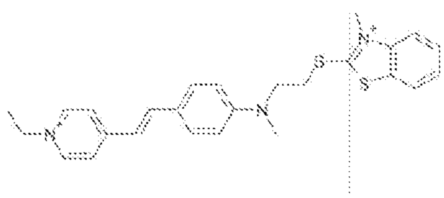 $2An^-$ |
| **47** | **48** |

(continued)

| | |
|---|---|
| 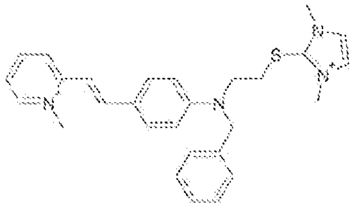 2An$^-$ | 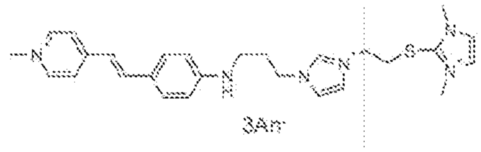 3An$^-$ |
| **49** | **50** |
| 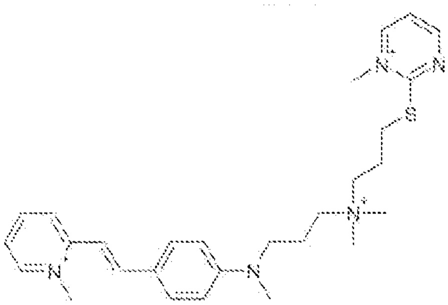 3An$^-$ | 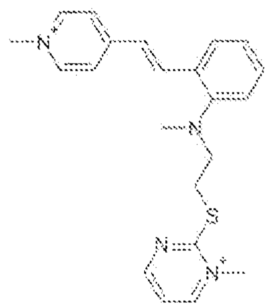 2An$^-$ |
| **51** | **52** |
| 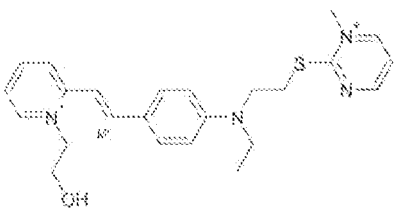 2An$^-$ | 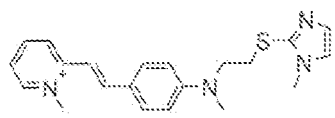 An$^-$ |
| **53** | **54** |
| 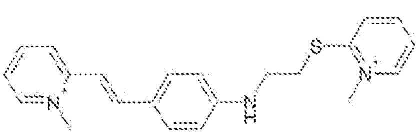 2An$^-$ | 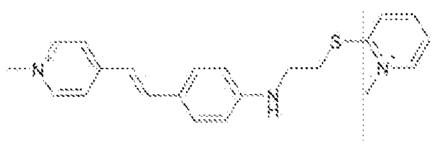 2An$^-$ |
| **55** | **56** |
| 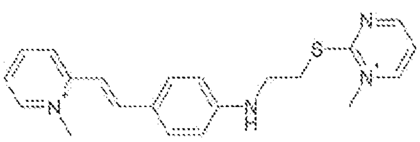 2An$^-$ | 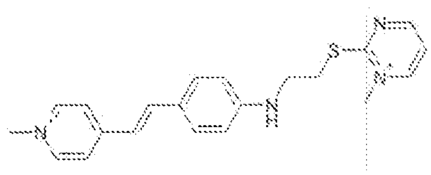 2An$^-$ |
| **57** | **58** |
| 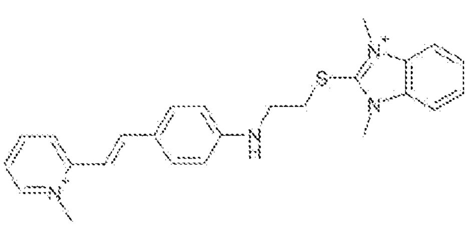 2An$^-$ | 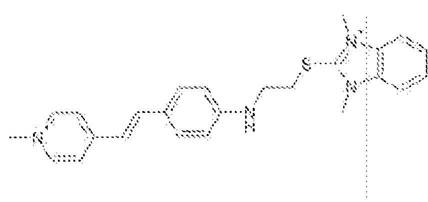 2An$^-$ |
| **59** | **60** |

(continued)

| | |
|---|---|
| 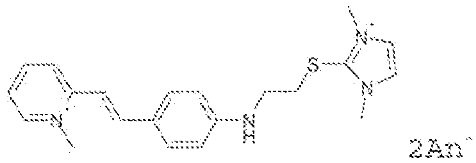 | 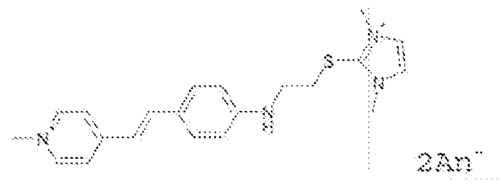 |
| **61** | **62** |
| 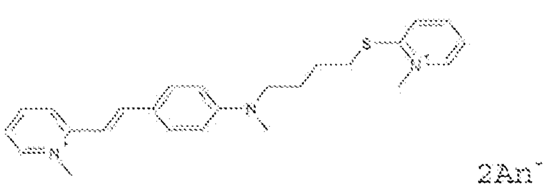 | 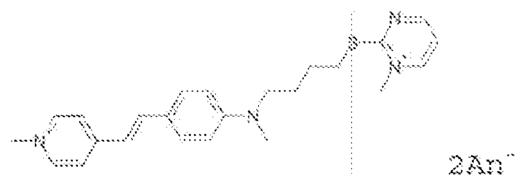 |
| **63** | **64** |
| 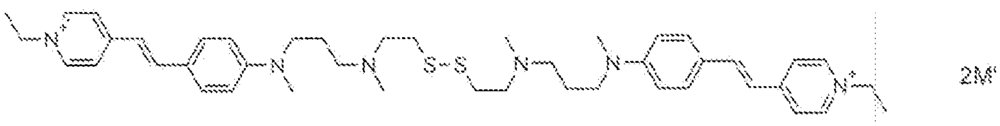 | |
| **65** | |
| 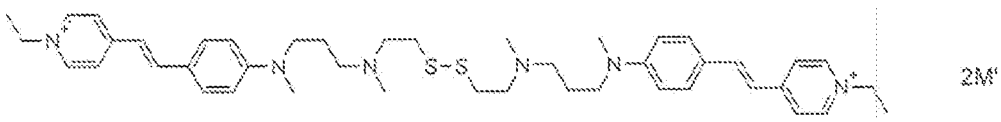 | |
| **66** | |
| 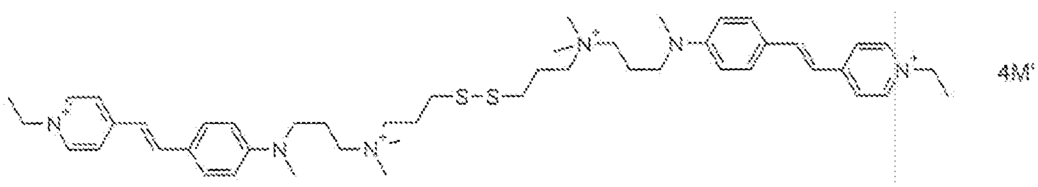 | |
| **67** | |
| 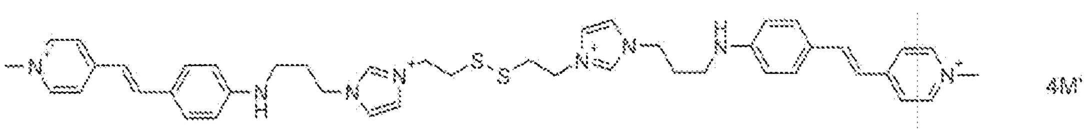 | |
| **68** | |
| 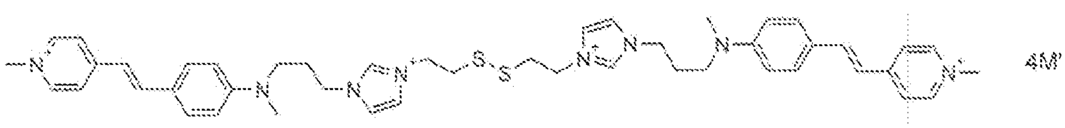 | |
| **69** | |
| 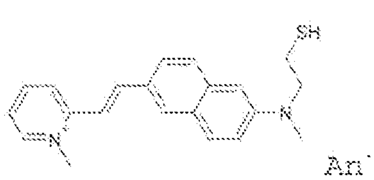 | |
| **70** | |

(continued)

| |
|---|
| 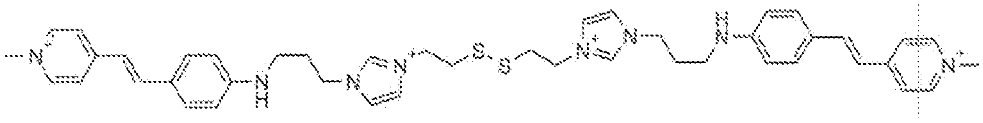 4M' |
| **71** |
| 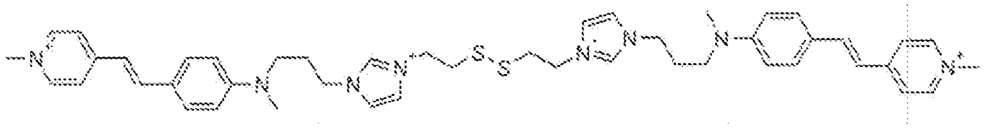 4M' |
| **72** |
| 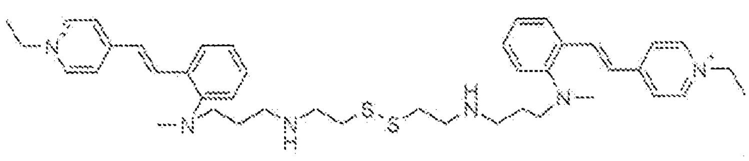 2M' |
| **73** |
| 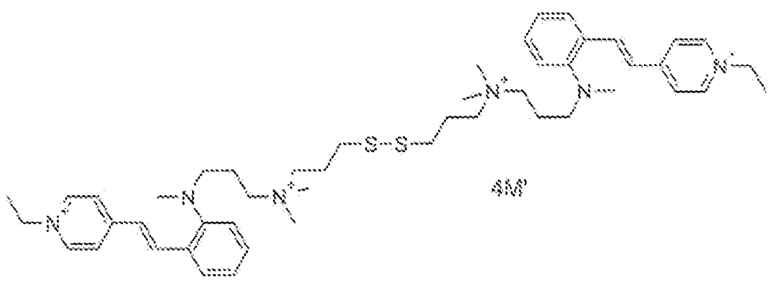 4M' |
| **74** |
| 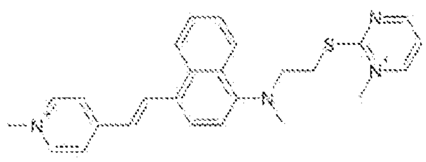 2An⁻ |
| **75** |
| 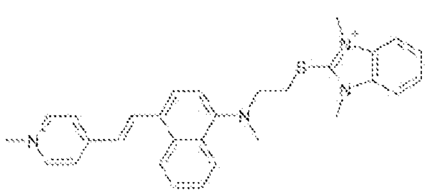 2An⁻ |
| **76** |
| 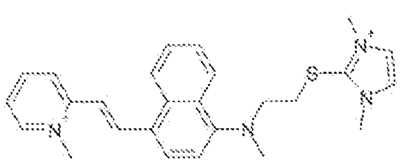 2An⁻ |
| **77** |

(continued)

| |
|---|
| 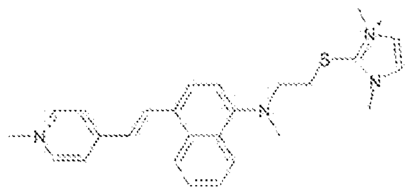 $2An^-$ |
| **78** |
| 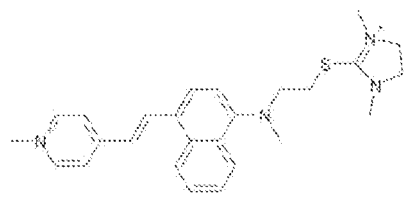 $2An^-$ |
| **79** |
| 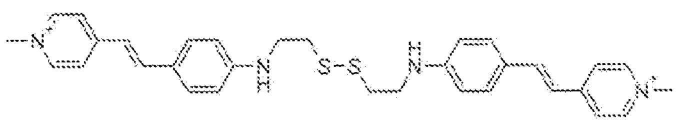 $2M^+$ |
| **80** |
| 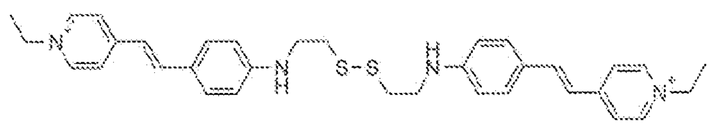 $2M^+$ |
| **82** |
| 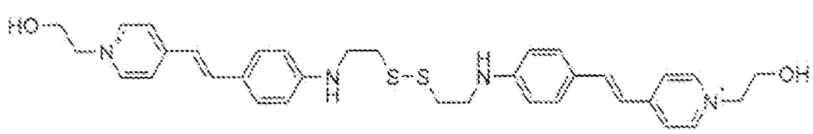 $2M^+$ |
| **83** |
| 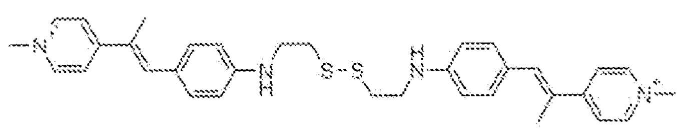 $2M^+$ |
| **84** |
| 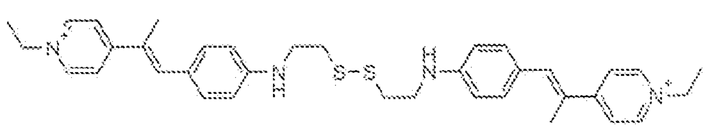 $2M^+$ |
| **85** |
| 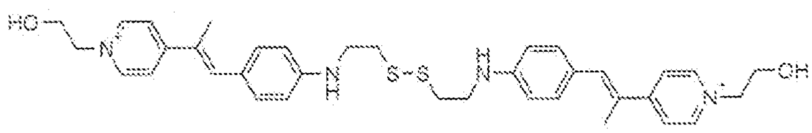 $2M^+$ |
| **86** |

(continued)

| |
|---|
| 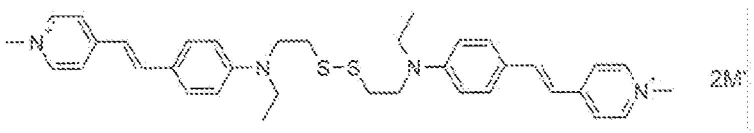 |
| **87** |
| 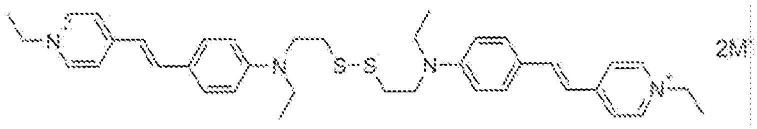 |
| **88** |
| 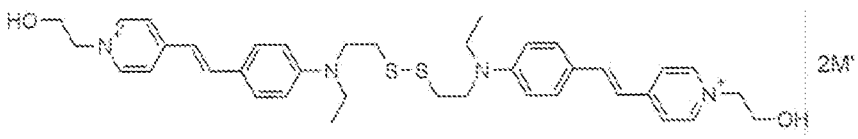 |
| **89** |
| 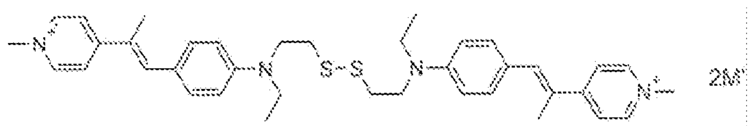 |
| **90** |
| 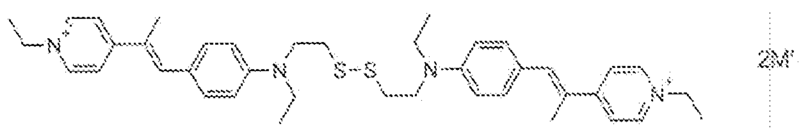 |
| **91** |
| 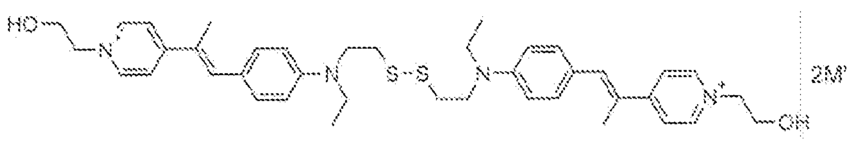 |
| **92** |
| 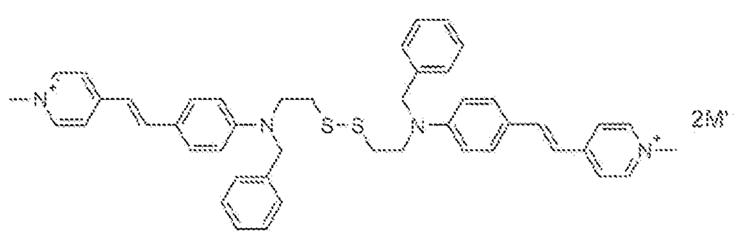 |
| **93** |
| 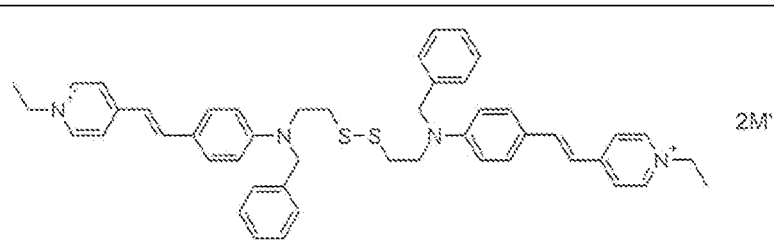 |
| **94** |

(continued)

| |
|---|
| 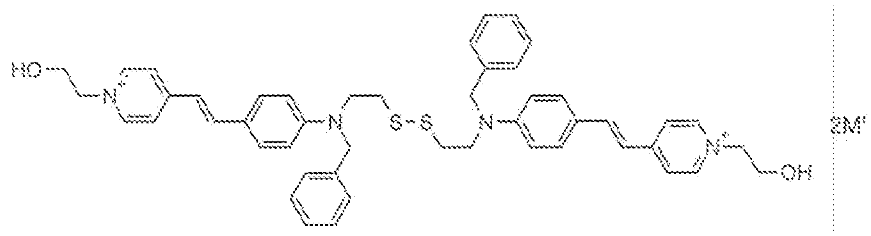 |
| **95** |
| 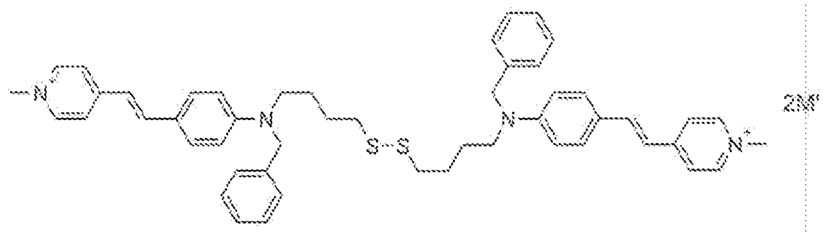 |
| **96** |
| 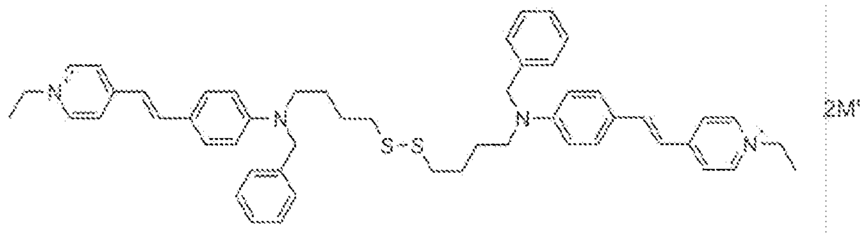 |
| **97** |
| 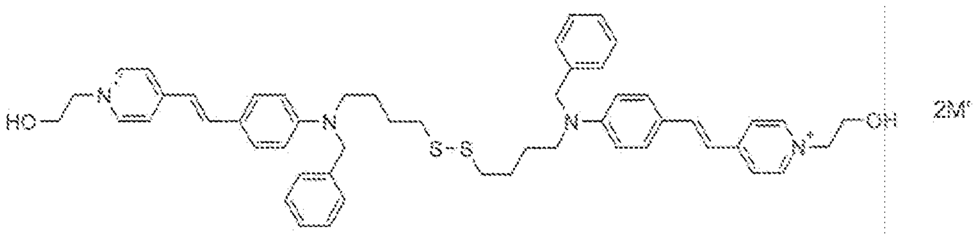 |
| **98** |
| 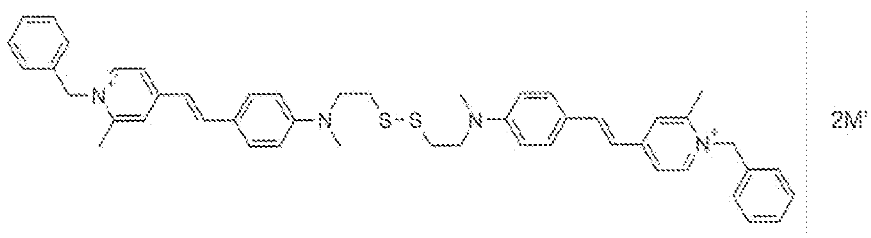 |
| **99** |
| 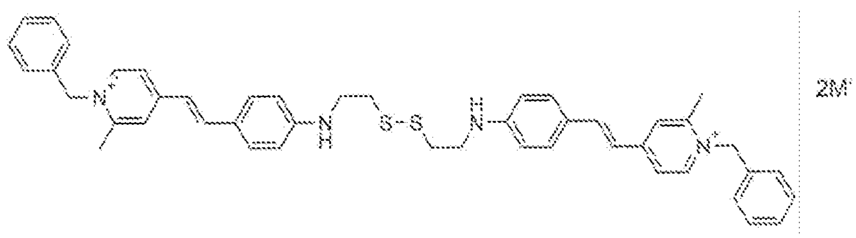 |

(continued)

| 100 |
|---|
| 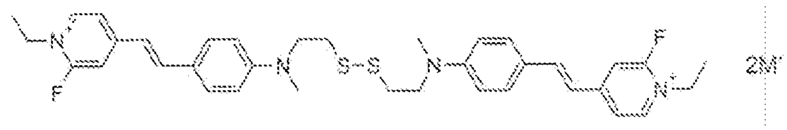 |
| 101 |
| 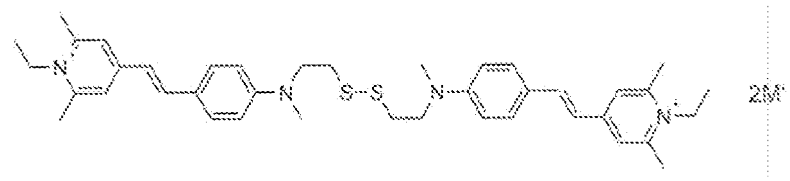 |
| 102 |
| 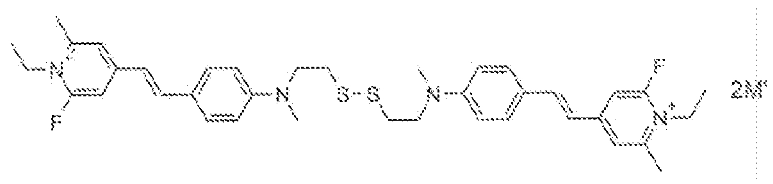 |
| 103 |
| 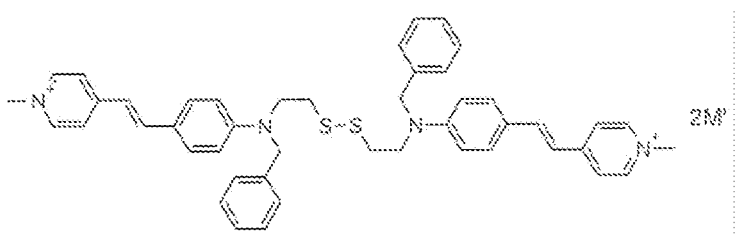 |
| 104 |
| 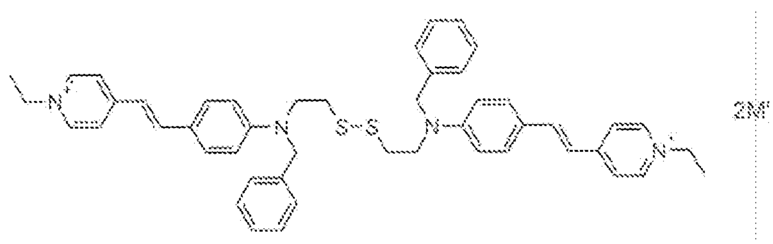 |
| 105 |
| 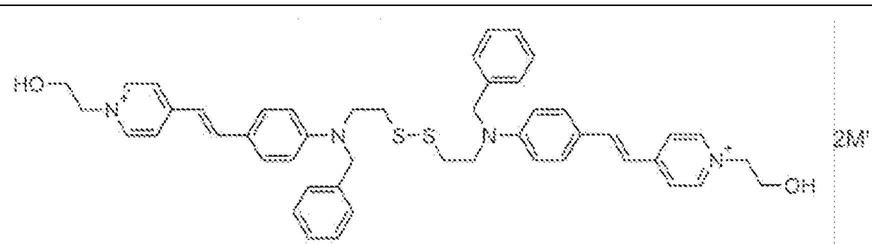 |
| 106 |

(continued)

| |
|---|
| 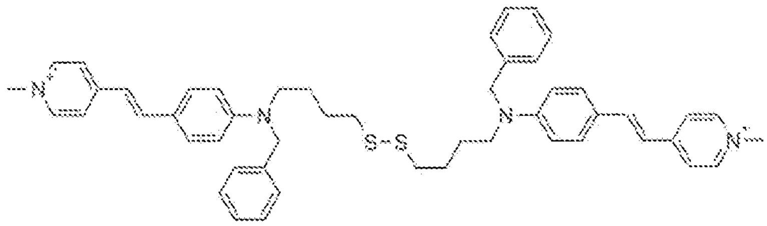 2M' |
| **107** |
| 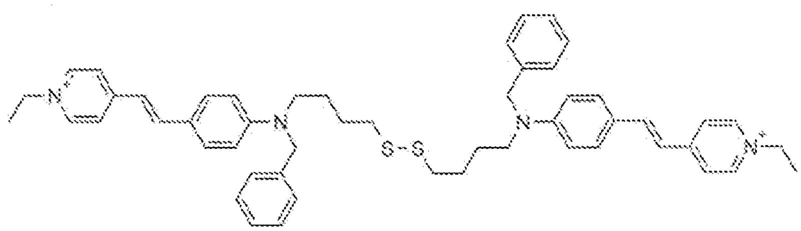 2M' |
| **108** |
| 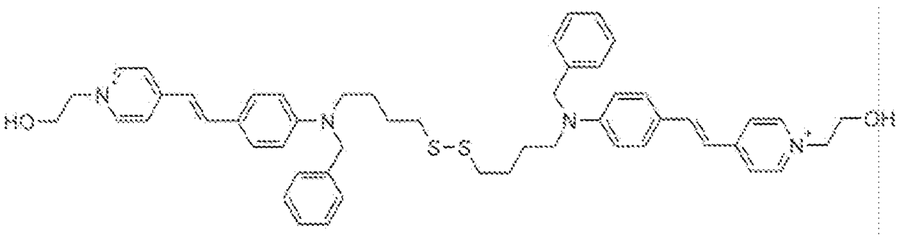 2M' |
| **109** |
| 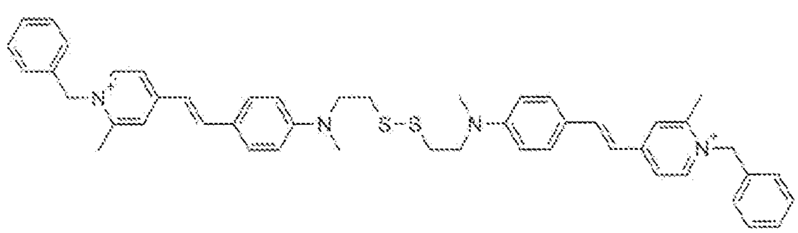 2M' |
| **110** |
| 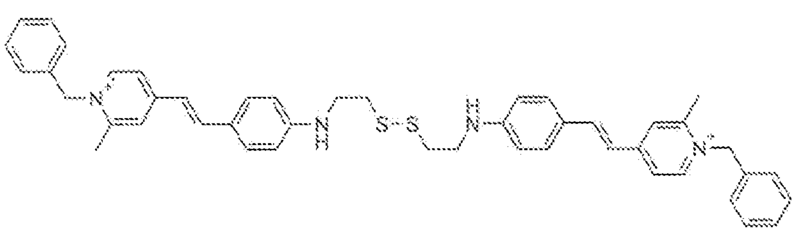 2M' |
| **111** |
| 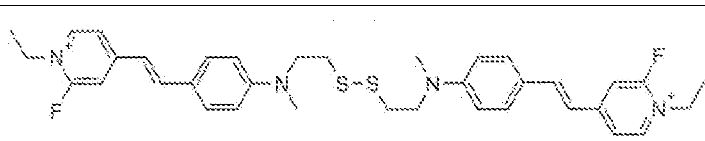 2M' |
| **112** |

(continued)

| |
|---|
| 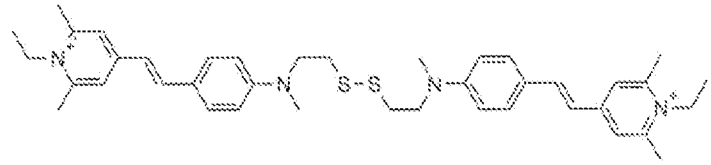 2M' |
| **113** |
| 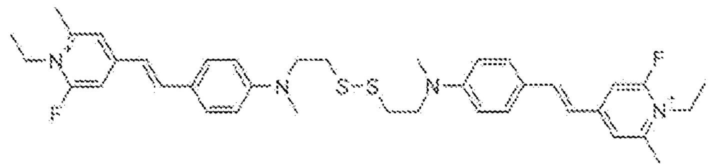 2M' |
| **114** |
| 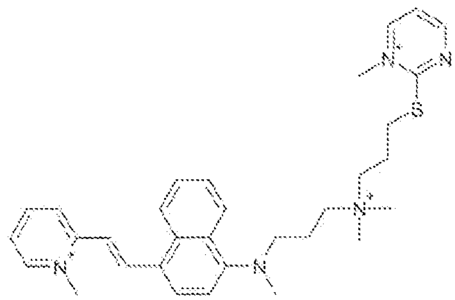 3 An⁻ |
| **115** |
| 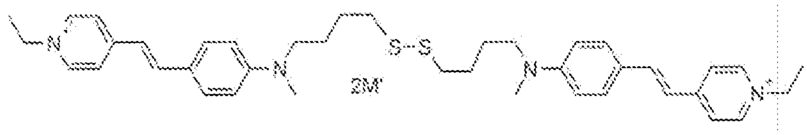 2M' |
| **116** |
| 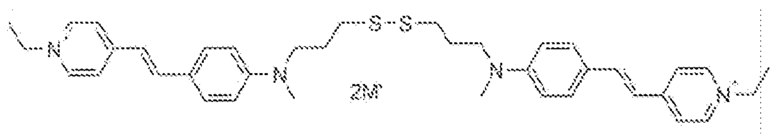 2M' |
| **117** |
| 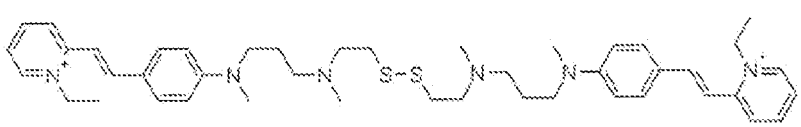 2M' |
| **118** |
| 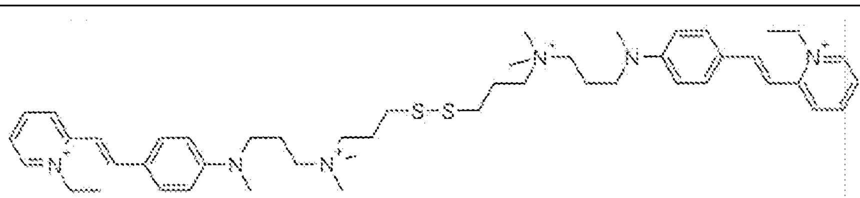 4M' |
| **119** |

(continued)

| |
|---|
| 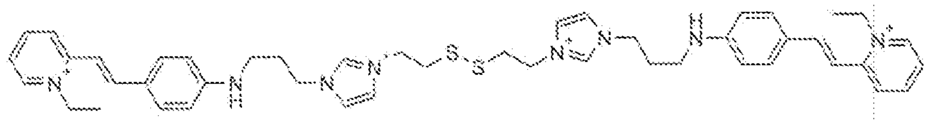 4M' |
| **120** |
| 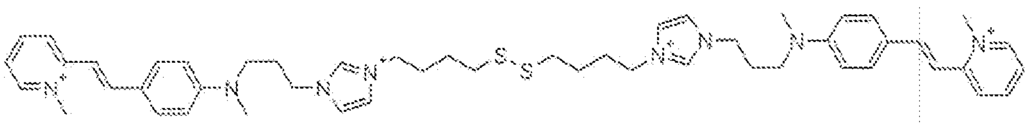 4M' |
| **121** |
| 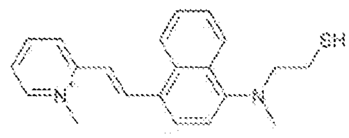 |
| **122** |
| 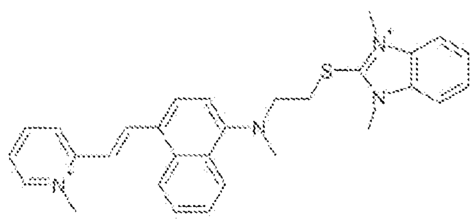 2M' |
| **123** |
| 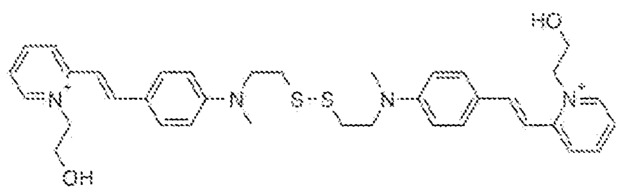 2M' |
| **124** |
| 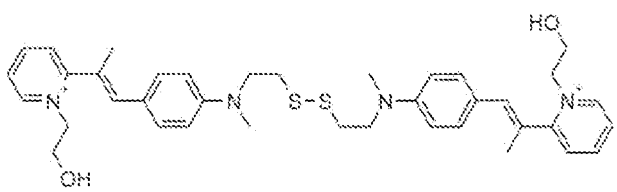 2M' |
| **125** |
| 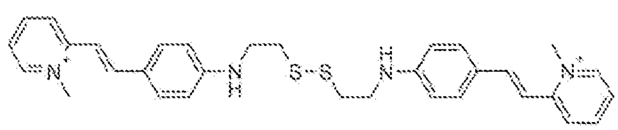 2M' |
| **126** |
| 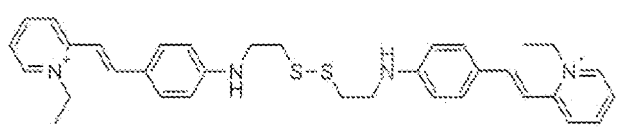 2M' |

(continued)

| 127 |
|---|
| 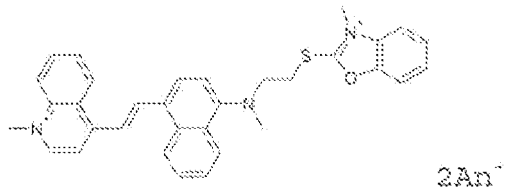 |
| 128 |
| 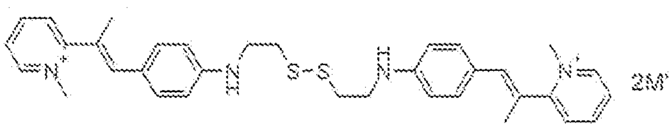 |
| 129 |
| 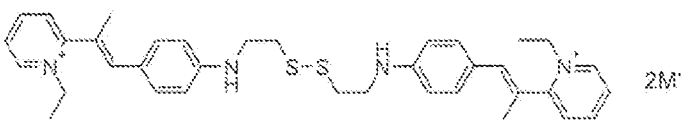 |
| 130 |
| 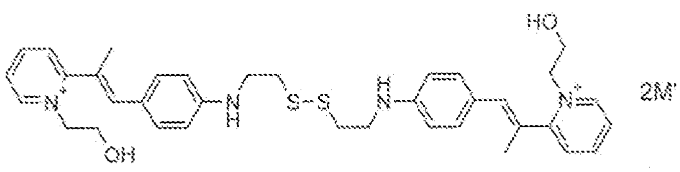 |
| 131 |
| 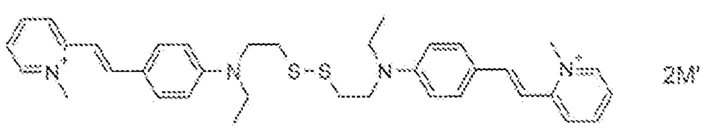 |
| 132 |
| 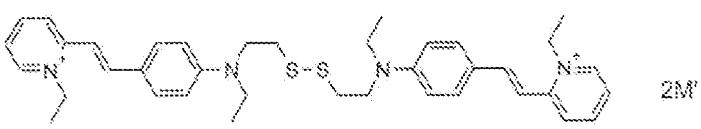 |
| 133 |
| 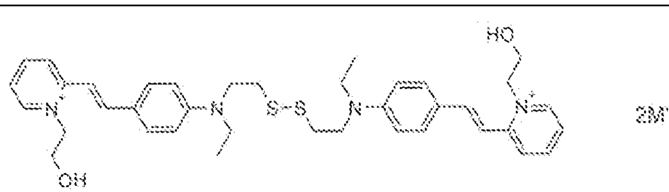 |
| 134 |

(continued)

| |
|---|
| 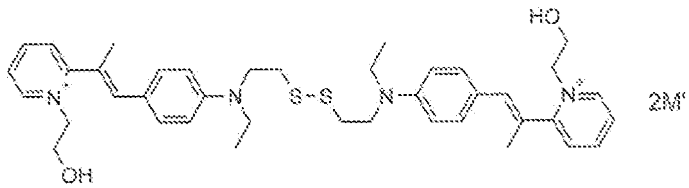 |
| **135** |
| 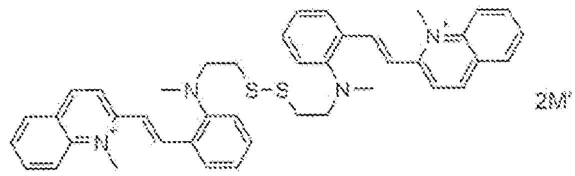 |
| **136** |
| 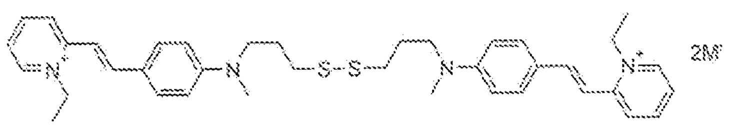 |
| **137** |
| 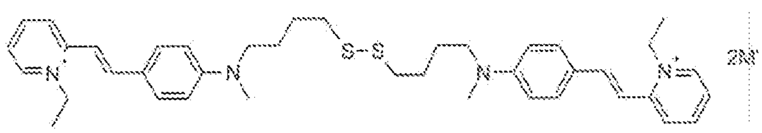 |
| **138** |
| 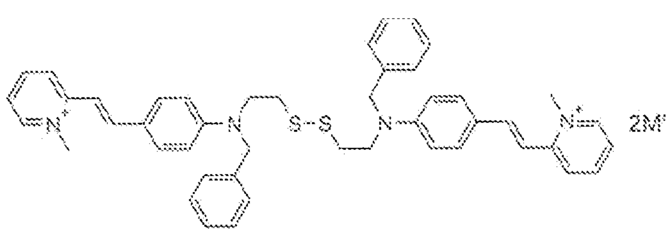 |
| **139** |
| 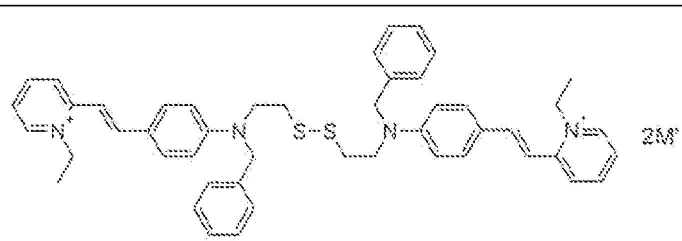 |
| **140** |
| 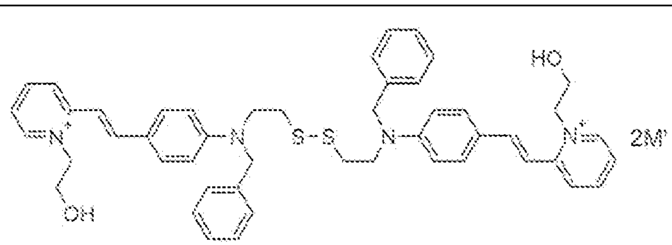 |

(continued)

| 141 | |
|---|---|
| 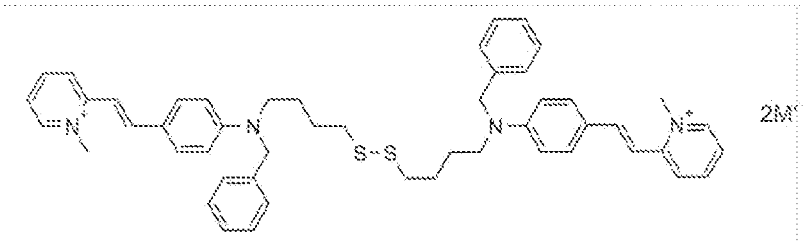 | |
| **142** | |
| 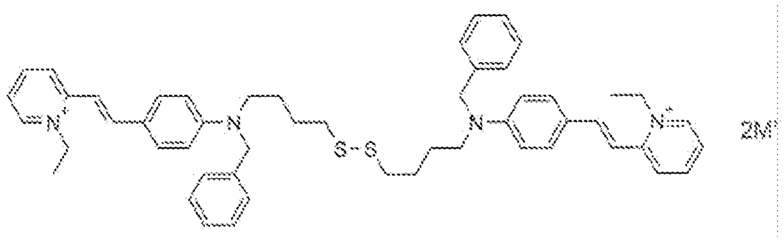 | |
| **143** | |
| 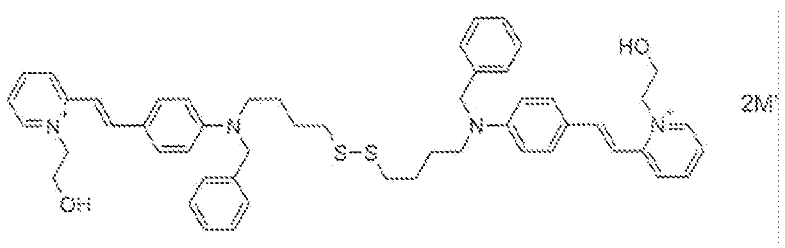 | |
| **144** | |
| 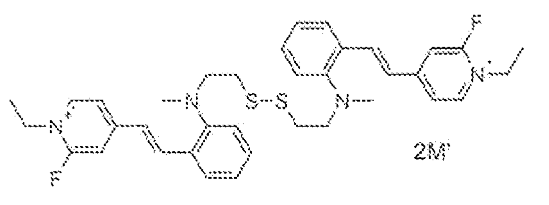 | 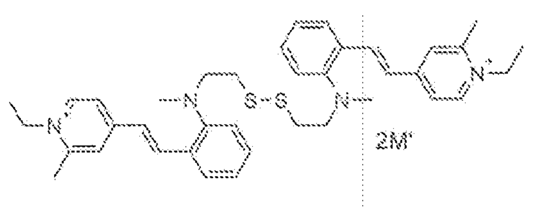 |
| **145** | **146** |
| 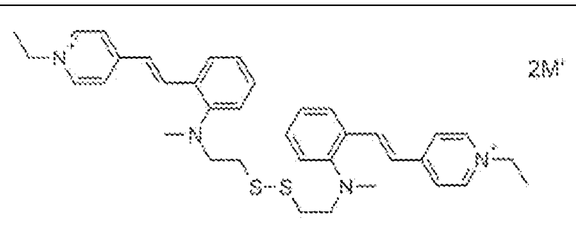 | 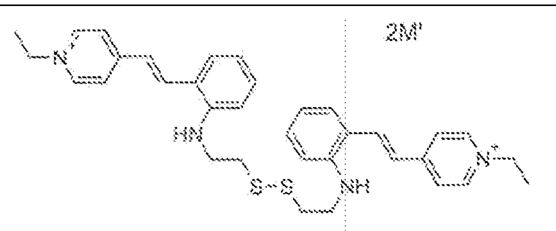 |
| **147** | **148** |
| 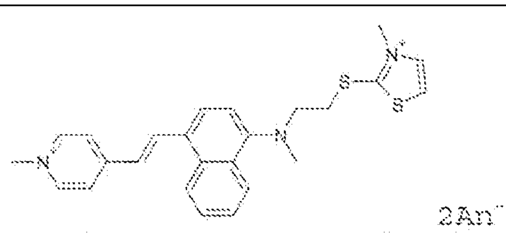 | |

with An⁻ and M', which may be identical or different, representing an anionic counterion; and $M^+$ representing an alkali metal.

**9.** Dye composition comprising, in a suitable cosmetic medium, a fluorescent dye of formula (I) or (II) as defined in any one of Claims 1 to 8.

**10.** Dye composition comprising, in a suitable cosmetic medium, at least one fluorescent dye of formulae (I) or (II) as defined in any one of Claims 1 to 8 and at least one reducing agent.

**11.** Process for dyeing keratin materials, in which a dye composition comprising, in a suitable cosmetic medium, at least one fluorescent dye of formula (I) or (II) as defined in any one of Claims 1 to 8, optionally in the presence of a reducing agent capable of reducing the disulphide bonds of the keratin materials, is applied to the materials.

**12.** Process for dyeing keratin materials according to Claim 11, **characterized in that** the keratin materials are dark keratin fibres.

**13.** Process according to Claims 11 or 12, comprising an additional step consisting in applying an oxidizing agent to the keratin fibres.

**14.** Process according to any one of Claims 11 to 13, in which the fluorescent dye of formula (I) or (II) is present in an amount of between 0.001% and 50% by weight relative to the total weight of the composition.

**15.** Multicompartment device in which a first compartment contains a dye composition comprising a fluorescent dye as defined in Claims 1 to 8 and a second compartment contains a reducing agent capable of reducing the disulphide bonds of the keratin materials.

**16.** Use of the fluorescent dyes of formula (I) or (II) as defined in Claims 1 to 8, for lightening dark keratin fibres.

## Patentansprüche

**1.** Fluoreszenzfarbstoff der folgenden Formel (I) oder (II):

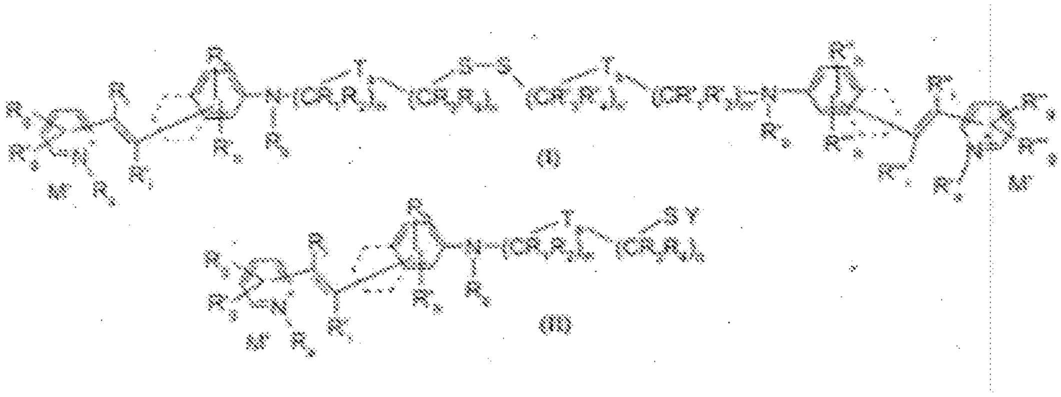

seine Salze mit einer organischen oder anorganischen Säure, optischen Isomere, geometrischen isomere und Solvate wie Hydrate;
in der Formel (I) oder (II):

> ➢ die Gruppen $R_a$ und $R'_a$, die gleich oder verschieden sind, bedeuten eine Aryl($C_{1-4}$)alkylgruppe oder eine ($C_{1-6}$)Alkylgruppe, die gegenbenenfalls substituiert ist mit Hydroxy oder Amino, (C1-4)Alkylamino oder (Di) ($C_{1-4}$)(alkyl)amino, wobei die beiden Alkylgruppen mit dem Stickstoffatom, von dem sie getragen werden, einen 5- bis 7-gliedrigen Heterocyclus bilden können, der gegebenenfalls ein weiteres Heteroatom enthält, das mit dem Stickstoffatom übereinstimmt oder davon verschieden ist;
> ➢ die Gruppen $R_b$ und $R'_b$, die gleich oder verschieden sind, bedeuten ein Wasserstoffatom, eine Aryl($C_{1-4}$) alkylgruppe oder eine ($C_{1-6}$)Alkylgruppe, die gegebenenfalls substituiert ist;

➢ die Gruppen $R_g$, $R'_g$, $R''_g$ und $R'''_g$, die gleich oder verschieden sind, bedeuten ein Wasserstoffatom, ein Halogenatom, eine Aminogruppe, eine $(C_{1-4})$Alkylaminogruppe, eine $(C_{1-4})$Dialkylaminogruppe, eine Trifluormethylgruppe, eine Acylaminogruppe, eine $(C_{1-4})$Alkoxygruppe, eine $(C_{1-4})$ Alkylcarbonyloxygruppe, eine $(C_{1-4})$ Alkoxycarbonylgruppe, eine $(C_{1-4})$Alkylcarbonylaminogruppe, eine $(C_{1-4})$Alkylsulfonylaminogruppe, eine $(C_{1-3})$ Alkylgruppe;

➢ die Gruppen $R_h$, $R'_h$, $R''_h$ und $R'''_h$, die gleich oder verschieden sind, bedeuten ein Wasserstoffatom, ein Halogenatom, eine $(C_{1-4})$Diakylamino. eine $(C_{1-4})$Alkylcarbonylaminogruppe, eine Acylaminogruppe, eine $(C_{1-4})$Alkylsulfonylaminogruppe oder eine $(C_{1-4})$Alkylgruppe;

➢ oder zwei Gruppen $R_g$ und $R'_g$; $R''_g$ und $R'''_g$; $R_h$ und $R'_h$; $R''_h$ und $R'''_h$, die von zwei benachbarten Kohlenstoffatomen getragen werden, bilden gemeinsam einen Benzoring, einen Indenoring, eine kondensierte Heterocycloalkylgruppe oder eine kondensierte Heteroarylgruppe; wobei der Benzoring, der Indenoring, die Heterocycloalkylgruppe und die Heteroarylgruppe gegebenenfalls substituiert sind mit Halogen, Amino, $(C_{1-4})$Alkylamino, $(C_{1-4})$Dialkylamino, Cyano, Carboxy, Hydroxy, Trifluormethyl, Acylamino, $(C_{1-4})$Alkoxy, $(C_{2-4})$(Poly)-hydroxyalkoxy, $(C_{1-4})$Alkylcarbonyloxy, $(C_{1-4})$Alkoxycarbonyl, $(C_{1-4})$Alkylcarbonylamino, Acylamino, Carbamoyl, $(C_{1-4})$Alkylsulfonylamino, Aminosulfonyl oder $(C_{1-16})$Alkyl, wobei diese Gruppe gegebenenfalls substituiert, ist mit einer Gruppe, die ausgewählt ist unter $(C_{1-4})$Alkoxy, Hydroxy, Cyano, Carboxy, Amino, $(C_{1-4})$Alkylamino und $(C_{1-4})$Dialkylamino, wobei die beiden Alkylgruppen, die von dem Stickstoffatom der Aminogruppe getragen werden, einen 5- bis 7-gliedrigen Heterocyclus bilden, der gegebenenfalls ein weiteres Heterocyclus bilden, der gegebenenfalls ein weiteres Heteroatom enthält, das mit dem Stickstoffatom übereinstimmt oder davon verschieden ist;

➢ die Gruppen $R_i$, $R'_i$, $R''_i$ und $R'''_i$, die gleich oder verschieden sind, bedeuten ein Wasserstoffatom oder eine $(C_{1-4})$Alkylgruppe;

➢ die Gruppen $R_1$, $R_2$, $R_3$, $R_4$, $R'_1$, $R'_2$, $R'_3$ und $R'_4$, die gleich oder verschieden sind, bedeuten ein Wasserstoffatom oder eine $(C_{1-4})$Alkylgruppe;

➢

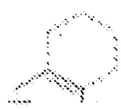

wobei diese Gruppe vorhanden oder nicht vorhanden ist, bedeutet eine Benzogruppe;

➢ die Gruppen $T_a$ et $T_b$, die gleich oder verschieden sind, bedeuten:

i) entweder eine kovalente σ-Bindung,
ii) oder eine Gruppe, die ausgewählt sind unter -N(R)-, $-N^+(R)(R^o)-$, wobei R und $R^o$, die gleich oder verschieden sind, ein Wasserstoffatom, eine $(C_{1-4})$Alkylgruppe, eine $(C_{1-4})$-Hydroxyalkylgruppe oder eine Aryl $(C_{1-4})$alkylgruppe bedeuten;
iii) oder eine Heterocycloalkylgruppe oder eine Heteroarylgruppe, die kanonisch oder nicht kationisch sind;

➢ m, m', n und n', die gleich oder verschieden sind, bedeuten eine ganze Zahl im Bereich von 0 bis 6 (Grenzen eingeschlossen) mit: m + n und m' + n', die gleich oder verschieden sind, bedeuten eine ganze Zahl im Bereich von 1 bis 10 (Grenzen eingeschlossen);

➢ wenn $T_a$ eine kovalente σ-Bindung ist, bedeutet Y eine Gruppe, die ausgewählt ist unter;

· Aryl, gegebenenfalls substituiert;
· Heteroaryl, gegebenenfalls substituiert;
· Heterocycloalkyl, gegebenenfalls substituiert;
· (Di)arylalkyl, gegebenenfalls substituiert;
· (Di)heteroarylalkyl, gegebenenfalls substituiert;
· cyclisch sterisch gehindert;

➢ wenn $T_a$ -N(R)-, $-N^+(R)(R^\circ)-$, eine Heterocycloalkylgruppe oder eine Heteroarylgruppe ist, die kationisch oder nicht kationisch vorliegen, bedeutet Y eine Gruppe, die ausgewählt ist unter: i) eineim Wasserstoffatom; ii) einem Alkalimetall; iii) einem Erdalkalimetall; iv) einer Ammoniumgruppe: $N^+R^\alpha R^\beta R^\gamma R^\delta$ oder eine Phosphoniumgruppe; $P^+R^\alpha R^\beta R^\gamma R^\delta$, wobei $R^\alpha$,$R^\beta$,$R^\gamma$ und $R^\delta$, die gleich oder verschieden sind, ein Wasserstoffatom oder eine $(C_{1-4})$Alkylgruppe bedeuten; oder v) eine Schutzgruppe für die Thiofunktion; und

➢ M' ist ein anionisches Gegenion;

mit der Maßgabe, dass:

w enn die Verbindung der Formel (I) oder (II) weitere kanonische Teile aufweist, sie mit einem oder mehreren anionischen Gegenionen kombiniert ist, mit denen die Elektroneutralität der Formeln (I) oder (II) erreicht werden kann.

**2.** Fluoreszierender Farbstoff der Formel (II) nach Anspruch 1, worin $T_a$ -N(R)-, -$N^+$(R)(R°)-, eine kationische oder nicht kationische Heterocycloalkyl- oder Heteroarylgruppe ist; und Y ein Wasserstoffatom oder ein Alkalimetall bedeutet.

**3.** Fluoreszierender Farbstoff der Formel (II) nach Anspruch 1, worin $T_a$ -N(R)-, -$N^+$(R)(R°)-, eine kationische oder nicht kationische Heterocycloalkyl- oder Heteroarylgruppe ist; und Y eine Schutzgruppe der Thiofunktion bedeutet, die unter den folgenden Gruppen ausgewählt ist:

➢ ($C_{1-4}$)Alkylcarbonyl;
➢ ($C_{1-4}$)Alkylthiocarbonyl;
➢ ($C_{1-4}$)Alkoxycarbonyl;
➢ ($C_{1-4}$)Alkoxythiocarbonyl;
➢ ($C_{1-4}$)Alkylthio-carbonyl;
➢ Di($C_{1-4}$)(alkyl)aminocarbonyl;
➢ Di($C_{1-4}$)(alkyl)aminothiocarbonyl;
➢ Arylcarbonyl;
➢ Aryloxycarbonyl;
➢ Aryl($C_{1-4}$)alkoxycarbonyl;
➢ Di($C_{1-4}$)(alkyl)aminocarbonyl;
➢ ($C_{1-4}$)(Alkyl)arylaminocarbonyl
➢ Carboxy;
➢ $SO_3^-$, $M^+$ mit $M^+$ bedeutet, ein Alkalimetall oder M' der Formel (II) und $M^+$ fehlen;
➢ Aryl, gegebenenfalls substituiert;
➢ Heteroaryl gegebenenfalls substituiert;
➢ Heterocycloalkyl, gegebenenfalls substituiert;
➢ Isothiouronium -C(NR'$^e$R'$^d$)=$N^+$R'$^e$R'$^f$; $An^+$, wobei R'$^e$, R'$^d$, R'$^e$ und R'$^f$, die gleich oder verschieden sind, ein Wasserstoffatom oder eine Alkylgruppe bedeuten;
➢ Isothioharnstoff -C(NR'$^e$R'$^d$)=NR'$^e$, wobei R'$^e$, R'$^d$ und R'$^e$ die oben angegebenen Bedeutungen aufweisen;
➢ (Di)arylalkyl, gegebenenfalls substituiert;
➢ (Di)heteroarylalkyl, gegebenfalls substituiert;
➢ CR'$^1$R'$^2$R'$^3$, wobei R'$^1$, R'$^2$ und R'$^3$, die gleich oder verschieden sind, ein Halogenatom oder eine Gruppe bedeuten, die ausgewählt ist unter:

· Alkyl, gegebenenfalls substituiert;
· Alkyl, gegebenenfalls substituiert;
· Alkoxy, gegebenenfalls substituiert;
· Aryl, gegebenenfalls substituiert;
· Heteroaryl, gegebenenfalls substituiert;

➢ P($Z^1$)R"$^1$R"$^2$R"$^3$, wobei R"$^1$ und R"$^2$, die gleich oder verschieden sind, Hydroxy, Alkoxy oder Alkyl bedeuten, R"$^3$ Hydroxy oder Alkoxy ist und $Z^1$ ein Sauerstoffatom oder ein Schwefelatom bedeutet;
➢ cyclisch sterisch gehindert; und
➢ Alkoxyalkyl, gegebenenfalls substituiert.

**4.** Fluoreszierender Farbstoff der Formel (II) nach einem der Ansprüche 1 und 3, worin $T_a$ -N(R)-, -$N^+$(R)(R°)-, eine kationische oder nicht kationische Heterocycloalkyl- oder Heteroarylgruppe ist; und Y eine Schutzgruppe bedeutet, die unter den folgenden Gruppen ausgewählt ist:

♦ ($C_{1-4}$)Alkylcarbonyl;
♦ Arylcarbonyl;

- ♦ $(C_{1-4})$Alkoxycarbonyl;
- ♦ Aryloxycarbonyl;
- ♦ Aryl$(C_{1-4})$alkoxycarbonyl;
- ♦ Di$(C_{1-4})$(alkyl)aminocarbonyl;
- ♦ $(C_{1-4})$(Alkyl)arylaminocarbonyl;
- ♦ Aryl, gegebenenfalls substituiert;
- ♦ 5- oder 6-gliedriges monocyclisches Heteroaryl;
- ♦ 8- bis 11-gliedriges kationisches bicyclisches Heteroaryl;
- ♦ dem kationischen Heterocyclus der folgenden Formel:

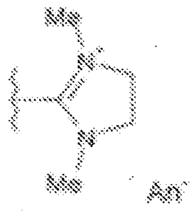

- ♦ Isothiouronium $-C(NH_2)-N^+H_2$, An-;
- ♦ Isothioharnstoff $-C(NH_2)=NH$; und
- ♦ $SO_3^-$,$M^+$ mit $M^+$ bedeutet ein Alkalimetall oder M' der Formel (II) und $M^+$ fehlen.

**5.** Fluoreszierender Farbstoff der Formel (II) nach Anspruch 1, worin, wenn $T_a$ eine Bindung ist, Y bedeutet; (i) ein 5- oder 6-gliedriges aromatisches kationisches monocyclisches Heteroaryl mit 1 bis 4 Heteroatomen, die unter Sauerstoff, Schwefel und Stickstoff ausgewählt sind; (ii) ein 8- bis 1-gliedriges kationisches bicyclisches Heteroaryl wobei diese mono- oder bicyclischen Gruppen gegebenenfalls mit einer oder mehreren Gruppen substituiert sind; iii) oder den folgenden Heterocyclus:

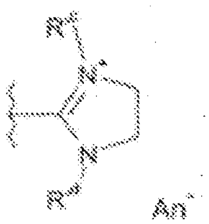

worin $R'^e$ und $R'^d$, die gleich oder verschieden sind, ein Wasserstoffatom oder eine Alkylgruppe bedeuten; und $An^-$ ein Gegenion bedeutet.

**6.** Fluoreszierender Farbstoff nach einem der vorhergehenden Ansprüche, der unter den fluoreszierenden Farbstoffen der Formel (Ia), (IIa), (Ib), (IIb), (Ic) oder (IIc) ausgewählt ist, wobei jeder eine Ethylengruppe aufweist, die den Pyridiniumteil mit dem Phenylteil in ortho- oder para-Stellung verbindet, d.h. den Positionen 4-4', 4-2', oder 2-4' für (Ia), (IIa), (Ib) oder (IIb), oder den Positionen 4-5' oder 2-5' für (Ic) oder (IIc):

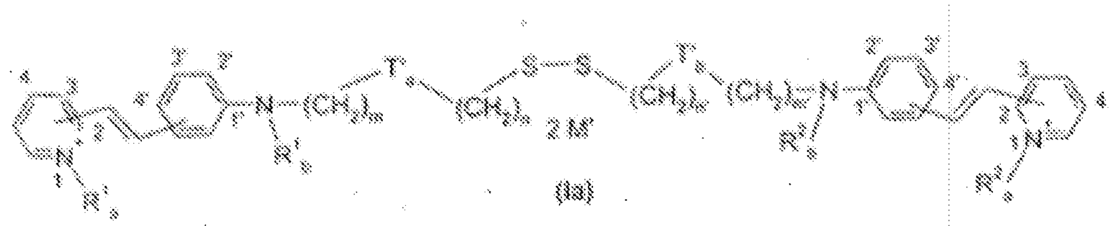

(Ia)

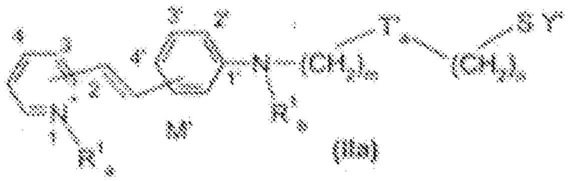

(IIa)

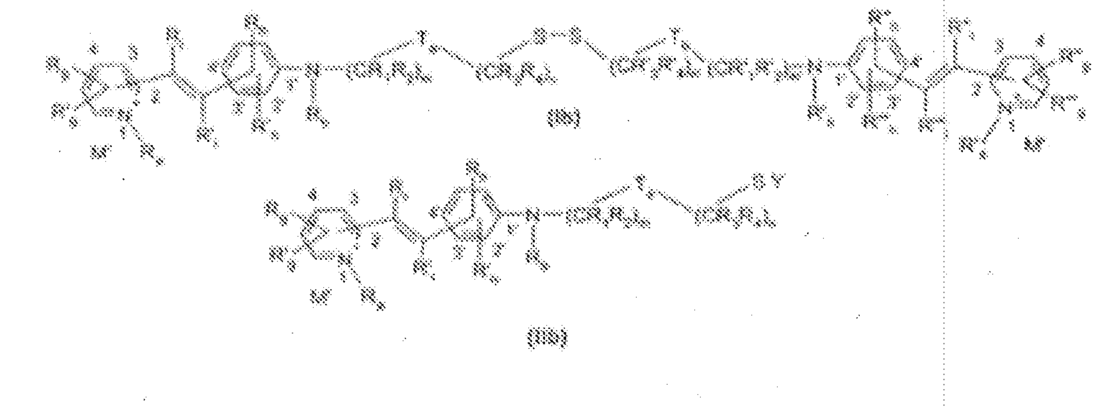

(Ib)

(IIb)

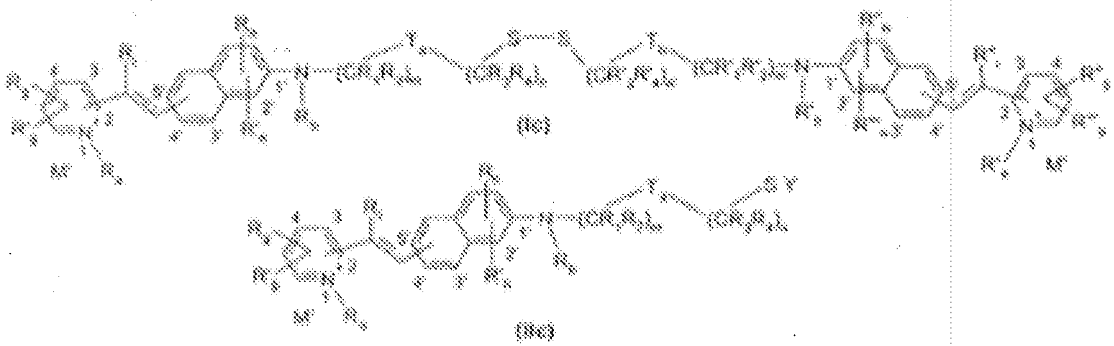

(Ic)

(IIc)

in den Formeln (Ia) oder (IIa):

- ♦ die Gruppen $R^1{}_a$ $R^2{}_a$, die gleich oder verschieden sind, bedeuten eine Benzyl-oder Alkylgruppe, die gegebenenfalls mit einer Hydroxygruppe substituiert ist;
- ♦ die Gruppen $R^1$b und $R^2$b, die gleich oder verschieden sind, bedeuten ein Wasserstoffatom; eine Benzyl- oder Alkylgruppe;
- ♦ die Gruppen $T_a$ und $T_b$, die gleich oder verschieden sind, bedeuten, eine σ-Bindung; eine Gruppe -$N^+$(R)(R°)-, wobei R und R°, die gleich oder verschieden sind, eine Alkylgruppe bedeuten; oder $T'_s$ und $T'_b$ bedeuten eine Imidazoliumgruppe;
- ♦ Y' bedeutet, eine Gruppe, die ausgewählt ist unter;
Imidazolium, Thiazolium; Oxazolium; 1,2,4-Triazolium; Pyridinium; Pyrimidinium; Benzoxazolium und Benzothiazolium; wobei diese Gruppen mit einer oder mehreren, identischen oder verschiedenen alkylgruppen substituiert sind;
- ♦ m, n, m' und n', die gleich oder verschieden sind, bedeuten eine ganze Zahl im Bereich von 1 bis 6 (Grenzen eingeschlossen) mit: m + n = m' + n' bedeuten eine ganze Zahl im Bereich von 2 bis 6 (Grenzen eingeschlossen);
- ♦ M' ist ein anionisches Gegenion;

in den Formeln (Ib) oder (IIb):

➢ $R_a$, $R'_a$, $R_b$, $R'_b$,$R_i$, $R'_i$, $R''_i$, $R'''_i$, $R_1$, $R'_1$, $R_2$, $R'_2$, $R_3$, $R'_3$, $R_4$, $R'_4$, $T_a$, $T_b$, m, m', n, n', Y haben die in einem der vorhergehenden Ansprüchen angegebenen Bedeutungen;

➢ $R_g$ und $R'_g$; $R''_g$ und $R'''_g$, die gleich oder verschieden sind, bedeuten ein Wasserstoffatom, ein Halogenatom, eine Aminogruppe, eine ($C_{1-4}$)Alkylaminogruppe, eine ($C_{1-4}$)Dialkylaminogruppe, eine Cyanogruppe, eine Carboxygruppe; eine Hydroxygruppe, eine Trifluormethylgruppe, eine Acylaminogruppe, eine ($C_{1-4}$)Alkoxygruppe, eine ($C_{2-4}$)(Poly)-hydroxyalkoxygruppe, eine ($C_{1-4}$)Alkylcarbonyloxygruppe, eine ($C_{1-4}$)Alkoxycarbonylgruppe, eine ($C_{1-4}$)Alkylcarbonylaminogruppe, eine Acylaminogruppe, eine Carbamoylgruppe, eine ($C_{1-4}$)Alkylsulfonylaminogruppe, eine Aminosulfonylgruppe oder eine ($C_{1-16}$)Alkylgruppe, die gegebenenfalls substituiert ist mit einer Gruppe, die ausgewählt ist unter ($C_{1-12}$)Alkoxy, Hydroxy, Cyano, Carboxy, Amino, ($C_{1-4}$)Alkyiamino und ($C_{1-4}$)Dialkylamino, wobei die beiden Alkylgruppen, die von dem Stickstoffatom der Aminogruppe getragen werden, einen 5- bis 7-gliedrigen Heterocyclus bilden, der gegebenenfalls ein weiteres Heteroatom enthält, das mit dem Stickstoffatom übereinstimmt oder davon verschieden ist;

➢ $R_h$ und $R'_h$; $R''_h$ und $R'''_h$, die von zwei benachbarten Kohlenstoffatomen getragen werden, bilden gemeinsam einen Benzoring, einen Indenoring, eine kondensierte Heterocycloalkylgruppe oder eine kondensierte Heteroarylgruppe; wobei der Benzoring, der Indenoring, die Heterocycloalkylgruppe und die Heteroarylgruppe gegebenenfalls substituiert sind mit Halogen, Amino, ($C_{1-4}$) Alkylamino, $C_{1-4}$)Dialkylamino, Cyano, Carboxy, Hydroxy, Trifluormethyl, Acylamino, ($C_{1-4}$)Alkoxy, ($C_{2-4}$)(Poly)hydroxyalkoxy, ($C_{1-4}$)Alkylcarbonyloxy, ($C_{1-4}$)Alkoxycarbonyl, ($C_{1-4}$) Alkylcarbonylamino, Acylamino, Carbamoyl, ($C_{1-4}$)Alkylsulfonylamino, Aminosulfonyl oder ($C_{1-16}$)Alkyl,wobei diese Gruppe gegebenenfalls substituiert ist mit einer Gruppe, die ausgewählt ist unter ($C_{1-12}$) Alkoxy, Hydroxy, Cyano, Amino, ($C_{1-4}$)Alkylamino und ($C_{1-4}$)Dialkylamino, wobei die beiden Alkylgruppen, die von dem Stickstoffatom der Aminogruppe getragen werden, einen 5- bis 7-gliedrigen Heterocyclus bilden, der gegebenenfalls ein weiteres Heteroatom enthält, das mit dem Stickstoffatom übereinstimmt oder davon verschieden ist;

und in den Formeln (Ic) oder (IIc):

➢ $R_a$, $R'_a$, $R_b$, $R'_b$, $R_1$, $R''_1$, $R_1$, $R'_1$, $R_2$, $R'_2$, $R_3$, $R'_3$, $R_4$, $R'_4$, $T_a$, $T_b$, m, m', n, n', Y haben die in einem, der vorhergehenden Ansprüchen angegebenen Bedeutungen;

➢ $R_g$, $R'_g$, $R''_g$, $R'''_g$, $R_h$, $R'_h$, $R''_h$ und $R'''_h$, die gleich oder verschieden sind, bedeuten ein Wasserstoffatom, ein Halogenatom, eine Aminogruppe, eine ($C_{1-4}$)Alkylaminogruppe, eine ($C_{1-4}$)Dialkylaminogruppe, eine Cyanogruppe, eine Carboxygruppe, eine Hydroxygruppe, eine Trifiuormethylgruppe, eine Acylaminogruppe, eine ($C_{1-4}$)-Alkoxygruppe, eine ($C_{2-4}$)(Poly)hydroxyalkoxygruppe, eine ($C_{1-4}$) Alkylcarbonyloxygruppe, eine ($C_{1-4}$) Alkoxycarbonylgruppe, eine ($C_{1-4}$)Alkylcarbonylaminogruppe, eine Acylaminogruppe, eine Carbamoylgruppe, eine ($C_{1-4}$)Alkylsulfonylaminogruppe, eine Aminosulfonylgruppe oder eine ($C_{1-16}$)Alkylgruppe, die gegebenenfalls substituiert ist mit einer Gruppe, die ausgewählt ist unter ($C_{1-12}$)Alkoxy, Hydroxy, Cyano, Carboxy, Amino, ($C_{1-4}$)Alkylamino und ($C_{1-4}$)Dialkylamino, wobei die beiden Alkylgruppen, die von dem Stickstoffatom der Aminogruppe getragen werden, einen 5- bis 7-gliedrigen Heterocyclus bilden, der gegebenenfalls ein weiteres Heteroatom enthält, das mit dem Stickstoffatom übereinstimmt oder davon verschieden ist;

mit der Maßgabe, dass;

- wenn die Verbindungen der Formel (Ia), (IIa), (Ib), (IIb), (Ic) oder (IIc) weitere kationische Teile aufweisen, sie mit einem oder mehreren anionischen Gegenionen, kombiniert ist, mit denen die Elektroneutralität der Formeln (Ia), (IIa), (Ib), (IIb), (Ic) oder (IIc) erreicht wird.

**7.** Fluoreszierender Farbstoff der Formel (I) nach einem der Ansprüche 1 oder 6, der symmetrisch ist.

**8.** Fluoreszierender Farbstoff nach einem der vorhergehenden Ansprüche, der unter den folgenden Farbstoffen ausgewählt ist:

| | |
|---|---|
| 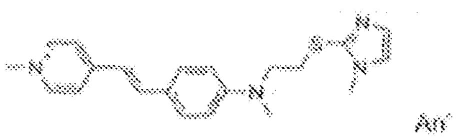 | 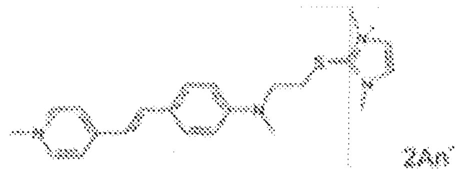 |
| **1** | **2** |
| 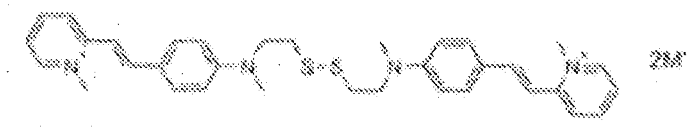 | |
| **3** | |
| 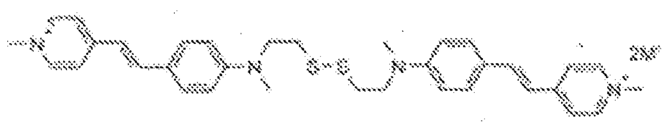 | |
| **4** | |
| 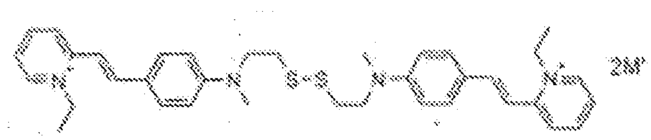 | |
| **5** | |
| 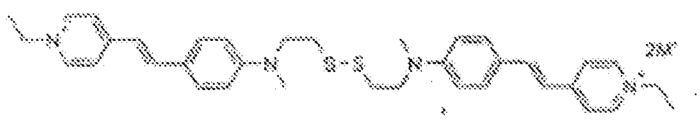 | |
| **6** | |
| 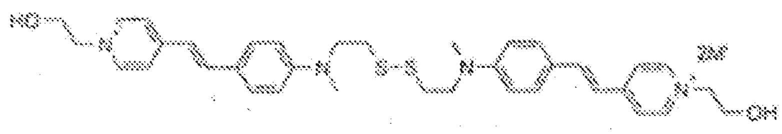 | |
| **7** | |
| 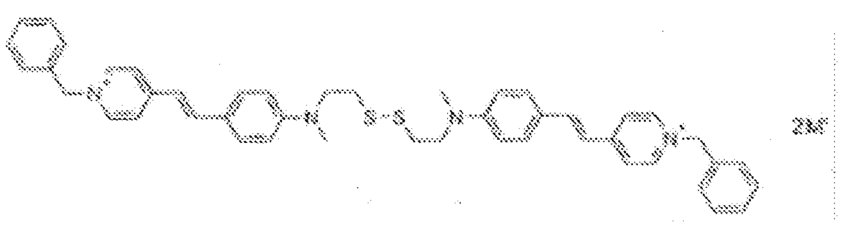 | |
| **8** | |
| 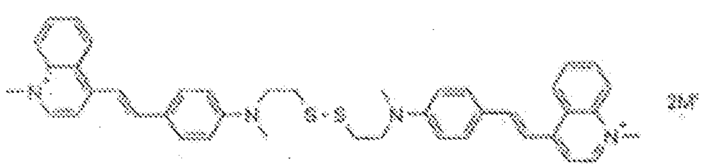 | |
| **9** | |

(fortgesetzt)

<table>
<tr><td colspan="2">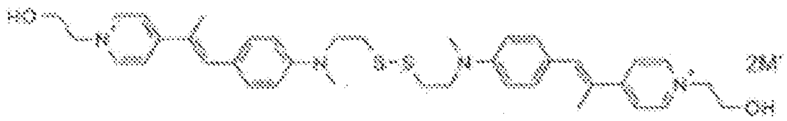</td></tr>
<tr><td colspan="2">10</td></tr>
<tr><td colspan="2">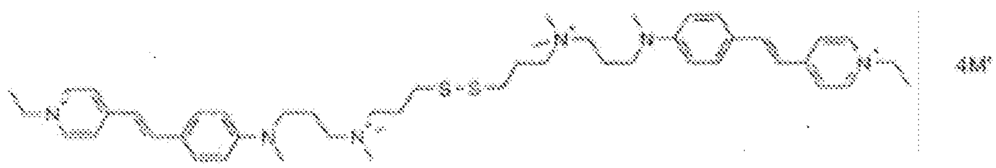</td></tr>
<tr><td colspan="2">11</td></tr>
<tr><td colspan="2">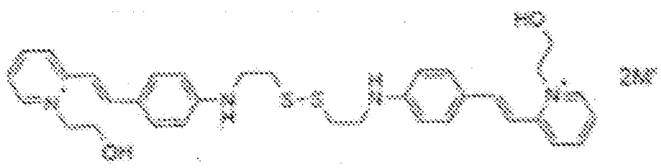</td></tr>
<tr><td colspan="2">12</td></tr>
<tr><td>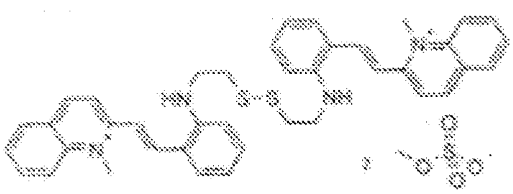</td><td>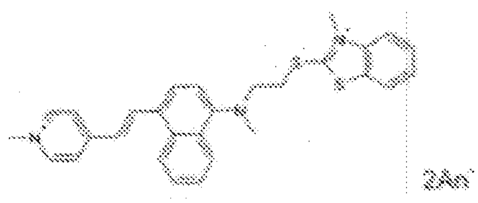</td></tr>
<tr><td>13</td><td>14</td></tr>
<tr><td>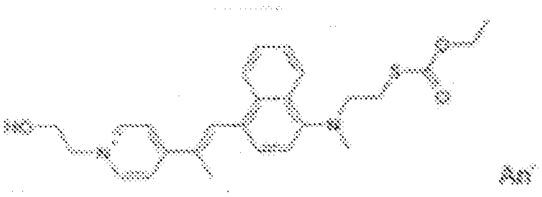</td><td>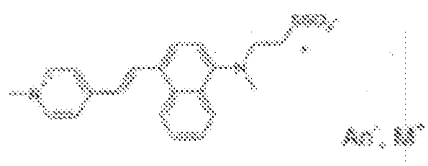</td></tr>
<tr><td></td><td>16</td></tr>
<tr><td>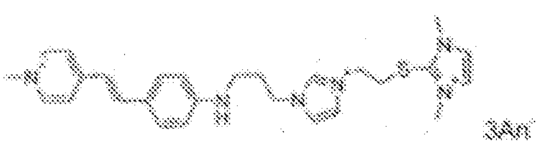</td><td>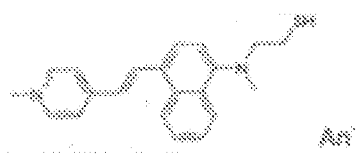</td></tr>
<tr><td>17</td><td>18</td></tr>
<tr><td>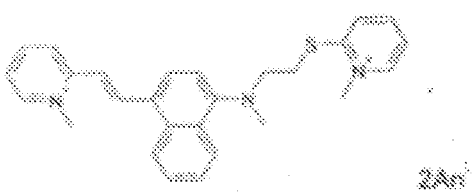</td><td>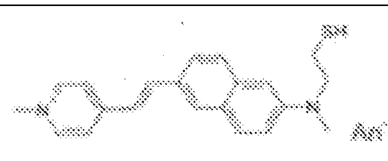</td></tr>
<tr><td>19</td><td>20</td></tr>
</table>

(fortgesetzt)

| | |
|---|---|
| 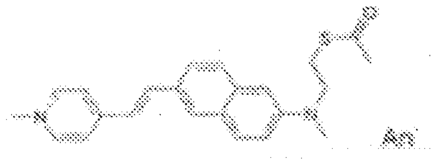 | 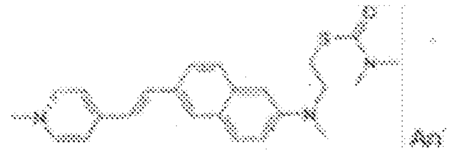 |
| **21** | **22** |
| 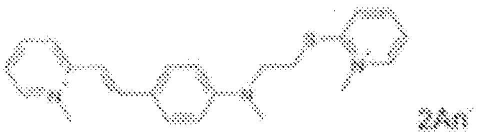 | 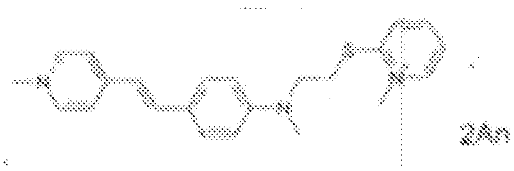 |
| **23** | **24** |
| 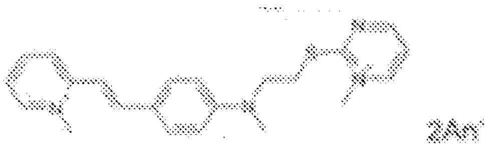 | 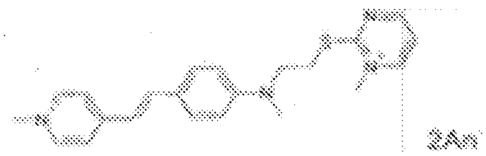 |
| **25** | **26** |
| 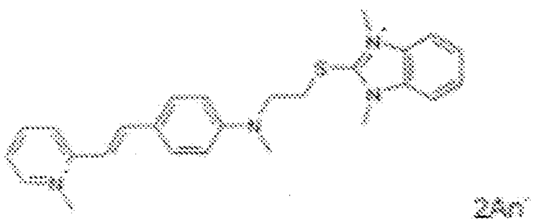 | 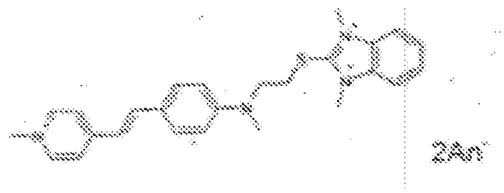 |
| **27** | **28** |
| 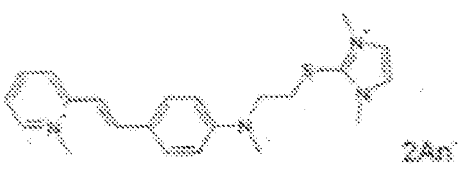 | 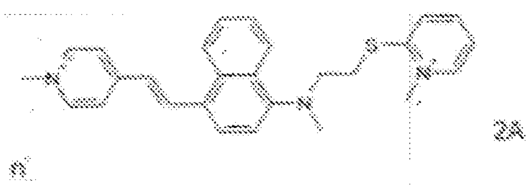 |
| **29** | **30** |
| 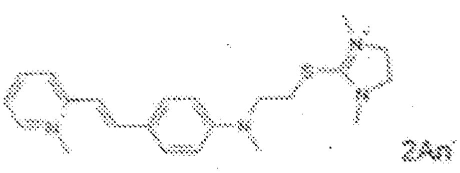 | 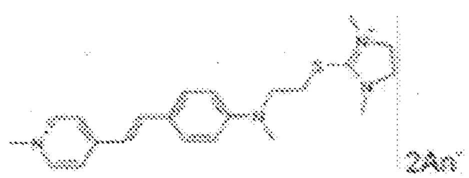 |
| **31** | **32** |
| 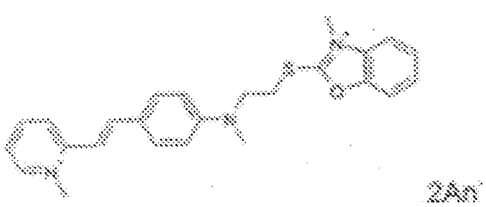 | 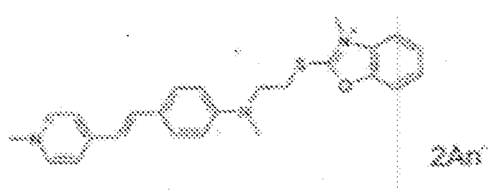 |
| **33** | **34** |

(fortgesetzt)

| | |
|---|---|
| 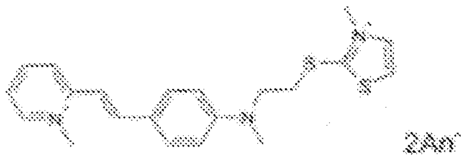 | 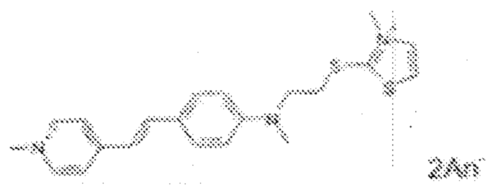 |
| **35** | **36** |
| 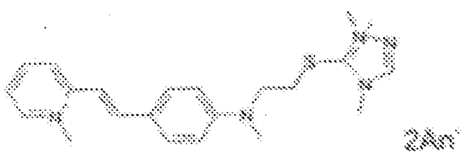 | 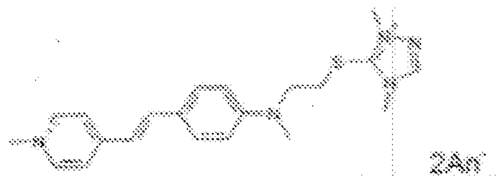 |
| **37** | **38** |
| 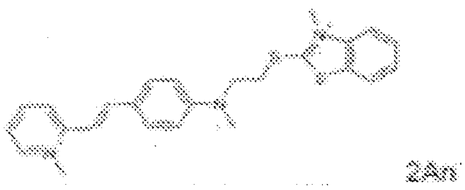 | 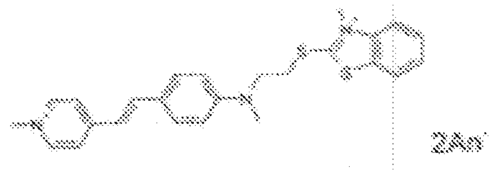 |
| **39** | **40** |
| 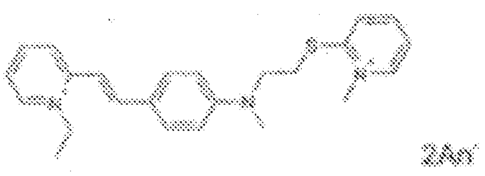 | 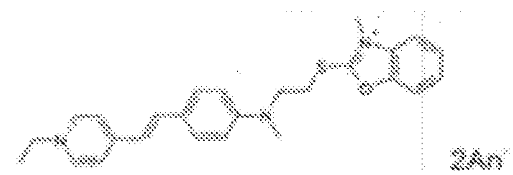 |
| **41** | **42** |
| 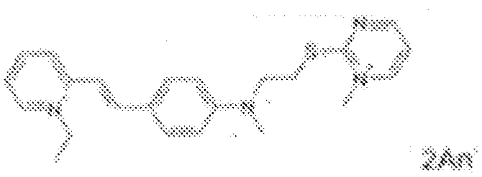 | 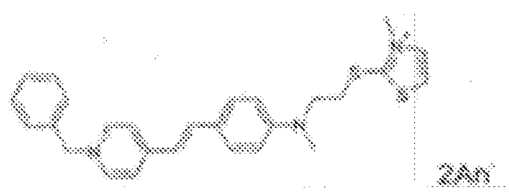 |
| **43** | **44** |
| 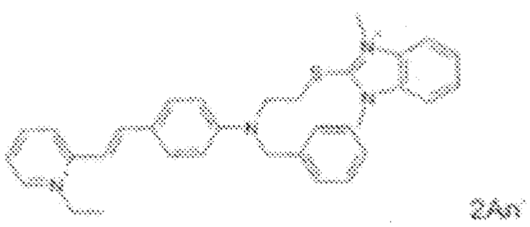 | 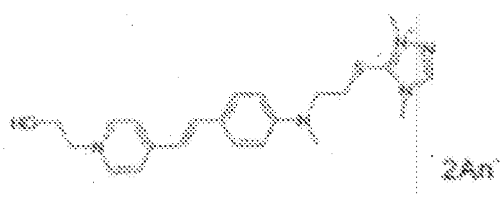 |
| **45** | **46** |
| 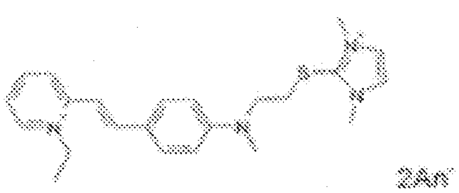 | 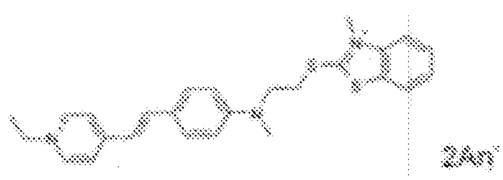 |

(fortgesetzt)

| 47 | |
|---|---|
| 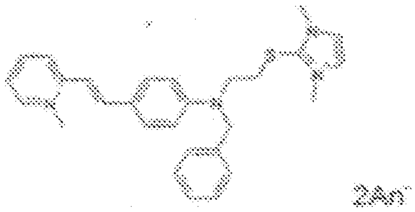 | 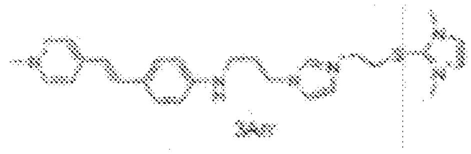 |
| 49 | 50 |
| 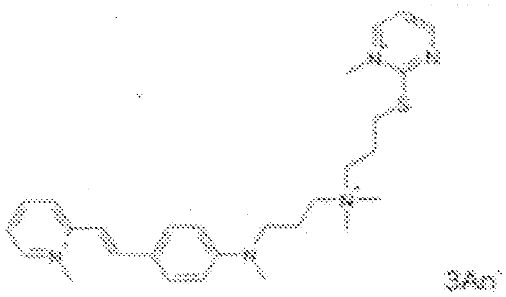 | 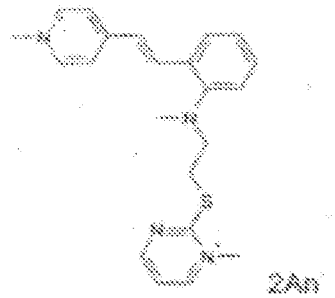 |
| 51 | 52 |
| 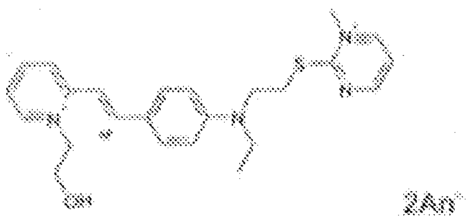 | 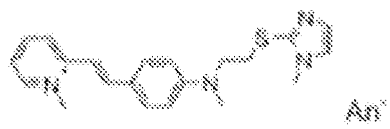 |
| 53 | 54 |
| 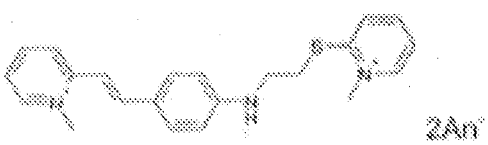 | 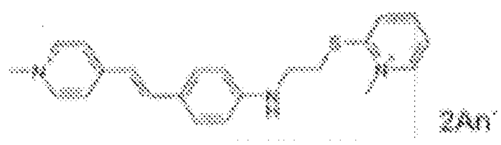 |
| 55 | 56 |
| 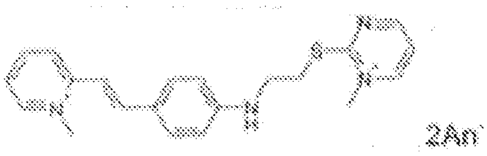 | 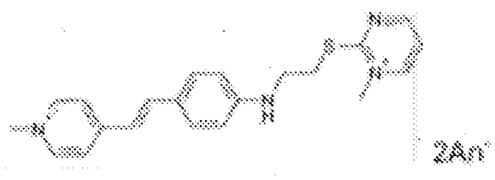 |
| 57 | 58 |
| 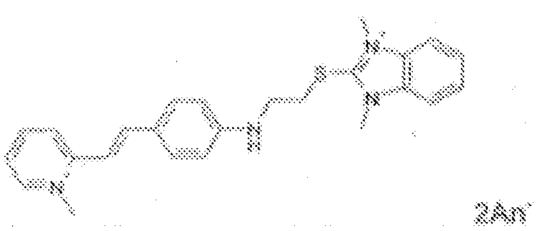 | 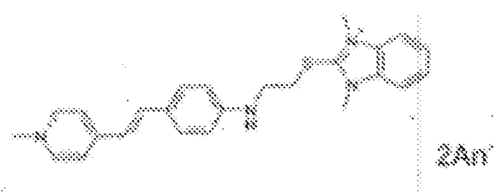 |
| 59 | 60 |

(fortgesetzt)

<table>
<tr><td>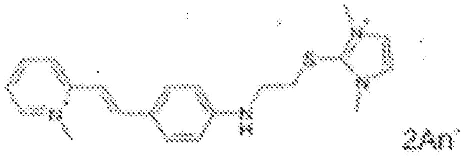</td><td>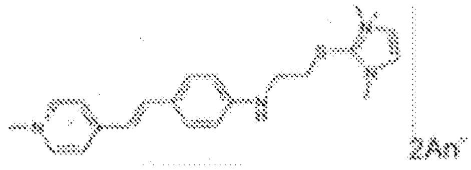</td></tr>
<tr><td>61</td><td>62</td></tr>
<tr><td>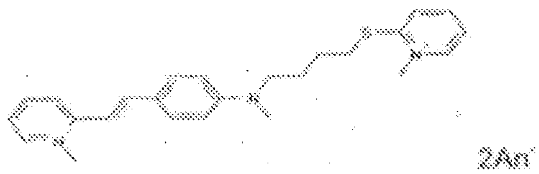</td><td>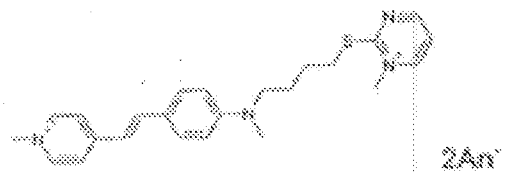</td></tr>
<tr><td>63</td><td>64</td></tr>
<tr><td colspan="2">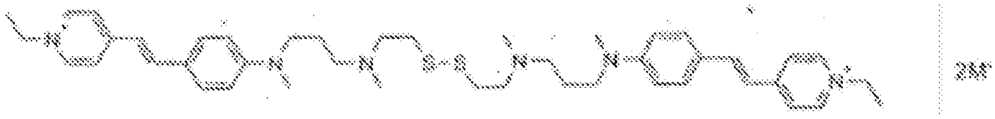</td></tr>
<tr><td colspan="2">65</td></tr>
<tr><td colspan="2">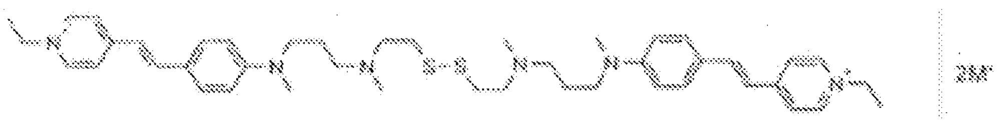</td></tr>
<tr><td colspan="2">66</td></tr>
<tr><td colspan="2">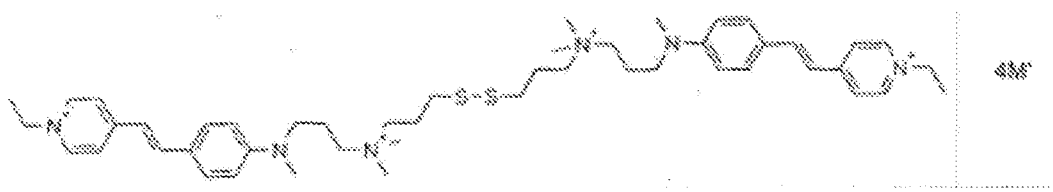</td></tr>
<tr><td colspan="2">67</td></tr>
<tr><td colspan="2">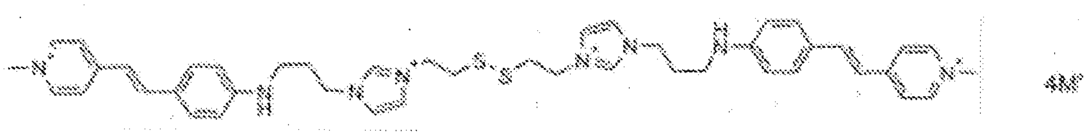</td></tr>
<tr><td colspan="2">68</td></tr>
<tr><td colspan="2">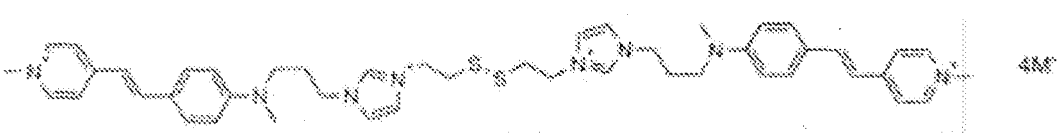</td></tr>
<tr><td colspan="2">69</td></tr>
<tr><td colspan="2">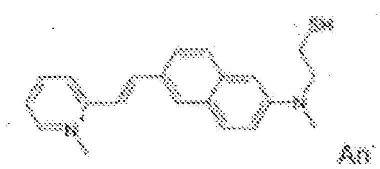</td></tr>
<tr><td colspan="2">70</td></tr>
</table>

(fortgesetzt)

| |
|---|
| 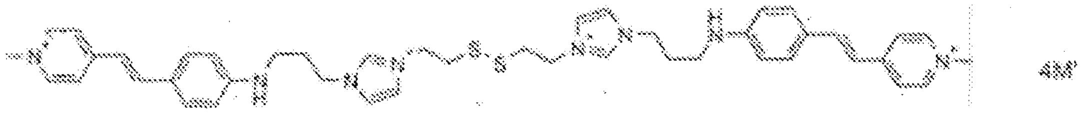 |
| **71** |
| 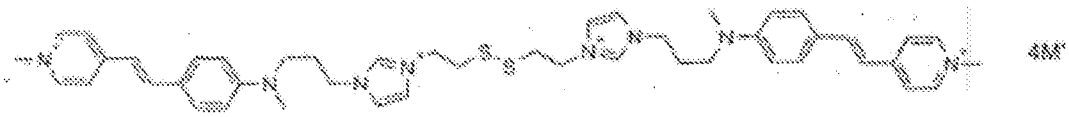 |
| **72** |
| 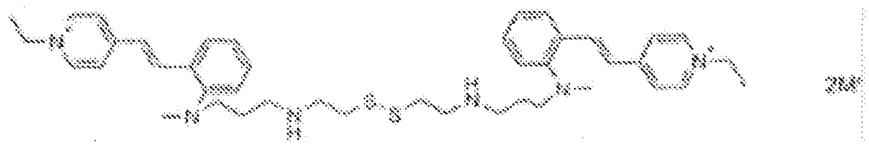 |
| **73** |
| 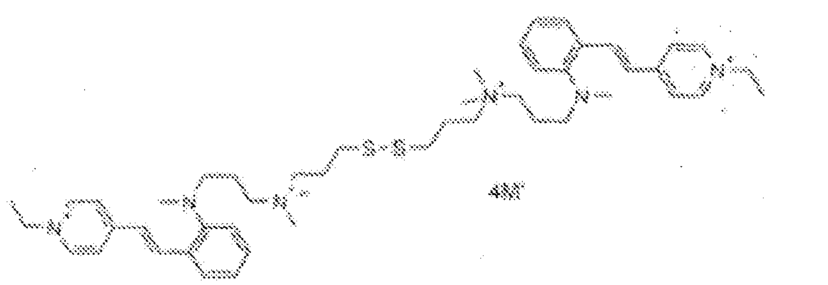 |
| **74** |
| 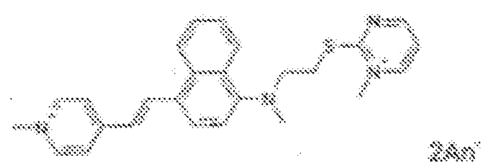 |
| **75** |
| 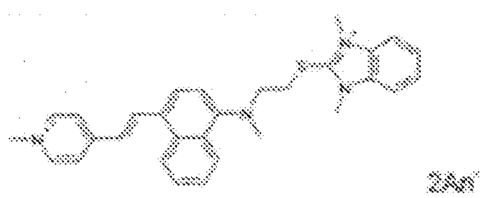 |
| **76** |
| 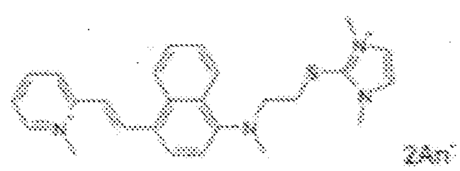 |
| **77** |

(fortgesetzt)

| |
|---|
| 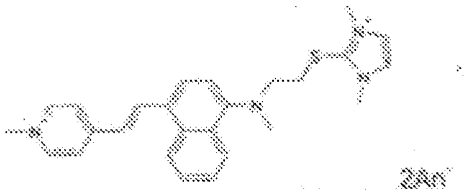 |
| **78** |
| 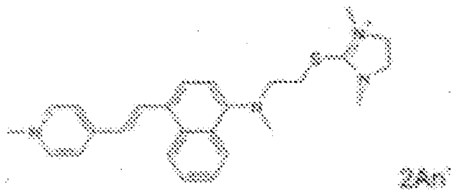 |
| **79** |
| 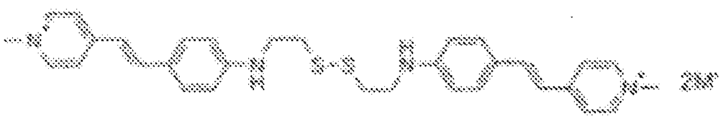 |
| **80** |
| 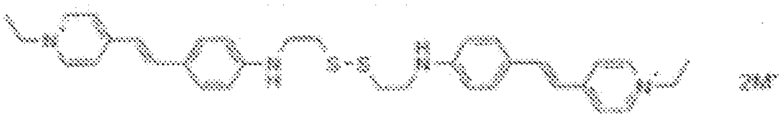 |
| **82** |
| 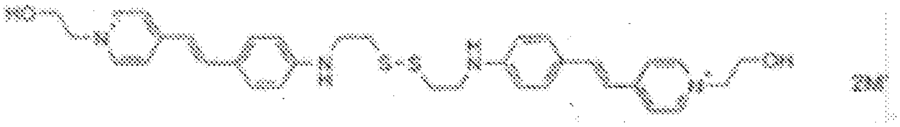 |
| **83** |
| 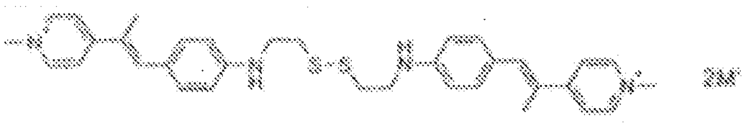 |
| **84** |
| 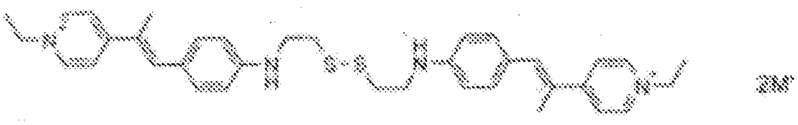 |
| **85** |
| 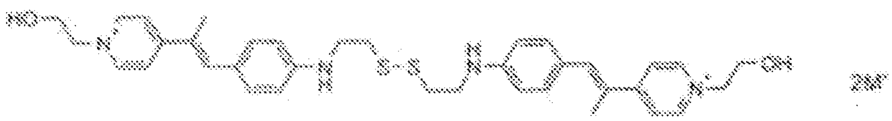 |
| **86** |

(fortgesetzt)

| |
|---|
| 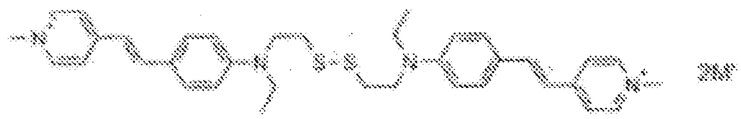 |
| **87** |
| 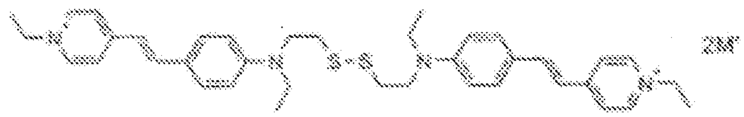 |
| **88** |
| 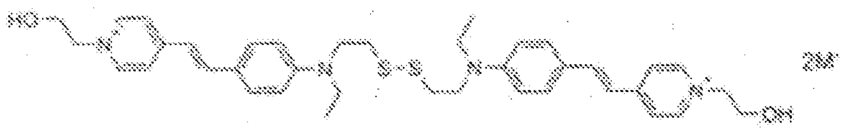 |
| **89** |
| 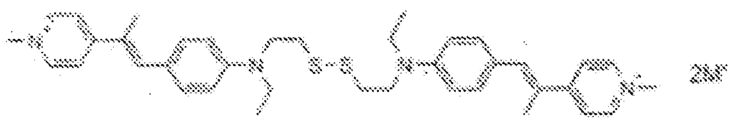 |
| **90** |
| 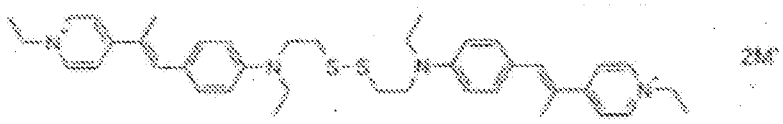 |
| **91** |
| 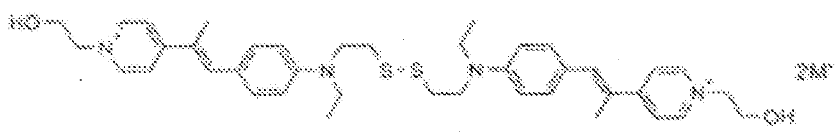 |
| **92** |
| 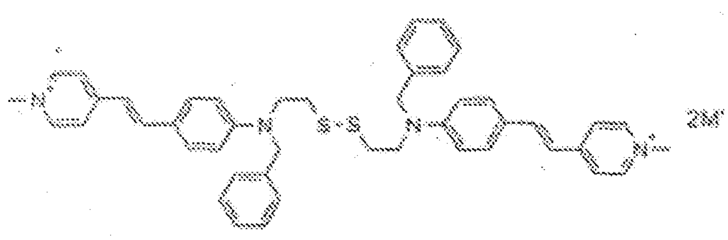 |
| **93** |
| 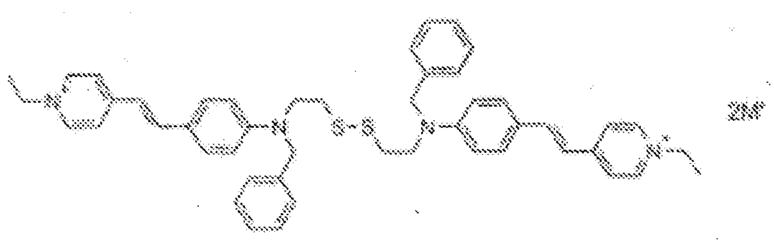 |
| **94** |

(fortgesetzt)

| |
|---|
| 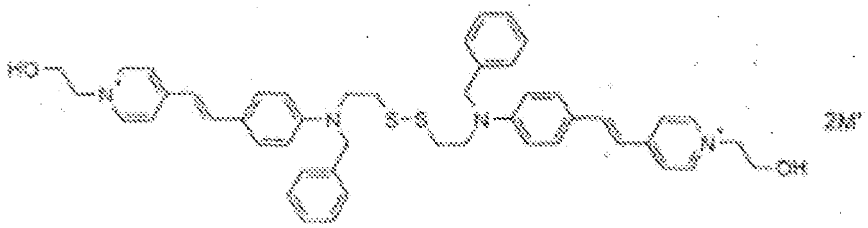 |
| **95** |
| 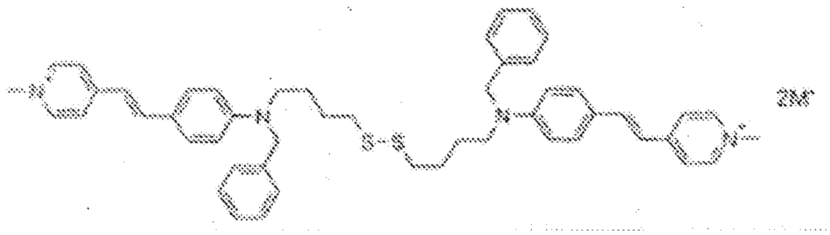 |
| **96** |
| 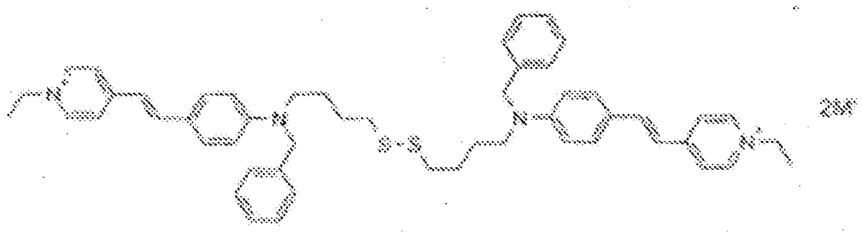 |
| **97** |
| 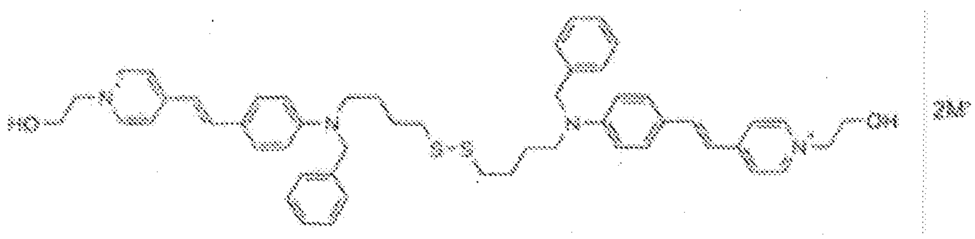 |
| **98** |
| 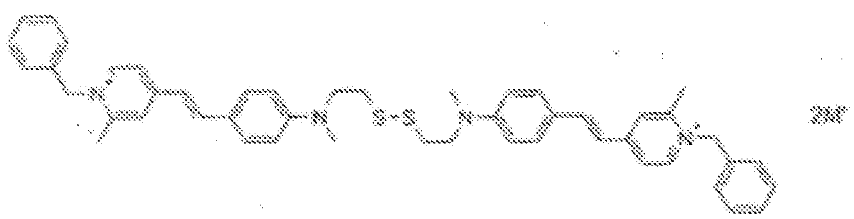 |
| **99** |
| 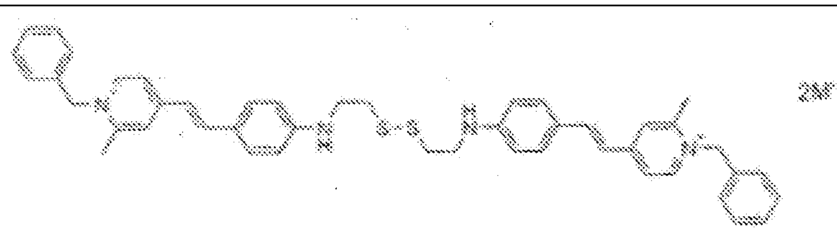 |
| **100** |

(fortgesetzt)

| |
|---|
| 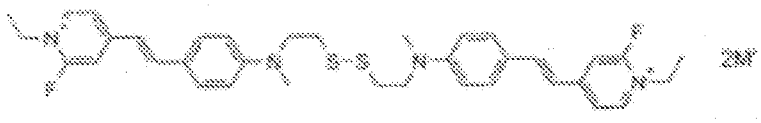 |
| **101** |
| 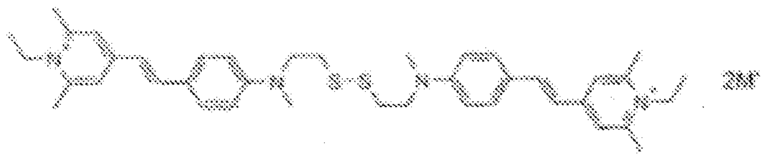 |
| **102** |
| 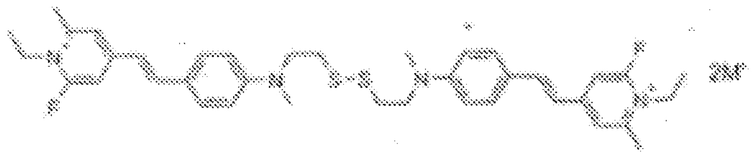 |
| **103** |
| 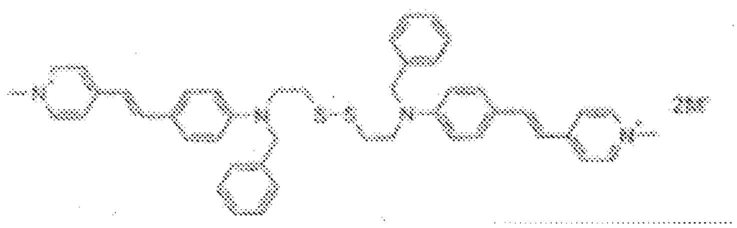 |
| **104** |
| 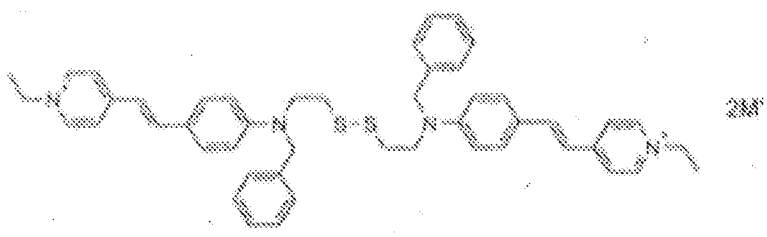 |
| **105** |
| 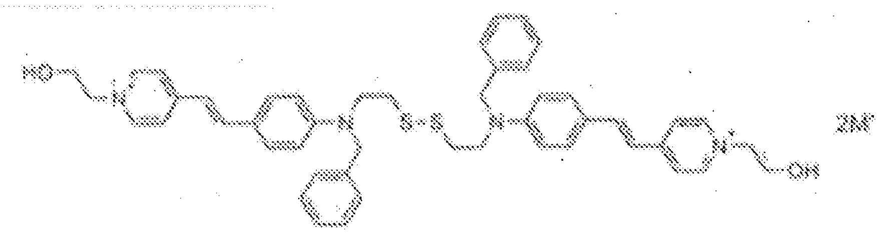 |
| **106** |
| 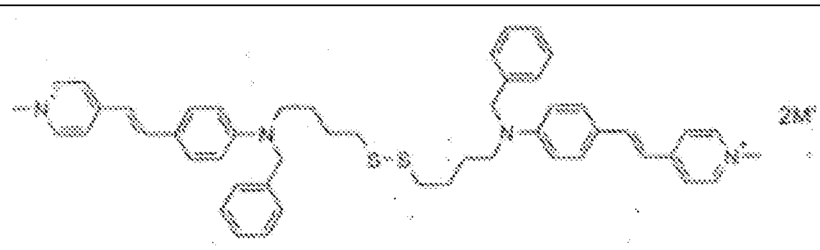 |

(fortgesetzt)

| |
|---|
| **107** |
| 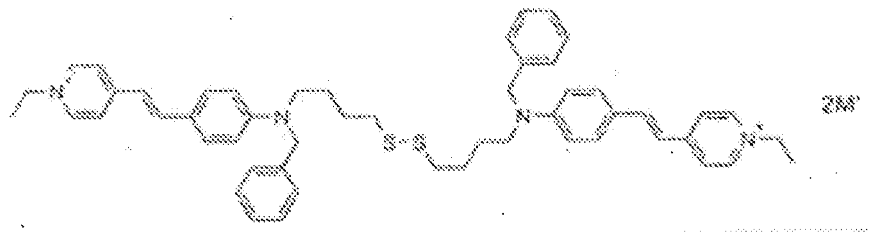 |
| **108** |
| 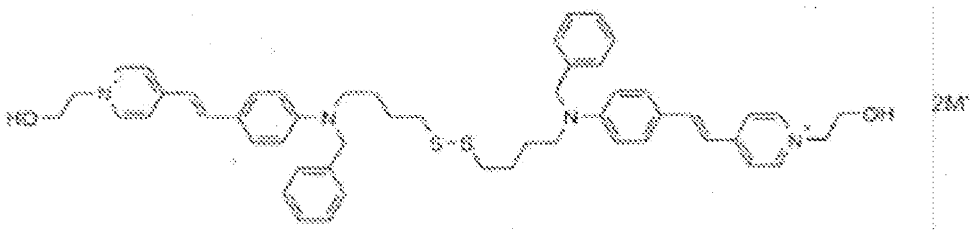 |
| **109** |
| 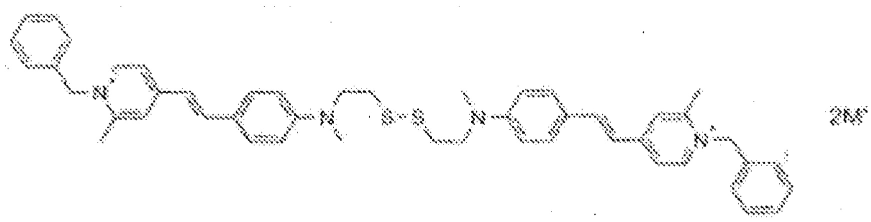 |
| **110** |
| 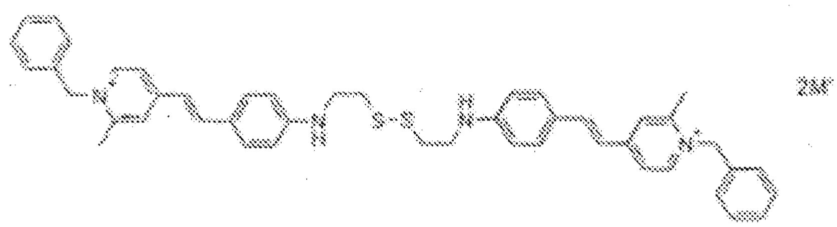 |
| **111** |
| 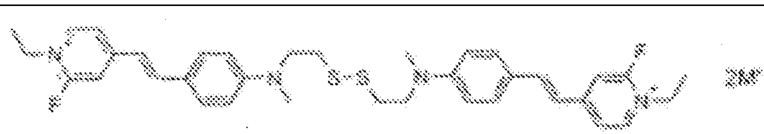 |
| **112** |
| 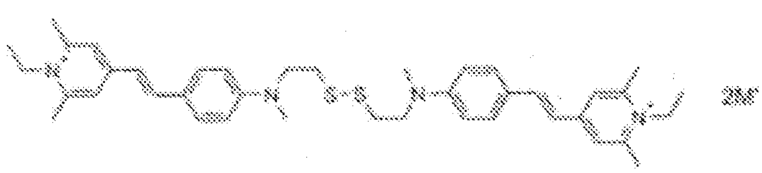 |
| **113** |

(fortgesetzt)

| |
|---|
| 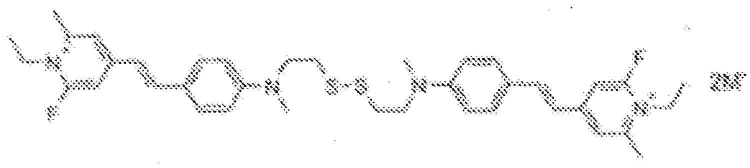 |
| **114** |
| 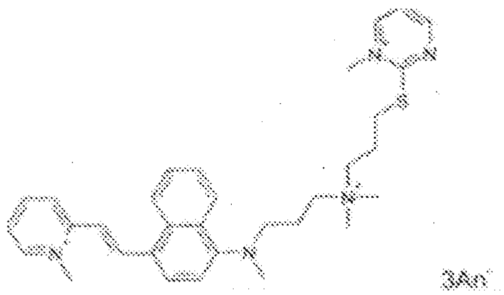 |
| **115** |
| 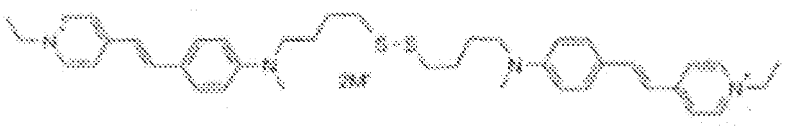 |
| **116** |
| 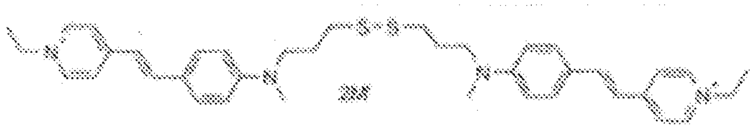 |
| **117** |
| 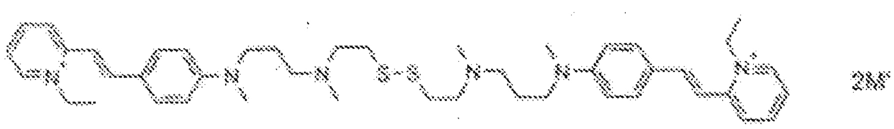 |
| **118** |
| 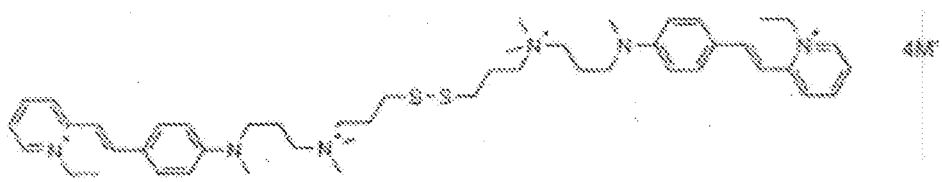 |
| **119** |
| 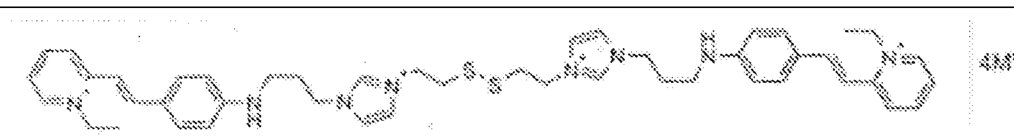 |
| **120** |
| 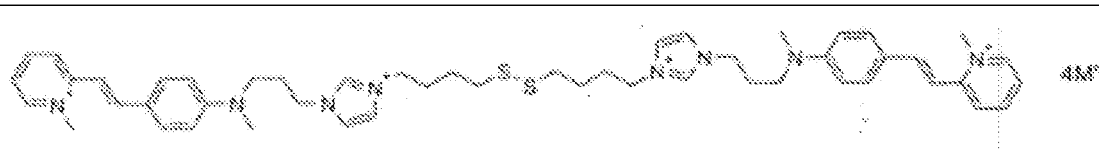 |

(fortgesetzt)

| 121 |
|---|
| 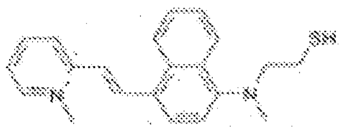 |
| **122** |
| 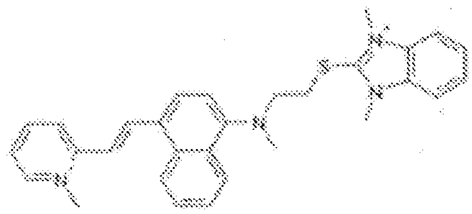 2M' |
| **123** |
| 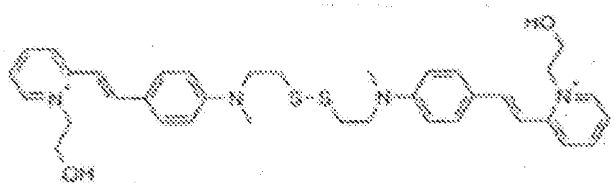 2M' |
| **124** |
| 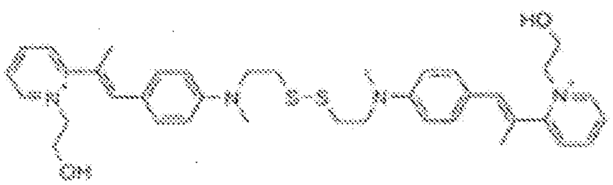 2M' |
| **125** |
| 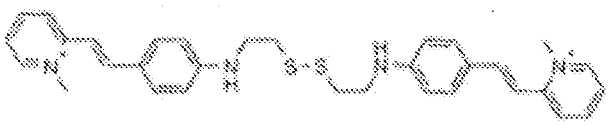 2M' |
| **126** |
| 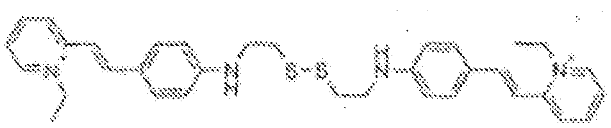 2M' |
| **127** |
| 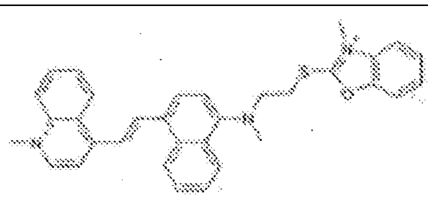 2An' |
| **128** |

(fortgesetzt)

| |
|---|
| 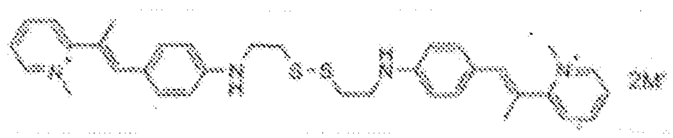 |
| **129** |
| 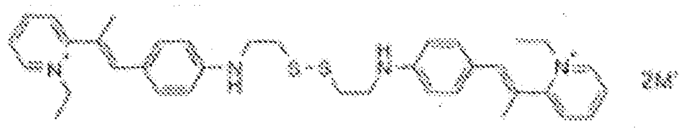 |
| **130** |
| 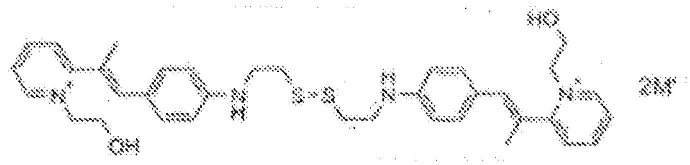 |
| **131** |
| 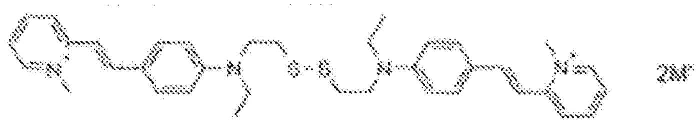 |
| **132** |
| 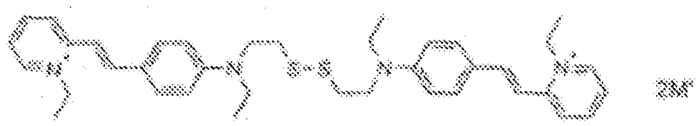 |
| **133** |
| 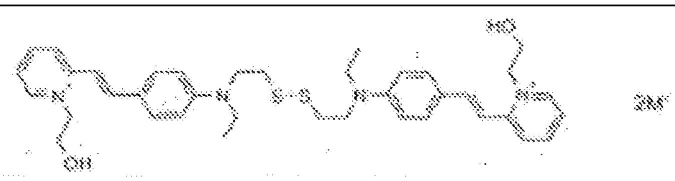 |
| **134** |
| 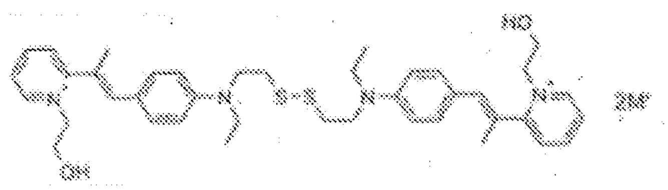 |
| **135** |
| 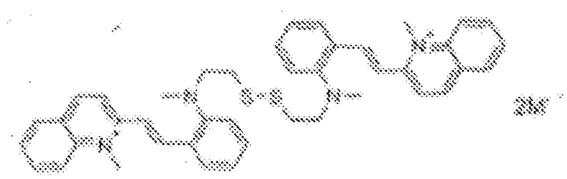 |
| **135** |

(fortgesetzt)

| |
|---|
| 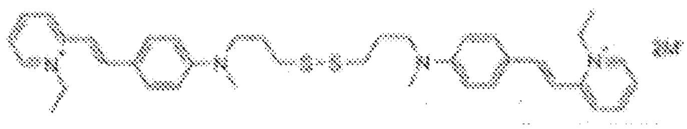 |
| **137** |
| 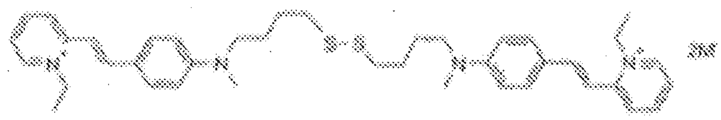 |
| **138** |
| 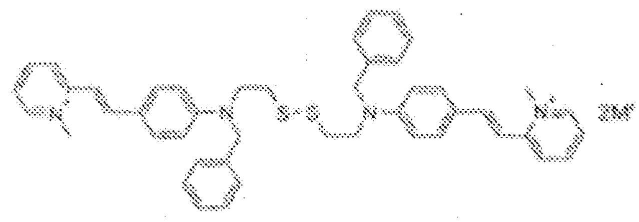 |
| **139** |
| 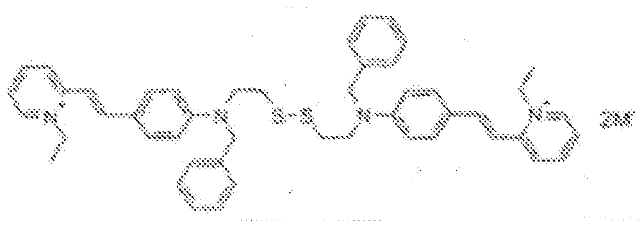 |
| **140** |
| 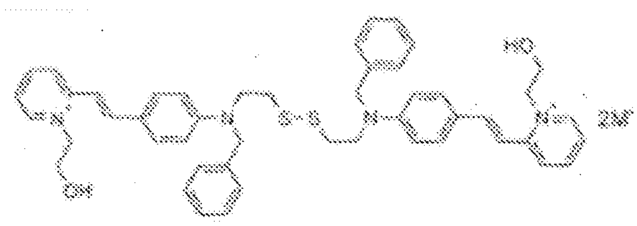 |
| **141** |
| 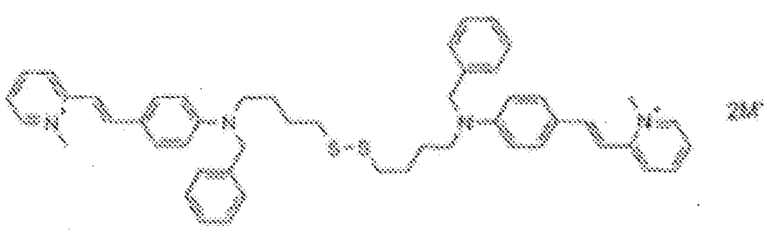 |
| **142** |

(fortgesetzt)

| | |
|---|---|
| 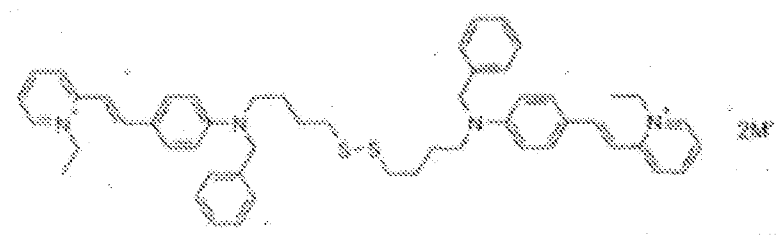 | |
| 143 | |
| 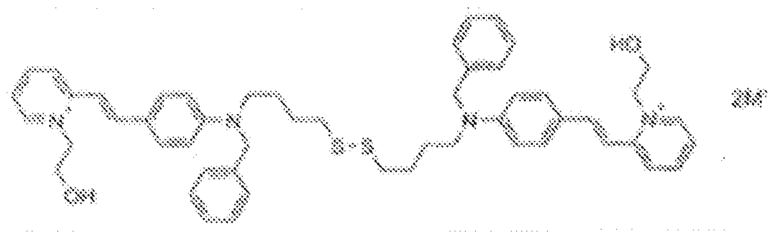 | |
| 144 | |
| 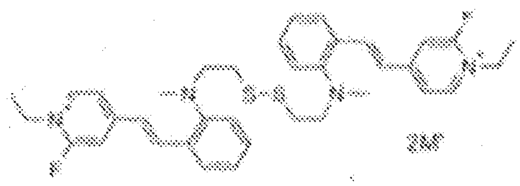 | 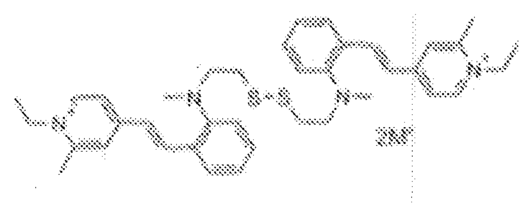 |
| 145 | 146 |
| 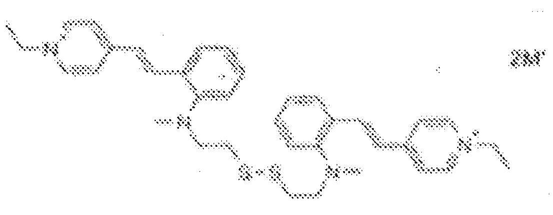 | 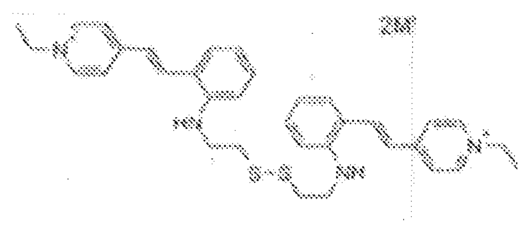 |
| 147 | 148 |
| 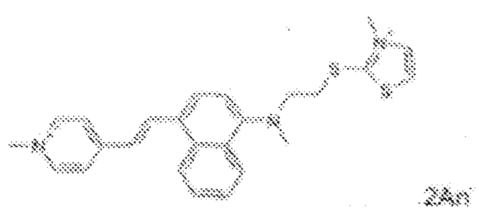 | |
| 149 | |

wobei $An^-$ und M', die gleich oder verschieden sind, ein anionisches Gegenion bedeuten; und $M^+$ ein Alkalimetall ist.

**9.** Farbmittelzusammensetzung, die in einem kosmetisch geeigneten Medium einen fluoreszierenden Farbstoff der Formel (I) oder (II) enthält, wie er in einem der Ansprüche 1 bis 8 definiert ist.

**10.** Farbmittelzusammensetzung, die in einem kosmetisch geeigneten Medium einen fluoreszierenden Farbstoff der Formel (I) oder (II), wie er in einem der Ansprüche 1 bis 8 definiert ist, und mindestens ein Reduktionsimttel enthält.

**11.** Verfahren zum Färben von Keratinsubstanzen, wobei auf die Keratinsubstanzen eine Färbmittelzusammensetzung aufgetragen wird, die in einem kosmetisch geeigneten Medium mindestens einen fluoreszierenden Farbstoff der

Formel (I) oder (II), wie er in einem der Ansprüche 1 bis 8 definiert ist, gegebenenfalls in Gegenwart eines Reduktionsmittels enthalt, das befähigt ist, die Disulfidbindungen von Keratinsubstanzen zu reduzieren.

**12.** Verfahren zum Färben von Keratinsubstanzen nach Anspruch 11, **dadurch gekennzeichnet, dass** die Keratinsubstanzen dunkle Keratinfasern sind.

**13.** Verfahren nach Anspruch 11 oder 12, das einen weiteren Schritt umfasst, der darin besteht, ein Oxidationsmittel auf die Keratinfasern aufzubringen.

**14.** Verfahren nach einem der Ansprüche 11 bis 13, bei dem der fluoreszierende Farbstoff der Formel (I) oder (II) in einem Mengenanteil von 0,001 bis 50 Gew.-%, bezogen auf das Gesamtgewicht der Zusammensetzung, enthalten ist.

**15.** Vorrichtung mit mehreren Abteilungen, wobei eine erste Abteilung eine Farbmittelzusammensetzung enthält, die einen fluoreszierenden Farbstoff, wie er in einem der Ansprüche 1 bis 8 definiert ist, aufweist, und eine zweite Abteilung ein Reduktionsmittel enthält, das befähigt ist, die Disulfidbindungen von Keratinsubstanzen zu reduzieren.

**16.** Verwendung von fluoreszierenden Farbstoffen der Formel (I) oder (II), wie sie in einem der Ansprüche 1 bis 8 definiert sind, zum Aufhellen von dunklen Keratinfasern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2830189
- WO 2004091473 A
- CA 2024509
- WO 9951194 A
- FR 1156407
- WO 2005097051 A
- DE 19951134
- FR 2586913


### Littérature non-brevet citée dans la description


- **Guise ; Stapleton.** *Journal of the Society of Dyers and Colourists,* 1975, vol. 91, 259-264
- *Journal of Cosmetic Chemistry,* 1991, vol. 42, 1-17
- **Charles ZVIAK.** Science des traitements capillaires. Ed. Masson, 1988, 215, 278
- **T. W. Greene.** Protective Groups in Organic Synthesis. John Willey & Sons, 1981, 193-217
- **P. Kocienski.** Protecting Groups. Thieme, 2005
- Thiols and organic Sulfides », « Thiocyanates and Isothiocyanates, organic. Ullmann's Encyclopedia. Wiley-VCH, 2005
- Reactions, Mechanisms and Structures. **J. March.** Advanced Organic Chemistry. John Willey & Sons, 1992
- **T. W. Greene.** Protective Groups in Organic Synthesis
- **T. W. Greene.** Protective Groups in Organic Synthesis. John Willey & Sons, 1981
- **J. March.** Advanced Organic Chemistry. John Willey & Sons, 1992